# EUROPEAN PATENT APPLICATION

(11) **EP 4 234 548 A1**
(43) Date of publication of application: **30.08.2023**
(21) Application number: 21882072.8
(22) Date of filing: 20.10.2021
(51) Int. Cl.: C07D 403/12, C07D 401/14, C07D 405/14, C07D 403/08, C07D 405/12, C07D 239/94, C07D 487/04, A61K 31/517, A61P 35/00

(54) **SUBSTITUTED BENZO OR PYRIDOPYRIMIDINE AMINE INHIBITOR, PREPARATION METHOD THEREFOR, AND APPLICATION THEREOF**

(30) Priority: 20.10.2020 CN 202011128302; 09.02.2021 CN 202110178999; 13.07.2021 CN 202110790488
(71) Applicant: Suzhou Zelgen Biopharmaceutical Co., Ltd., Jiangsu 215300 (CN); Shanghai Zelgen Pharma.Tech Co., Ltd., Shanghai 201203 (CN)
(72) Inventor: LV, Binhua, Shanghai 201203 (CN); CUI, Dawei, Shanghai 201203 (CN); ZHANG, Qing, Shanghai 201203 (CN); CHAI, Chuanke, Shanghai 201203 (CN); LIANG, Hui, Shanghai 201203 (CN); PANG, Xudong, Shanghai 201203 (CN)
(74) Representative: dompatent von Kreisler Selting Werner - Partnerschaft von Patent- und Rechtsanwälten mbB
(86) International application number: PCT/CN2021/125084
(87) International publication number: WO 2022/083657

(57) **Abstract**

The present invention relates to a substituted benzo or pyridopyrimidine amine inhibitor, a preparation method therefor, and an application thereof. Specifically, a compound of the present invention is of a structure as shown in formula (I). The present invention further discloses a preparation method for the compound and a use of the compound as an SOS1 inhibitor; and the compound of the present invention has a good selective inhibitory effect on SOS1 and has better pharmacodynamic and pharmacokinetic properties and smaller toxic and side effects.

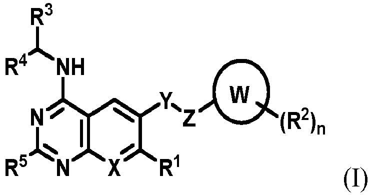

## Description

### TECHNICAL FIELD

The present invention relates to the field of pharmaceuticals, and particularly to a substituted benzo- or pyrido-pyrimidinamine inhibitor, a method for preparing the same, and use thereof.

### BACKGROUND

Lung cancer is one of the leading causes of cancer deaths worldwide. According to cell type, lung cancer can be divided into small cell lung cancer (SCLC) and non-small cell lung cancer (NSCLC), with NSCLC accounting for 85% among all lung cancer patients. According to statistics, the global NSCLC market was approximately $20.9 billion in 2016, of which the US market occupied half, followed by Japan, Germany, and China. Based on current trends, the non-small cell lung cancer market will continuoulsy grow and is expected to reach $54 billion worldwide by 2023 (Nature, 2018; 553(7689):446-454).

At present, the major therapeutics for NSCLC include chemotherapies, targeted therapies, tumor immunotherapies, and the like. Among them, the chemotherapeutics mainly include gemcitabine, paclitaxel, platinum-based drugs, and the like, but such drugs generally possess poor selectivity and high toxicity, leading to relatively strong adverse effects. In recent years, the targeted therapies have gradually become a research hotspot due to their obvious advantages such as high selectivity, milder adverse effects, and the potential in precision medicine. Existing targeted therapies for NSCLC include EGFR inhibitors (such as afatinib, gefitinib, erlotinib, lapatinib, dacomitinib, icotinib, pyrotinib, rociletinib, osimertinib, etc.), ALK inhibitors (such as ceritinib, alectinib, brigatinib, lorlatinib, ocatinib, etc.), and VEGFR inhibitors (sorafenib, regorafenib, cabozantinib, sunitinib, donafenib, etc.) (Current Medicinal Chemistry, 2019, 26, 1-39).

KRAS mutations occur in 20-40% of lung adenocarcinomas, with a higher prevalence in Western population (vs. Asian population; 26% vs 11%) and in smokers (vs non-smokers; 30% vs 10%). The most common mutations occur in codons 12 and 13, including G12C, G12V, and G12D. To date, no drug targeting KRAS mutation has been approved for marketing.

KRAS protein transitions between inactivated and activated states within the cells. KRAS is in the inactivated state when it binds to guanosine diphosphate (GDP); it is in the activated state and can activate downstream signaling pathways when it binds to guanosine triphosphate (GTP). The transition between inactivated and activated states of KRAS is regulated by two types of factors. One type is guanine nucleotide exchange factor (GEF), including the SOS1 protein. Such proteins catalyze the binding of KRAS to GTP, thereby promoting the activation of KRAS. Another type is GTPase-activating protein (GAP), which promotes the hydrolysis of GTP binding to KRAS to GDP, thereby inhibiting KRAS activity.

To date, three major groups of RAS-specific GEFs have been identified, with SOS proteins being primarily found involved in tumors. SOS proteins are widely expressed *in vivo* and contain two isoforms SOS1 and SOS2. Published data indicate a critical role of SOS1 in mutant KRAS activation and oncogenic signaling in cancers. Depleting SOS1 levels decreased the proliferation rate and survival of tumor cells carrying a KRAS mutation whereas no effect was observed in KRAS wild type cell lines. Loss of SOS1 could not be rescued by introduction of SOS1 with mutations at the catalytic site, demonstrating the essentiality of SOS1's GEF activity in KRAS mutant cancer cells (see WO2019122129A1).

Since the binding of KRAS, whether mutant or wild-type, to GTP is dependent on SOS1, selective inhibition of SOS1, regardless of KRAS mutation, may prevent the interaction between SOS1 and KRAS, ultimately inhibiting KRAS activation.

Since the target protein SOS1 is pathologically associated with a variety of diseases, there is also a need for novel SOS1 inhibitors for clinical therapy. For highly selective and active SOS1 inhibitors with potentials to treat diseases such as cancers caused by KRAS mutations more effectively and to reduce off-target effects, there is a more urgent clinical need.

### SUMMARY

The present invention is intended to provide a compound with selective inhibition against SOS 1 and/or better pharmacodynamic performance, and use thereof.

In a first aspect of the present invention, provided is a substituted benzo- or pyrido-pyrimidinamine compound having a structure of general formula (I), or a stereoisomer, a tautomer, a crystalline form, a pharmaceutically acceptable salt, a hydrate, a solvate, or a prodrug thereof: 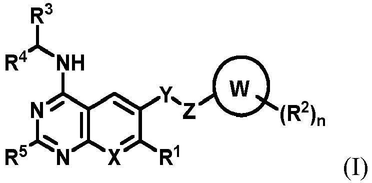
wherein in the formula,
X is selected from: CR⁶ and N, wherein R⁶ is selected from: hydrogen, deuterium, halogen, cyano, C₁-C₆ alkyl, C₃-C₆ cycloalkyl, and 4- to 6-membered heterocyclyl;
Y is selected from the group consisting of: O, NH, NR⁷, S, SO, SO₂, C=C, substituted or unsubstituted 4- to 20-membered heterocyclyl, substituted or unsubstituted C₆-C₁₄ aryl, and 5-to 14-membered heteroaryl, wherein R⁷ is selected from the group consisting of the following substituted or unsubstituted groups: C₁-C₆ alkyl, C₃-C₆ cycloalkyl, and 4- to 6-membered heterocyclyl;
Z is selected from the group consisting of the following substituted or unsubstituted groups: a bond and substituted or unsubstituted C₁-C₁₈ alkylene;
W is selected from the group consisting of the following substituted or unsubstituted groups: a bond, C₃-C₂₀ cycloalkylene, 4- to 20-membered heterocyclylene, OR¹¹, NR¹¹R¹², SO₂, NR¹²SO₂, CO, and NR¹²CO; R¹¹ is independently selected from the group consisting of the following substituted or unsubstituted groups: C₃-C₂₀ cycloalkylene, 4- to 20-membered heterocyclylene, C₃-C₂₀ cycloalkylene C₁-C₁₈ alkylene, 4- to 20-membered heterocyclylene C₁-C₁₈ alkylene, C₆-C₁₄ aryl, and 5- to 14-membered heteroaryl; R¹² is independently selected from the group consisting of the following substituted or unsubstituted groups: hydrogen, deuterium, C₁-C₆ alkyl, and C₃-C₆ cycloalkyl;
R¹ and R² are each independently selected from the group consisting of: hydrogen, deuterium, halogen, cyano, -(CH₂)ₘR⁸, -(CH₂)ₘ(CH=CH)R⁸, -(CH₂)ₘ(C≡C)R⁸, -(CH₂)ₘO(CH₂)ₚR⁸, -(CH₂)ₘSR⁸, -(CH₂)ₘCOR⁸, -(CH₂)ₘC(O)OR⁸, -(CH₂)ₘS(O)_{q}R⁸, -(CH₂)ₘNR⁸R⁹, -(CH₂)ₘC(O)NR⁸R⁹, -(CH₂)ₘNR⁸C(O)R⁹, -(CH₂)ₘNR⁸C(O)NR⁹R¹⁰, -(CH₂)ₘS(O)_{q}NR⁸R⁹, -(CH₂)ₘNR⁸S(O)_{q}R⁹, -(CH₂)ₘNR⁸S(O)_{q}NR⁹R¹⁰, wherein H in CH₂ can be optionally substituted; R⁸, R⁹, and R¹⁰ are each independently selected from the group consisting of the following substituted or unsubstituted groups: hydrogen, C₁-C₁₈ alkyl, C₃-C₂₀ cycloalkyl, 4- to 20-membered heterocyclyl, C₆-C₁₄ aryl, and 5- to 14-membered heteroaryl; or in -(CH₂)ₘNR⁸R⁹, -(CH₂)ₘC(O)NR⁸R⁹, or -(CH₂)ₘS(O)_{q}NR⁸R⁹, R⁸ and R⁹, together with the N atom adjacent thereto, form a substituted or unsubstituted 4- to 8-membered heterocyclyl by cyclization; or in -(CH₂)ₘNR⁸C(O)R⁹, -(CH₂)ₘNR⁸C(O)NR⁹R¹⁰, -(CH₂)ₘNR⁸S(O)_{q}R⁹, or -(CH₂)ₘNR⁸S(O)_{q}NR⁹R¹⁰, R⁸ and R⁹, together with the N atom adjacent thereto, form a substituted or unsubstituted 4- to 8-membered heterocyclyl by cyclization, or R⁹ and R¹⁰, together with the atom adjacent thereto, form a substituted or unsubstituted 4- to 8-membered heterocyclyl by cyclization;
R³ is selected from the group consisting of the following substituted or unsubstituted groups: C₃-C₁₈ cycloalkyl, 4- to 20-membered heterocyclyl, C₆-C₁₄ aryl, and 5- to 14-membered heteroaryl;
R⁴ and R⁵ are each independently selected from the group consisting of the following substituted or unsubstituted groups: C₁-C₆ alkyl, C₃-C₆ cycloalkyl, 4- to 6-membered heterocyclyl, ester group, COOH, CONH₂, C₂-C₆ alkenyl, and C₂-C₆ alkynyl;
wherein the above substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₃-C₂₀ cycloalkyl-O-, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, 4- to 20-membered heterocyclyl-O-, halogen, oxo C₁-C₆ alkyl, nitro, hydroxy, cyano, C₂-C₆ ester group, C₁-C₆ amino, C₁-C₆ acyl, C₁-C₆ amido, C₁-C₆ sulfonyl, C₁-C₆ sulfonamido, and C₁-C₆ ureido; wherein the C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₃-C₂₀ cycloalkyl-O-, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, or 4- to 20-membered heterocyclyl-O- may be further substituted with one or more R^{a}, wherein R^{a} is selected from: C₁-C₆ alkyl, deuterated C₁-C₆ alkyl, halogenated C₁-C₆ alkyl, halogenated C₁-C₆ alkylhydroxy, C₃-C₆ cycloalkyl, C₃-C₆ cycloalkyl-O-, C₁-C₆ alkoxy, deuterated C₁-C₆ alkoxy, halogenated C₁-C₆ alkoxy, C₆-C₁₄ aryl, 5- to 14-membered heteroaryl, 4- to 6-membered heterocyclyl, 4- to 6-membered heterocyclyl-O-, halogen, oxo (=O), nitro, hydroxy, cyano, C₂-C₆ ester group, C₁-C₆ amino, C₁-C₆ amido, C₁-C₆ sulfonamido, and C₁-C₆ ureido; or two substituents on the same carbon atom together form -(CH₂)ₙ- or =O;
m and n are each independently 0, 1, 2, 3, 4, or 5;
p is 0, 1, 2, 3, 4, or 5;
q is 1 or 2;
provided that when Y is selected from the group consisting of: O, NH, and NR⁷, and when Z is a bond and W is C₃-C₂₀ cycloalkylene or 4- to 20-membered heterocyclylene, R¹ is not hydrogen, deuterium, halogen, cyano, R⁸, O(CH₂)ₚR⁸, COR⁸, -C(O)OR⁸, NR⁸R⁹, C(O)NR⁸R⁹, -NR⁸C(O)R⁹, or -NR⁸C(O)NR⁹R¹⁰.

In another preferred embodiment, for the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof: 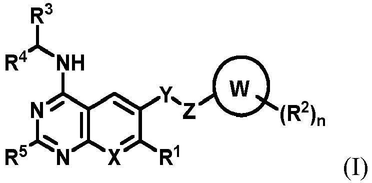
wherein in the formula,
X is selected from: CR⁶ and N, wherein R⁶ is selected from: hydrogen, deuterium, halogen, cyano, C₁-C₆ alkyl, C₃-C₆ cycloalkyl, and 4- to 6-membered heterocyclyl;
Y is selected from the group consisting of: O, NH, NR⁷, S, SO, SO₂, C=C, wherein R⁷ is selected from the group consisting of the following substituted or unsubstituted groups: C₁-C₆ alkyl, C₃-C₆ cycloalkyl, and 4- to 6-membered heterocyclyl;
Z is selected from the group consisting of the following substituted or unsubstituted groups: a bond and substituted or unsubstituted C₁-C₁₈ alkylene (preferably deuterated C₁-C₁₈ alkylene or halogenated C₁-C₁₈ alkylene);
W is selected from the group consisting of the following substituted or unsubstituted groups: a bond, C₃-C₂₀ cycloalkylene, 4- to 20-membered heterocyclylene, OR¹¹, NR¹¹R¹², SO₂, NR¹²SO₂, CO, and NR¹²CO; R¹¹ is independently selected from the group consisting of the following substituted or unsubstituted groups: C₃-C₂₀ cycloalkylene, 4- to 20-membered heterocyclylene, C₃-C₂₀ cycloalkylene C₁-C₁₈ alkylene, 4- to 20-membered heterocyclylene C₁-C₁₈ alkylene, C₆-C₁₄ aryl, and 5- to 14-membered heteroaryl; R¹² is independently selected from the group consisting of the following substituted or unsubstituted groups: hydrogen, deuterium, C₁-C₆ alkyl, and C₃-C₆ cycloalkyl;
R¹ and R² are each independently selected from the group consisting of: hydrogen, deuterium, halogen, cyano, -(CH₂)ₘR⁸, -(CH₂)ₘ(CH=CH)R⁸, -(CH₂)ₘ(C≡C)R⁸, -(CH₂)ₘO(CH₂)ₚR⁸, -(CH₂)ₘSR⁸, -(CH₂)ₘCOR⁸, -(CH₂)ₘC(O)OR⁸, -(CH₂)ₘS(O)_{q}R⁸, -(CH₂)ₘNR⁸R⁹, -(CH₂)ₘC(O)NR⁸R⁹, -(CH₂)ₘNR⁸C(O)R⁹, -(CH₂)ₘNR⁸C(O)NR⁹R¹⁰, -(CH₂)ₘS(O)_{q}NR⁸R⁹, -(CH₂)ₘNR⁸S(O)_{q}R⁹, and -(CH₂)ₘNR⁸S(O)_{q}NR⁹R¹⁰, wherein H in CH₂ can be optionally substituted; R⁸, R⁹, and R¹⁰ are each independently selected from the group consisting of the following substituted or unsubstituted groups: hydrogen, C₁-C₁₈ alkyl, C₁-C₁₈ alkoxy, C₃-C₂₀ cycloalkyl, 4- to 20-membered heterocyclyl, C₆-C₁₄ aryl, and 5- to 14-membered heteroaryl; or in -(CH₂)ₘNR⁸R⁹, -(CH₂)ₘC(O)NR⁸R⁹, or -(CH₂)ₘS(O)_{q}NR⁸R⁹, R⁸ and R⁹, together with the N atom adjacent thereto, form a substituted or unsubstituted 4- to 8-membered heterocyclyl by cyclization; or in -(CH₂)ₘNR⁸C(O)R⁹, -(CH₂)ₘNR⁸C(O)NR⁹R¹⁰, -(CH₂)ₘNR⁸S(O)_{q}R⁹, or -(CH₂)ₘNR⁸S(O)_{q}NR⁹R¹⁰, R⁸ and R⁹, together with the N atom adjacent thereto, form a substituted or unsubstituted 4- to 8-membered heterocyclyl by cyclization, or R⁹ and R¹⁰, together with the atom adjacent thereto, form a substituted or unsubstituted 4- to 8-membered heterocyclyl by cyclization;
R³ is selected from the group consisting of the following substituted or unsubstituted groups: C₃-C₁₈ cycloalkyl, 4- to 20-membered heterocyclyl, C₆-C₁₄ aryl, and 5- to 14-membered heteroaryl;
R⁴ and R⁵ are each independently selected from the group consisting of the following substituted or unsubstituted groups: C₁-C₆ alkyl, C₃-C₆ cycloalkyl, 4- to 6-membered heterocyclyl, ester group, COOH, CONH₂, C₂-C₆ alkenyl, and C₂-C₆ alkynyl;
wherein the above substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₃-C₂₀ cycloalkyl-O-, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, 4- to 20-membered heterocyclyl-O-, halogen, oxo C₁-C₆ alkyl, nitro, hydroxy, cyano, C₂-C₆ ester group, C₁-C₆ amino, C₂-C₆ amido, C₂-C₆ sulfonamido, and C₁-C₆ ureido; wherein the C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₃-C₂₀ cycloalkyl-O-, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, or 4- to 20-membered heterocyclyl-O- may be further substituted with one or more R^{a}, wherein R^{a} is selected from: C₁-C₆ alkyl, deuterated C₁-C₆ alkyl, halogenated C₁-C₆ alkyl, halogenated C₁-C₆ alkylhydroxy, C₃-C₆ cycloalkyl, C₃-C₆ cycloalkyl-O-, C₁-C₆ alkoxy, deuterated C₁-C₆ alkoxy, halogenated C₁-C₆ alkoxy, C₆-C₁₄ aryl, 5- to 14-membered heteroaryl, 4- to 6-membered heterocyclyl, 4- to 6-membered heterocyclyl-O-, halogen, oxo C₁-C₆ alkyl, nitro, hydroxy, cyano, C₂-C₆ ester group, C₁-C₆ amino, C₂-C₆ amido, C₂-C₆ sulfonamido, and C₁-C₆ ureido; or two substituents on the same carbon atom together form -(CH₂)ₙ- or =O;
m and n are each independently 0, 1, 2, 3, 4, or 5;
p is 0, 1, 2, 3, 4, or 5;
q is 1 or 2;
provided that when Y is selected from the group consisting of: O, NH, and NR⁷, and when Z is a bond and W is C₃-C₂₀ cycloalkylene or 4- to 20-membered heterocyclylene, R¹ is not hydrogen, deuterium, halogen, cyano, R⁸, O(CH₂)ₚR⁸, COR⁸, -C(O)OR⁸, NR⁸R⁹, C(O)NR⁸R⁹, -NR⁸C(O)R⁹, or -NR⁸C(O)NR⁹R¹⁰.

In another preferred embodiment, for the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof: 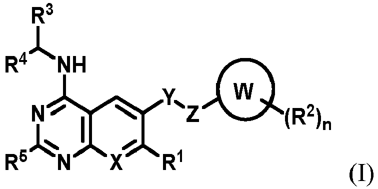
wherein in the formula,
X is selected from: CR⁶ and N, wherein R⁶ is selected from: hydrogen, deuterium, halogen, cyano, C₁-C₆ alkyl, C₃-C₆ cycloalkyl, and 4- to 6-membered heterocyclyl;
Y is selected from the group consisting of: O, NH, NR⁷, S, SO, SO₂, C=C, wherein R⁷ is selected from the group consisting of the following substituted or unsubstituted groups: C₁-C₆ alkyl, C₃-C₆ cycloalkyl, and 4- to 6-membered heterocyclyl;
Z is selected from the group consisting of the following substituted or unsubstituted groups: a bond, C₁-C₁₈ alkylene, deuterated C₁-C₁₈ alkylene, and halogenated C₁-C₁₈ alkylene;
W is selected from the group consisting of the following substituted or unsubstituted groups: a bond, C₃-C₂₀ cycloalkylene, 4- to 20-membered heterocyclylene, OR¹¹, NR¹¹R¹², SO₂, NR¹²SO₂, CO, and NR¹²CO; R¹¹ is independently selected from the group consisting of the following substituted or unsubstituted groups: C₃-C₂₀ cycloalkylene, 4- to 20-membered heterocyclylene, C₃-C₂₀ cycloalkylene C₁-C₁₈ alkylene, and 4- to 20-membered heterocyclylene C₁-C₁₈ alkylene; R¹² is independently selected from the group consisting of the following substituted or unsubstituted groups: hydrogen, deuterium, C₁-C₆ alkyl, and C₃-C₆ cycloalkyl;
R¹ and R² are each independently selected from the group consisting of: hydrogen, deuterium, halogen, cyano, -(CH₂)ₘR⁸, -(CH₂)ₘ(CH=CH)R⁸, -(CH₂)ₘ(C≡C)R⁸, -(CH₂)ₘO(CH₂)ₚR⁸, -(CH₂)ₘSR⁸, -(CH₂)ₘCOR⁸, -(CH₂)ₘC(O)OR⁸, -(CH₂)ₘS(O)_{q}R⁸, -(CH₂)ₘNR⁸R⁹, -(CH₂)ₘC(O)NR⁸R⁹, -(CH₂)ₘNR⁸C(O)R⁹, -(CH₂)ₘNR⁸C(O)NR⁹R¹⁰, -(CH₂)ₘS(O)_{q}NR⁸R⁹, -(CH₂)ₘNR⁸S(O)_{q}R⁹, -(CH₂)ₘNR⁸S(O)_{q}NR⁹R¹⁰, wherein H in CH₂ can be optionally substituted; R⁸, R⁹, and R¹⁰ are each independently selected from the group consisting of the following substituted or unsubstituted groups: hydrogen, C₁-C₁₈ alkyl, C₃-C₂₀ cycloalkyl, and 4-to 20-membered heterocyclyl; or in -(CH₂)ₘNR⁸R⁹, -(CH₂)ₘC(O)NR⁸R⁹, or -(CH₂)ₘS(O)_{q}NR⁸R⁹, R⁸ and R⁹, together with the N atom adjacent thereto, form a substituted or unsubstituted 4- to 8-membered heterocyclyl by cyclization; or in -(CH₂)ₘNR⁸C(O)R⁹, -(CH₂)ₘNR⁸C(O)NR⁹R¹⁰, -(CH₂)ₘNR⁸S(O)_{q}R⁹, or -(CH₂)ₘNR⁸S(O)_{q}NR⁹R¹⁰, R⁸ and R⁹, together with the N atom adjacent thereto, form a substituted or unsubstituted 4- to 8-membered heterocyclyl by cyclization, or R⁹ and R¹⁰, together with the atom adjacent thereto, form a substituted or unsubstituted 4- to 8-membered heterocyclyl by cyclization;
R³ is selected from the group consisting of the following substituted or unsubstituted groups: C₃-C₁₈ cycloalkyl, 4- to 20-membered heterocyclyl, C₆-C₁₄ aryl, and 5- to 14-membered heteroaryl;
R⁴ and R⁵ are each independently selected from the group consisting of the following substituted or unsubstituted groups: C₁-C₆ alkyl, C₃-C₆ cycloalkyl, and 4- to 6-membered heterocyclyl;
wherein the above substitution refers to substitution with one or more (e.g., 2, 3, or 4) groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido;
m and n are each independently 0, 1, 2, 3, 4, or 5;
p is 0, 1, 2, 3, 4, or 5;
q is 1 or 2;
provided that when Y is selected from: O, NH, and NR⁷, and when Z is a bond and W is C₃-C₂₀ cycloalkylene or 4- to 20-membered heterocyclylene, R¹ is not hydrogen, deuterium, halogen, cyano, R⁸, O(CH₂)ₚR⁸, COR⁸, -C(O)OR⁸, NR⁸R⁹, C(O)NR⁸R⁹, -NR⁸C(O)R⁹, or -NR⁸C(O)NR⁹R¹⁰.

In another preferred embodiment, for the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof: 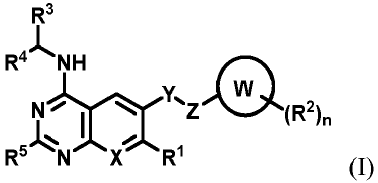
in the formula,
X is selected from: CR⁶ and N, wherein R⁶ is selected from: hydrogen, deuterium, halogen, cyano, C₁-C₆ alkyl, C₃-C₆ cycloalkyl, and 4- to 6-membered heterocyclyl;
Y is selected from the group consisting of: O, NH, and NR⁷, wherein R⁷ is selected from: C₁-C₆ alkyl, C₃-C₆ cycloalkyl, and 4- to 6-membered heterocyclyl;
Z is selected from the group consisting of the following substituted or unsubstituted groups: C₁-C₁₈ alkylene, deuterated C₁-C₁₈ alkylene, and halogenated C₁-C₁₈ alkylene;
W is selected from the group consisting of the following substituted or unsubstituted groups: C₃-C₂₀ cycloalkylene, 4- to 20-membered heterocyclylene, OR¹¹, NR¹¹R¹², SO₂, NR¹²SO₂, CO, and NR¹²CO; R¹¹ is independently selected from the group consisting of the following substituted or unsubstituted groups: C₃-C₂₀ cycloalkylene, 4- to 20-membered heterocyclylene, C₃-C₂₀ cycloalkylene C₁-C₁₈ alkylene, and 4- to 20-membered heterocyclylene C₁-C₁₈ alkylene; R¹² is independently selected from the group consisting of the following substituted or unsubstituted groups: hydrogen, deuterium, C₁-C₆ alkyl, and C₃-C₆ cycloalkyl;
R¹ and R² are independently selected from the group consisting of: hydrogen, deuterium, halogen, cyano, -(CH₂)ₘR⁸, -(CH₂)ₘO(CH₂)ₚR⁸, -(CH₂)ₘSR⁸, -(CH₂)ₘCOR⁸, -(CH₂)ₘC(O)OR⁸, -(CH₂)ₘS(O)_{q}R⁸, -(CH₂)ₘNR⁵R⁸, -(CH₂)ₘC(O)NR⁸R⁹, -(CH₂)ₘNR⁸C(O)R⁹, -(CH₂)ₘNR⁸C(O)NR⁹R¹⁰, -(CH₂)ₘS(O)_{q}NR⁸R⁹, -(CH₂)ₘNR⁸S(O)_{q}R⁹, and -(CH₂)ₘNR⁸S(O)_{q}NR⁹R¹⁰, wherein H in CH₂ can be optionally substituted; R⁸, R⁹, and R¹⁰ are independently selected from the group consisting of the following substituted or unsubstituted groups: hydrogen, C₁-C₁₈ alkyl, C₃-C₂₀ cycloalkyl, and 4- to 20-membered heterocyclyl;
R³ is selected from the group consisting of the following substituted or unsubstituted groups: C₃-C₁₈ cycloalkyl, 4- to 20-membered heterocyclyl, C₆-C₁₄ aryl, and 5- to 14-membered heteroaryl;
R⁴ and R⁵ are independently selected from the group consisting of the following substituted or unsubstituted groups: C₁-C₆ alkyl, C₃-C₆ cycloalkyl, and 4- to 6-membered heterocyclyl;
wherein the above substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, nitro, hydroxy, cyano, ester group, amino, amido, sulfonamido, and ureido;
m and n are each independently 0, 1, 2, 3, 4, or 5;
p is 0, 1, 2, 3, 4, or 5;
q is 1 or 2. In another preferred embodiment, R¹ is selected from the group consisting of: hydrogen, deuterium, halogen, cyano, -(CH₂)ₘ₁R⁸, -(CH₂)_{m'1}(CH=CH)R⁸, -(CH₂)_{m'1}(C≡C)R⁸, -(CH₂)ₘ₁O(CH₂)ₚ₁R⁸, -(CH₂)_{m'1}SR⁸, -(CH₂)ₘ₁COR⁸, -(CH₂)ₘ₁C(O)OR⁸, -(CH₂)_{m'1}S(O)_{q1}R⁸, -(CH₂)ₘ₁NR⁸R⁹, -(CH₂)ₘ₁C(O)NR⁸R⁹, -(CH₂)ₘ₁NR⁸C(O)R⁹, -(CH₂)ₘ₁NR⁸C(O)NR⁹R¹⁰, -(CH₂)_{m'1}(O)_{q1}R⁸R⁹, -(CH₂)_{m'1}R⁸S(O)_{q1}R⁹, and -(CH₂)_{m'1}R⁸S(O)_{q1}R⁹R¹⁰, wherein H in CH₂ can be optionally substituted; R⁸, R⁹, and R¹⁰ are each independently selected from the group consisting of the following substituted or unsubstituted groups: hydrogen, C₁-C₁₈ alkyl, C₃-C₂₀ cycloalkyl, and 4- to 20-membered heterocyclyl; or in -(CH₂)ₘ₁NR⁸R⁹, -(CH₂)ₘ₁C(O)NR⁸R⁹, or -(CH₂)_{m'1}S(O)_{q1}NR⁸R⁹, R⁸ and R⁹, together with the N atom adjacent thereto, form a substituted or unsubstituted 4- to 8-membered heterocyclyl by cyclization; or in -(CH₂)ₘ₁NR⁸C(O)R⁹, -(CH₂)ₘ₁NR⁸C(O)NR⁹R¹⁰, -(CH₂)_{m'1}NR⁸S(O)_{q1}R⁹, or -(CH₂)_{m'1}NR⁸S(O)_{q1}NR⁹R¹⁰, R⁸ and R⁹, together with the N atom adjacent thereto, form a substituted or unsubstituted 4- to 8-membered heterocyclyl by cyclization, or R⁹ and R¹⁰, together with the atom adjacent thereto, form a substituted or unsubstituted 4- to 8-membered heterocyclyl by cyclization;
m1 is 0, 1, 2, 3, 4, or 5;
m'1 is 0, 1, 2, 3, 4, or 5;
p1 is 0, 1, 2, 3, 4, or 5;
q1 is 1 or 2;
wherein the substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido.

In another preferred embodiment, Y is selected from: O, NH, and NR⁷, Z is a bond, and W is C₃-C₂₀ cycloalkylene or 4- to 20-membered heterocyclylene; R¹ is not hydrogen, deuterium, halogen, or cyano, and m1 is not 0.

In another preferred embodiment, each R² is independently selected from the group consisting of: hydrogen, deuterium, halogen, cyano, -(CH₂)ₘ₂R⁸, -(CH₂)_{m'2}(CH=CH)R⁸, -(CH₂)_{m'2}(C≡C)R⁸, -(CH₂)ₘ₂O(CH₂)ₚ₂R⁸, -(CH₂)_{m'2}SR⁸, -(CH₂)ₘ₂COR⁸, -(CH₂)ₘ₂C(O)OR⁸, -(CH₂)_{m'2}S(O)_{q2}R⁸, -(CH₂)ₘ₂NR⁸R⁹, -(CH₂)ₘ₂C(O)NR⁸R⁹, -(CH₂)ₘ₂NR⁸C(O)R⁹, -(CH₂)ₘ₂NR⁸C(O)NR⁹R¹⁰, -(CH₂)_{m'2}S(O)_{q2}NR⁸R⁹, -(CH₂)_{m'2}NR⁸S(O)_{q2}R⁹, and -(CH₂)_{m'2}NR⁸S(O)_{q2}NR⁹R¹⁰, wherein H in CH₂ can be optionally substituted; R⁸, R⁹, and R¹⁰ are each independently selected from the group consisting of the following substituted or unsubstituted groups: hydrogen, C₁-C₁₈ alkyl, C₃-C₂₀ cycloalkyl, and 4- to 20-membered heterocyclyl; or in -(CH₂)ₘ₂NR⁸R⁹, -(CH₂)ₘ₂C(O)NR⁸R⁹, or -(CH₂)_{m'2}S(O)_{q2}NR⁸R⁹, R⁸ and R⁹, together with the N atom adjacent thereto, form a substituted or unsubstituted 4- to 8-membered heterocyclyl by cyclization; or in -(CH₂)ₘ₂NR⁸C(O)R⁹, -(CH₂)ₘ₂NR⁸C(O)NR⁹R¹⁰, -(CH₂)_{m'2}NR⁸S(O)_{q2}R⁹, or -(CH₂)_{m'2}NR⁸S(O)_{q2}NR⁹R¹⁰, R⁸ and R⁹, together with the N atom adjacent thereto, form a substituted or unsubstituted 4- to 8-membered heterocyclyl by cyclization, or R⁹ and R¹⁰, together with the atom adjacent thereto, form a substituted or unsubstituted 4- to 8-membered heterocyclyl by cyclization;
m2 is 0, 1, 2, 3, 4, or 5;
m'2 is 0, 1, 2, 3, 4, or 5;
p2 is 0, 1, 2, 3, 4, or 5;
q2 is 1 or 2;
wherein the substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido.

In another preferred embodiment, the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof has a structure of general formula (II): 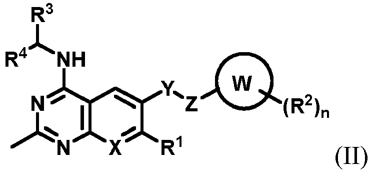 wherein in the formula, R¹, R², R³, R⁴, X, Y, Z, W, and n are as defined above.

In another preferred embodiment, the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof has a structure of general formula (III): 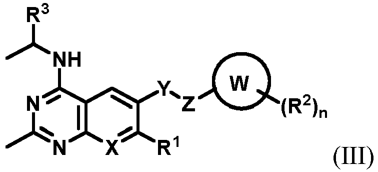 wherein in the formula, R¹, R², R³, X, Y, Z, W, and n are as defined above.

In another preferred embodiment, the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof has a structure of general formula (IV): 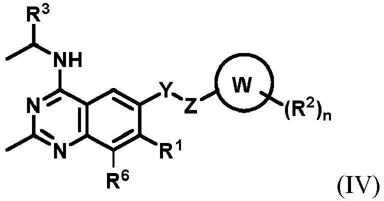
wherein in the formula,
R¹, R², R³, R⁶, X, Y, Z, W, and n are as defined above.

In another preferred embodiment, the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof has a structure of general formula (V): 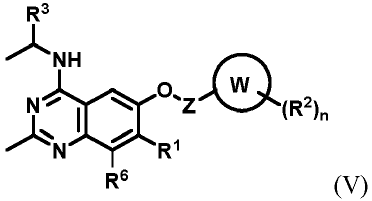
wherein in the formula,
R¹, R², R³, R⁶, Z, W, and n are as defined above.

In another preferred embodiment, in formulas I-V, Z is selected from the group consisting of the following substituted or unsubstituted groups: a bond, C₁-C₆ alkylene, deuterated C₁-C₆ alkylene, and halogenated C₁-C₆ alkylene; wherein the substitution refers to substitution with one or more groups selected from the group consisting of: deuterium, C₁-C₆ alkyl, deuterated C₁-C₆ alkyl, halogenated C₁-C₆ alkyl, halogenated C₁-C₆ alkylhydroxy, C₃-C₆ cycloalkyl, C₁-C₆ alkoxy, deuterated C₁-C₆ alkoxy, halogenated C₁-C₆ alkoxy, C₆-C₁₀ aryl, 5- to 10-membered heteroaryl, 4- to 6-membered heterocyclyl, halogen, oxo, nitro, hydroxy, cyano, ester group, amino, amido, sulfonamido, and ureido.

In another preferred embodiment, W is selected from the group consisting of: a bond, substituted or unsubstituted C₃-C₁₂ cycloalkylene, substituted or unsubstituted 4- to 12-membered heterocyclylene, OR¹¹, NR¹¹R¹², SO₂, NR¹²SO₂, CO, and NR¹²CO;
wherein R¹¹ is selected from the group consisting of the following substituted or unsubstituted groups: C₃-C₁₂ cycloalkylene, 4- to 12-membered heterocyclylene, C₃-C₁₂ cycloalkylene C₁-C₆ alkylene, 4- to 12-membered heterocyclylene C₁-C₆ alkylene, C₆-C₁₄ aryl, and 5- to 14-membered heteroaryl; preferably, R¹¹ is selected from the group consisting of the following substituted or unsubstituted groups: C₃-C₆ cycloalkylene, 4- to 6-membered heterocyclylene, C₃-C₆ cycloalkylene C₁-C₃ alkylene, 4- to 6-membered heterocyclylene C₁-C₃ alkylene, C₆-C₁₄ aryl, and 5- to 14-membered heteroaryl;
R¹² is independently selected from the group consisting of the following substituted or unsubstituted groups: hydrogen, deuterium, C₁-C₆ alkyl, and C₃-C₆ cycloalkyl;
wherein the above substitution refers to substitution with one or more (e.g., 2, 3, or 4) groups selected from the group consisting of: deuterium, C₁-C₆ alkyl, deuterated C₁-C₆ alkyl, halogenated C₁-C₆ alkyl, halogenated C₁-C₆ alkylhydroxy, C₃-C₆ cycloalkyl, C₁-C₆ alkoxy, deuterated C₁-C₆ alkoxy, halogenated C₁-C₆ alkoxy, C₆-C₁₀ aryl, 5- to 10-membered heteroaryl, 4- to 6-membered heterocyclyl, halogen, oxo, nitro, hydroxy, cyano, ester group, amino, amido, sulfonamido, and ureido;
provided that when Z is a bond and W is C₃-C₂₀ cycloalkylene or 4- to 20-membered heterocyclylene, R¹ is not hydrogen, deuterium, halogen, cyano, R⁸, O(CH₂)ₚR⁸, COR⁸, -C(O)OR⁸, NR⁸R⁹, C(O)NR⁸R⁹, -NR⁸C(O)R⁹, or -NR⁸C(O)NR⁹R¹⁰.

In another preferred embodiment, the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof has a structure of formula (VI): 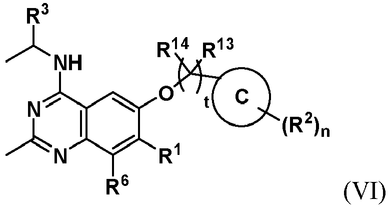
wherein in the formula,
R¹³ and R¹⁴ are each independently selected from: H, C₁-C₆ alkyl, deuterated C₁-C₆ alkyl, halogenated C₁-C₆ alkyl, halogen, oxo, nitro, hydroxy, cyano, ester group, amino, amido, sulfonamido, and ureido;
ring C is selected from the group consisting of the following substituted or unsubstituted groups: C₃-C₁₂ cycloalkylene and 4- to 12-membered heterocyclylene;
each R² is identical or different and is independently selected from the group consisting of: -(CH₂)ₘO(CH₂)ₚR⁸, -(CH₂)ₘ(CH=CH)ₚR⁸, (CH₂)ₘ(C=C)ₚR⁸, -(CH₂)ₘSR⁸, -(CH₂)ₘCOR⁸, -(CH₂)ₘC(O)OR⁸, -(CH₂)ₘS(O)_{q}R⁸, -(CH₂)ₘNR⁸R⁹, -(CH₂)ₘC(O)NR⁸R⁹, -(CH₂)ₘNR⁸C(O)R⁹, -(CH₂)ₘNR⁸C(O)NR⁹R¹⁰, -(CH₂)ₘS(O)_{q}NR⁸R⁹, -(CH₂)ₘNR⁸S(O)_{q}R⁹, and -(CH₂)ₘNR⁸S(O)_{q}NR⁹R¹⁰, wherein H in CH₂ can be optionally substituted; m is selected from 1, 2, 3, 4, and 5;
the substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido;
t is 1, 2, 3, 4, 5, or 6;
R¹, R³, R⁶, R⁸, R⁹, R¹⁰, p, q, and n are as defined above.

In another preferred embodiment, in formula (VI), the moiety 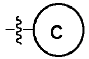 selected from: 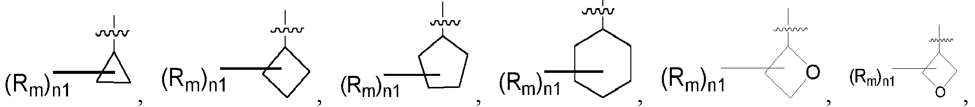 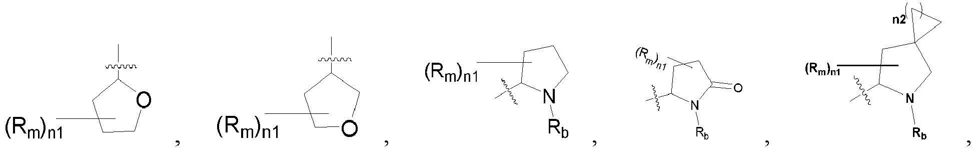 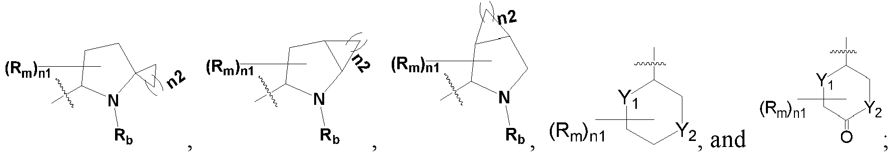
wherein Y₁ and Y₂ are each independently selected from: NR_{b}, and O;
Rₘ is independently selected from: hydrogen, deuterium, C₁-C₆ alkyl, deuterated C₁-C₆ alkyl, halogenated C₁-C₆ alkyl, halogenated C₁-C₆ alkylhydroxy, C₃-C₆ cycloalkyl, C₁-C₆ alkoxy, deuterated C₁-C₆ alkoxy, halogenated C₁-C₆ alkoxy, C₆-C₁₀ aryl, 5- to 10-membered heteroaryl, 4- to 6-membered heterocyclyl, halogen, nitro, hydroxy, oxo, cyano, ester group, amino, amido, sulfonamido, and ureido;
R_{b} is independently selected from: H, C₁-C₆ alkyl, deuterated C₁-C₆ alkyl, halogenated C₁-C₆ alkyl, C₃-C₆ cycloalkyl, 4- to 6-membered heterocyclyl, SO₂R³⁰, COR³⁰, and ester group;
R³⁰ is each independently selected from: hydrogen, substituted or unsubstituted C₁-C₆ alkyl, substituted or unsubstituted C₃-C₆ cycloalkyl, and substituted or unsubstituted 4- to 6-membered heterocyclyl;
n1 is 0, 1, 2, 3, or 4;
n2 is 1, 2, 3, or 4;
wherein the above substitution refers to substitution with one or more (e.g., 2, 3, or 4) groups selected from the group consisting of: hydrogen, deuterium, C₁-C₆ alkyl, deuterated C₁-C₆ alkyl, halogenated C₁-C₆ alkyl, halogenated C₁-C₆ alkylhydroxy, C₃-C₆ cycloalkyl, C₁-C₆ alkoxy, deuterated C₁-C₆ alkoxy, halogenated C₁-C₆ alkoxy, C₆-C₁₀ aryl, 5- to 10-membered heteroaryl, 4- to 6-membered heterocyclyl, halogen, oxo, nitro, hydroxy, cyano, ester group, amino, amido, sulfonamido, and ureido.

In another preferred embodiment, the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof has a structure of formula (VII): 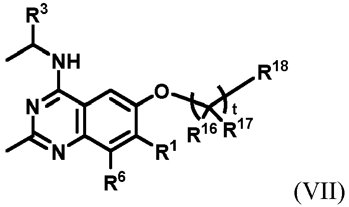
wherein in the formula,
R¹⁶ and R¹⁷ are each independently selected from: H, C₁-C₆ alkyl, deuterated C₁-C₆ alkyl, halogenated C₁-C₆ alkyl, halogen, oxo, nitro, hydroxy, cyano, ester group, amino, amido, sulfonamido, and ureido;
R¹⁸ is selected from: OR¹¹, NR¹¹R¹², NR¹²SO₂R², COR², and NR¹²COR²; R¹¹ is independently selected from: substituted C₃-C₁₂ cycloalkyl, substituted or unsubstituted 4- to 12-membered heterocyclyl, substituted or unsubstituted C₃-C₁₂ cycloalkylene C₁-C₆ alkylene, substituted or unsubstituted 4- to 12-membered heterocyclylene C₁-C₆ alkylene, substituted or unsubstituted C₆-C₁₄ aryl, and substituted or unsubstituted 5- to 14-membered heteroaryl; R¹² is independently selected from the group consisting of the following substituted or unsubstituted groups: hydrogen, deuterium, C₁-C₆ alkyl, and C₃-C₆ cycloalkyl;
or R¹⁸ is selected from: -(CH₂)ₘ(CH=CH)R⁸, -(CH₂)ₘ(C≡C)R⁸, -(CH₂)ₘO(CH₂)ₚR⁸, -(CH₂)ₘSR⁸, -(CH₂)ₘCOR⁸, -(CH₂)ₘC(O)OR⁸, -(CH₂)ₘS(O)_{q}R⁸, -(CH₂)ₘNR⁸R⁹, -(CH₂)ₘC(O)NR⁸R⁹, -(CH₂)ₘNR⁸C(O)R⁹, -(CH₂)ₘNR⁸C(O)NR⁹R¹⁰, -(CH₂)ₘS(O)_{q}NR⁸R⁹, -(CH₂)ₘNR⁸S(O)_{q}R⁹, and -(CH₂)ₘNR⁸S(O)_{q}NR⁹R¹⁰, wherein H in CH₂ can be optionally substituted; R⁸, R⁹, and R¹⁰ are each independently selected from: substituted or unsubstituted C₁-C₁₈ alkyl, substituted or unsubstituted C₃-C₂₀ cycloalkyl, and substituted or unsubstituted 4-to 20-membered heterocyclyl;
wherein the substitution in R⁸, R⁹, and R¹⁰ refers to substitution with one or more groups selected from the group consisting of: C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido;
t is 1, 2, 3, 4, 5, or 6;
wherein unless otherwise stated, the above substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido;
R¹, R², R³, R⁶, R⁸, R⁹, R¹⁰, m, p, and q are as defined above.

In another preferred embodiment, R¹ is selected from the group consisting of: H, cyano, halogen, -(CH₂)ₘR⁸, -(CH₂)ₘO(CH₂)ₚR⁸, -(CH₂)ₘSR⁸, -(CH₂)ₘS(O)_{q}R⁸, and -(CH₂)ₘ(C≡C)R⁸; wherein H in CH₂ can be optionally substituted; R⁸ is selected from the group consisting of the following substituted or unsubstituted groups: hydrogen, C₁-C₁₈ alkyl, C₁-C₁₈ alkoxy, C₃-C₈ cycloalkyl, 4- to 10-membered heterocyclyl, C₆-C₁₄ aryl, and 5- to 14-membered heteroaryl.

In another preferred embodiment, R¹ is selected from the group consisting of: halogen, cyano, -(CH₂)ₘR⁸, -(CH₂)ₘ(C≡CH), -(CH₂)ₘ(C≡C)R⁸, and -(CH₂)ₘO(CH₂)ₚR⁸, and preferably R⁸ is substituted or unsubstituted C₁-C₁₈ alkyl (preferably C₁-C₆ alkyl).

In another preferred embodiment, R¹ is selected from the group consisting of: H, cyano, halogen, hydroxy, C₁-C₆ alkyl, C₁-C₆ alkoxy, halogenated C₁-C₆ alkyl, halogenated C₁-C₆ alkyl-O-, deuterated C₁-C₆ alkyl-O-, substituted or unsubstituted C₃-C₆ cycloalkyl-O-, substituted or unsubstituted 4- to 6-membered heterocyclyl-O-, C₁-C₆ alkoxy C₁-C₆ alkyl-O-, substituted or unsubstituted phenyl, substituted or unsubstituted 5- to 6-membered heteroaryl, substituted or unsubstituted phenyl-O-, substituted or unsubstituted 5- to 6-membered heteroaryl-O-, and substituted or unsubstituted C₂-C₆ alkynyl; wherein the substitution refers to substitution with one or more (e.g., 2, 3, or 4) groups selected from the group consisting of: halogen, C₁-C₆ alkyl, C₃-C₆ cycloalkyl, oxo C₁-C₆ alkyl, and C₂-C₆ ester group.

In another preferred embodiment, in formula VII, R¹⁸ is selected from: OR¹¹, NR¹¹R¹², and NR¹²SO₂R²; wherein R¹¹ is independently selected from: substituted C₃-C₁₂ cycloalkyl, substituted or unsubstituted 4- to 12-membered heterocyclyl, substituted or unsubstituted C₃-C₁₂ cycloalkylene C₁-C₆ alkylene, substituted or unsubstituted 4- to 12-membered heterocyclylene C₁-C₆ alkylene, substituted or unsubstituted C₆-C₁₄ aryl, and substituted or unsubstituted 5- to 14-membered heteroaryl; R¹² is independently selected from the group consisting of: hydrogen, deuterium, substituted or unsubstituted C₁-C₆ alkyl, and substituted or unsubstituted C₃-C₆ cycloalkyl;
or R¹⁸ is selected from: -(CH₂)ₘO(CH₂)ₚR⁸; wherein H in CH₂ can be optionally substituted; R⁸ is selected from: substituted or unsubstituted C₁-C₁₈ alkyl, substituted or unsubstituted C₃-C₂₀ cycloalkyl, and substituted or unsubstituted 4- to 20-membered heterocyclyl; preferably, R⁸ is substituted or unsubstituted C₁-C₁₈ alkyl (preferably C₁-C₆ alkyl);
the above substitution refers to substitution with one or more (e.g., 2, 3, or 4) groups selected from the group consisting of: C₁-C₆ alkyl, deuterated C₁-C₆ alkyl, halogenated C₁-C₆ alkyl, halogenated C₁-C₆ alkylhydroxy, C₃-C₆ cycloalkyl, C₁-C₆ alkoxy, deuterated C₁-C₆ alkoxy, halogenated C₁-C₆ alkoxy, C₆-C₁₀ aryl, 5- to 10-membered heteroaryl, 4- to 6-membered heterocyclyl, halogen, oxo, nitro, hydroxy, cyano, ester group, amino, amido, sulfonamido, and ureido.

In another preferred embodiment, R³ is selected from: substituted C₆-C₁₄ aryl and substituted 5-to 14-membered heteroaryl; the substitution refers to substitution with one or more groups selected from the group consisting of: R^{3a}, hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₃-C₂₀ cycloalkyl-O-, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, 4- to 20-membered heterocyclyl-O-, halogen, oxo, nitro, hydroxy, cyano, ester group, amino, amido, sulfonamido, and ureido; wherein the C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₃-C₂₀ cycloalkyl-O-, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, and 4- to 20-membered heterocyclyl-O- may be further substituted with one or more R^{a}, wherein R^{a} is selected from: C₁-C₆ alkyl, deuterated C₁-C₆ alkyl, halogenated C₁-C₆ alkyl, halogenated C₁-C₆ alkylhydroxy, C₃-C₆ cycloalkyl, C₃-C₆ cycloalkyl-O-, C₁-C₆ alkoxy, deuterated C₁-C₆ alkoxy, halogenated C₁-C₆ alkoxy, C₆-C₁₄ aryl, 5- to 14-membered heteroaryl, 4- to 6-membered heterocyclyl, 4- to 6-membered heterocyclyl-O-, halogen, oxo, nitro, hydroxy, cyano, ester group, amino, amido, sulfonamido, and ureido; provided that at least one R^{3a} substituent is present;
wherein R^{3a} is selected from: C₁-C₁₈ alkyl substituted with hydroxy, C₁-C₁₈ haloalkyl substituted with hydroxy, C₁-C₁₈ deuteroalkyl substituted with hydroxy, C₁-C₁₈ alkyl substituted with alkoxy, C₁-C₁₈ haloalkyl substituted with alkoxy, C₁-C₁₈ deuteroalkyl substituted with alkoxy, C₁-C₁₈ alkyl substituted with cycloalkyloxy, C₁-C₁₈ haloalkyl substituted with cycloalkyloxy, C₁-C₁₈ deuteroalkyl substituted with cycloalkyloxy, C₁-C₁₈ alkyl substituted with heterocyclyloxy, C₁-C₁₈ haloalkyl substituted with heterocyclyloxy, C₁-C₁₈ deuteroalkyl substituted with heterocyclyloxy, C₁-C₁₈ haloalkyl substituted with cycloalkyl, C₁-C₁₈ haloalkyl substituted with heterocyclyl, C₁-C₁₈ haloalkyl substituted with amino, C₁-C₁₈ haloalkyl substituted with cyano, C₁-C₁₈ haloalkyl substituted with amido, substituted C₃-C₁₂ cycloalkyl, substituted 4- to 12-membered heterocyclyl, substituted or unsubstituted haloalkyloxy, substituted or unsubstituted cycloalkyloxy, substituted or unsubstituted heterocyclyloxy, substituted or unsubstituted sulfonamido, and substituted or unsubstituted cycloalkylsulfonyl; the above substitution refers to substitution with one or more groups selected from the group consisting of: C₁-C₆ alkyl, deuterated C₁-C₆ alkyl, halogenated C₁-C₆ alkyl, halogenated C₁-C₆ alkylhydroxy, C₃-C₆ cycloalkyl, C₁-C₆ alkoxy, deuterated C₁-C₆ alkoxy, halogenated C₁-C₆ alkoxy, C₆-C₁₀ aryl, 5- to 10-membered heteroaryl, 4- to 6-membered heterocyclyl, halogen, oxo, nitro, hydroxy, cyano, ester group, amino, amido, sulfonamido, and ureido.

In another preferred embodiment, the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof has a structure of formula (VIII): 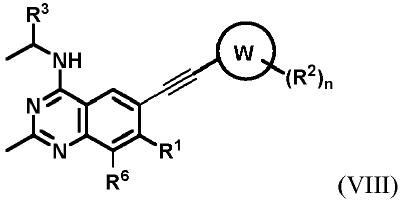 wherein in the formula, R¹, R², R³, R⁶, and W are as defined above.

In another preferred embodiment, the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof has a structure of formula (IX-A) or (IX-B): 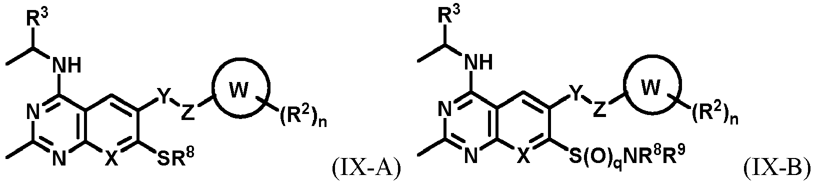 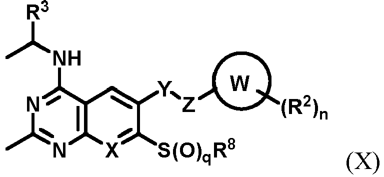 wherein in the formula, R², R³, R⁸, R⁹, X, Y, Z, W, n, and q are as defined above.

In another preferred embodiment, the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof has a structure of formula (X):
wherein in the formula, R⁸ is selected from the group consisting of the following substituted or unsubstituted groups: C₃-C₂₀ cycloalkyl and 4- to 20-membered heterocyclyl;
wherein the substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido;
R², R³, X, Y, Z, W, n, and q are as defined above.

In another preferred embodiment, R⁸ is selected from the group consisting of the following substituted or unsubstituted groups: C₃-C₆ cycloalkyl and 4- to 6-membered heterocyclyl; wherein the substitution refers to substitution with one or more groups selected from the group consisting of: hydrogen, deuterium, C₁-C₆ alkyl, deuterated C₁-C₆ alkyl, halogenated C₁-C₆ alkyl, halogenated C₁-C₆ alkylhydroxy, C₃-C₆ cycloalkyl, C₁-C₆ alkoxy, deuterated C₁-C₆ alkoxy, halogenated C₁-C₆ alkoxy, C₆-C₁₄ aryl, 5- to 14-membered heteroaryl, 4- to 6-membered heterocyclyl, halogen, oxo, nitro, hydroxy, cyano, ester group, amino, amido, sulfonamido, and ureido.

In another preferred embodiment, Z and W are both bonds.

In another preferred embodiment, Y is O, and Z and W are bonds.

In another preferred embodiment, R³ is selected from the group consisting of the following substituted groups: phenyl, pyridyl, pyrimidinyl, and pyridazinyl; wherein the substitution refers to substitution with one or more (e.g., 2, 3, or 4) groups selected from the group consisting of: C₁-C₆ alkyl, deuterated C₁-C₆ alkyl, halogenated C₁-C₆ alkyl, halogenated C₁-C₆ alkylhydroxy, C₁-C₆ alkoxy, deuterated C₁-C₆ alkoxy, halogenated C₁-C₆ alkoxy, halogen, oxo, nitro, hydroxy, cyano, ester group, amino, amido, sulfonamido, and ureido; preferably, the substituent is selected from 1, 2, or 3 of halogenated C₁-C₆ alkyl, halogenated C₁-C₆ alkylhydroxy, C₁-C₆ alkoxy, halogen, hydroxy, cyano, ester group, amino, amido and sulfonamido.

In another preferred embodiment R¹ is methoxy.

In another preferred embodiment, 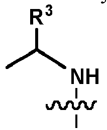 is 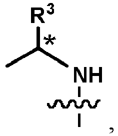 wherein * represents R or S configuration.

Preferably, R³ is selected from:

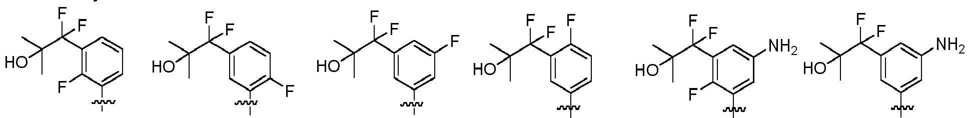

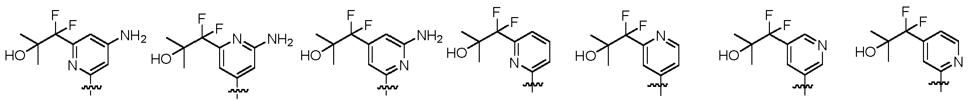

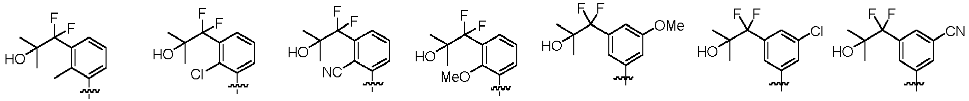

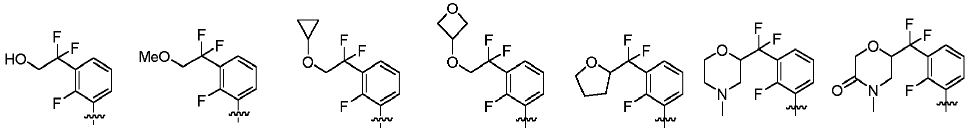

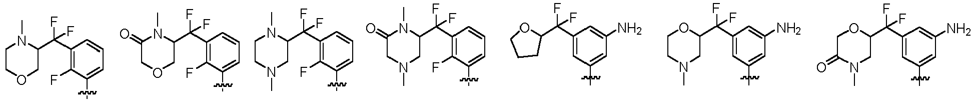

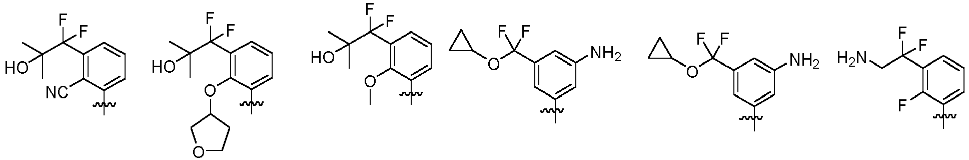

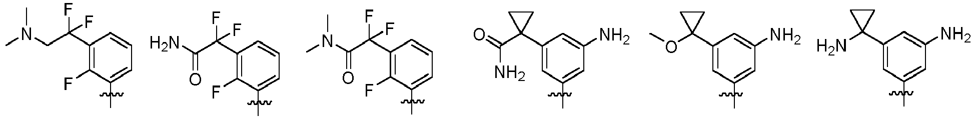

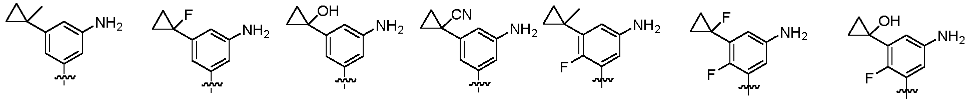

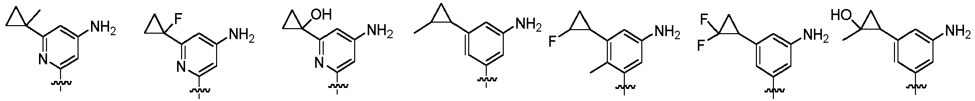

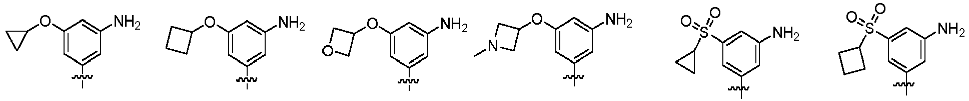

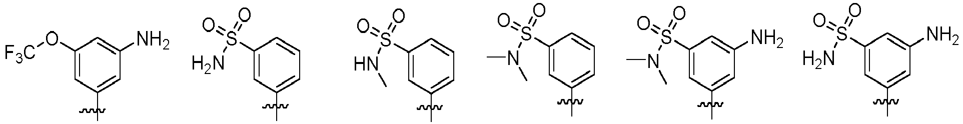

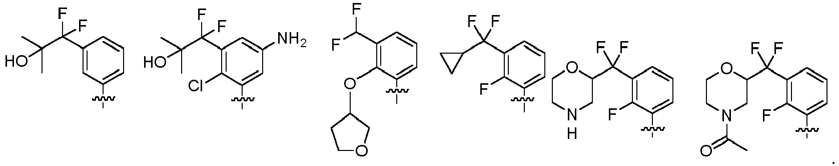

In another preferred embodiment, R³ is selected from:

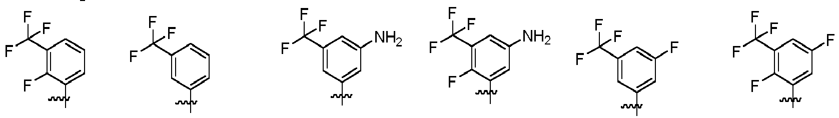

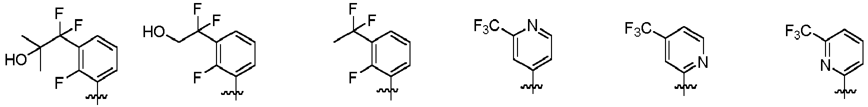

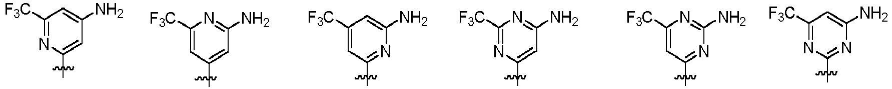

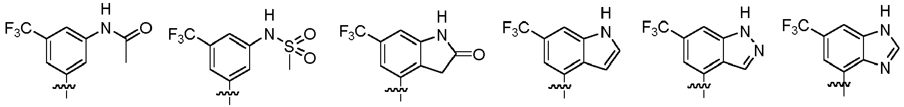

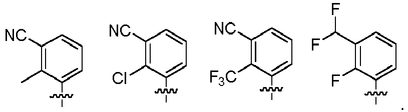

In another preferred embodiment, R⁶ is selected from: hydrogen, deuterium, halogen, cyano, and C₁-C₆ alkyl.

In another preferred embodiment, R¹, R², R³, R⁴, R⁵, R⁶, X, Y, Z, W, and n are the specific groups corresponding to the specific compounds in the examples.

In another preferred embodiment, for the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof, the compound is selected from the group consisting of:

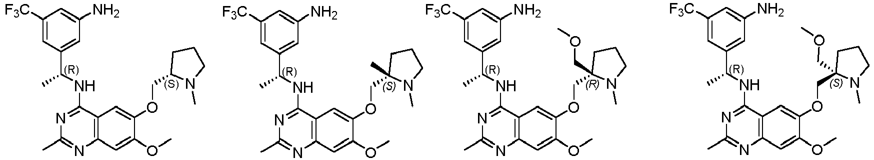

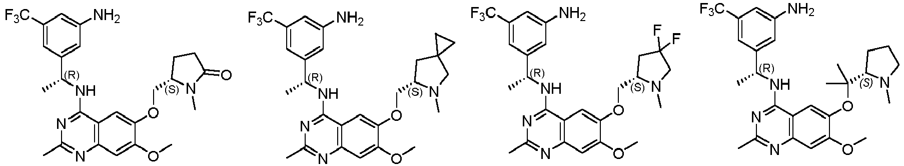

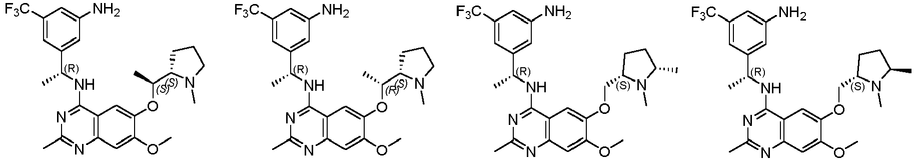

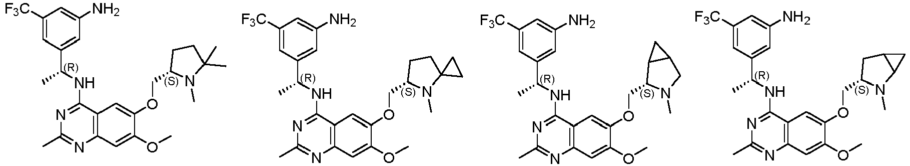

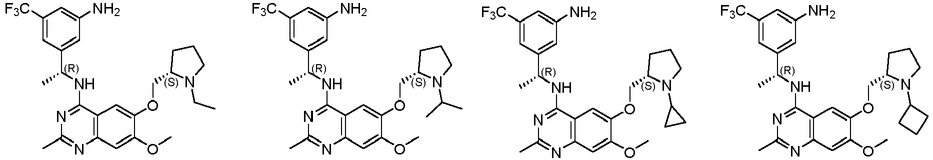

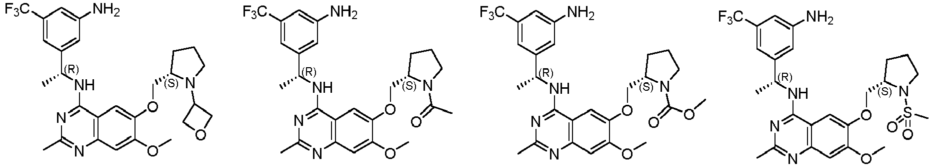

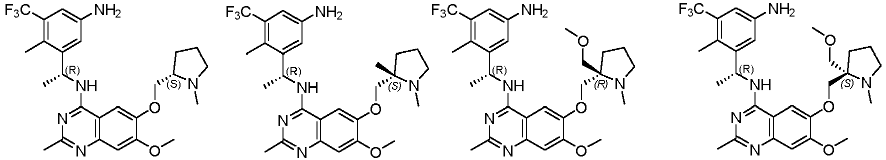

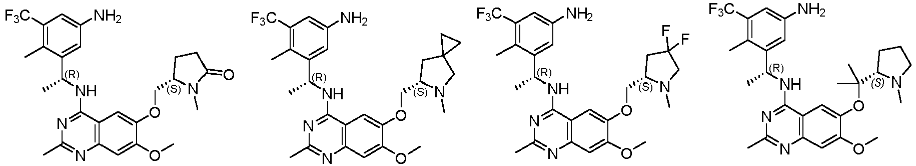

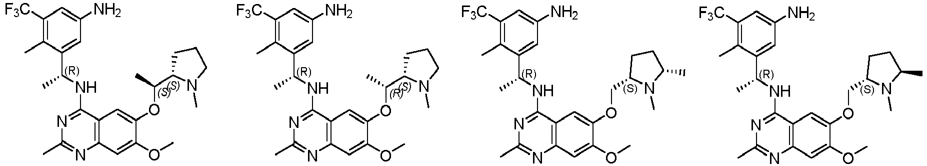

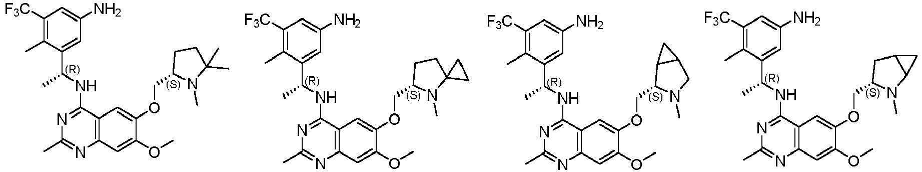

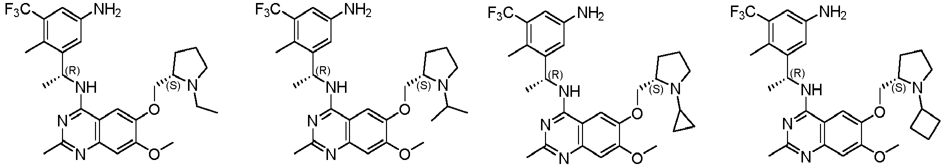

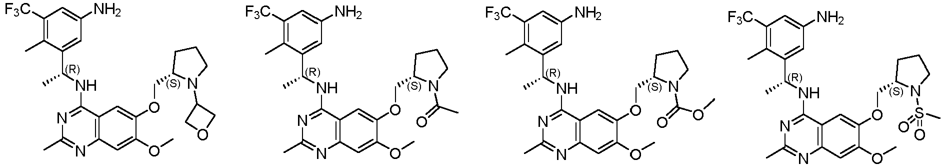

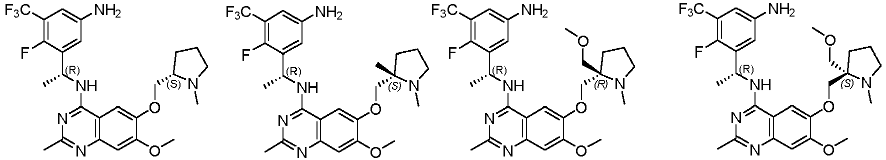

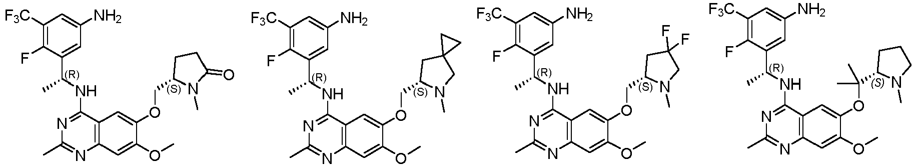

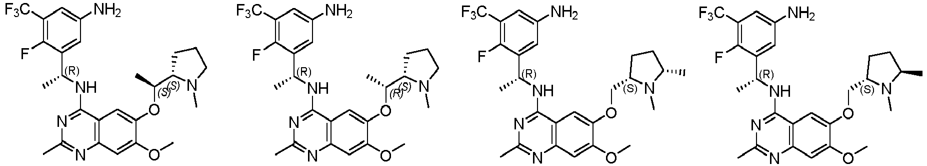

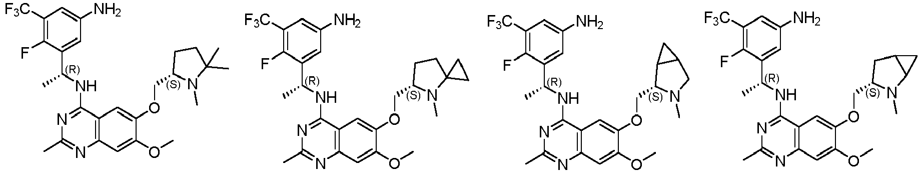

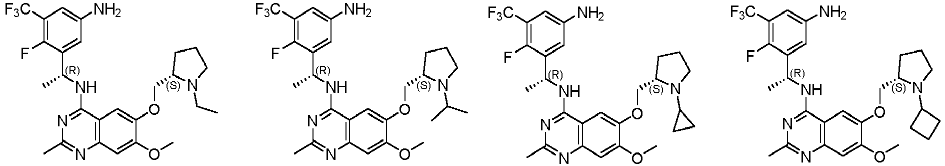

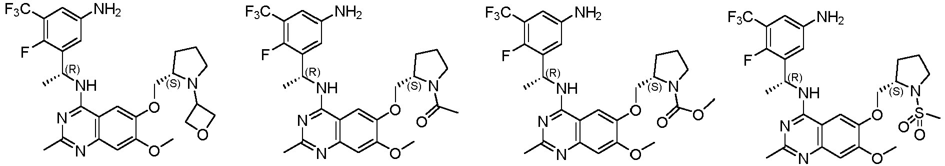

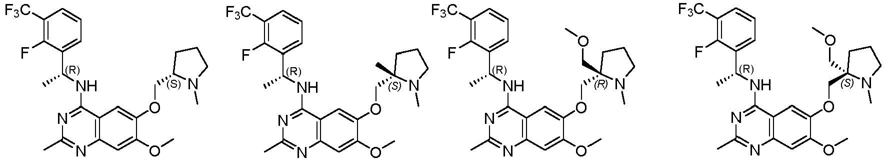

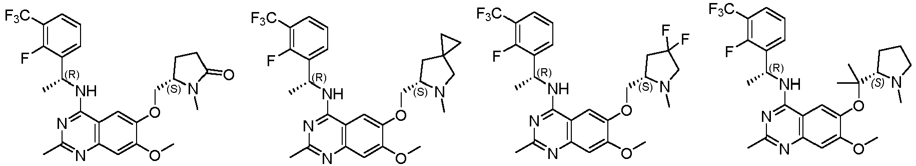

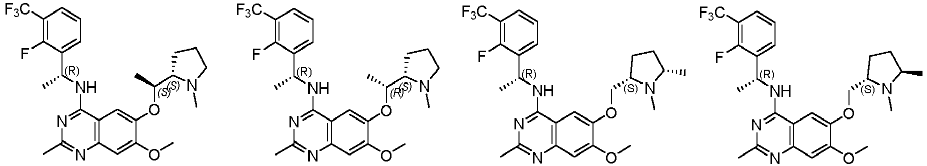

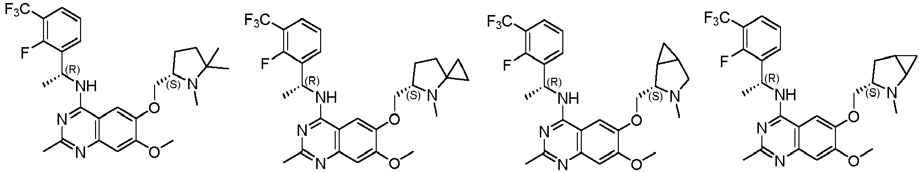

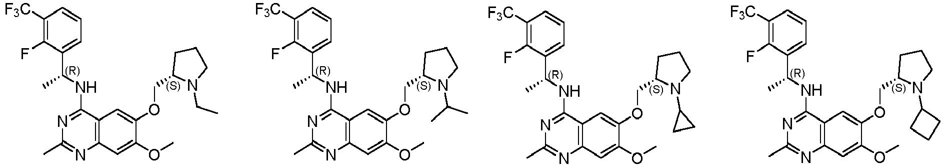

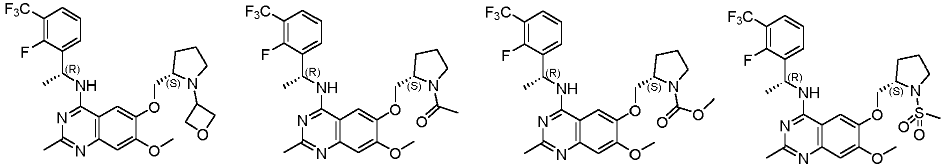

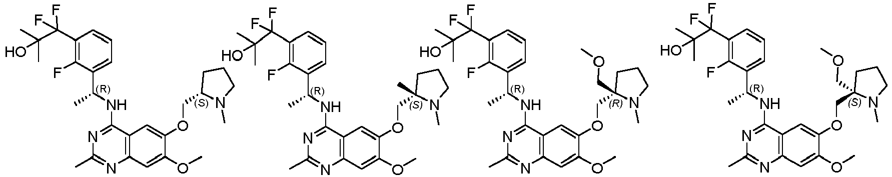

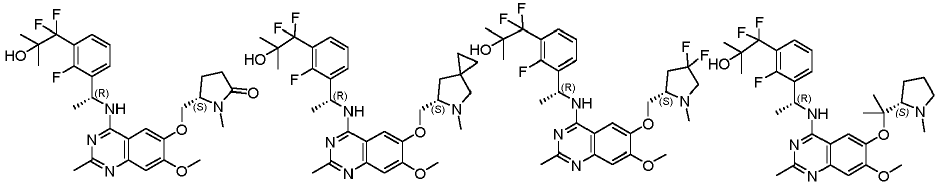

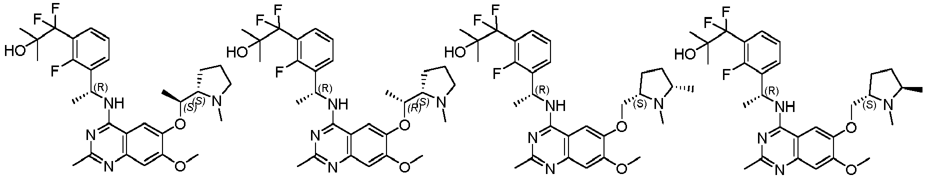

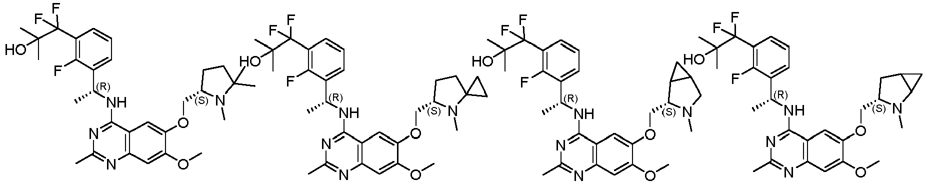

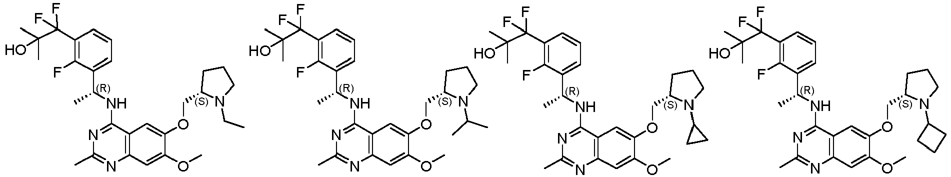

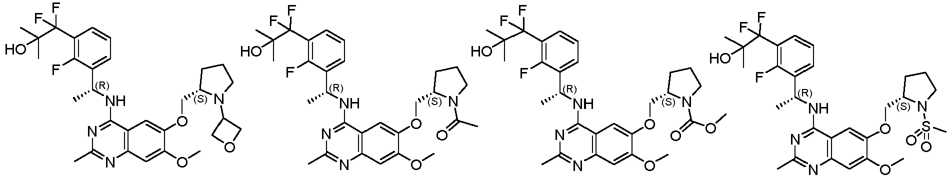

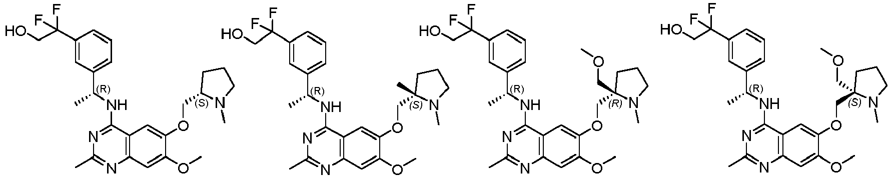

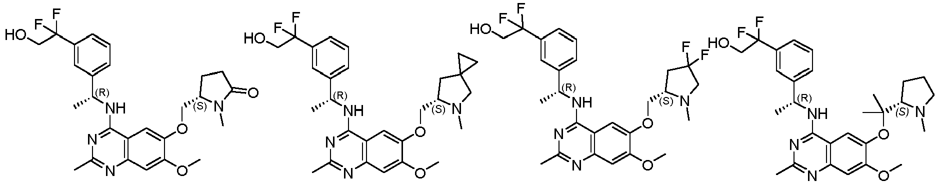

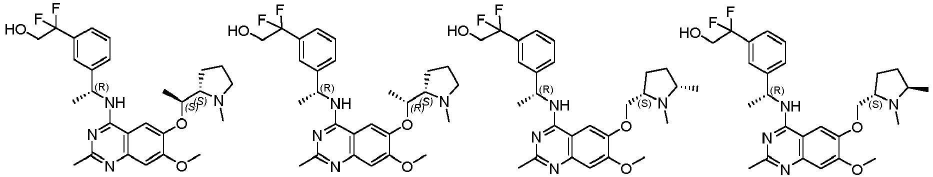

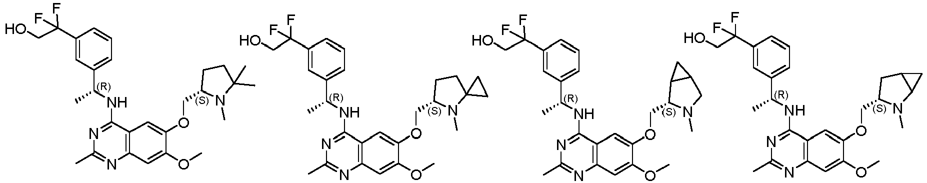

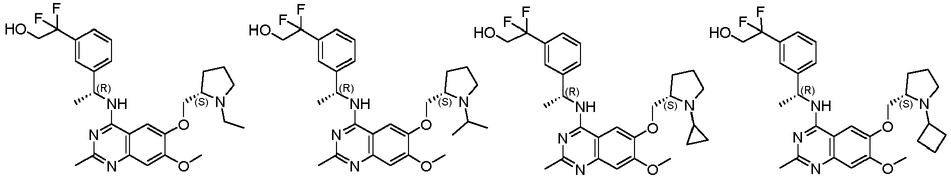

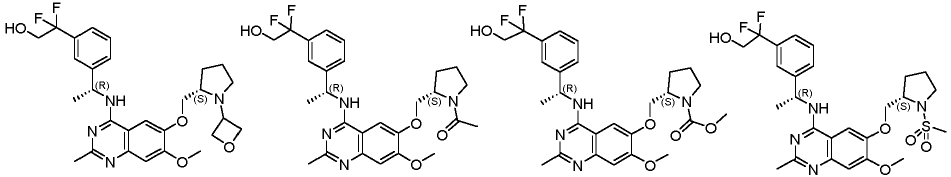

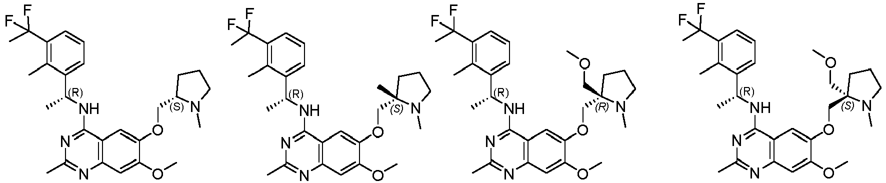

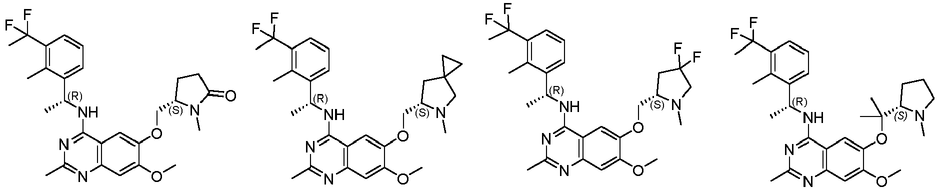

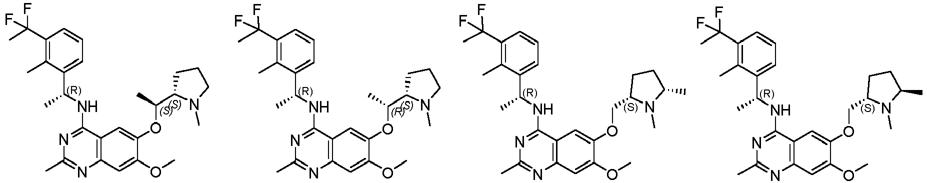

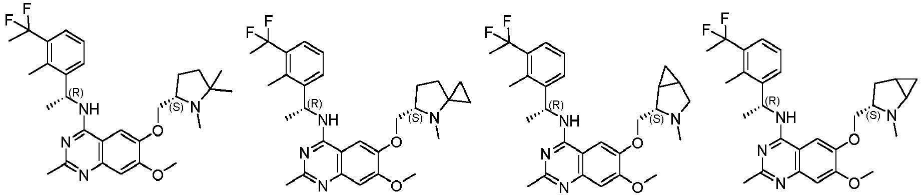

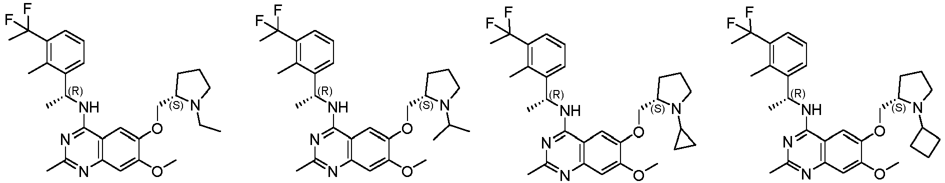

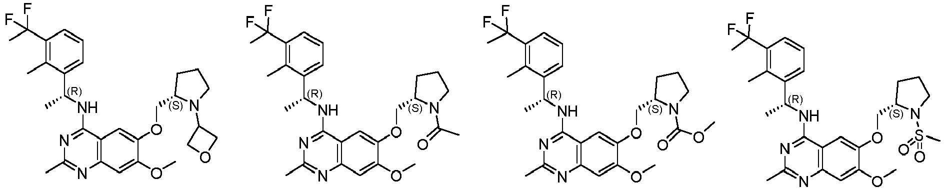

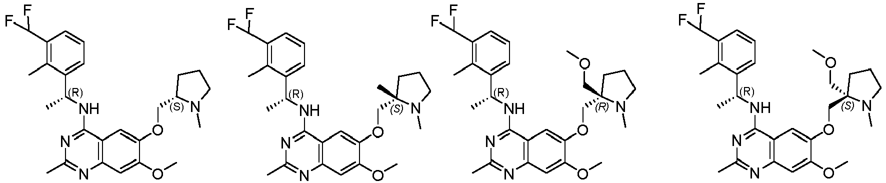

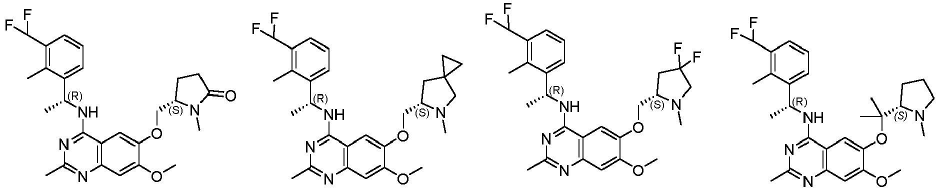

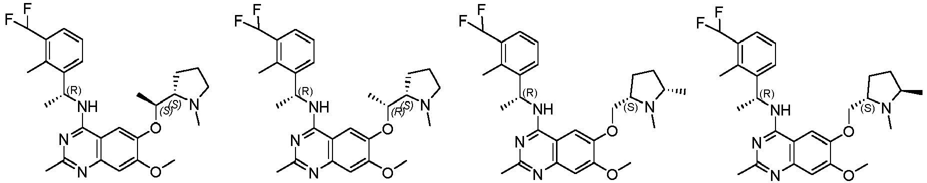

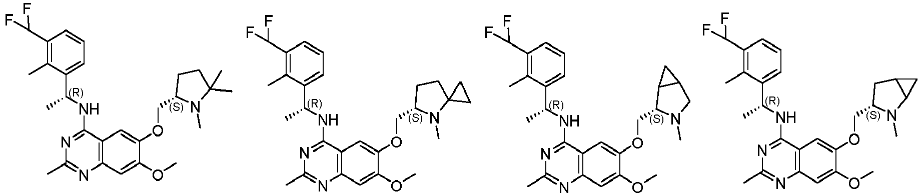

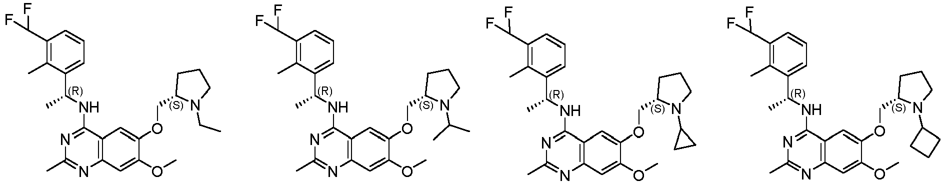

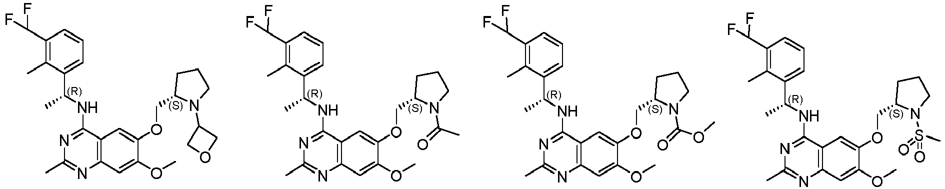

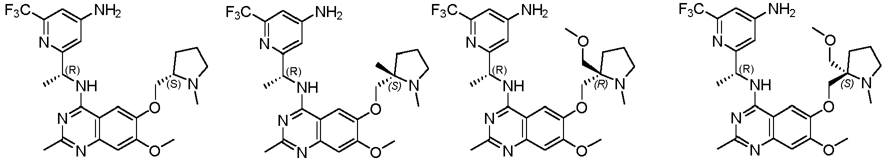

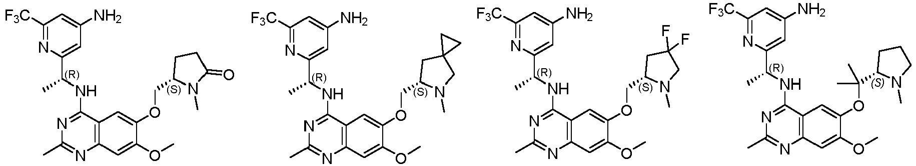

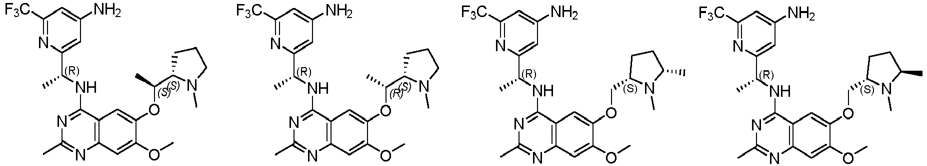

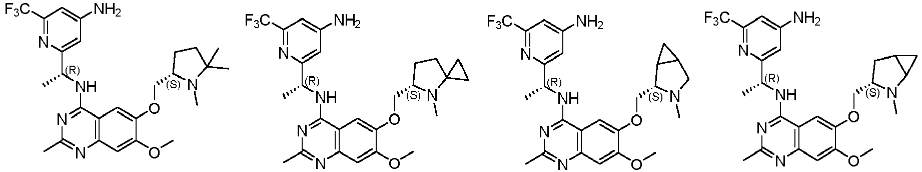

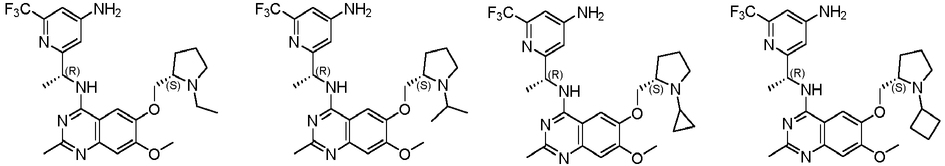

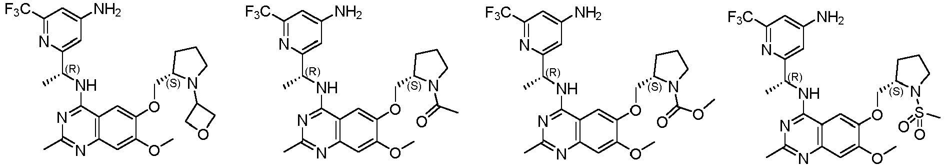

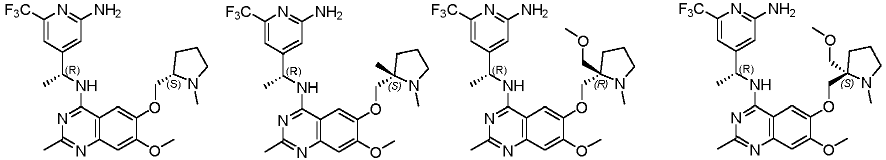

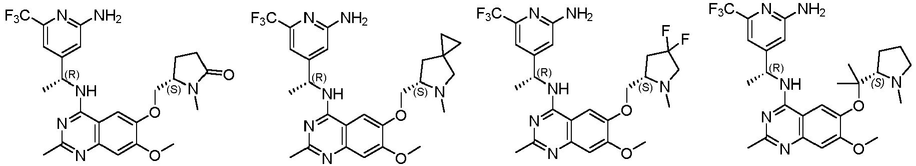

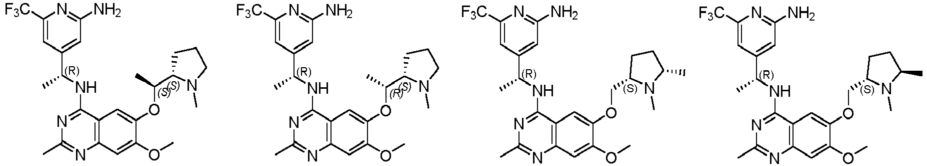

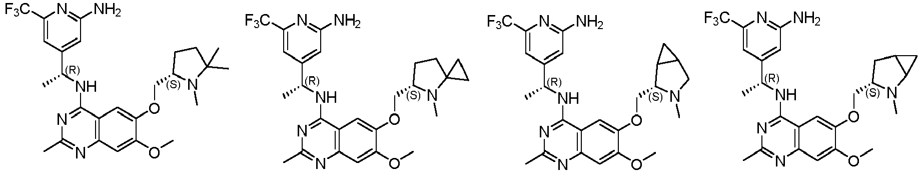

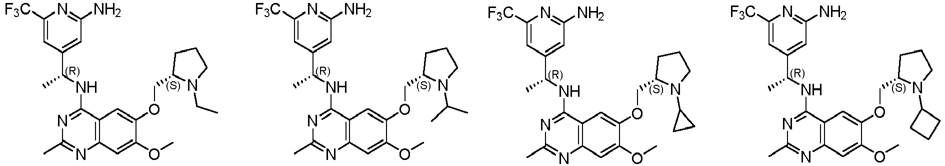

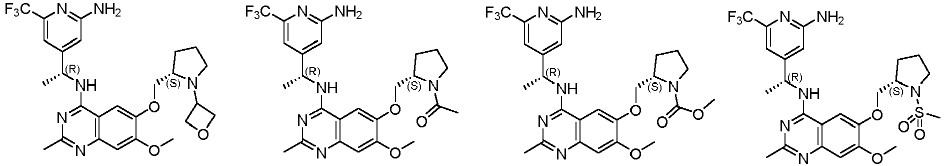

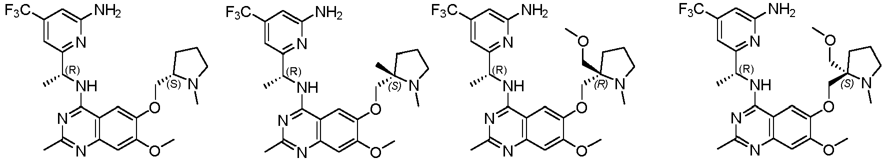

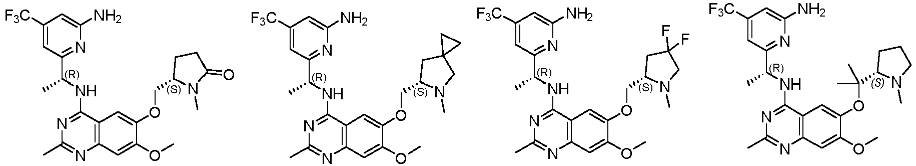

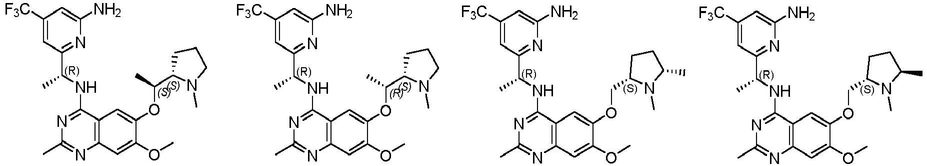

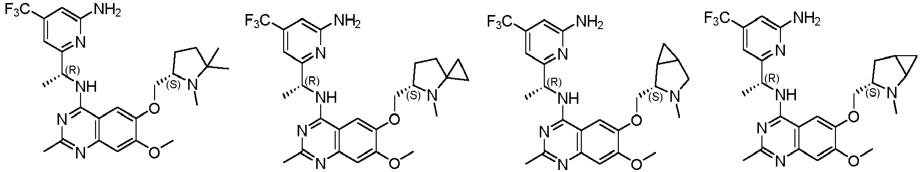

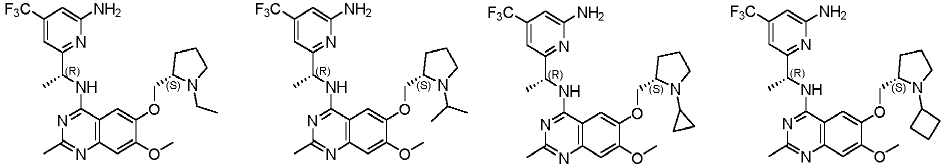

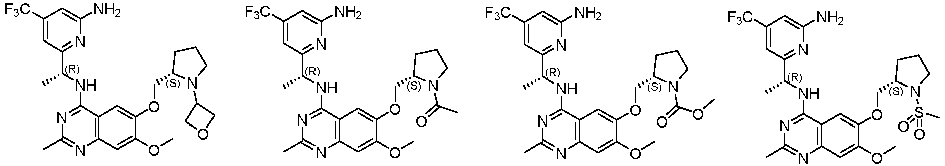

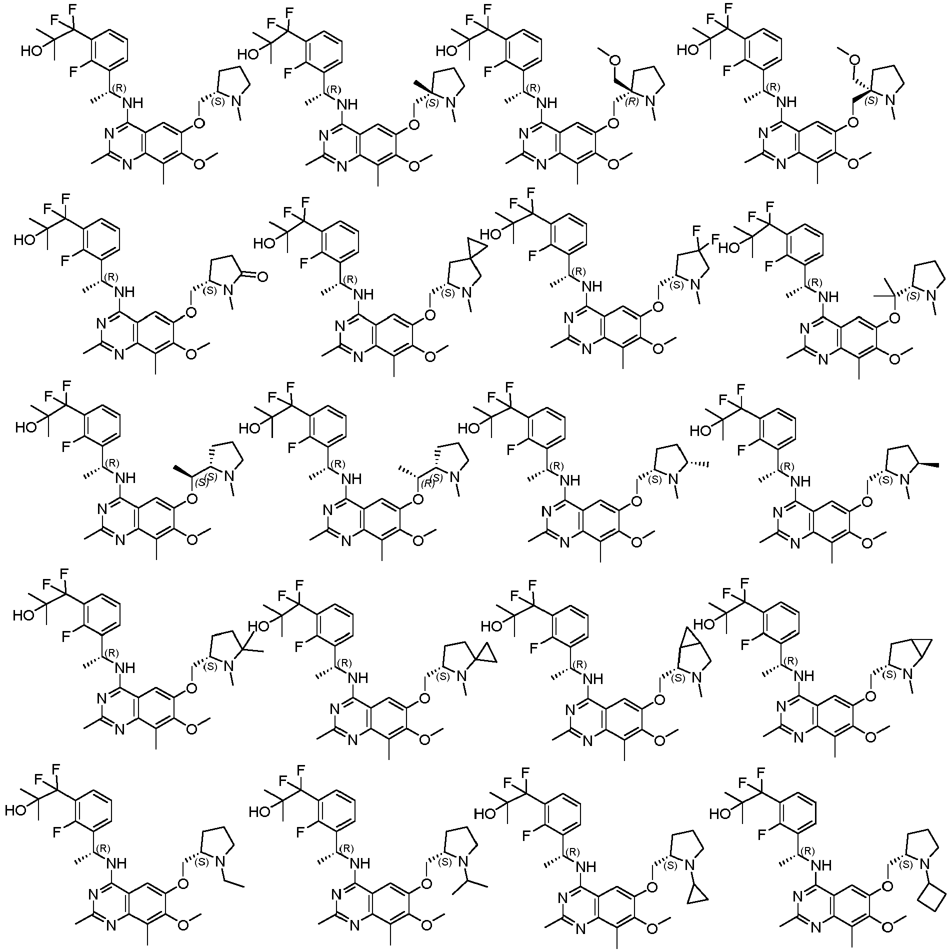

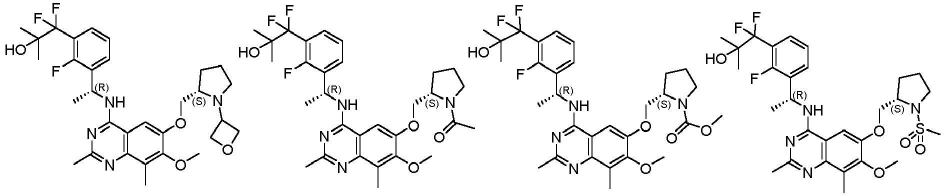

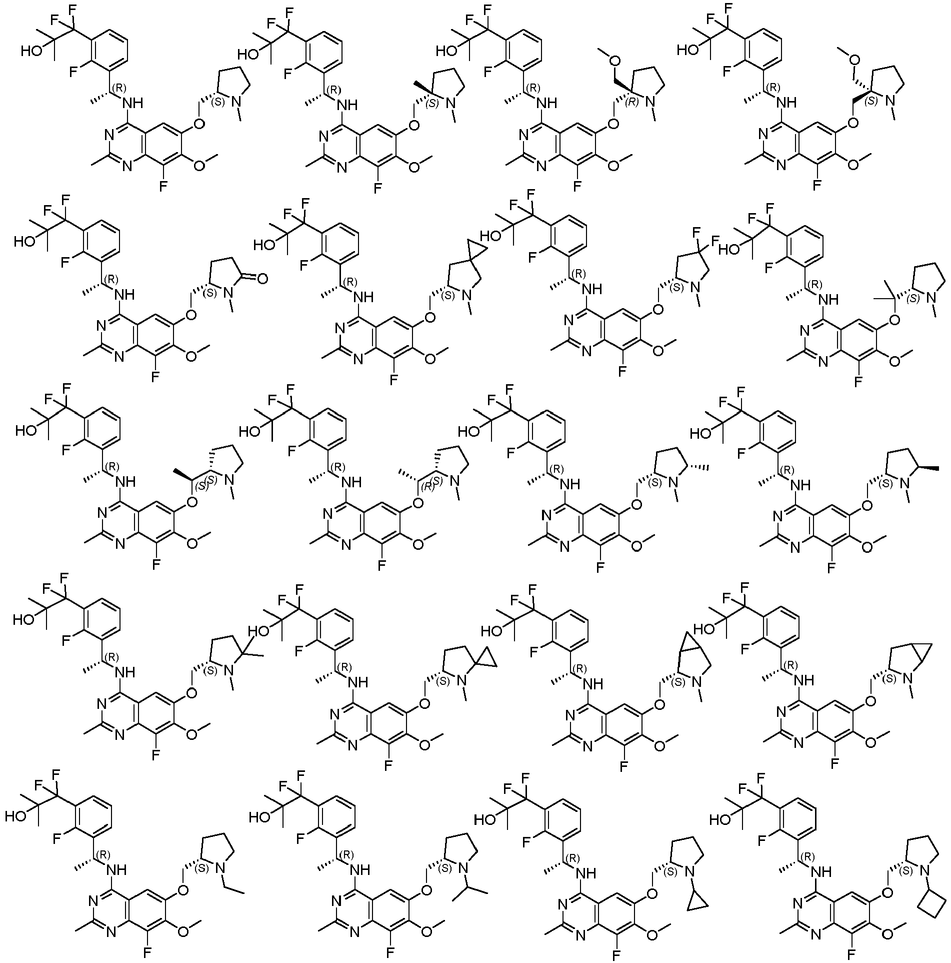

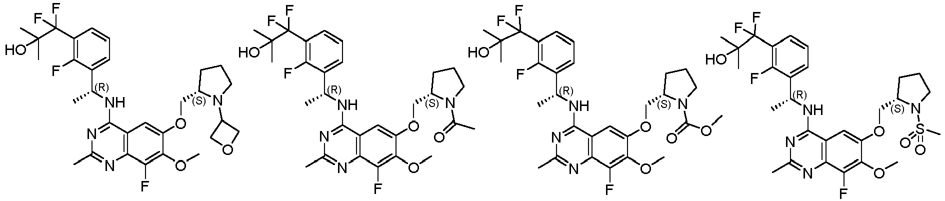

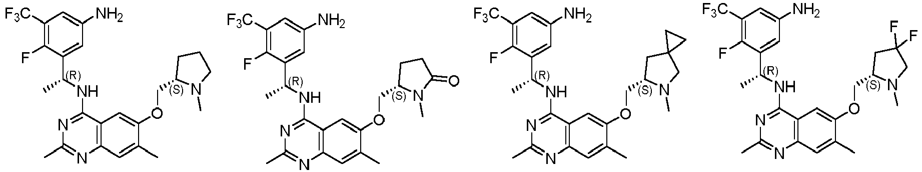

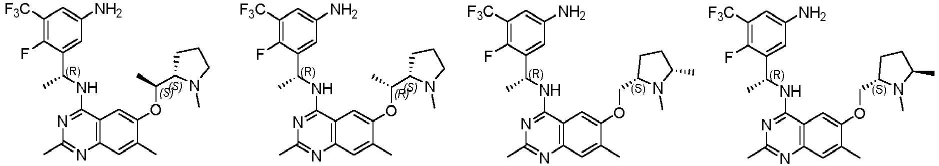

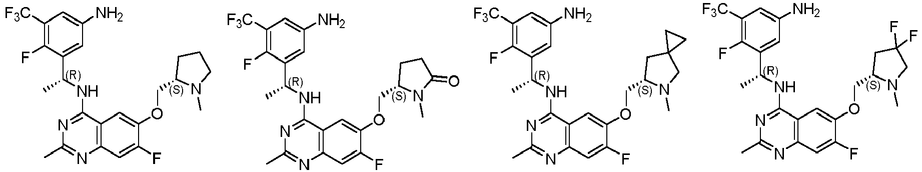

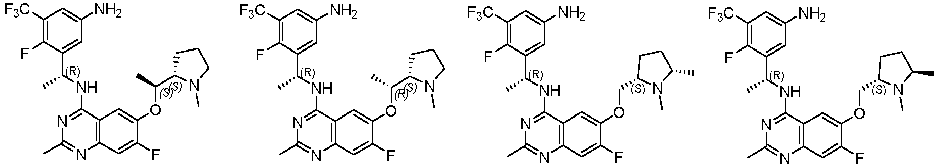

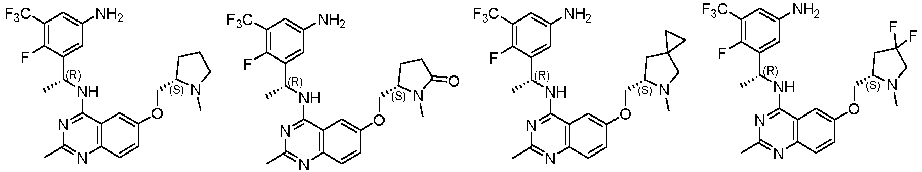

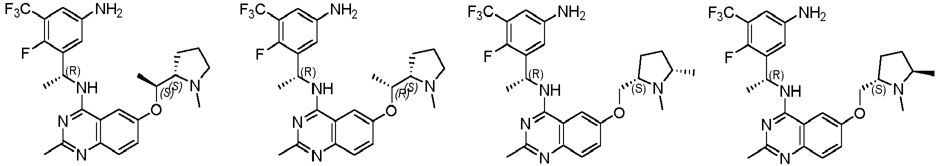

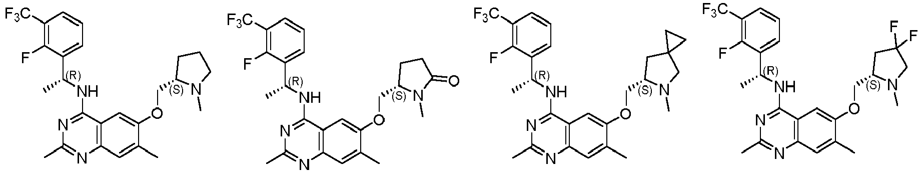

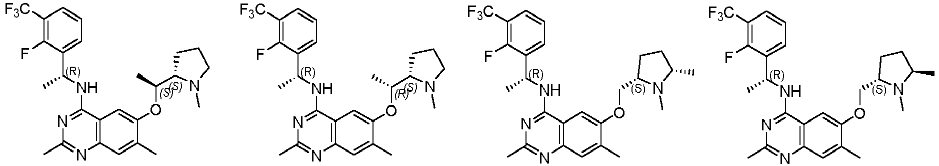

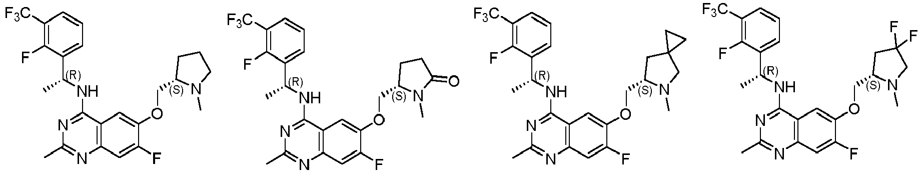

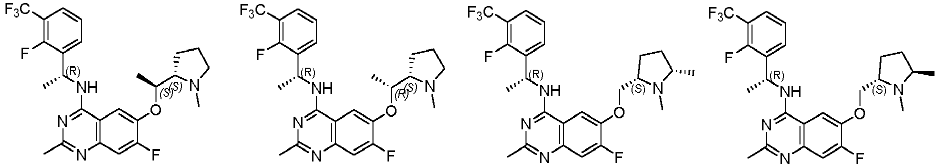

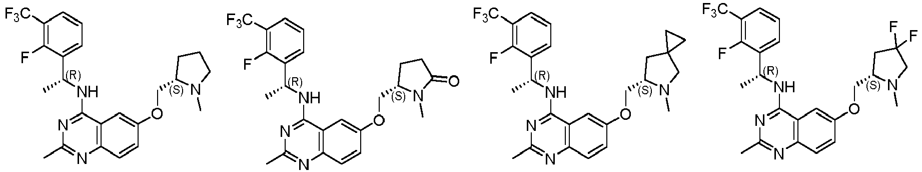

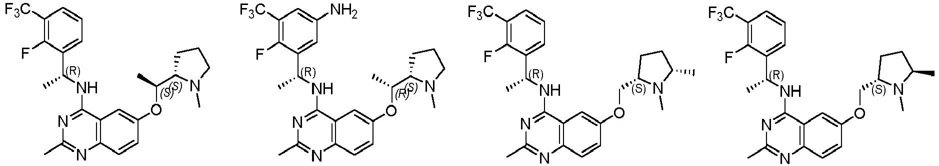

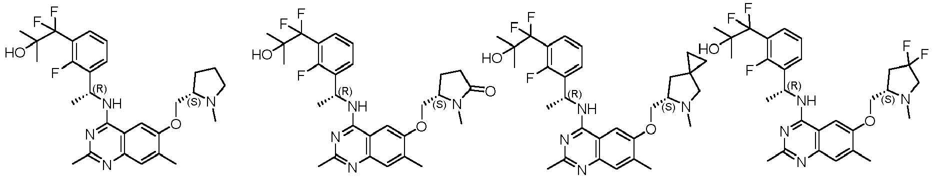

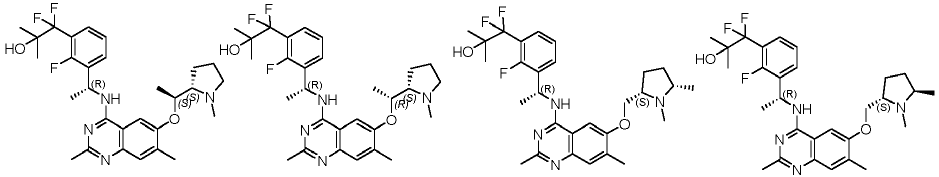

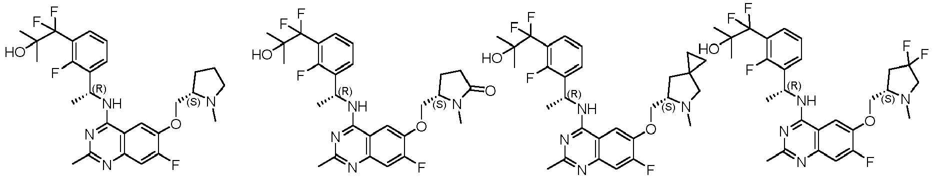

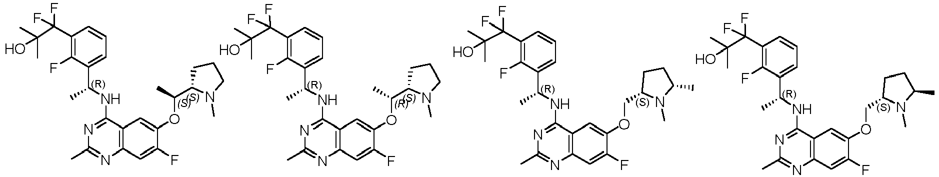

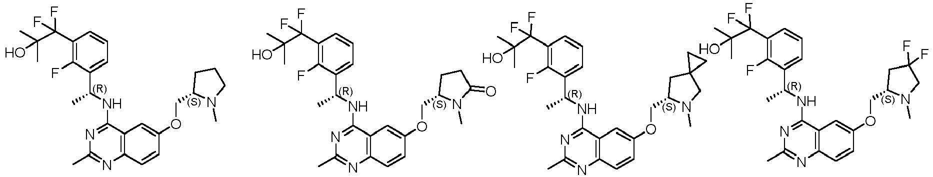

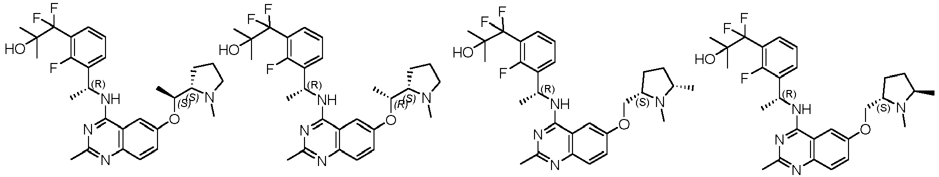

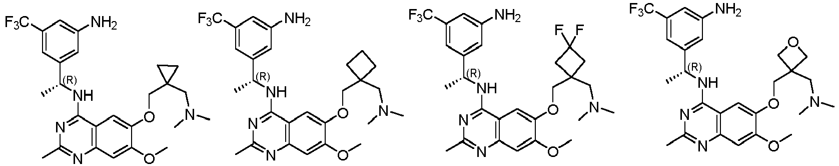

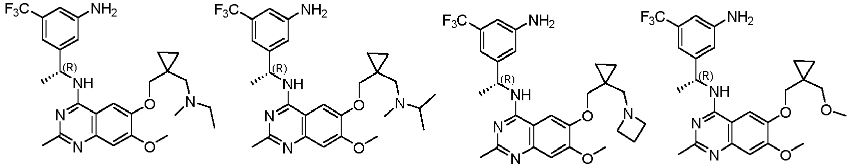

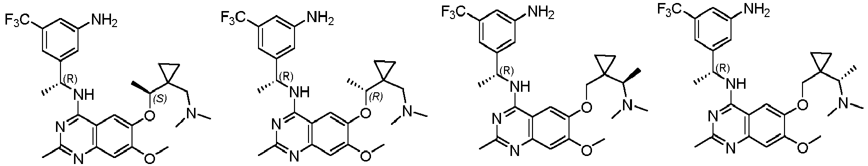

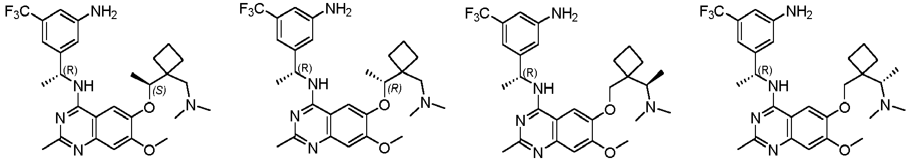

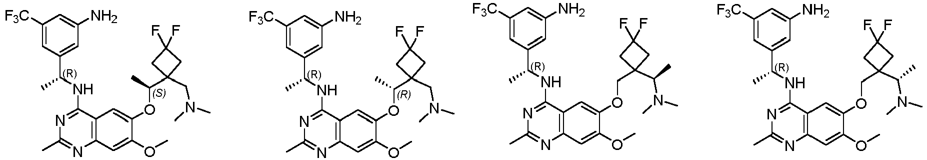

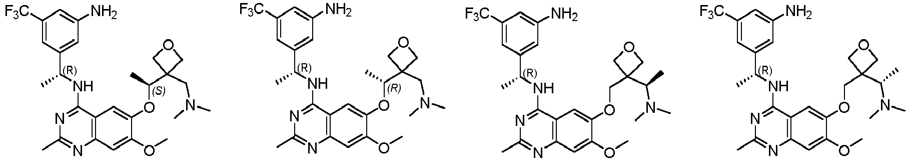

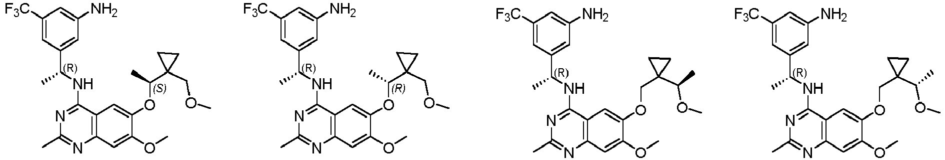

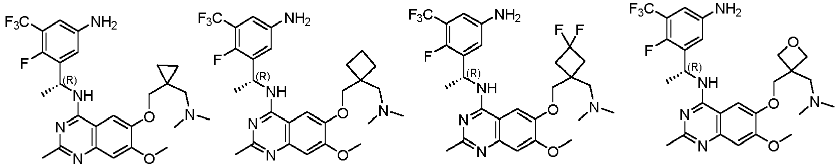

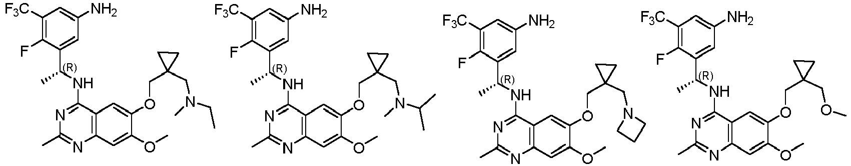

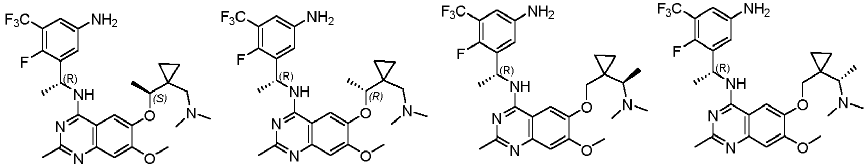

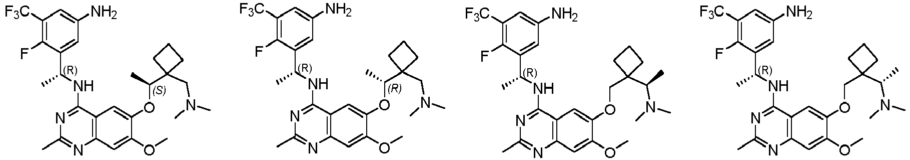

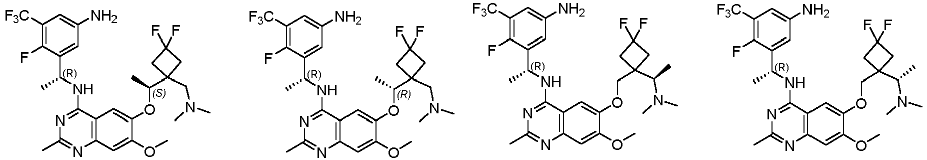

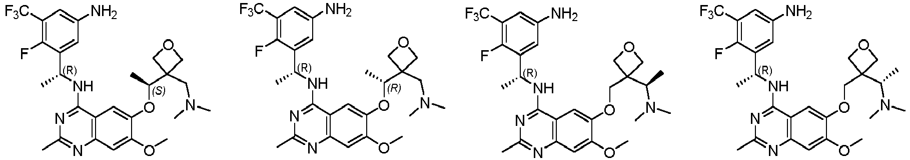

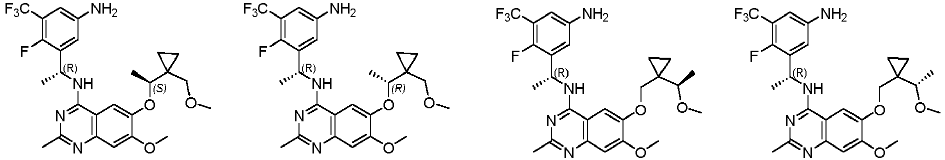

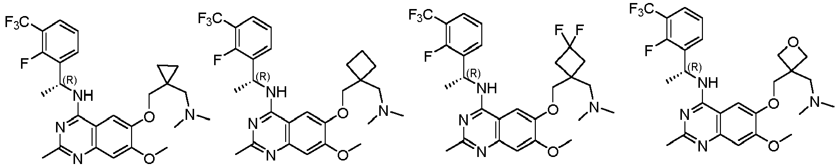

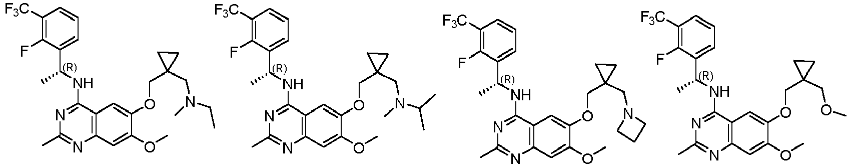

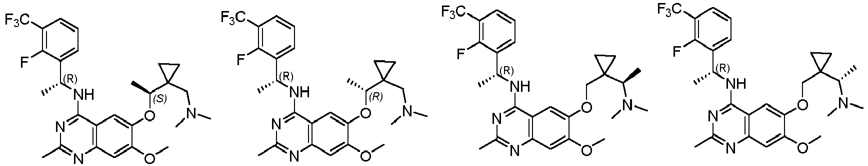

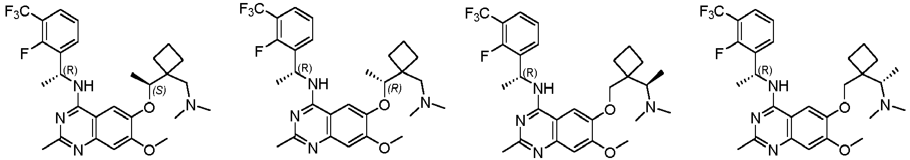

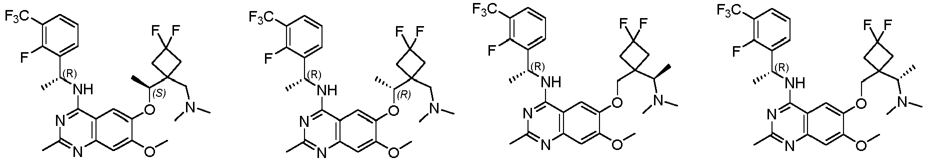

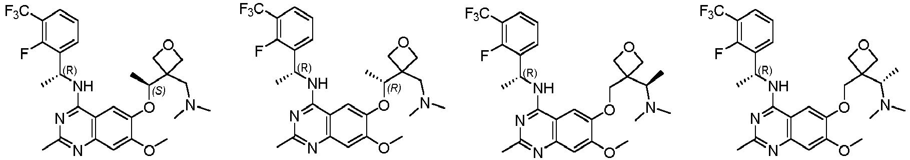

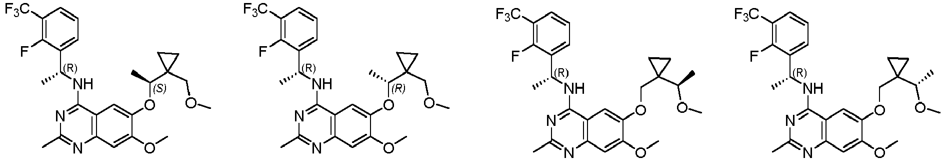

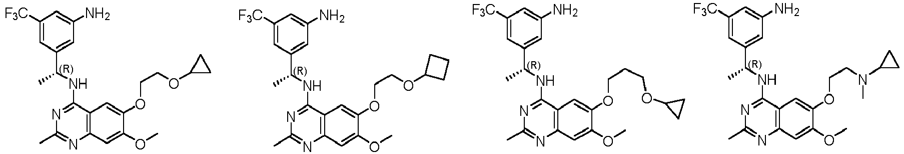

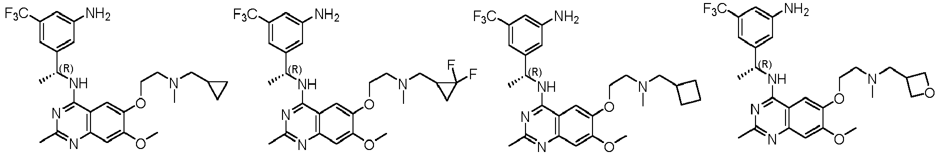

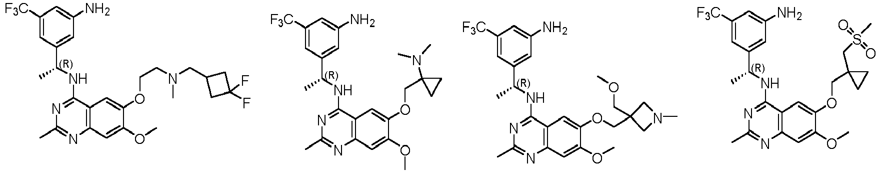

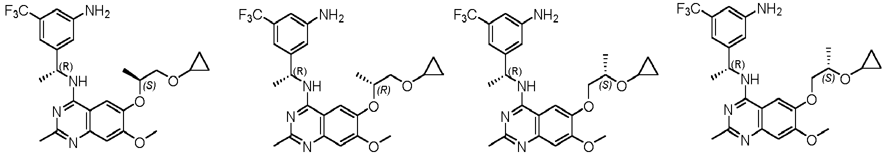

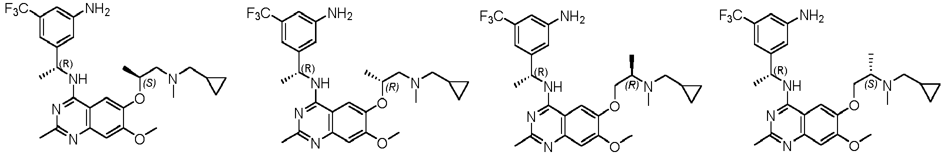

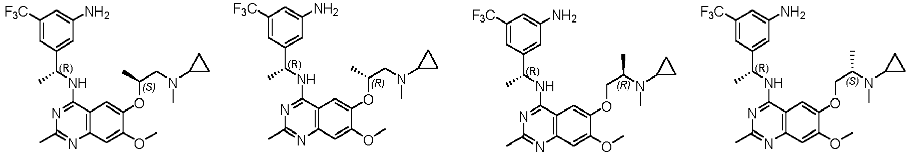

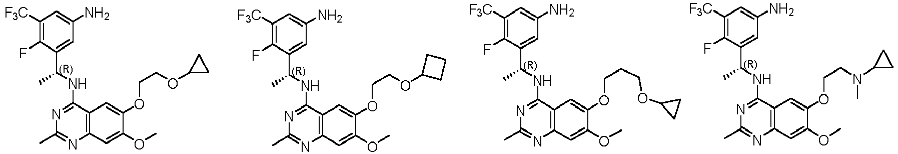

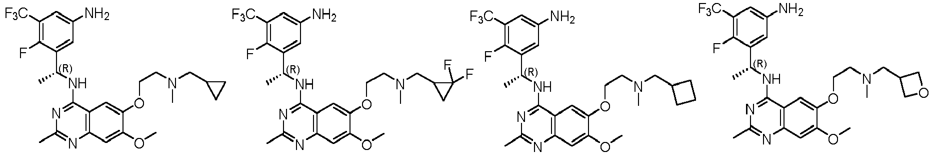

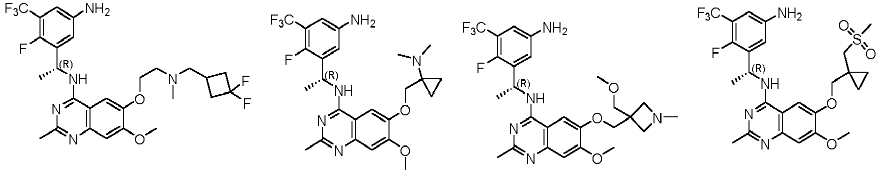

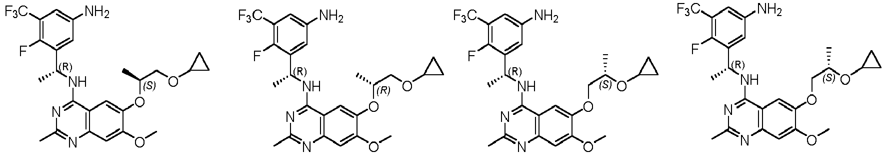

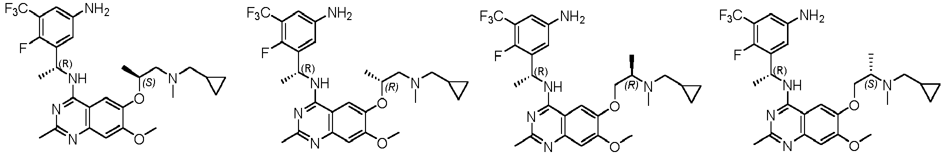

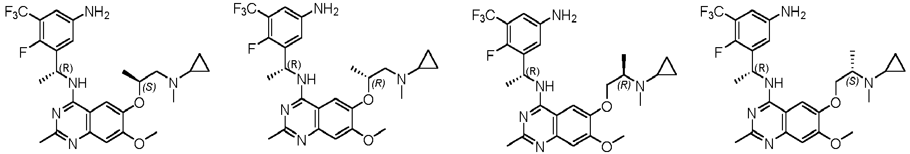

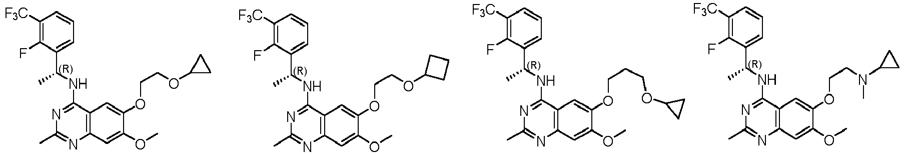

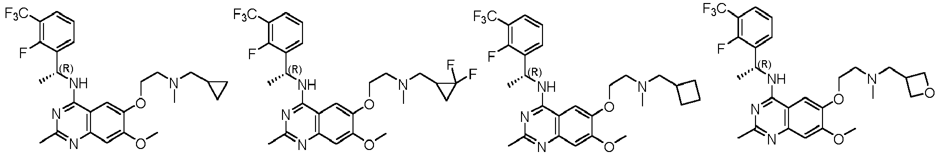

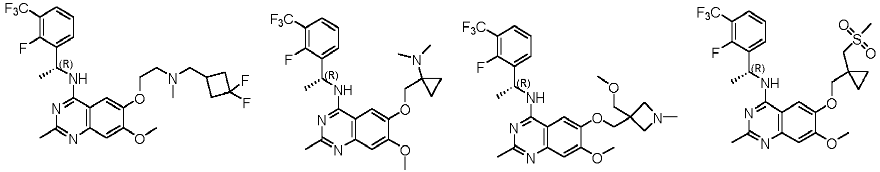

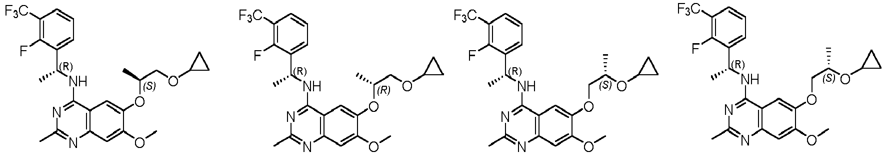

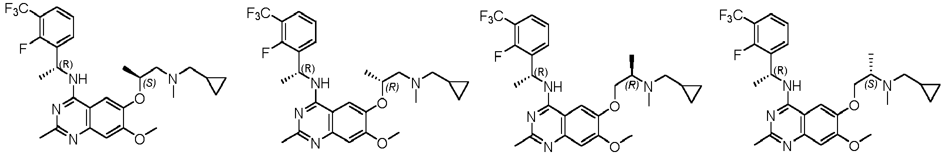

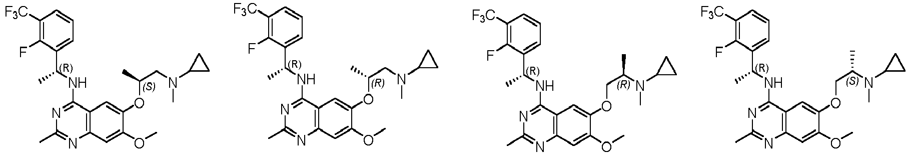

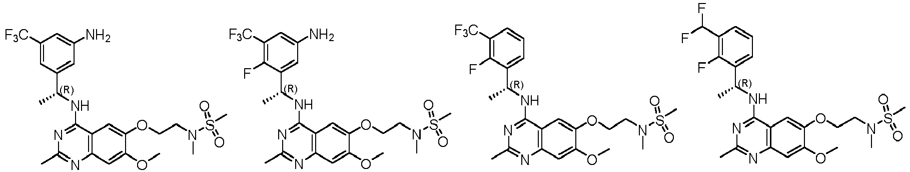

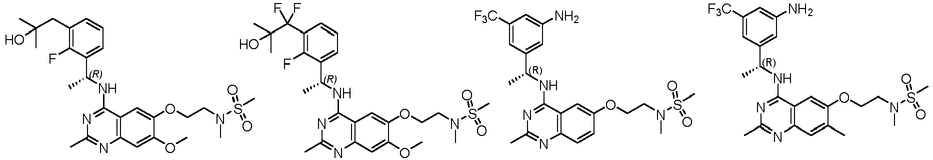

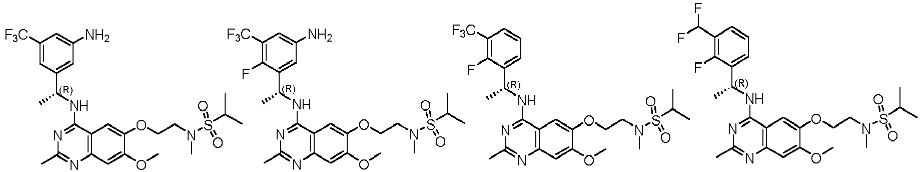

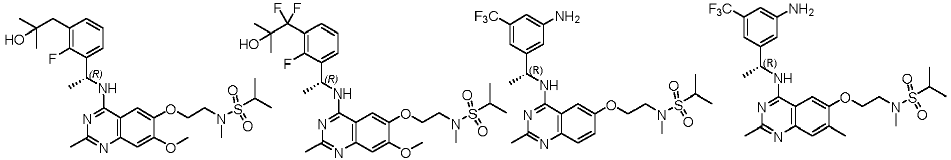

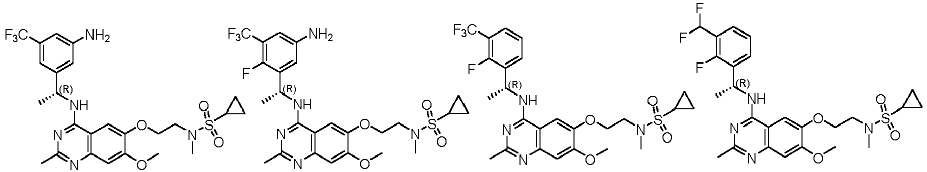

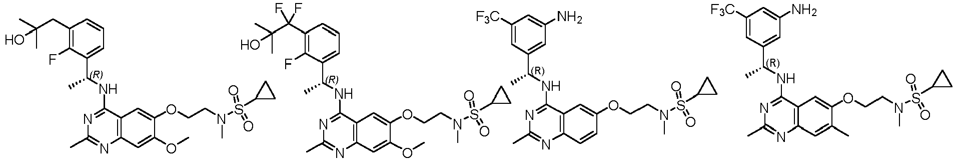

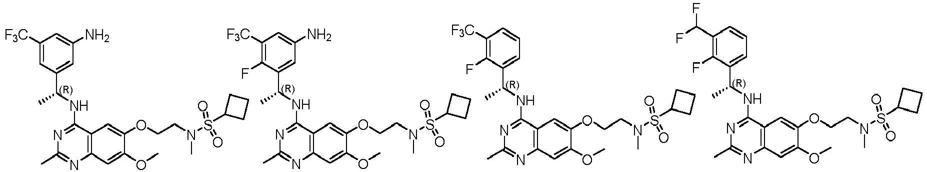

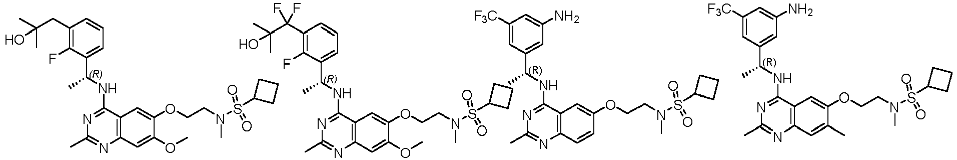

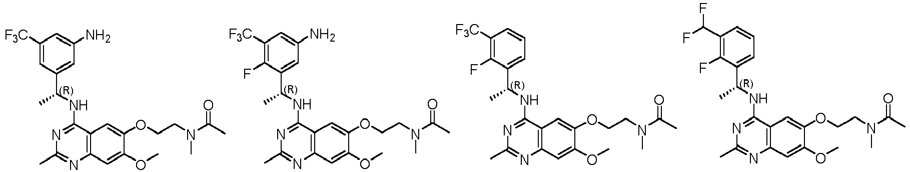

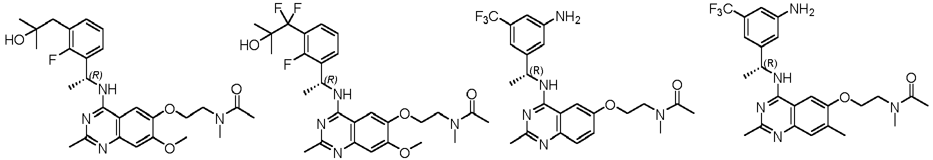

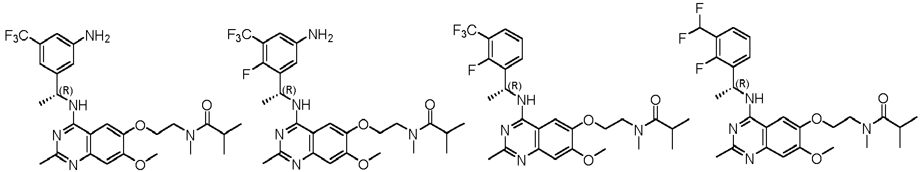

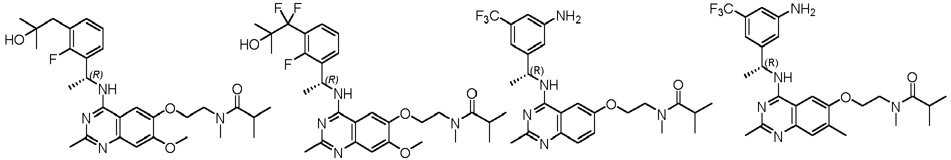

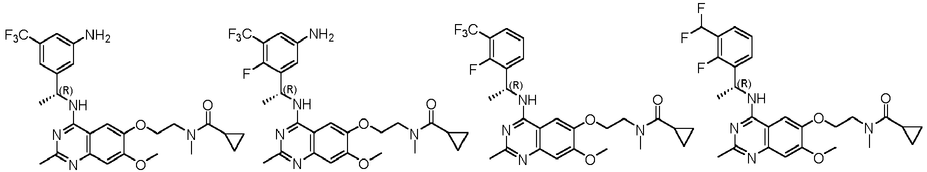

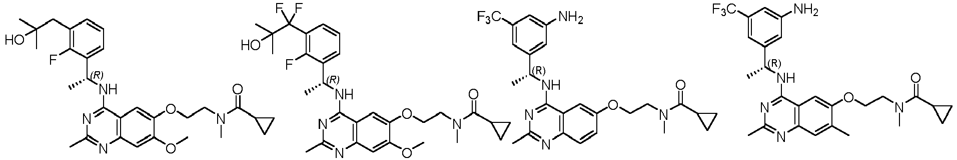

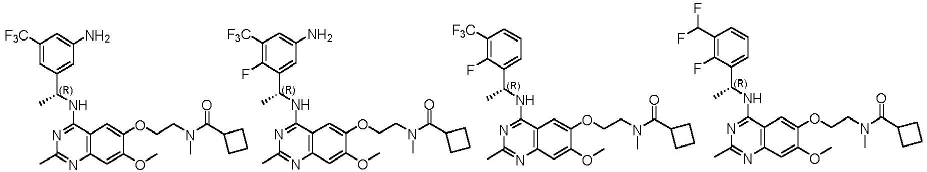

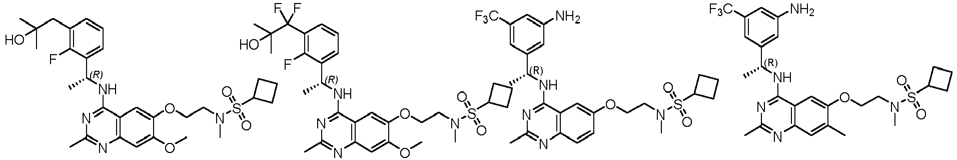

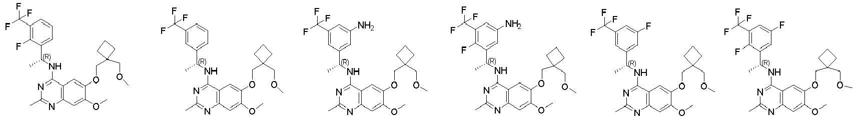

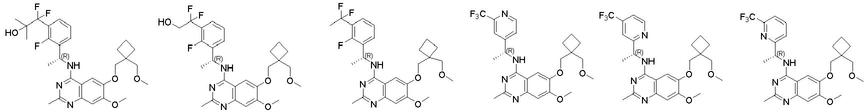

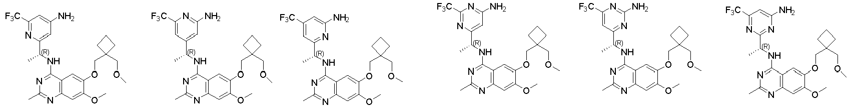

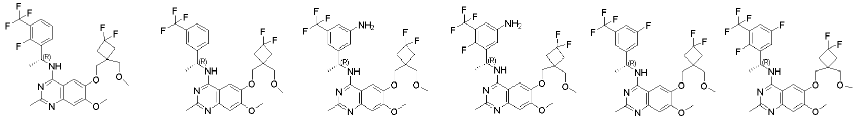

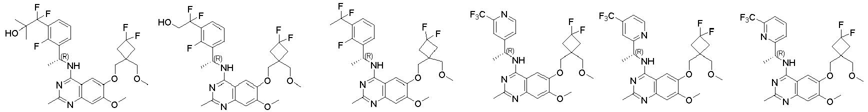

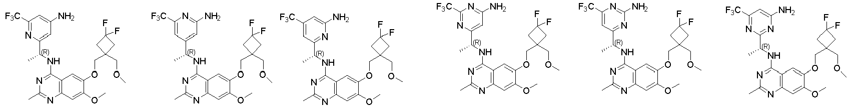

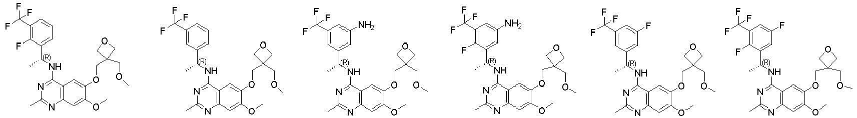

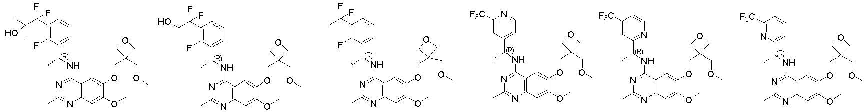

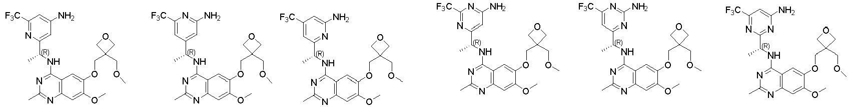

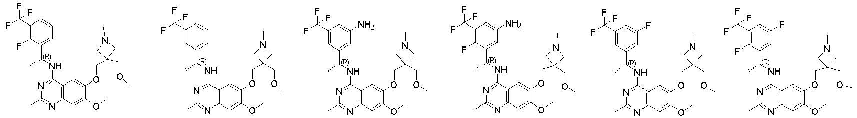

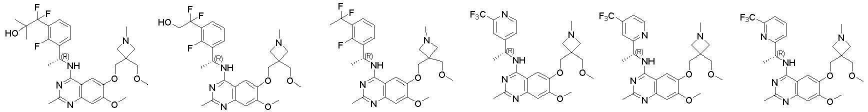

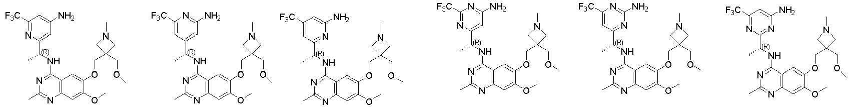

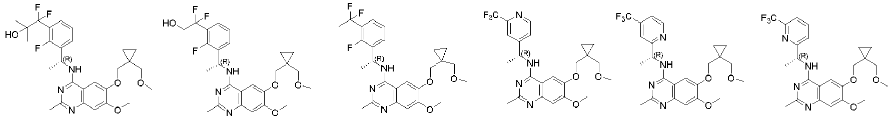

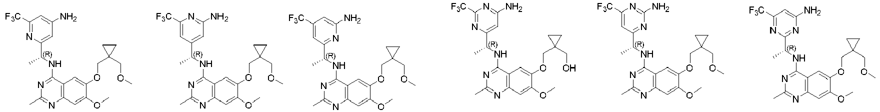

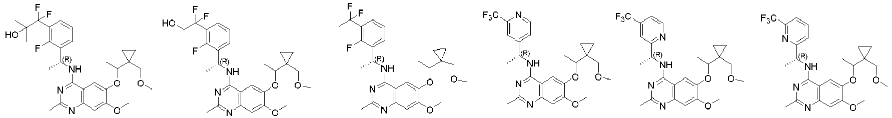

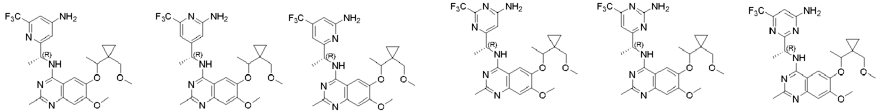

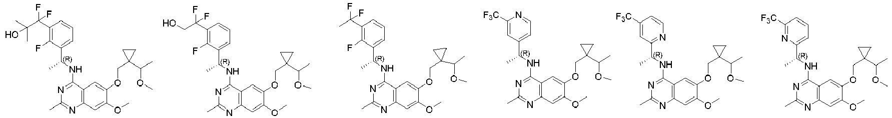

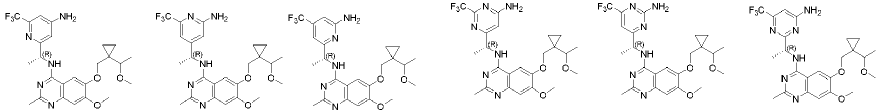

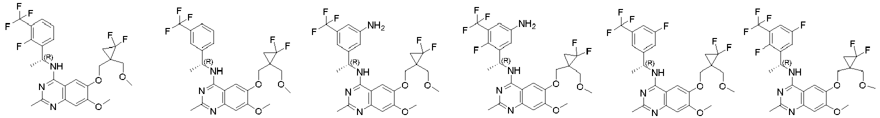

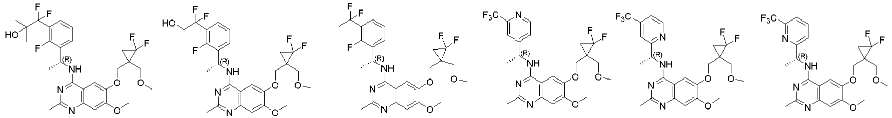

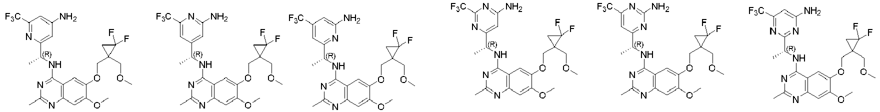

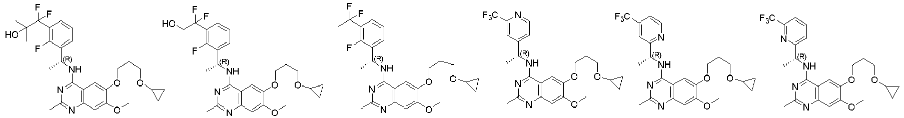

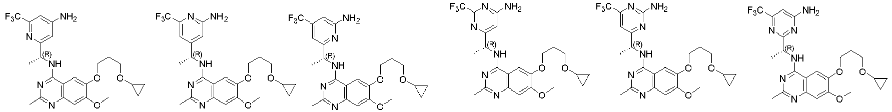

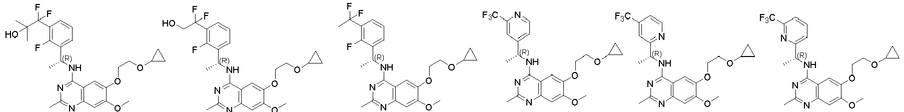

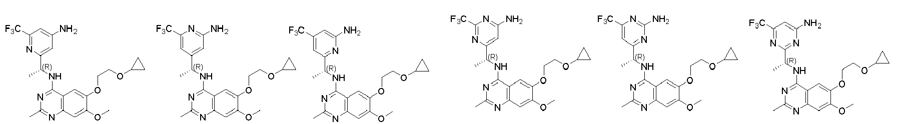

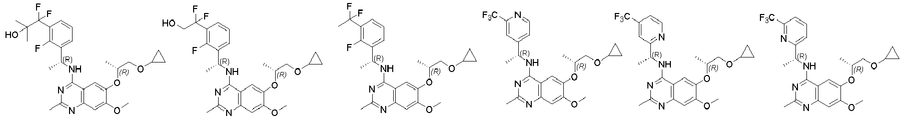

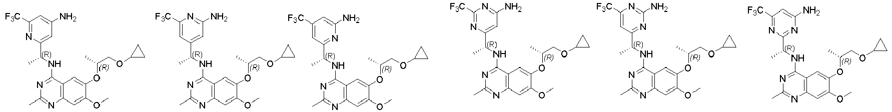

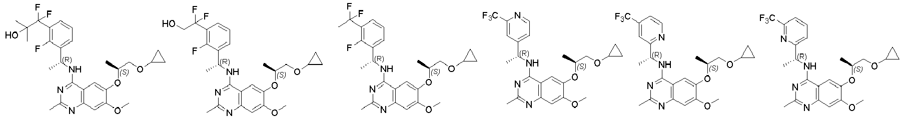

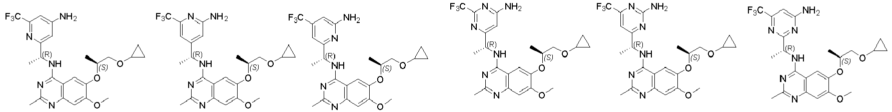

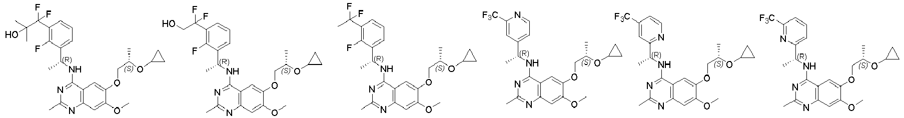

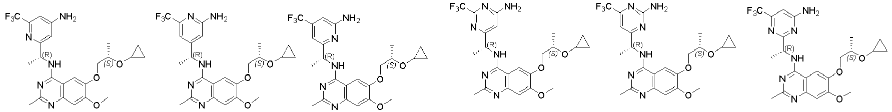

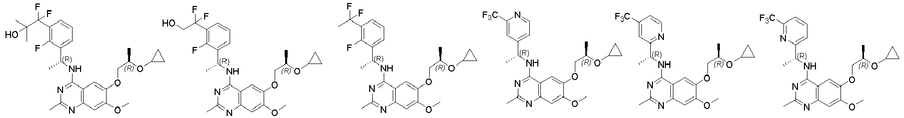

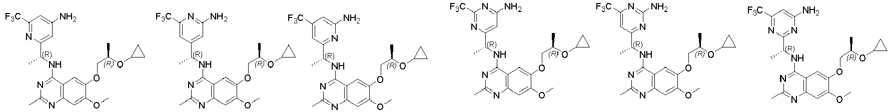

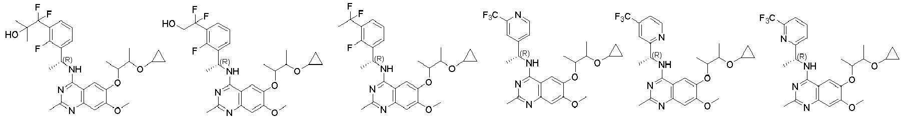

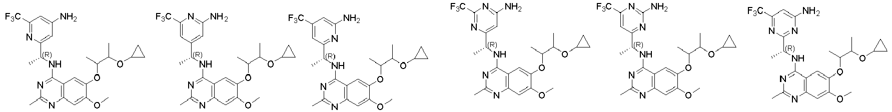

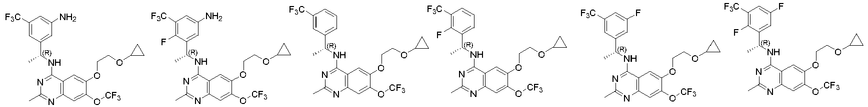

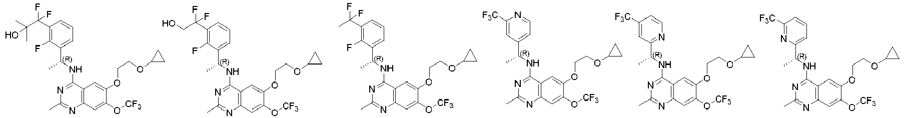

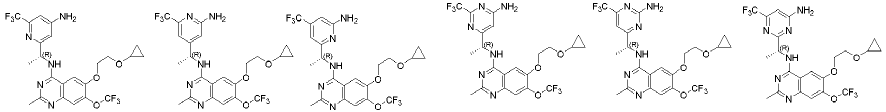

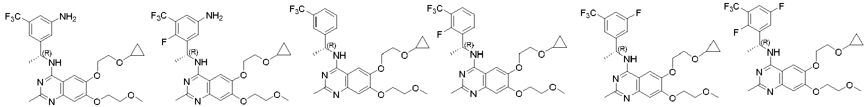

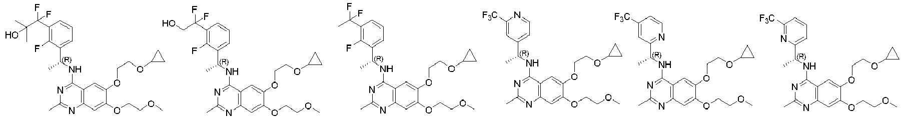

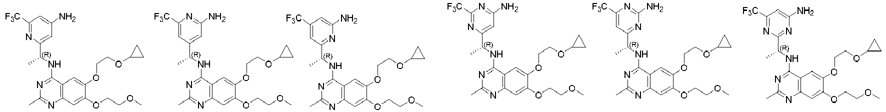

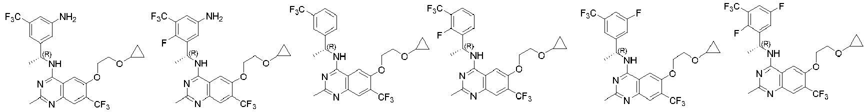

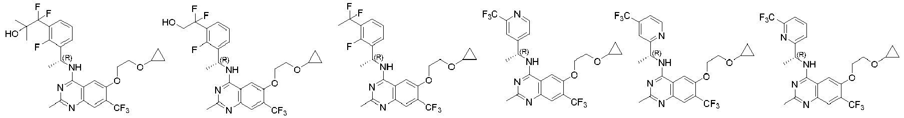

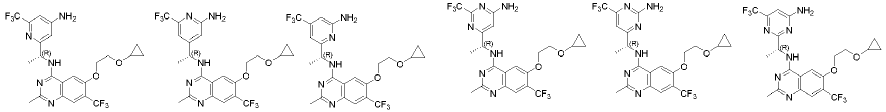

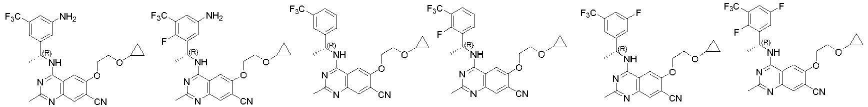

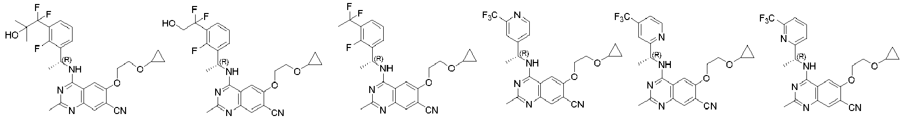

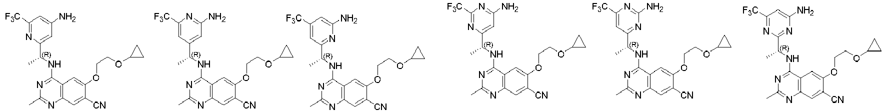

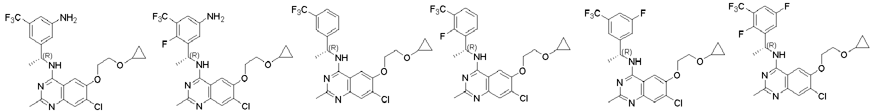

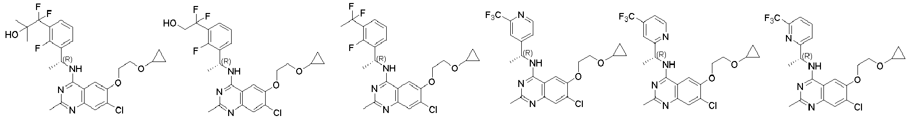

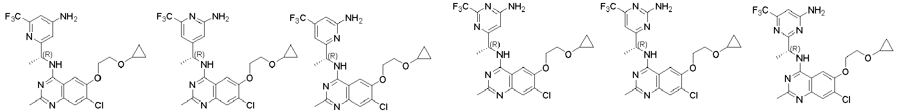

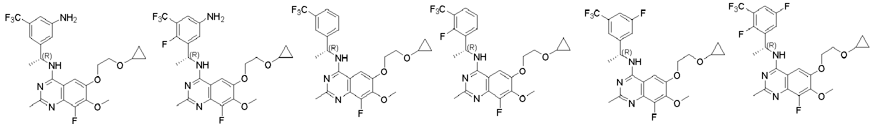

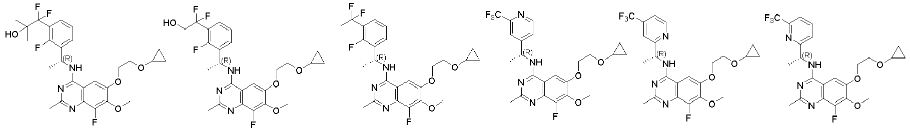

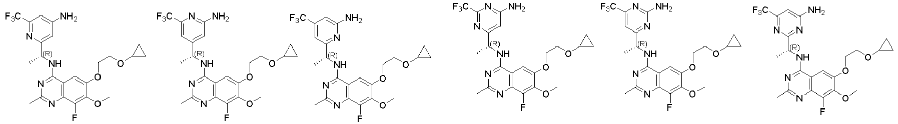

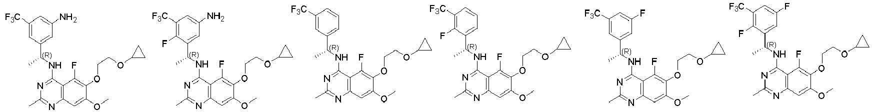

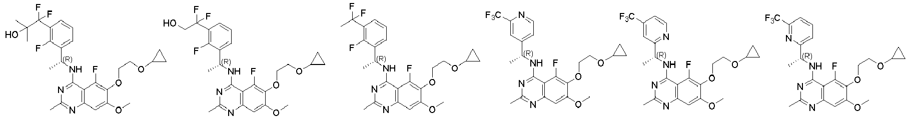

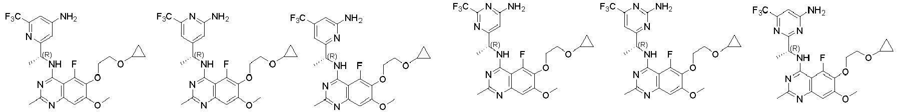

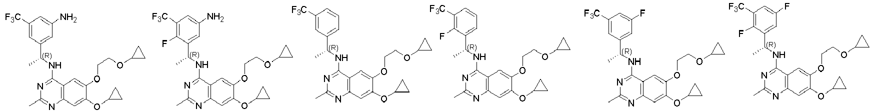

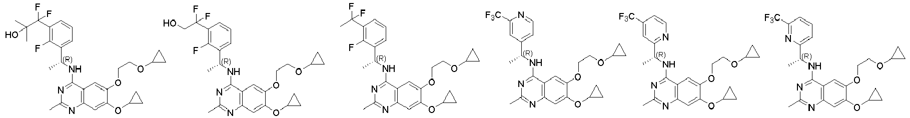

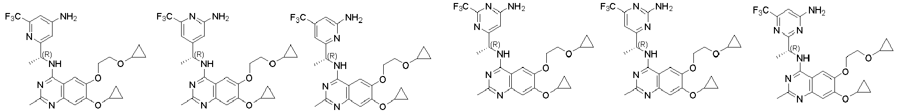

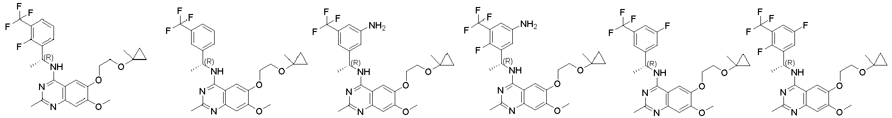

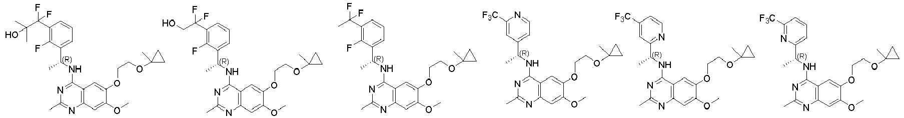

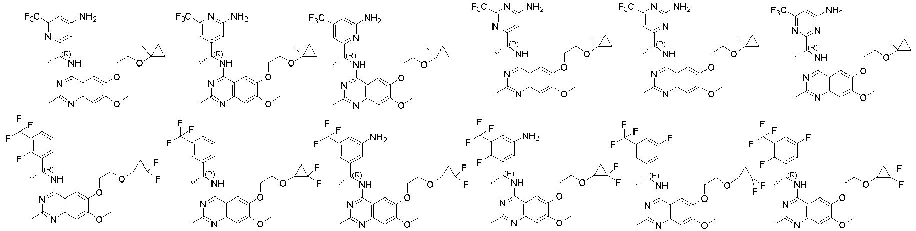

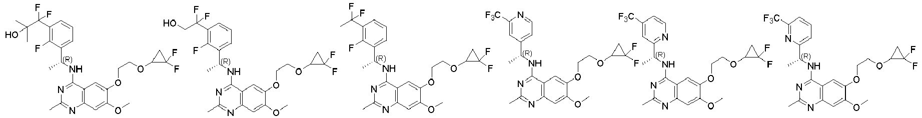

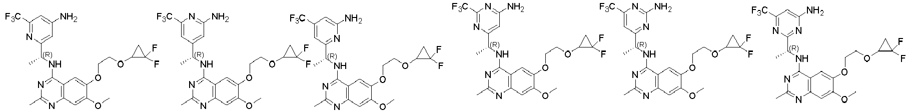

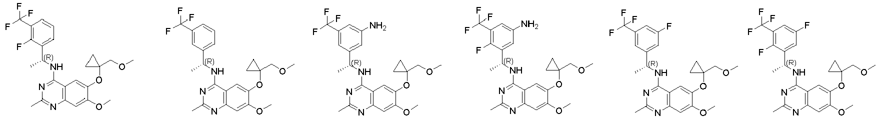

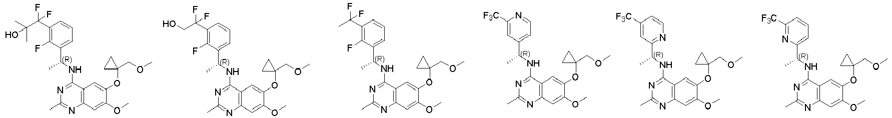

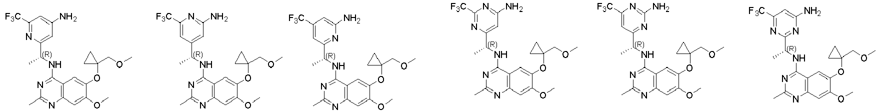

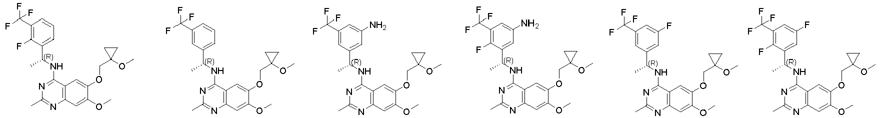

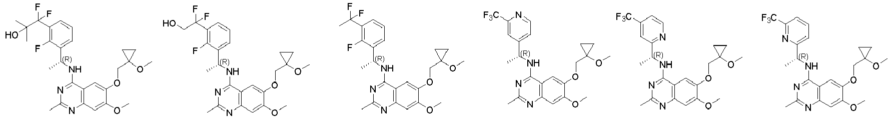

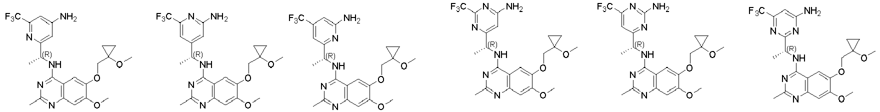

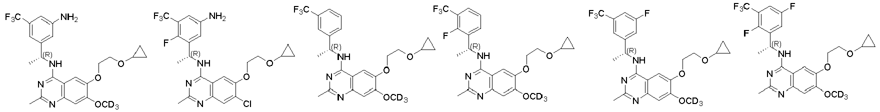

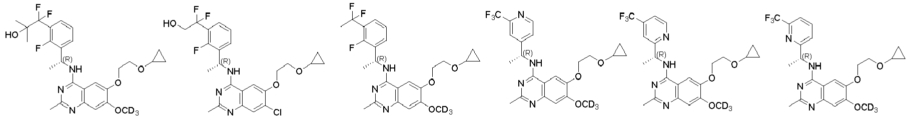

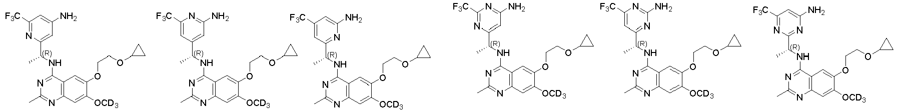

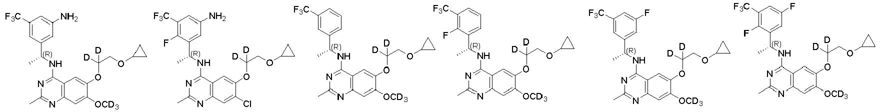

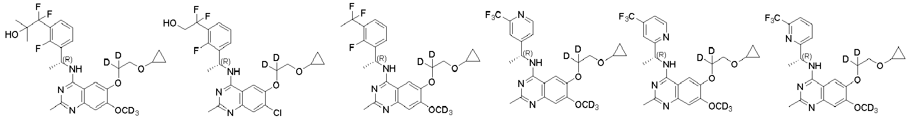

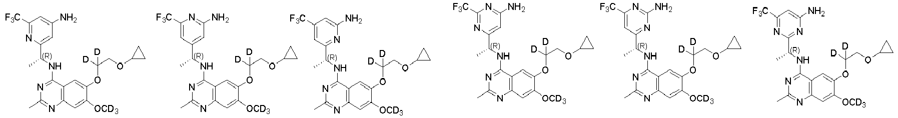

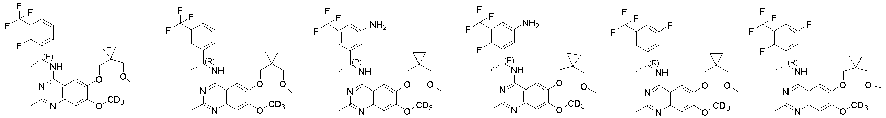

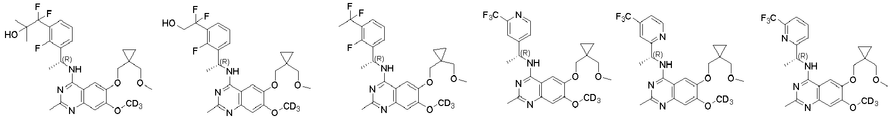

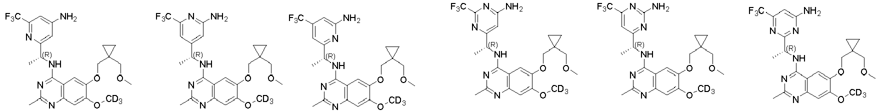

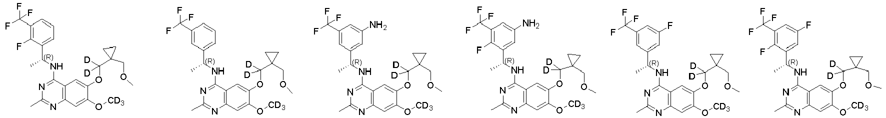

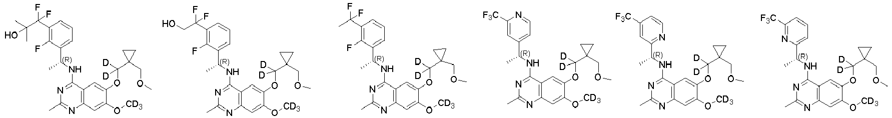

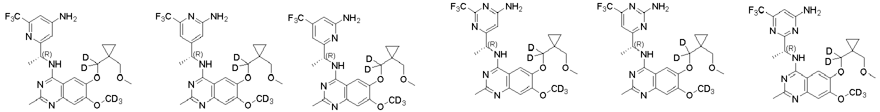

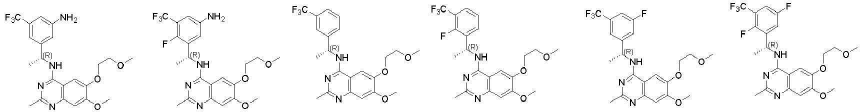

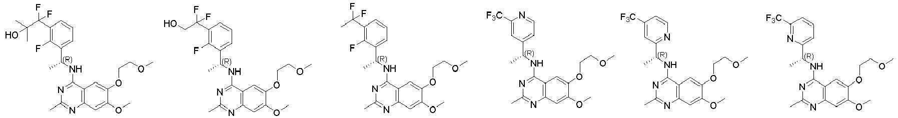

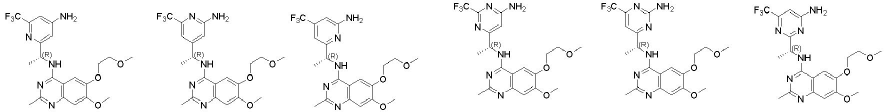

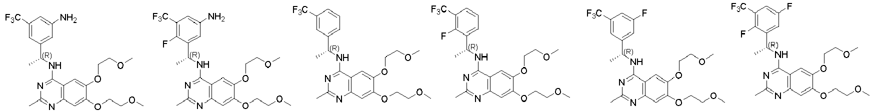

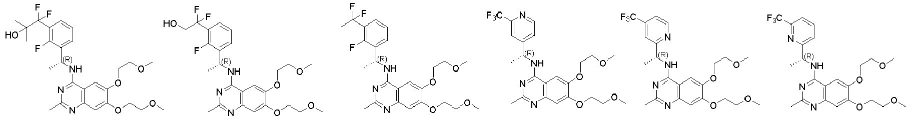

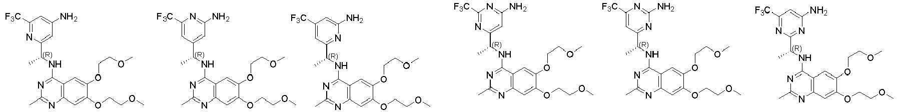

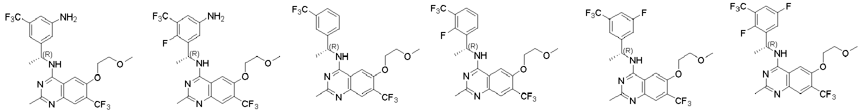

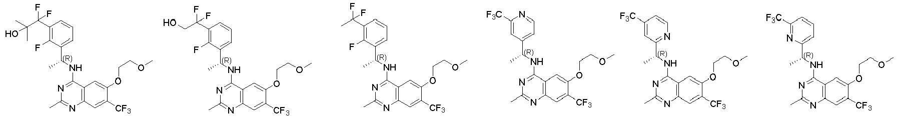

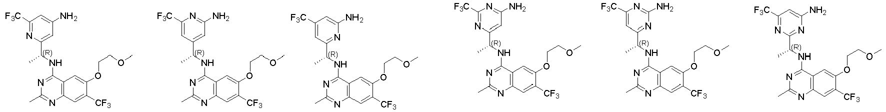

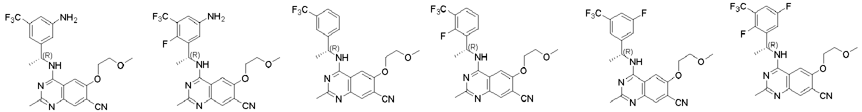

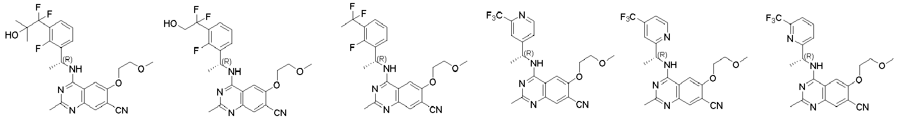

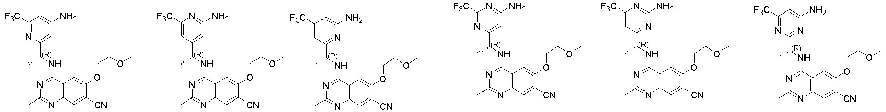

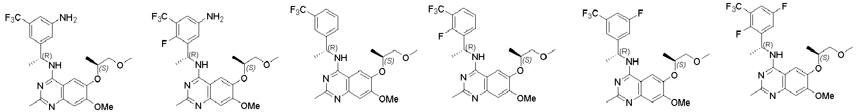

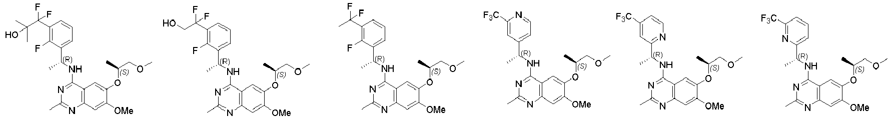

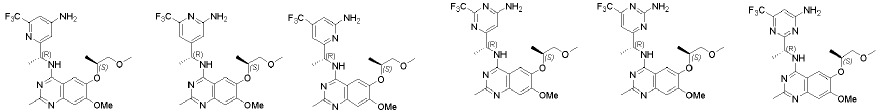

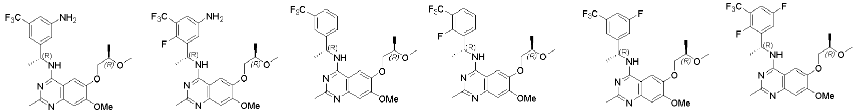

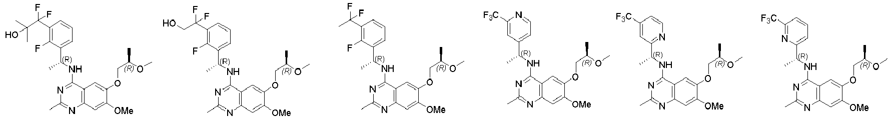

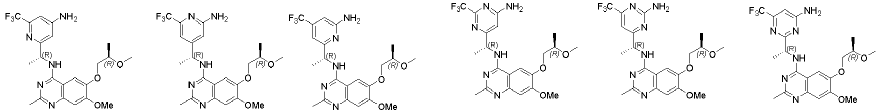

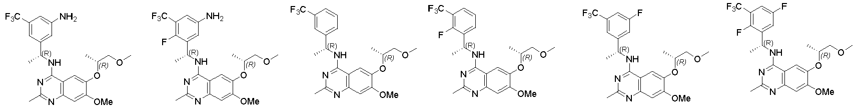

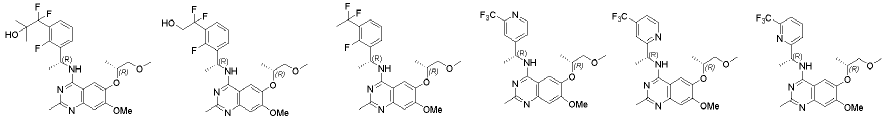

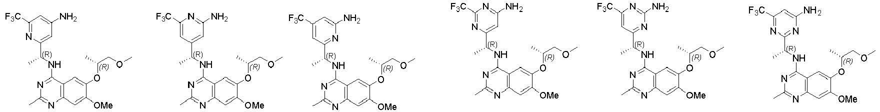

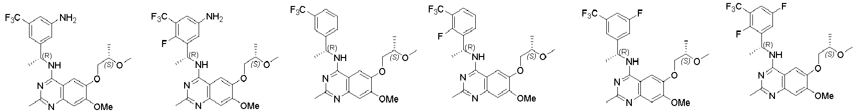

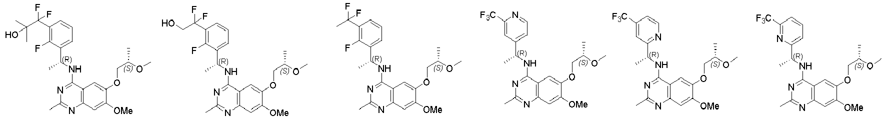

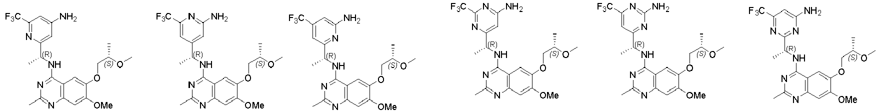

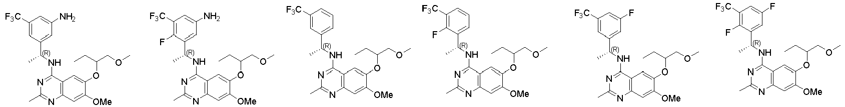

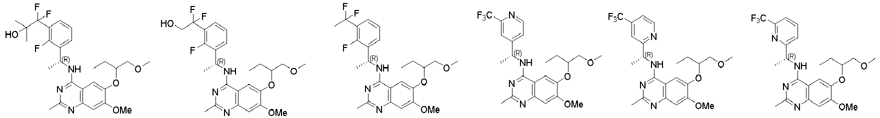

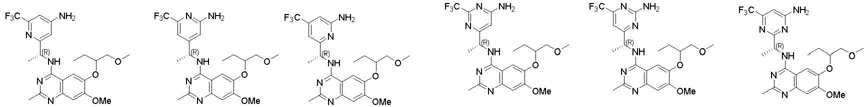

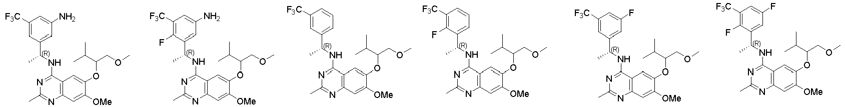

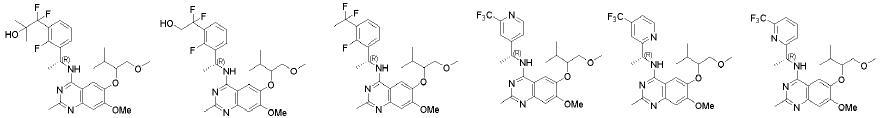

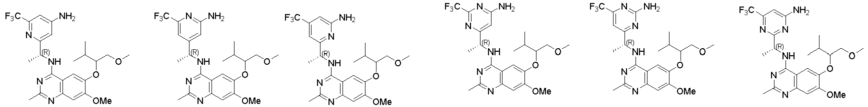

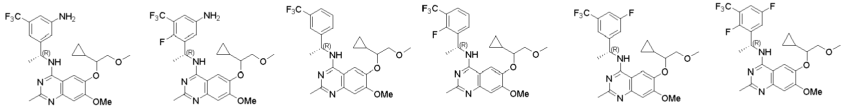

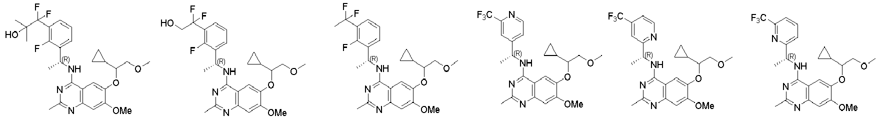

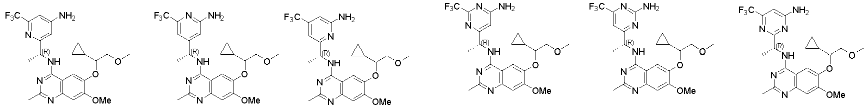

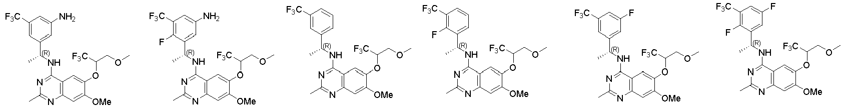

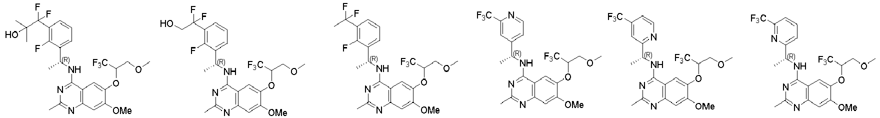

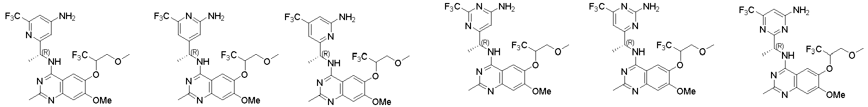

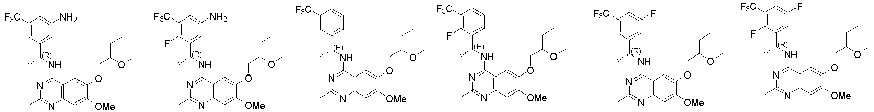

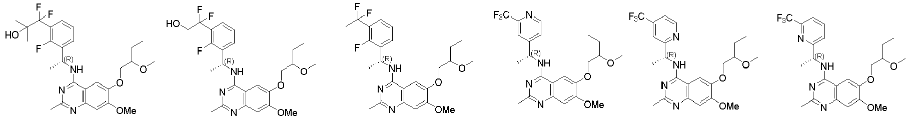

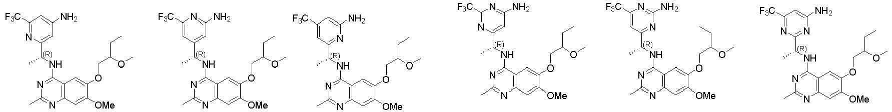

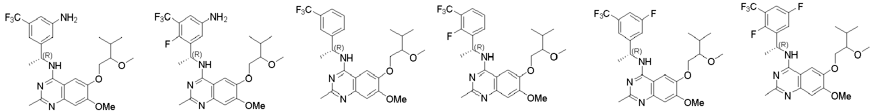

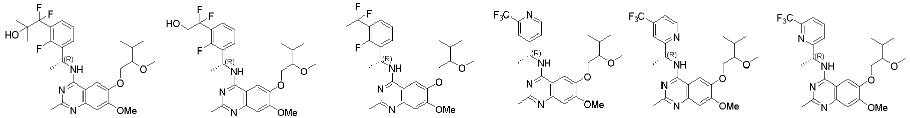

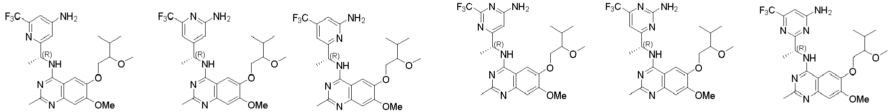

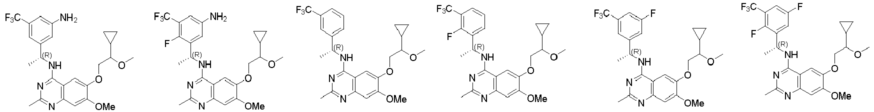

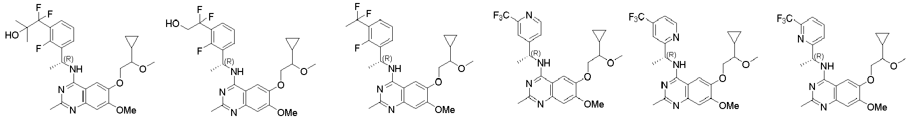

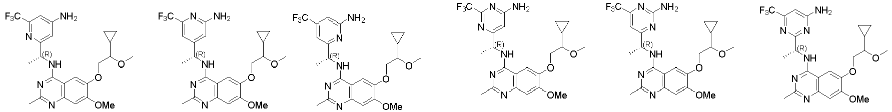

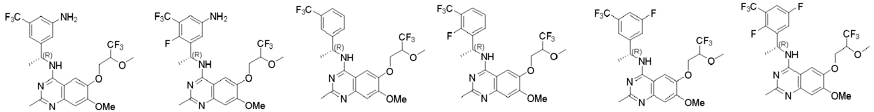

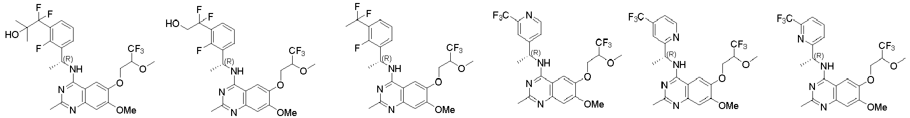

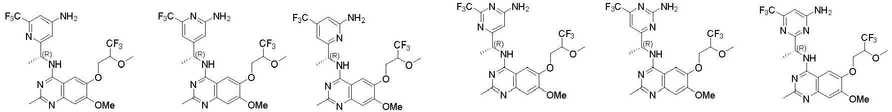

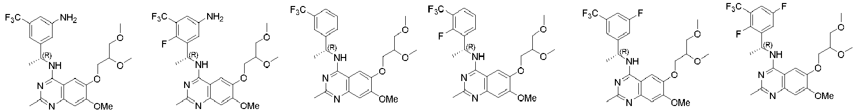

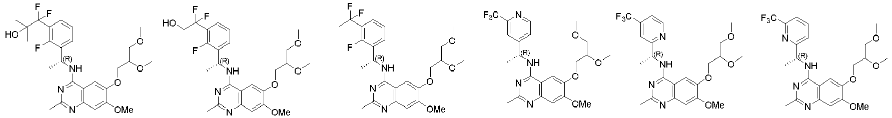

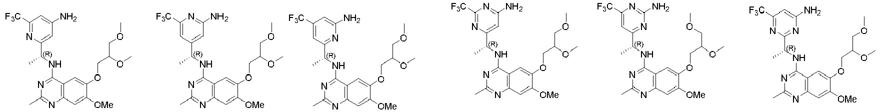

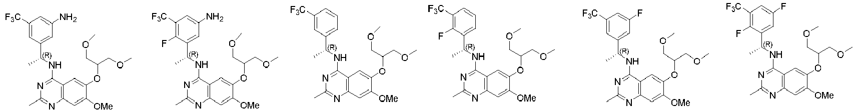

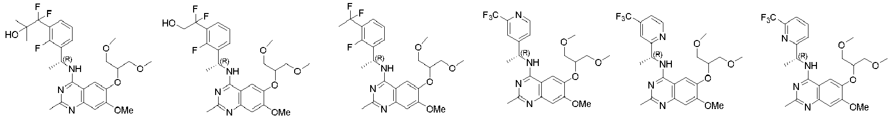

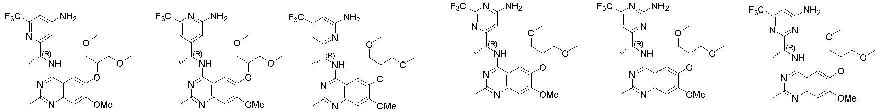

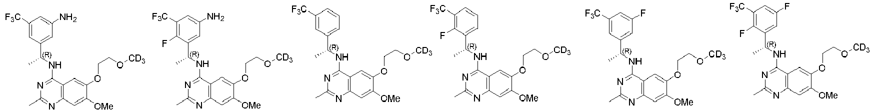

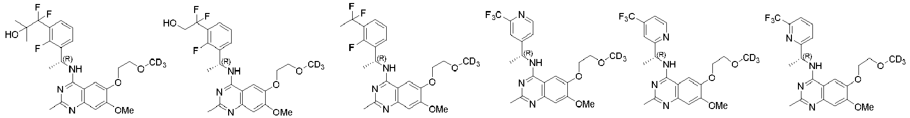

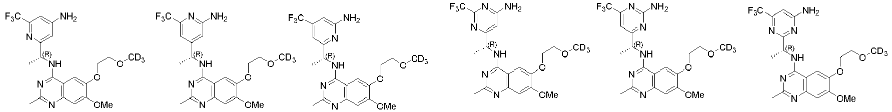

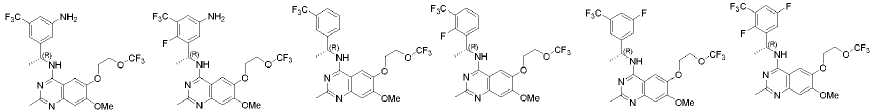

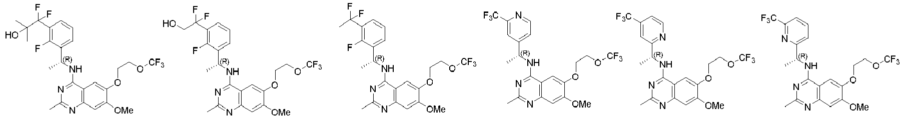

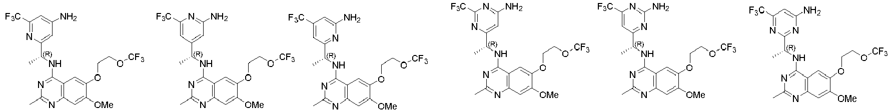

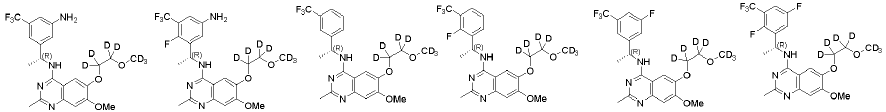

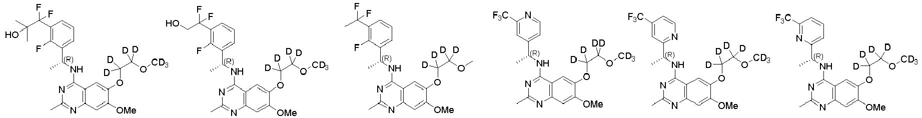

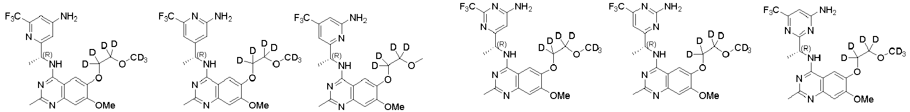

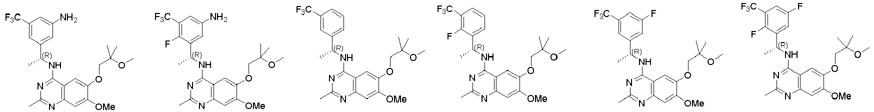

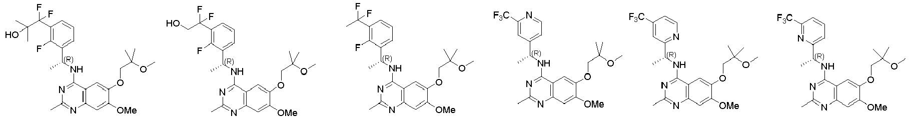

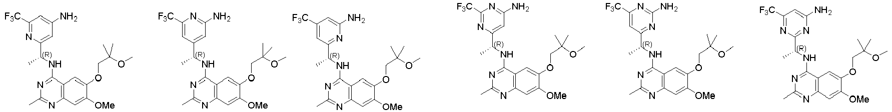

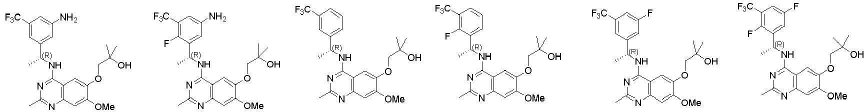

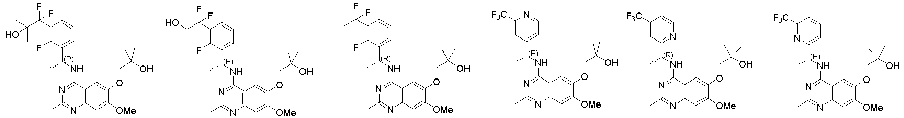

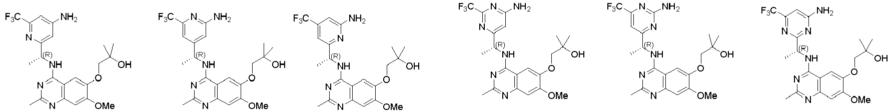

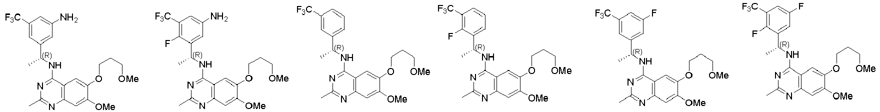

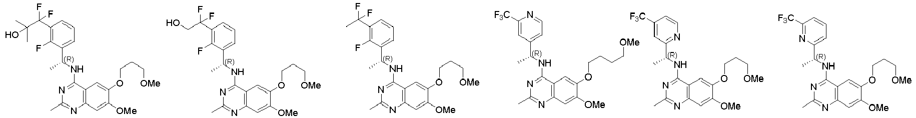

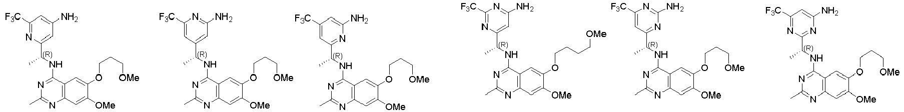

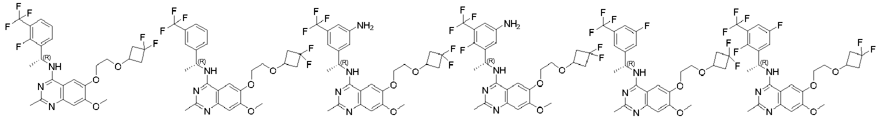

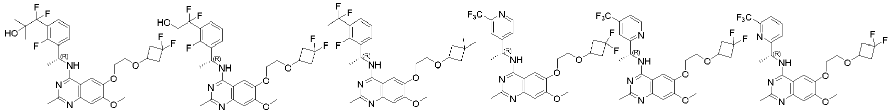

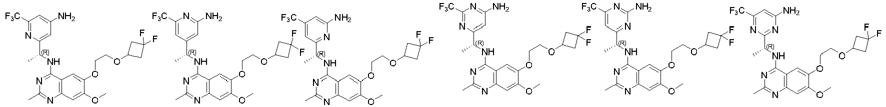

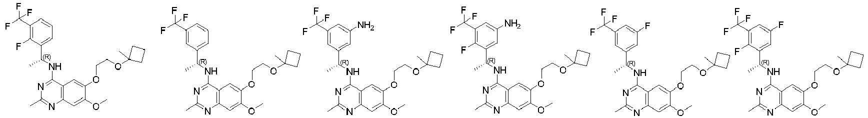

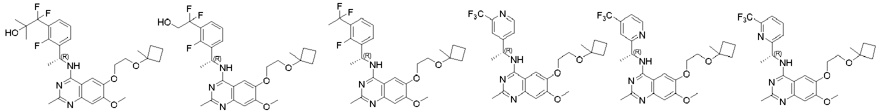

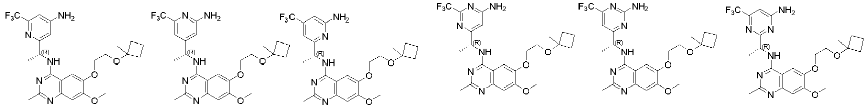

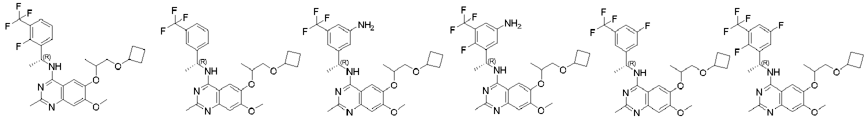

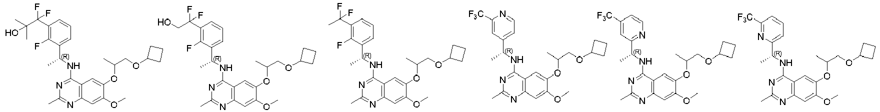

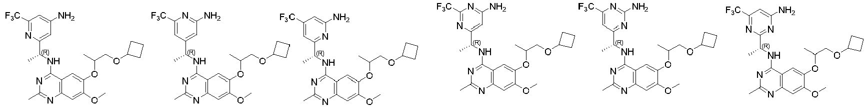

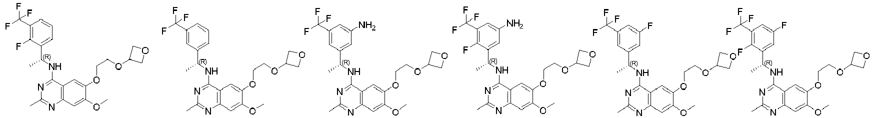

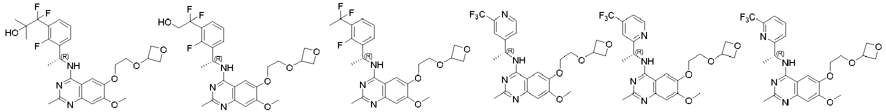

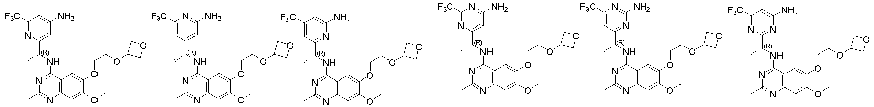

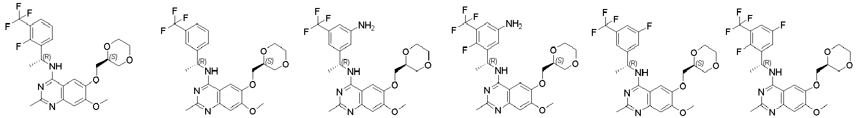

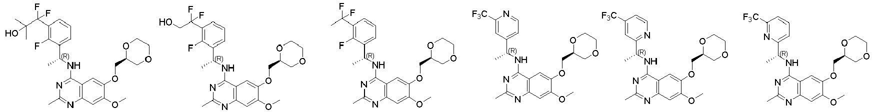

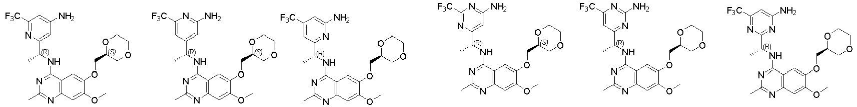

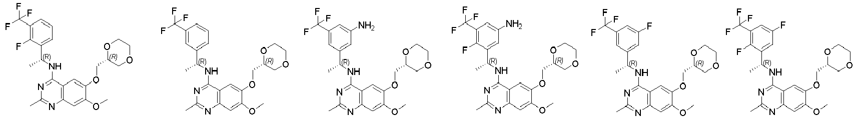

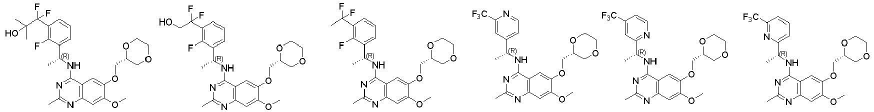

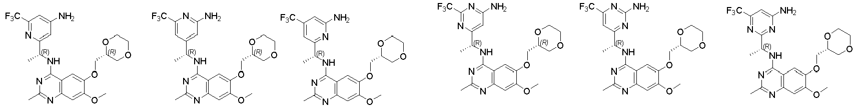

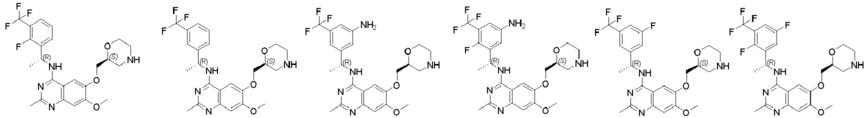

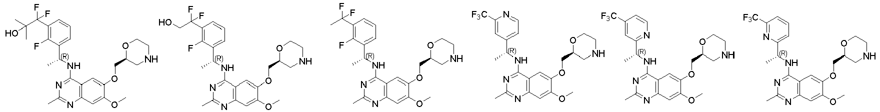

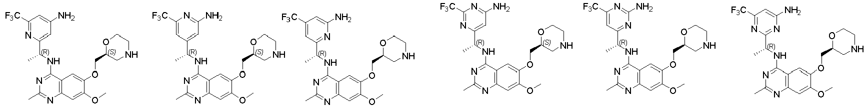

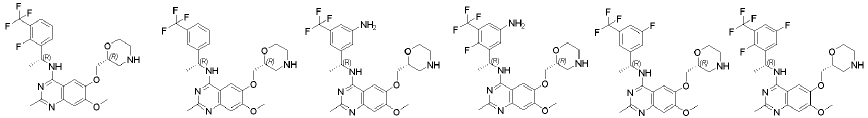

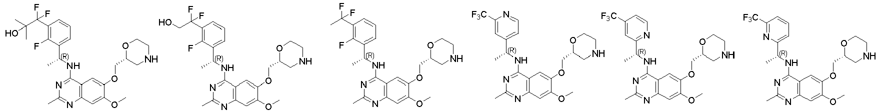

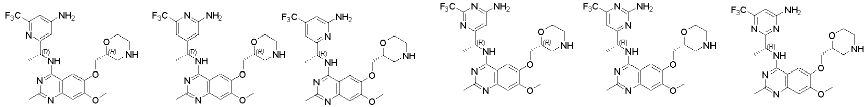

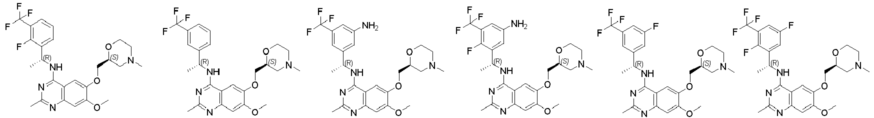

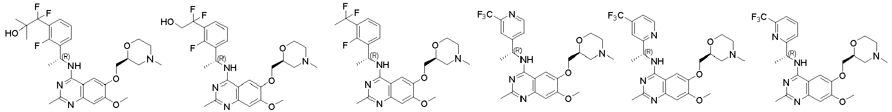

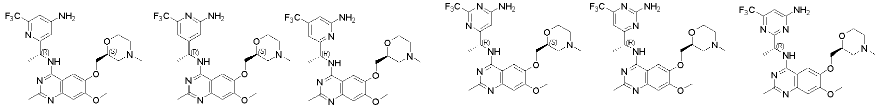

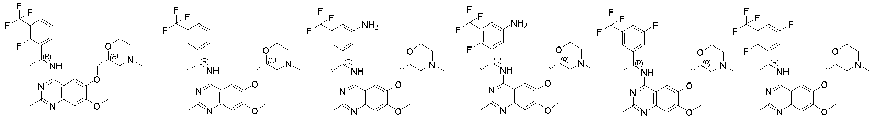

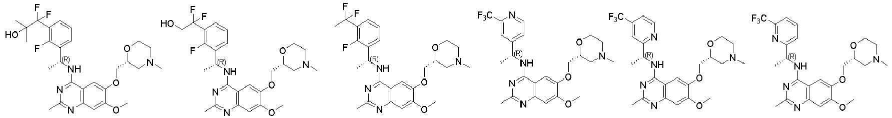

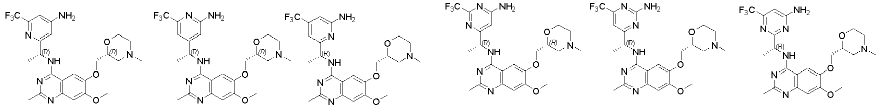

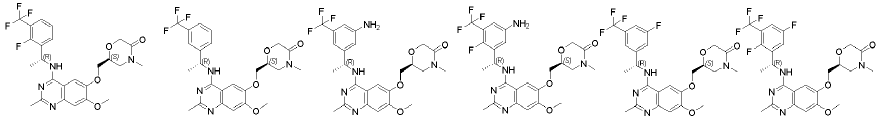

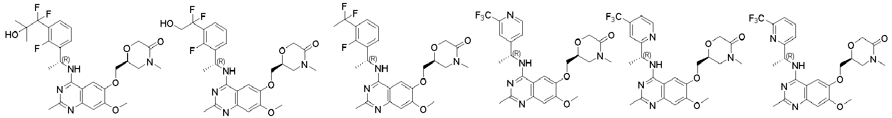

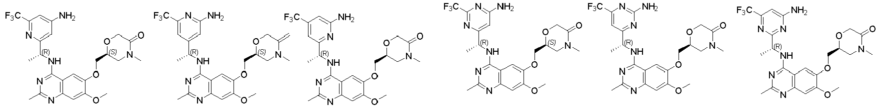

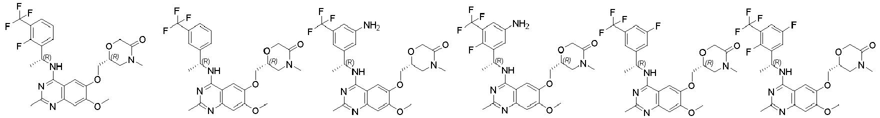

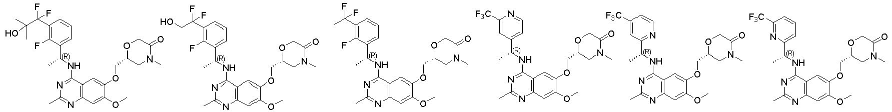

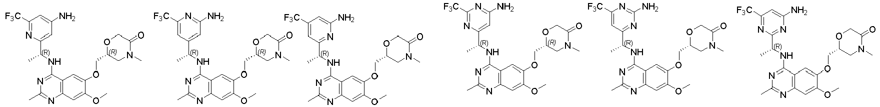

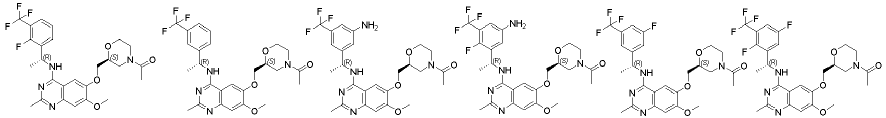

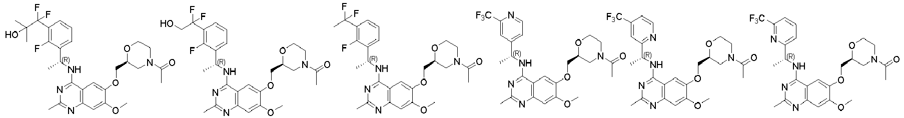

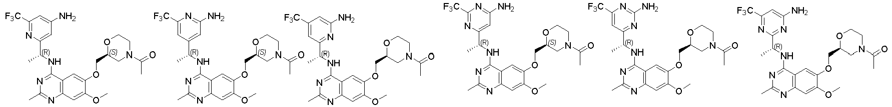

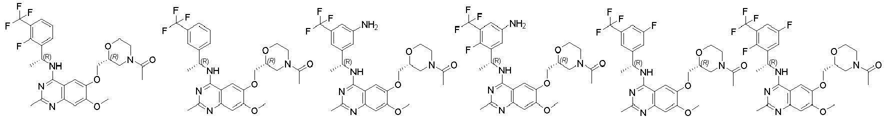

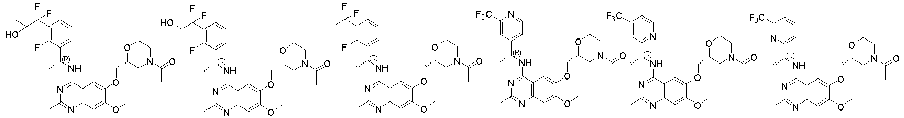

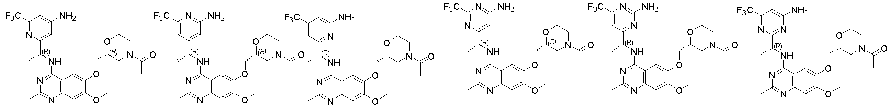

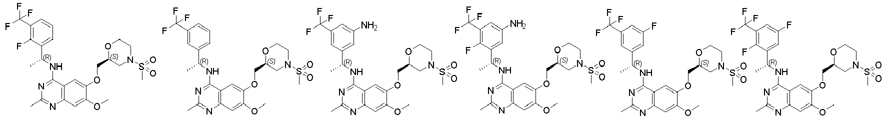

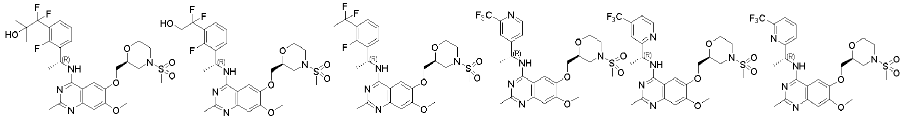

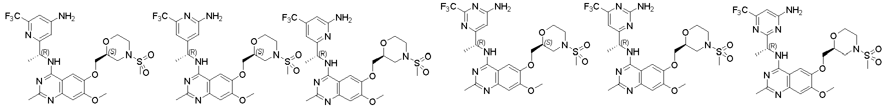

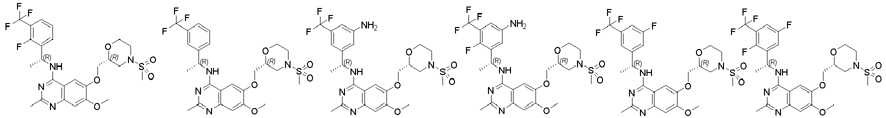

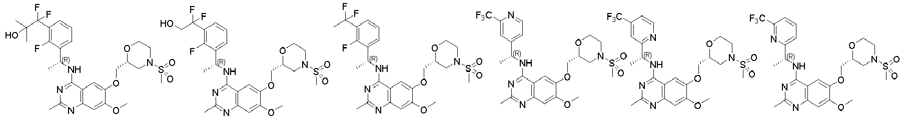

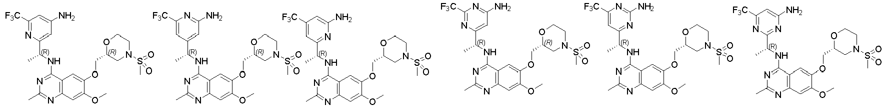

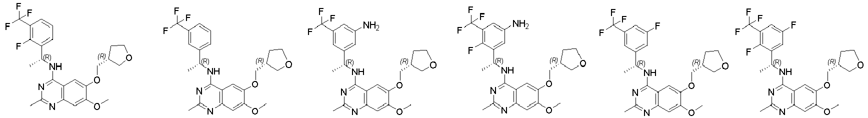

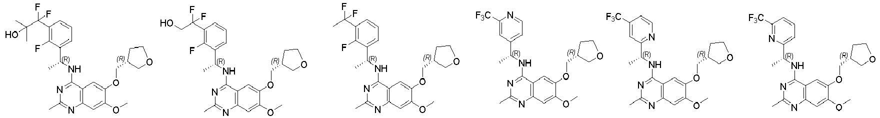

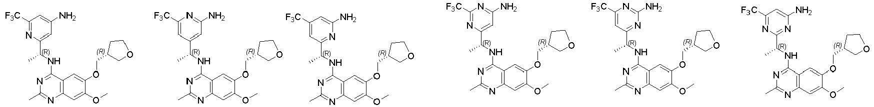

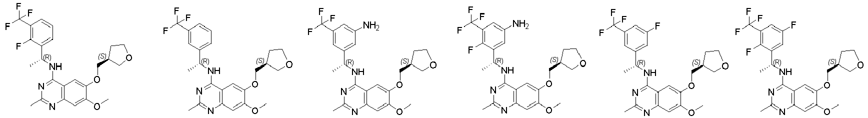

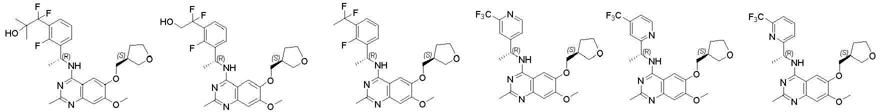

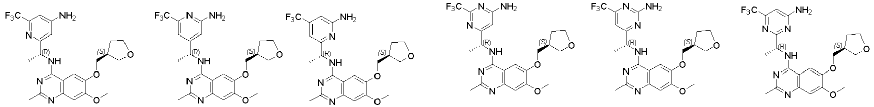

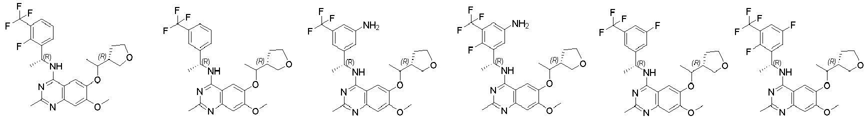

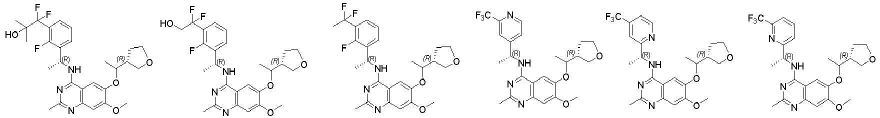

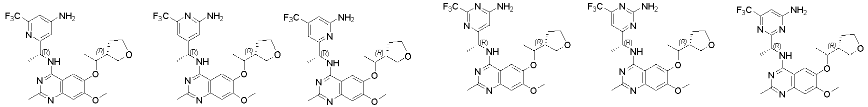

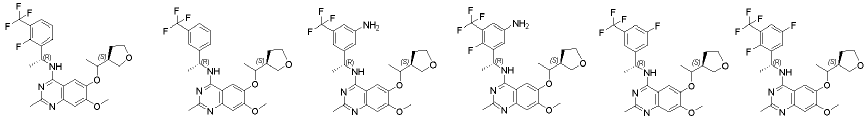

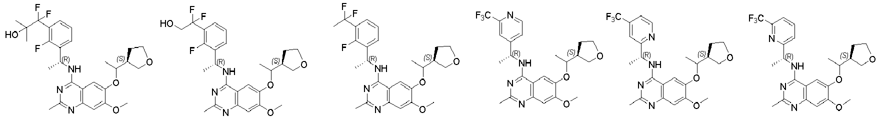

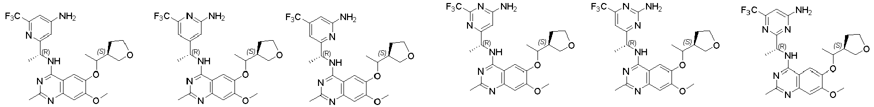

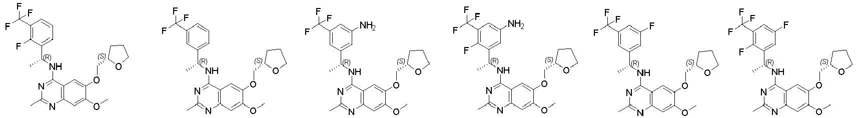

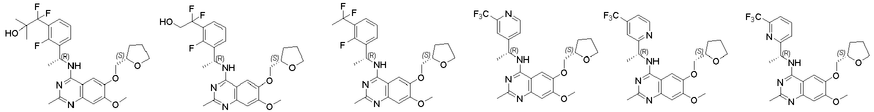

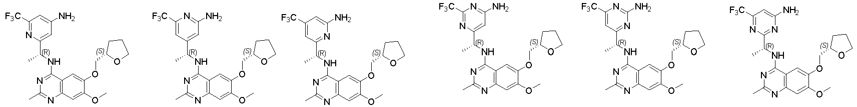

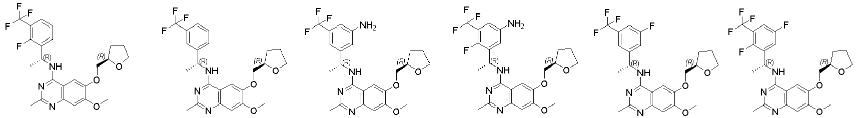

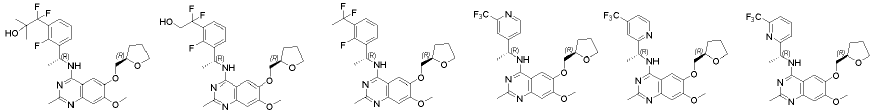

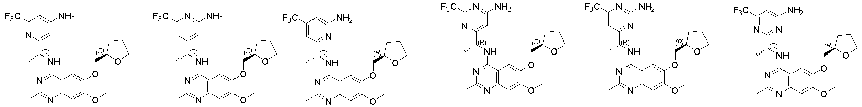

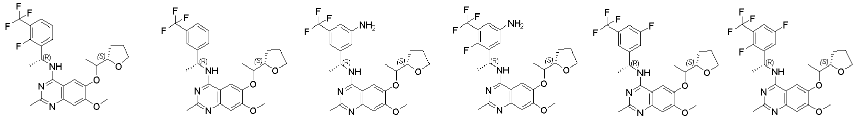

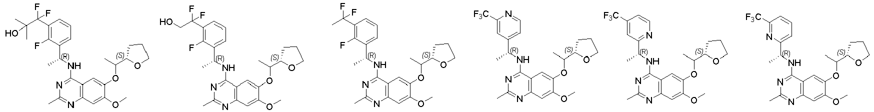

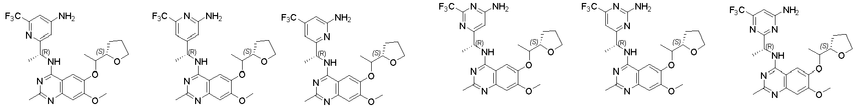

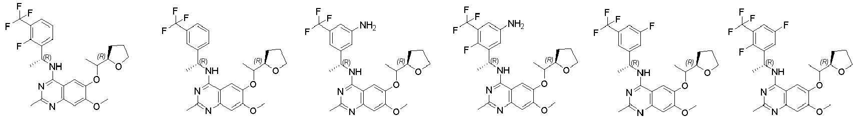

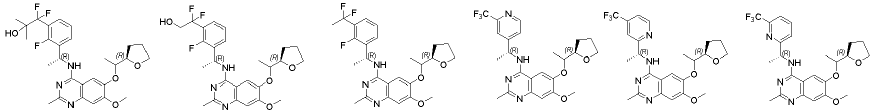

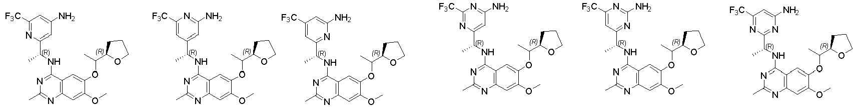

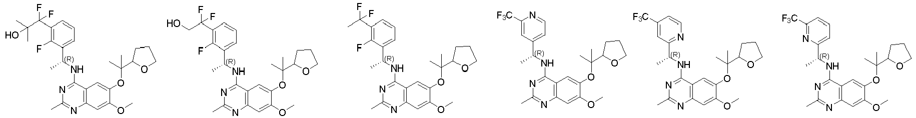

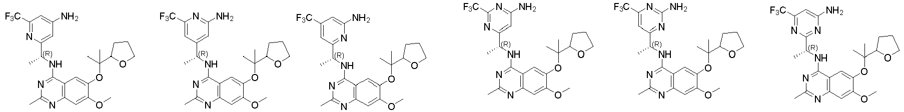

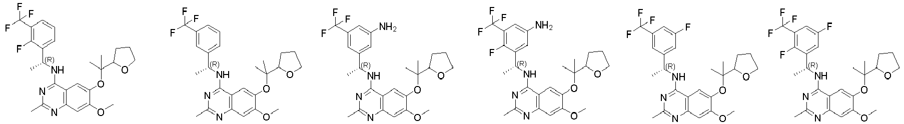

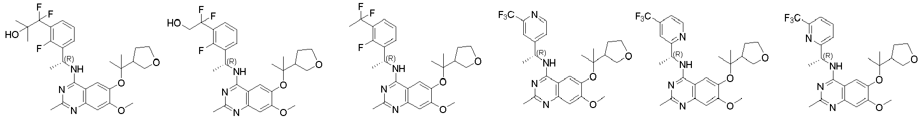

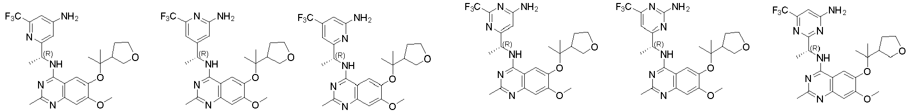

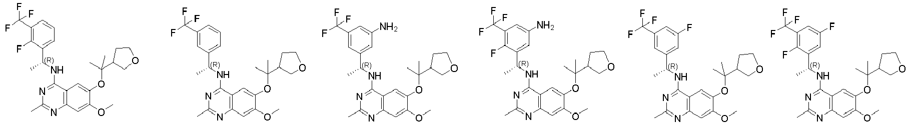

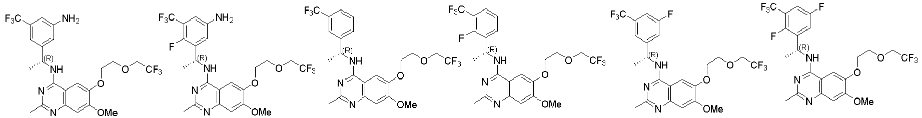

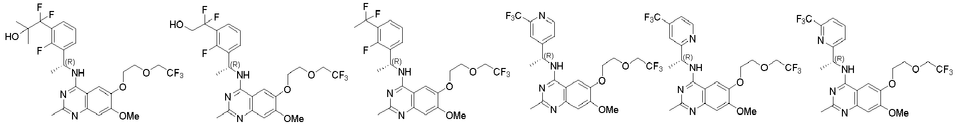

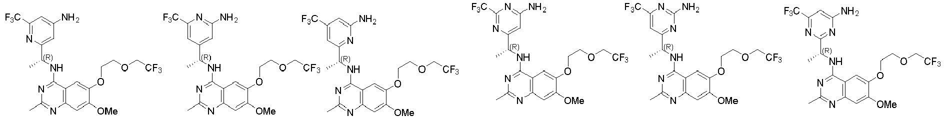

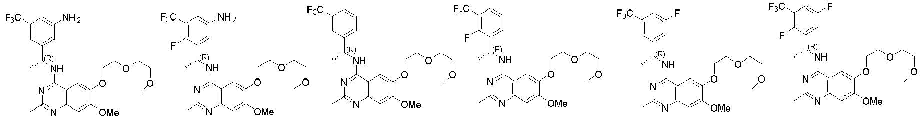

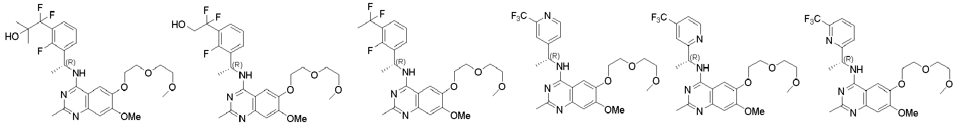

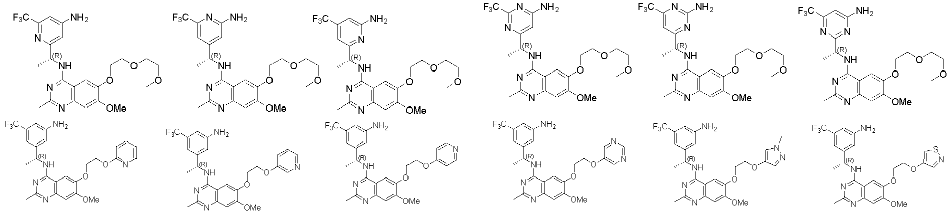

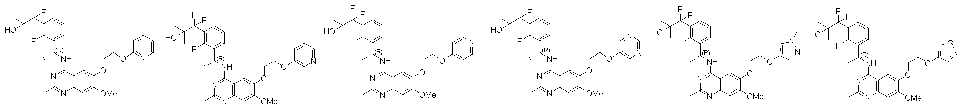

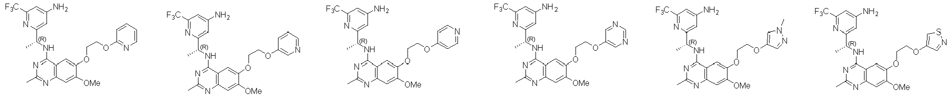

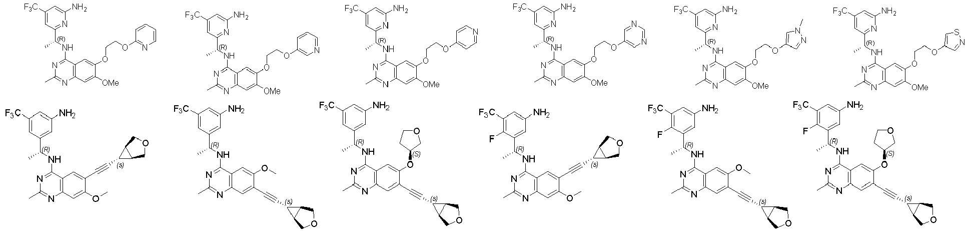

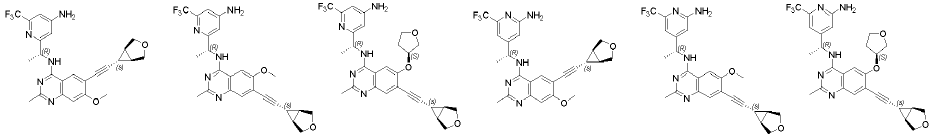

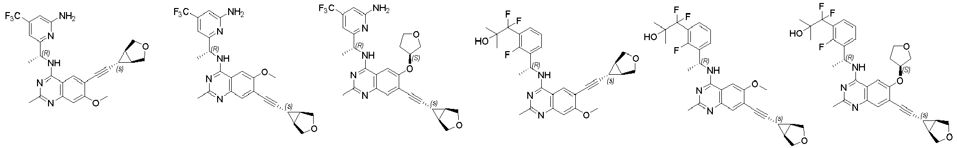

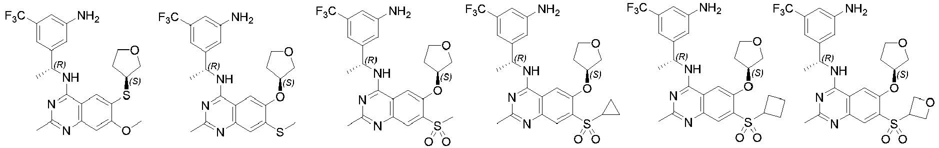

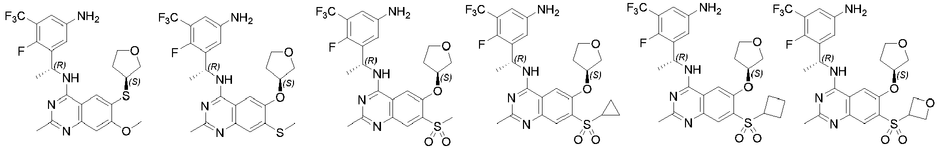

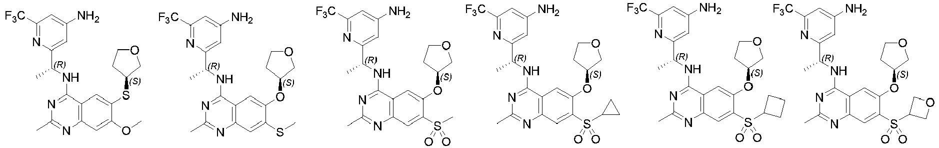

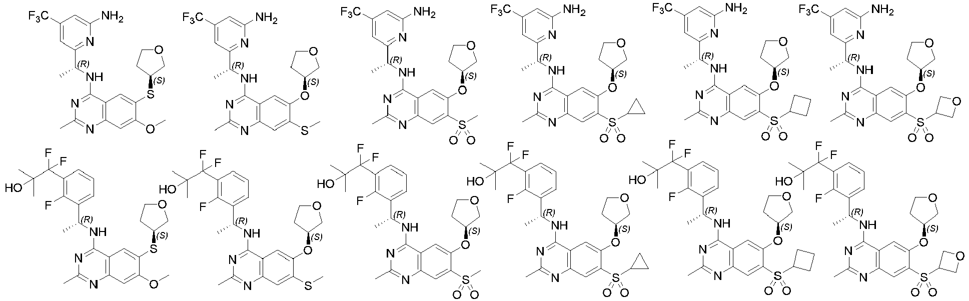

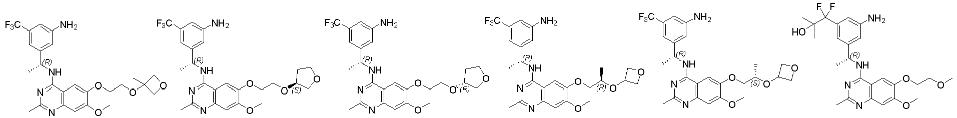

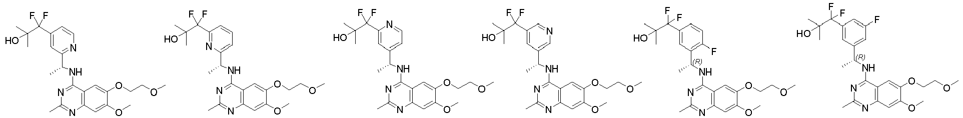

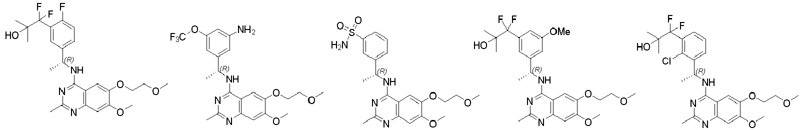

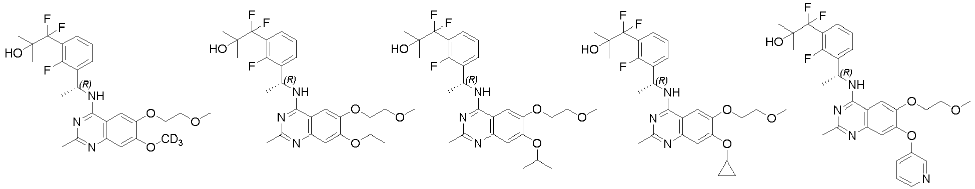

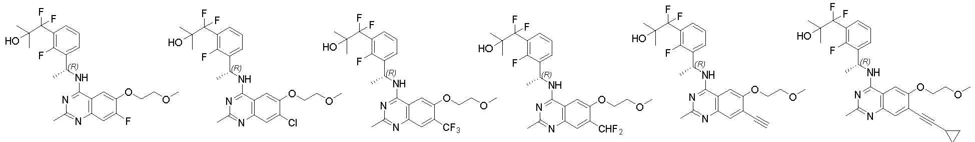

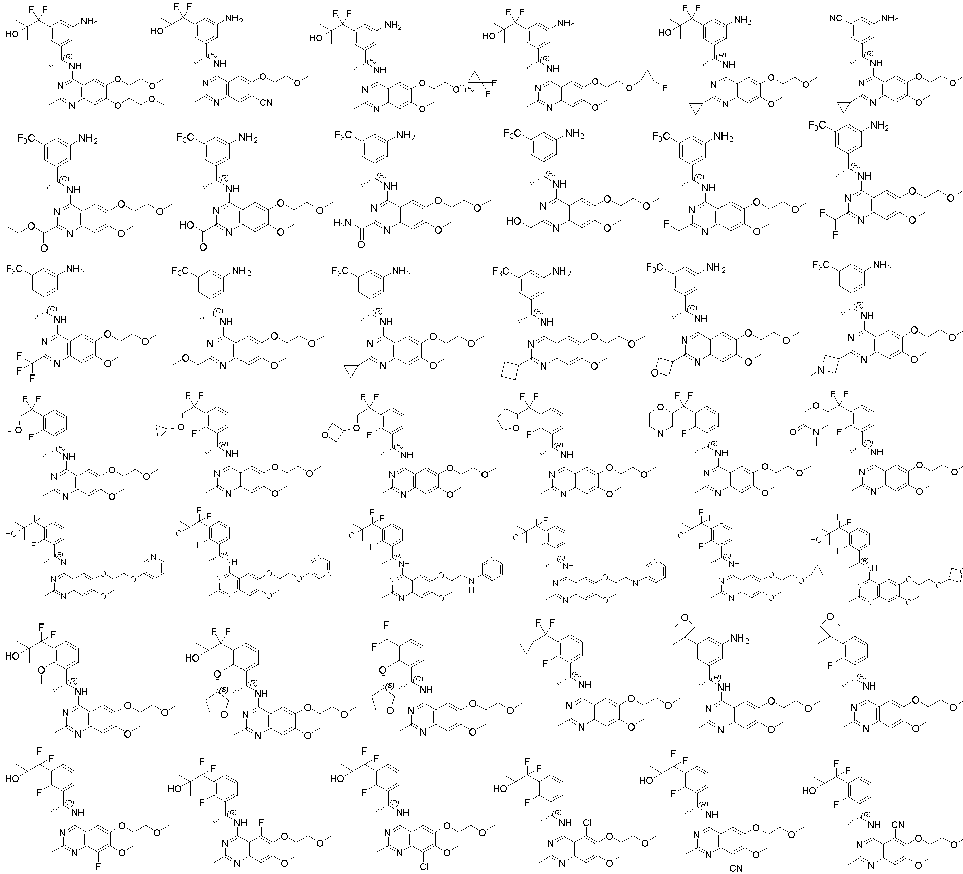

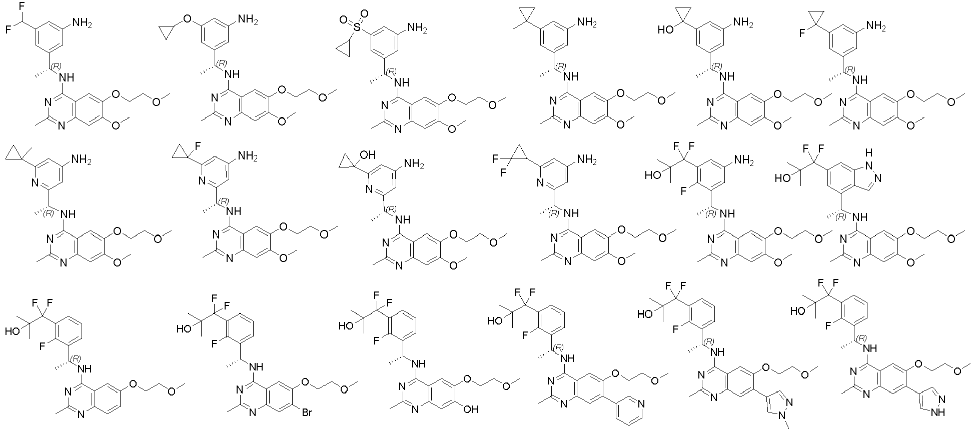

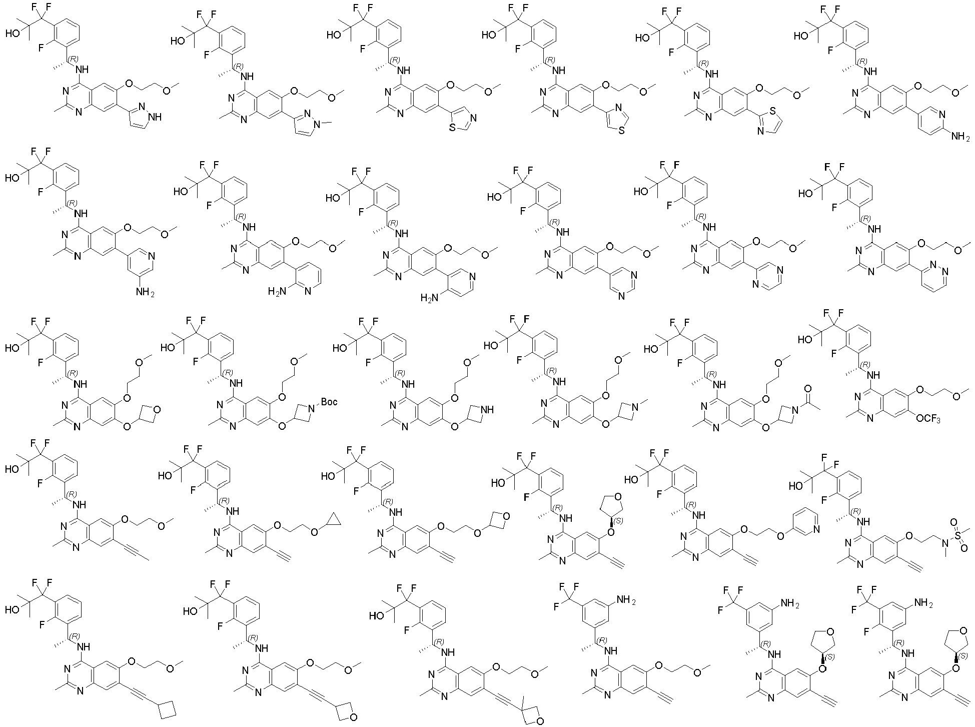

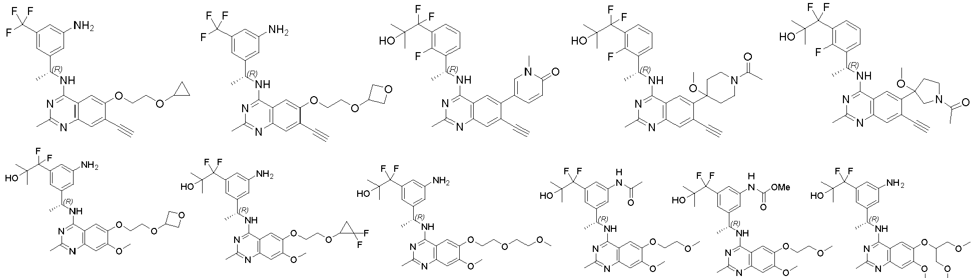

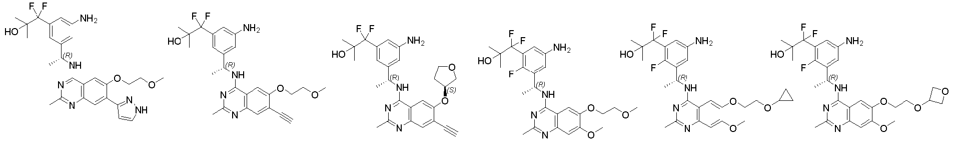

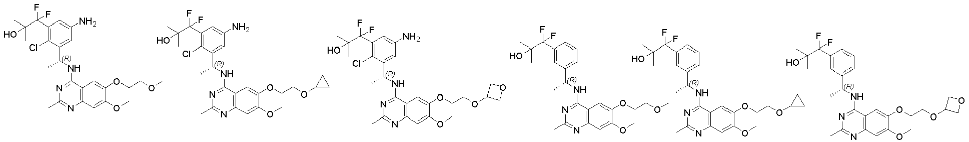

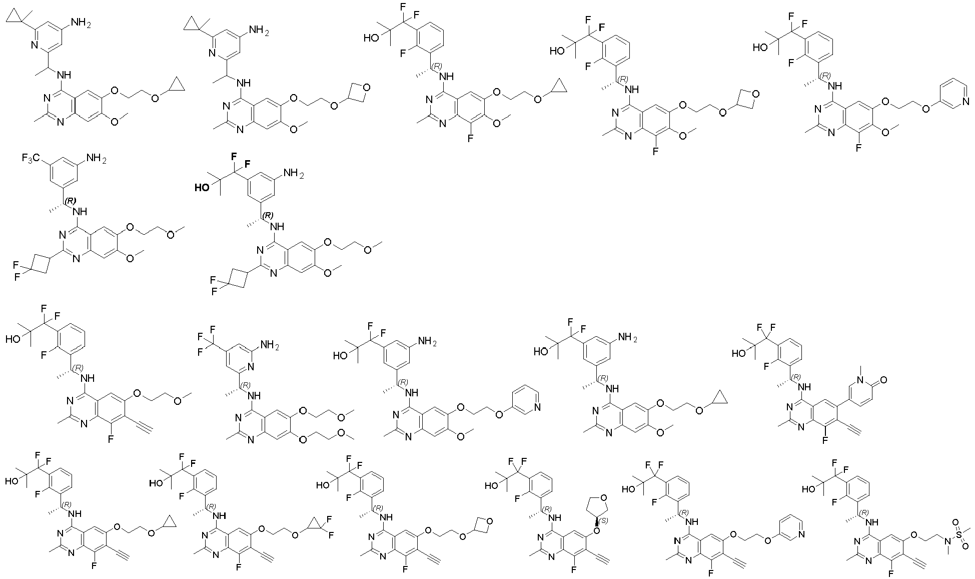

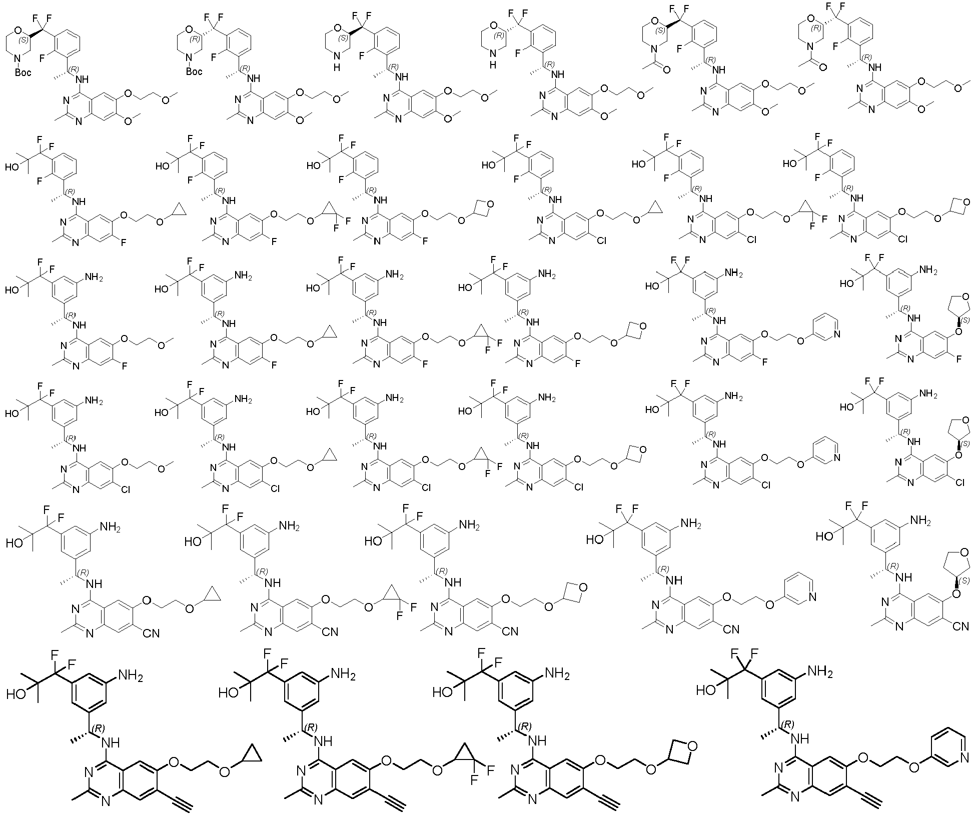

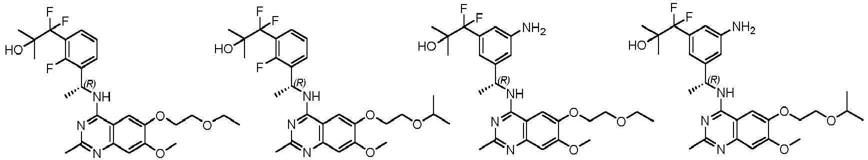

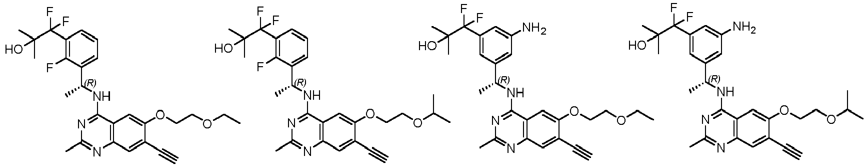

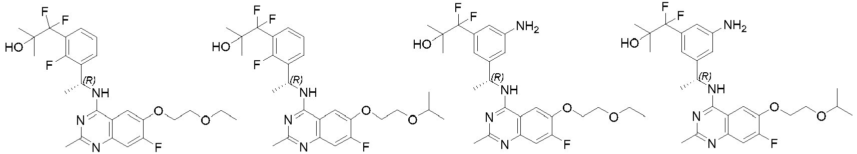

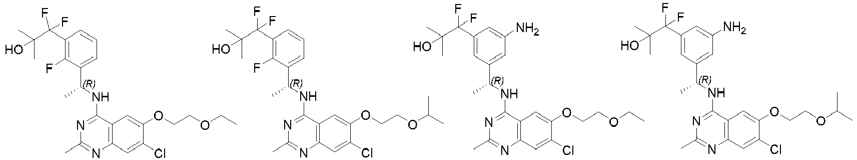

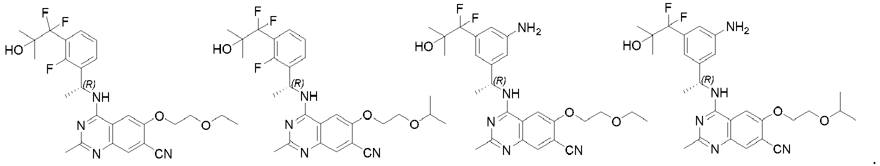

In another preferred embodiment, the compound is preferably a compound prepared in the examples.

In a second aspect of the present invention, provided is a method for preparing the substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof, comprising: 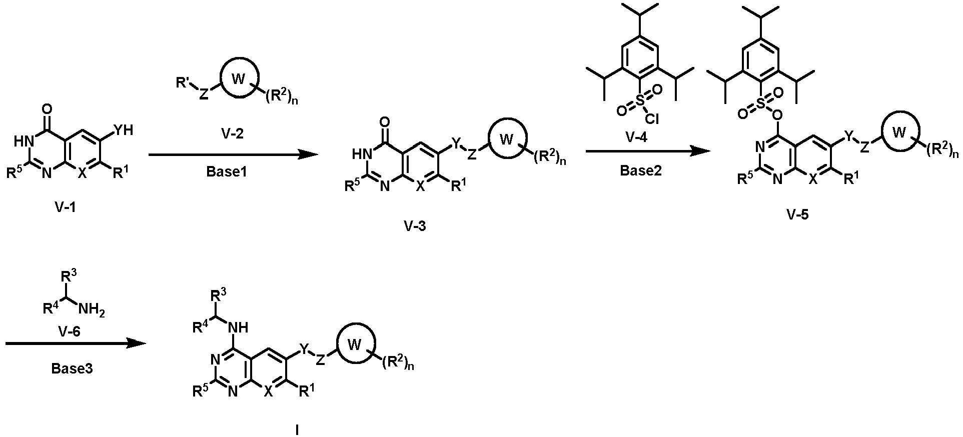
(i) reacting a compound of formula V-1 with a compound of formula V-2 in the presence of a first base to give a compound of formula V-3;
(ii) reacting the compound of formula V-3 with sulfonyl chloride (V-4) in the presence of a second base and a catalyst (e.g., DMAP) to give a compound of formula V-5; and
(iii) reacting the compound of formula V-5 with an amine (formula V-6) in the presence of a third base to give a compound of formula (I);

wherein in the formula,
R' is selected from: halogen, OTs, or OMs;
R¹, R², R³, R⁴, R⁵, X, Y, Z, W, and n are as defined above.

In another preferred embodiment, the first base is potassium carbonate, cesium carbonate, or the like.

In another preferred embodiment, the second base is TEA, DIPEA, or the like.

In another preferred embodiment, the third base is TEA, DIPEA, or the like.

In a third aspect of the present invention, provided is a pharmaceutical composition comprising i) one or more compounds, or stereoisomers, tautomers, crystalline forms, pharmaceutically acceptable salts, hydrates, solvates or prodrugs thereof according to the first aspect; and ii) a pharmaceutically acceptable carrier.

In another preferred embodiment, the pharmaceutical composition further comprises one or more therapeutic agents selected from the group consisting of: PD-1 inhibitors (e.g., nivolumab, pembrolizumab, pidilizumab, cemiplimab, JS-001, SHR-120, BGB-A317, IBI-308, GLS-010, GB-226, STW204, HX008, HLX10, BAT1306, AK105, and LZM009, or a biosimilar thereof), PD-L1 inhibitors (e.g., durvalumab, atezolizumab, avelumab, CS1001, KN035, HLX20, SHR-1316, BGB-A333, JS003, CS1003, KL-A167, F520, GR1405, and MSB2311, or a biosimilar thereof), CD20 antibodies (e.g., rituximab, obinutuzumab, ofatumumab, veltuzumab, tositumomab, 1311-tositumomab, ibritumomab, 90Y-ibritumomab, 90In-ibritumomab, and ibritumomab tiuxetan), CD47 antibodies (e.g., Hu5F9-G4, CC-90002, TTI-621, TTI-622, OSE-172, SRF-231, ALX-148, NI-1701, SHR-1603, IBI188, or IMM01), ALK inhibitors (e.g., ceritinib, alectinib, brigatinib, lorlatinib, and ocatinib), PI3K inhibitors (e.g., idelalisib, duvelisib, dactolisib, taselisib, bimiralisib, omipalisib, and buparlisib), BTK inhibitors (e.g., ibrutinib, tirabrutinib, acalabrutinib, zanubrutinib, and vecabrutinib), EGFR inhibitors (e.g., afatinib, gefitinib, erlotinib, lapatinib, dacomitinib, icotinib, canertinib, sapitinib, naquotinib, pyrotinib, rociletinib, and osimertinib), VEGFR inhibitors (e.g., sorafenib, pazopanib, regorafenib, sitravatinib, ningetinib, cabozantinib, sunitinib, and donafenib), HDAC inhibitors (e.g., givinostat, tucidinostat, vorinostat, fimepinostat, droxinostat, entinostat, dacinostat, quisinostat, and tacedinaline), CDK inhibitors (e.g., palbociclib, ribociclib, abemaciclib, milciclib, trilaciclib, and lerociclib), MEK inhibitors (e.g., selumetinib (AZD6244), trametinib (GSK1120212), PD0325901, U0126, pimasertib (AS-703026), and PD184352 (CI-1040)), mTOR inhibitors (e.g., vistusertib), and SHP2 inhibitors (e.g., RMC-4630, JAB-3068, and TNO155), or a combination thereof.

In a fourth aspect of the present invention, provided is of the compound, or the stereoisomer, the tautomer, the crystalline form, the pharmaceutically acceptable salt, the hydrate, the solvate, or the prodrug thereof according to the first aspect, or the pharmaceutical composition according to the third aspect in preparing a pharmaceutical composition for preventing and/or treating a disease associated with the activity or expression level of SOS1.

In another preferred embodiment, the disease is cancer.

In another preferred embodiment, the cancer is selected from: lung cancer, breast cancer, prostate cancer, esophageal cancer, colorectal cancer, bone cancer, kidney cancer, gastric cancer, liver cancer, colon cancer, melanoma, lymphoma, blood cancer, brain tumor, myeloma, soft tissue sarcoma, pancreatic cancer, and skin carcinoma.

In a fifth aspect of the present invention, provided is a non-diagnostic and non-therapeutic method for inhibiting SOS1, comprising: administering to a patient in need an effective amount of the compound of general formula (I), or the stereoisomer, the tautomer, the crystalline form, the pharmaceutically acceptable salt, the hydrate, the solvate, or the prodrug thereof according to the first aspect, or administering the pharmaceutical composition according to the third aspect.

It will be appreciated that within the scope of the present invention, the various technical features of the present invention described above and the technical features specifically described hereinafter (as in the examples) may be combined with each other to constitute a new or preferred technical scheme. Due to limited space, such schemes are not described herein.

### DETAILED DESCRIPTION

The inventors, through extensive and intensive studies in a long period of time, have surprisingly found a novel class of compounds which selectively inhibit SOS1 and/or have improved pharmacodynamic performance. The present invention is implemented on this basis.

### Terminology

In the present invention, unless otherwise specified, the terms as used have the ordinary meaning known to those skilled in the art.

The term "alkyl" refers to a linear or branched or cyclic alkane group containing 1 to 20 carbon atoms, such as 1 to 18 carbon atoms, especially 1 to 18 carbon atoms. Typical "alkyl" include methyl, ethyl, propyl, isopropyl, *n*-butyl, *t*-butyl, isobutyl, 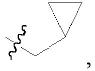 pentyl, isopentyl, heptyl, 4,4-dimethylpentyl, octyl, 2,2,4-trimethylpentyl, nonyl, decyl, undecyl, dodecyl, and the like.

The term "C1-C18 alkyl" refers to a linear or branched or cyclic alkyl including 1 to 18 carbon atoms, such as methyl, ethyl, propyl, isopropyl 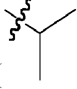 ), *n*-butyl, *t*-butyl, isobutyl (e.g., 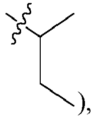 *n*-pentyl, isopentyl, *n*-hexyl, isohexyl, *n*-heptyl, and isoheptyl. The "substituted alkyl" means that one or more positions in the alkyl are substituted with a substituent, especially 1 to 4 substituents, wherein the substitution may occur in any position. Typical substituents include, but are not limited to, one or more of the following groups: such as hydrogen, deuterium, halogen (e.g., a monohalogen substituent or polyhalogen substituent such as trifluoromethyl or alkyl containing Cl₃), nitrile group, nitro, oxygen (e.g., =O), trifluoromethyl, trifluoromethoxy, cycloalkyl, alkenyl, cycloalkenyl, alkynyl, heterocycle, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, and NR_{b}P(=O)₂Rₑ, wherein the R^{a} herein may independently represent hydrogen, deuterium, alkyl, cycloalkyl, alkenyl, cycloalkenyl, alkynyl, heterocycle or aromatic ring, R_{b}, R_{c}, and R_{d} may independently represent hydrogen, deuterium, alkyl, cycloalkyl, alkenyl, cycloalkenyl, alkynyl, heterocycle, or aromatic ring, or R_{b} and R_{c}, together with the N atom, may form a heterocycle; Rₑ may independently represent hydrogen, alkyl, cycloalkyl, alkenyl, cycloalkenyl, alkynyl, heterocycle, or aromatic ring. The aforementioned typical substituents such as alkyl, cycloalkyl, alkenyl, cycloalkenyl, alkynyl, heterocycle, or aromatic ring may be optionally substituted.

The term "alkylene" refers to a group formed by further removal of one hydrogen atom from an "alkyl", such as methylene, ethylene, propylene, isopropylene (e.g., 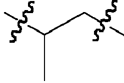 ), butylene (e.g., 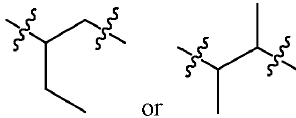 ), pentylene (e.g., 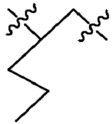 ), hexylene (e.g., 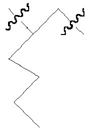 or 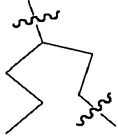 ), and heptylene (e.g., 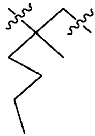 ).

The term "cycloalkyl" refers to a fully saturated cyclic hydrocarbon group comprising 1 to 4 rings each containing 3 to 8 carbon atoms. The "substituted cycloalkyl" means that one or more positions in the cycloalkyl are substituted with a substituent, especially 1 to 4 substituents, wherein the substitution may occur in any position. Typical substituents include, but are not limited to, one or more of the following groups: such as hydrogen, deuterium, halogen (e.g., a monohalogen substituent or polyhalogen substituent such as trifluoromethyl or alkyl containing Cl₃), nitrile group, nitro, oxygen (e.g., =O), trifluoromethyl, trifluoromethoxy, cycloalkyl, alkenyl, cycloalkenyl, alkynyl, heterocycle, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)2NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, and NR_{b}P(=O)₂Rₑ, wherein the R^{a} herein may independently represent hydrogen, deuterium, alkyl, cycloalkyl, alkenyl, cycloalkenyl, alkynyl, heterocycle or aromatic ring, R_{b}, R_{c}, and R_{d} may independently represent hydrogen, deuterium, alkyl, cycloalkyl, alkenyl, cycloalkenyl, alkynyl, heterocycle, or aromatic ring, or R_{b} and R_{c}, together with the N atom, may form a heterocycle; Rₑ may independently represent hydrogen, deuterium, alkyl, cycloalkyl, alkenyl, cycloalkenyl, alkynyl, heterocycle, or aromatic ring. The aforementioned typical substituents may be optionally substituted. Typical substituents also include spiro, bridged or fused ring substituents, especially spirocycloalkyl, spirocycloalkenyl, spiroheterocycle (excluding heteroaromatic ring), bridged cycloalkyl, bridged cycloalkenyl, bridged heterocycle (excluding heteroaromatic ring), fused cycloalkyl, fused cycloalkenyl, fused heterocyclyl, and fused aryl, wherein the above cycloalkyl, cycloalkenyl, heterocyclyl, and heterocycloaryl may be optionally substituted. Any two or more atoms on the ring may be further fused with other cycloalkyl, heterocyclyl, aryl, or heteroaryl.

The term "cycloalkylene" refers to a group formed by removal of two hydrogen atoms from cycloalkyl, such as:

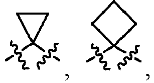

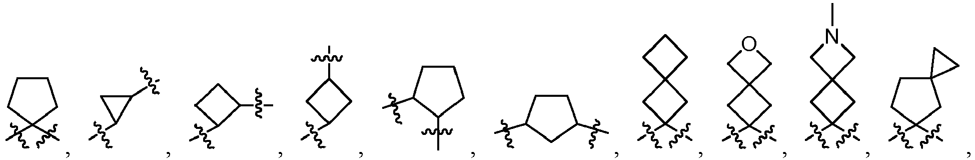

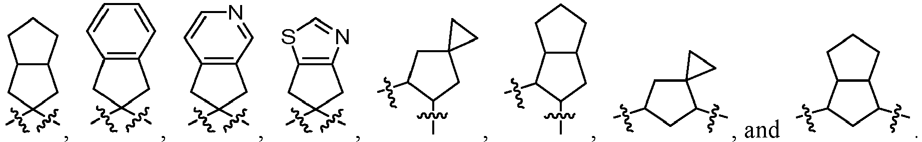

The term "alkylene cycloalkylene" refers to a group formed by removal of two hydrogen atoms from the aforementioned cycloalkyl alkyl or alkyl cycloalkyl, wherein "C1-C18 alkylene C3-C20 cycloalkylene" or "C3-C20 cycloalkylene C1-C18 alkylene" has the same meaning, preferably C1-C6 alkylene C3-C12 cycloalkylene, including but not limited to:

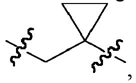

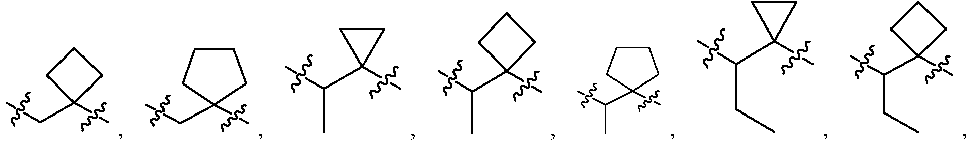

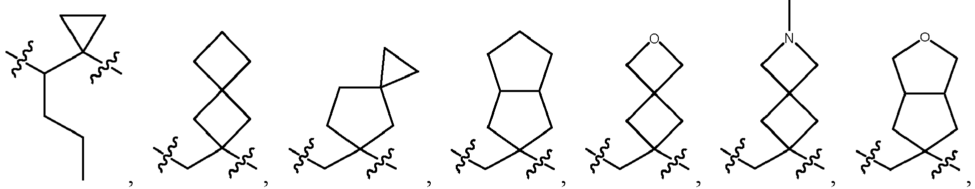

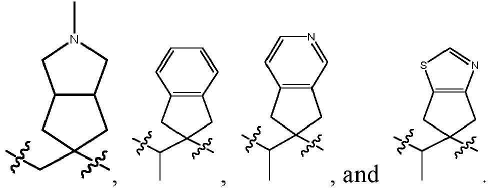

The term "heterocyclyl" refers to a fully saturated or partially unsaturated cyclic group (including but not limited to, for example, 3- to 7-membered monocyclic, 6- to 11-membered bicyclic, or 8- to 16-membered tricyclic ring systems), wherein at least one heteroatom is present in a ring containing at least one carbon atom. Each heterocycle containing heteroatoms may carry 1, 2, 3, or 4 heteroatoms selected from a nitrogen atom, an oxygen atom, and a sulfur atom, wherein the nitrogen or sulfur atom may be oxidized, and the nitrogen atom may be quaternized. The heterocyclyl may be attached to the residue of any heteroatom or carbon atom of the ring or cyclic molecule. Exemplary monocyclic heterocycles include, but are not limited to, azetidinyl, pyrrolidyl, oxetanyl, pyrazolinyl, imidazolinyl, imidazolidinyl, oxazolidinyl, isoxazolidinyl, thiazolidinyl, isothiazolidinyl, tetrahydrofuryl, piperidyl, piperazinyl, 2-oxopiperazinyl, 2-oxopiperidyl, 2-oxopyrrolidyl, hexahydroazepinyl, 4-piperidinonyl, tetrahydropyranyl, morpholinyl, thiomorpholinyl, thiomorpholinyl-sulfoxyl, thiomorpholine-sulfonyl, 1,3-dioxanyl, and tetrahydro-1,1-dioxothienyl. A polycyclic heterocyclyl includes spiro, fused, and bridged heterocyclyls. The spiro, fused, and bridged heterocyclyls involved are optionally linked to other groups via single bonds, or are further fused with other cycloalkyl, heterocyclyl, aryl and heteroaryl via any two or more atoms of the ring. The heterocyclyl may be substituted or unsubstituted, and when substituted, the substituents are preferably one or more groups independently selected from alkyl, deuteroalkyl, haloalkyl, alkoxy, haloalkoxy, alkenyl, alkynyl, alkylthio, alkylamino, halogen, amino, nitro, hydroxy, mercapto, cyano, cycloalkyl, heterocyclyl, aryl, heteroaryl, cycloalkylthio, oxo, carboxyl, and carboxylate group, wherein any two or more atoms on the ring may be further fused with other cycloalkyl, heterocyclyl, aryl, or heteroaryl.

The term "heterocyclylene" refers to a group formed by removal of two hydrogen atoms from the aforementioned heterocyclyl, including but not limited to: 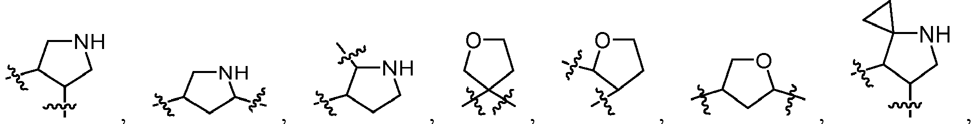 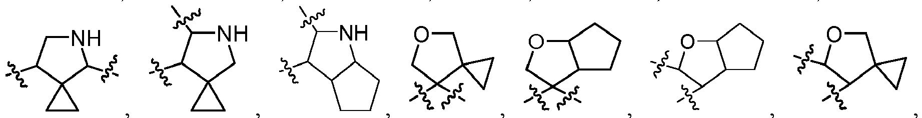 and

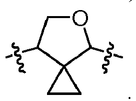

The term "heterocycloalkylene alkylene" refers to a group formed by removal of two hydrogen atoms from the cycloalkyl alkyl or alkyl cycloalkyl, wherein "4- to 20-membered heterocycloalkylene C1-C18 alkylene" or "C1-C18 alkylene 4- to 20-membered heterocycloalkylene" has the same meaning, preferably 4- to 12-membered heterocycloalkylene C1-6 alkylene, including but not limited to: 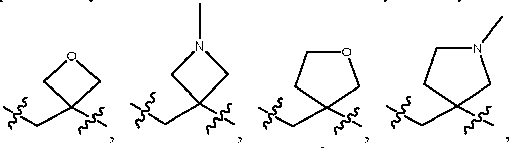 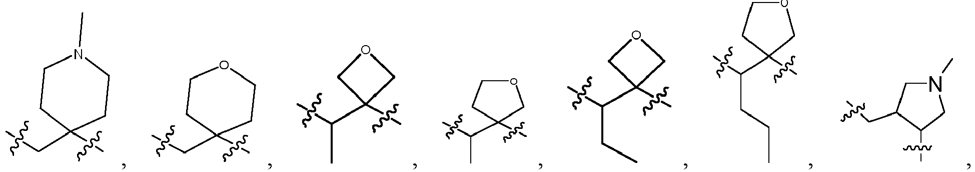 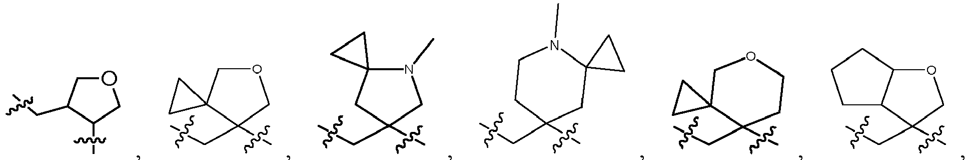 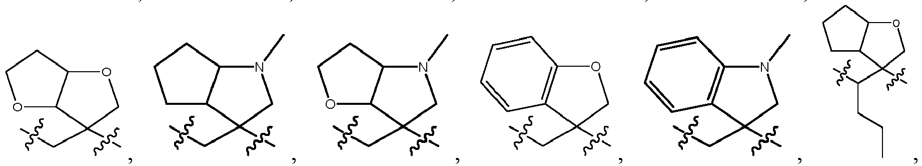 and

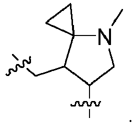

The term "aryl" refers to an aromatic cyclic hydrocarbon group having 1 to 5 rings, especially monocyclic and bicyclic groups such as phenyl, biphenyl or naphthyl. When having two or more aromatic rings (bicyclic and the like), the aromatic rings of aryl may be linked by a single bond (such as biphenyl) or fused (such as naphthalene and anthracene). The "substituted aryl" means that one or more positions in the aryl are substituted with a substituent, especially 1 to 3 substituents, wherein the substitution may occur in any position. Typical substituents include, but are not limited to, one or more of the following groups: such as hydrogen, deuterium, halogen (e.g., a monohalogen substituent or polyhalogen substituent such as trifluoromethyl or alkyl containing Cl₃), nitrile group, nitro, oxygen (e.g., =O), trifluoromethyl, trifluoromethoxy, cycloalkyl, alkenyl, cycloalkenyl, alkynyl, heterocycle, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, and NR_{b}P(=O)₂Rₑ, wherein the Rₐ herein may independently represent hydrogen, deuterium, alkyl, cycloalkyl, alkenyl, cycloalkenyl, alkynyl, heterocycle or aromatic ring, R_{b}, R_{c}, and R_{d} may independently represent hydrogen, deuterium, alkyl, cycloalkyl, alkenyl, cycloalkenyl, alkynyl, heterocycle, or aromatic ring, or R_{b} and R_{c}, together with the N atom, may form a heterocycle; Rₑ may independently represent hydrogen, deuterium, alkyl, cycloalkyl, alkenyl, cycloalkenyl, alkynyl, heterocycle, or aromatic ring. The aforementioned typical substituents may be optionally substituted. Typical substituents also include fused ring substituents, especially fused cycloalkyl, fused cycloalkenyl, fused heterocyclyl, and fused aryl, wherein the above cycloalkyl, cycloalkenyl, heterocyclyl, and heterocycloaryl may be optionally substituted.

The term "heteroaryl" refers to a heteroaromatic system containing 1-4 heteroatoms and 5-14 ring atoms, wherein the heteroatoms are selected from the group consisting of oxygen, nitrogen, and sulfur. The heteroaryl is preferably a 5- to 10-membered ring, more preferably a 5- or 6-membered ring, such as pyrrolyl, pyrazolyl, imidazolyl, oxazolyl, isoxazolyl, thiazolyl, thiadiazolyl, isothiazolyl, furyl, pyridyl, pyrazinyl, pyrimidinyl, pyridazinyl, triazinyl, triazolyl, tetrazolyl, and the like. The "heteroaryl" may be substituted or unsubstituted, and when it is substituted, the substituent is preferably one or more groups independently selected from alkyl, deuteroalkyl, haloalkyl, alkoxy, haloalkoxy, alkenyl, alkynyl, alkylthio, alkylamino, halogen, amino, nitro, hydroxy, mercapto, cyano, cycloalkyl, heterocyclyl, aryl, heteroaryl, cycloalkylthio, oxo, carboxyl, and carboxylate group.

The term "C1-C18 alkoxy" refers to a linear or branched or cyclic alkoxy having 1 to 18 carbon atoms, including, without limitation, methoxy, ethoxy, propoxy, isopropoxy, butoxy, and the like. C1-C8 alkoxy is preferred, and C1-C6 alkoxy is more preferred.

The term "C1-C18 alkyleneoxy" refers to a group formed by the removal of one hydrogen atom from "C1-C18 alkoxy".

The term "halogen" or "halo" refers to chlorine, bromine, fluorine, or iodine.

The term "halogenated" means being substituted with a halogen.

The term "deuterated" means being substituted with deuterium.

The term "hydroxy" refers to a group with a structure of OH.

The term "nitro" refers to a group with a structure of NO₂.

The term "cyano" refers to a group with a structure of CN.

The term "acyl" refers to a group with a structure of -COR, wherein R represents hydrogen, alkyl or substituted alkyl, cycloalkyl or substituted cycloalkyl, cycloalkenyl or substituted cycloalkenyl, aryl or substituted aryl, or heterocycle or substituted heterocycle. Preferably, the acyl is "C₂-C₆ acyl" (e.g., -COC₁-C₅ alkyl). Examples of acyl include, but are not limited to: -COCH₃, -COCH₂CH₃, -COCH₂CH₂CH₃, or -COCH₂CH(CH₃)₂.

The term "ester group" refers to a group with a structure of -COOR, wherein R represents hydrogen, alkyl or substituted alkyl, cycloalkyl or substituted cycloalkyl, cycloalkenyl or substituted cycloalkenyl, aryl or substituted aryl, or heterocycle or substituted heterocycle. Preferably, the ester group is "C₂-C₆ ester group" (e.g., -COOC₁-C₅ alkyl). Examples of ester group include, but are not limited to: -COOCH₃, -COOCH₂CH₃, -COOCH₂CH₂CH₃, or -COOCH₂CH(CH₃)₂.

The term "amino" refers to a group with a structure of -NRR', wherein R and R' may independently represent hydrogen, alkyl or substituted alkyl, cycloalkyl or substituted cycloalkyl, cycloalkenyl or substituted cycloalkenyl, aryl or substituted aryl, or heterocycle or substituted heterocycle, as defined above. R and R' in the dialkylamine moiety may be identical or different. Preferably, the amine group is a C₁-C₆ amine group (i.e., an alkylamino containing 1-6 carbon atoms, such as C₁-C₆ alkyl-NH-). Examples of amine group include, but are not limited to: NH₂, methylamino, dimethylamino, ethylamino, diethylamino, propylamino, dipropylamino, isopropylamino, diisopropylamino, anilino, diphenylamino, and the like.

The term "amido" refers to a group with a structure of -CONRR', wherein R and R' may independently represent hydrogen, alkyl or substituted alkyl, cycloalkyl or substituted cycloalkyl, cycloalkenyl or substituted cycloalkenyl, aryl or substituted aryl, or heterocycle or substituted heterocycle, as defined above. Preferably, the amido is "C₁-C₆ amido" (e.g., -CONHC₁-C₅ alkyl or -CONH₂). R and R' in the dialkylamine moiety may be identical or different. Examples of amido include, but are not limited to: -CONH₂, -CONHCH₃, -CON(CH₃)₂, and the like.

The term "sulfonamido" refers to a group with a structure of -SO₂NRR' or RSO₂NR'-, wherein R and R' may independently represent hydrogen, alkyl or substituted alkyl, cycloalkyl or substituted cycloalkyl, cycloalkenyl or substituted cycloalkenyl, aryl or substituted aryl, or heterocycle or substituted heterocycle, as defined above. R and R' in the dialkylamine moiety may be identical or different. Examples of sulfonamido include, but are not limited to: -SO₂NH₂, -SO₂NHCH₃, -SO₂N(CH₃)₂, CH₃SO₂NH-, CH₃SO₂NCH₃-, and the like. As used herein, "C₁-C₆ sulfonamido" refers to C₁-C₆ alkylsulfonamido, that is, the total number of carbon atoms in R and R' is 1-6.

The term "ureido" refers to a group with a structure of -NRCONR'R", wherein R, R' and R" may independently represent hydrogen, alkyl or substituted alkyl, cycloalkyl or substituted cycloalkyl, cycloalkenyl or substituted cycloalkenyl, aryl or substituted aryl, or heterocycle or substituted heterocycle, as defined above. R, R', and R" in the dialkylamine moiety may be identical or different. Examples of ureido include, but are not limited to: -NHCONH₂, -NHCONHCH₃, -NHCON(CH₃)₂, and the like. As used herein, "C₁-C₆ ureido" refers to C₁-C₆ alkylureido, that is, the total number of carbon atoms in R, R', and R" is 1-6.

The term "alkylaminoalkyl" refers to a group with a structure of -RNHR', wherein R and R' may independently represent hydrogen, alkyl or substituted alkyl, cycloalkyl or substituted cycloalkyl, cycloalkenyl or substituted cycloalkenyl, aryl or substituted aryl, or heterocycle or substituted heterocycle, as defined above. R and R' may be identical or different. Examples of alkylaminoalkyl include, but are not limited to, -CH₂NHCH₃, -CH₂CH₂NHCH₃, and the like. The term "dialkylaminoalkyl" refers to a group with a structure of -RNR'R", wherein R, R' and R" may independently represent alkyl or substituted alkyl, cycloalkyl or substituted cycloalkyl, cycloalkenyl or substituted cycloalkenyl, aryl or substituted aryl, or heterocycle or substituted heterocycle, as defined above. R, R', and R" in the dialkylamine moiety may be identical or different. Examples of dialkylaminoalkyl include, but are not limited to: -CH₂N(CH₃)₂, -CH₂CH₂N(CH₃)₂, and the like.

The term "sulfonyl" refers to a group with a structure of -SO₂R', where R' may independently represent hydrogen, alkyl or substituted alkyl, cycloalkyl or substituted cycloalkyl, cycloalkenyl or substituted cycloalkenyl, aryl or substituted aryl, or heterocycle or substituted heterocycle, as defined above. Examples of sulfonyl include, but are not limited to: -SO₂CH₃, -SO₂CH₂CH₃, -SO₂-cyclopropyl, -SO₂-cyclobutyl, -SO₂-cyclopentyl, or -SO₂-cyclohexyl.

The term "heterocyclylalkyl" refers to a group with a structure of -RR', wherein R may independently represent alkyl or substituted alkyl, cycloalkyl or substituted cycloalkyl, cycloalkenyl or substituted cycloalkenyl, or aryl or substituted aryl; R' represents heterocycle or substituted heterocycle. Examples of heterocyclylalkyl include, but are not limited to: azetidinyl-CH₂-, oxetanyl-CH₂-, azolidinyl-CH₂-, oxolanyl-CH₂-, azanyl-CH₂-, or oxanyl-CH₂-. According to the present invention, the term "substituted" means that one or more hydrogen atoms on a specific group are substituted with a specific substituent. Specific substituents are those described correspondingly in the preceding text or as present in the examples. Unless otherwise specified, a substituted group may have substituents selected from a specific group at any substitutable positions of the group. The substituents may be identical or different at the positions. It will be understood by those skilled in the art that combinations of substituents contemplated by the present invention are those stable or chemically available. The examples of such substituents include (but are not limited to): halogen, hydroxy, cyano, carboxyl (-COOH), C1-C6 alkyl, C2-C6 alkenyl, C2-C6 alkynyl, C3-C8 cycloalkyl, 3- to 12-membered heterocyclyl, aryl, heteroaryl, C1-C8 aldehyde group, C2-C10 acyl, C2-C10 ester group, amino, C1-C6 alkoxy, C1-C10 sulfonyl, C1-C6 ureido, and the like.

Unless otherwise stated, it is assumed that any heteroatom with insufficient valence has enough hydrogen atoms to supplement its valence state.

When the substituent is a non-terminal substituent, it is a "-ylene group" of the corresponding group. For example, the corresponding "-ylene group" of alkyl is alkylene, the corresponding "-ylene group" of cycloalkyl is cycloalkylene, the corresponding "-ylene group" of heterocyclyl is heterocyclylene, the corresponding "-ylene group" of alkoxy is alkyleneoxy, and so on.

### Active Ingredient

As used herein, "compound of the present invention" refers to a compound of formula I, and also includes a stereoisomer or optical isomer, a pharmaceutically acceptable salt, a prodrug or a solvate of the compound of formula I.

The compound of formula I has the following structure: 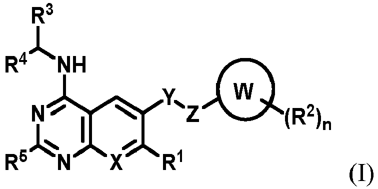 wherein in the formula, R¹, R², R³, R⁴, R⁵, X, Y, Z, W, and n are as defined above.

Preferably, the compound of formula I has a structure of general formula (II): 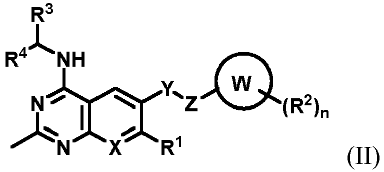 wherein in the formula, R¹, R², R³, R⁴, X, Y, Z, W, and n are as defined above.

Preferably, the compound of formula I has a structure of general formula (III): 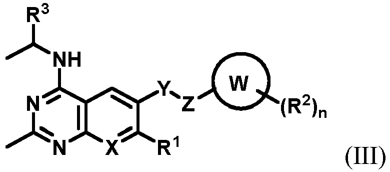 wherein in the formula, R¹, R², R³, X, Y, Z, W, and n are as defined above.

Preferably, the compound of formula I has a structure of general formula (IV): 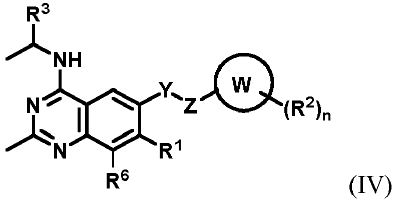
wherein in the formula,
R¹, R², R³, R⁶, X, Y, Z, W, and n are as defined above.

Preferably, the compound of formula I has a structure of general formula (V): 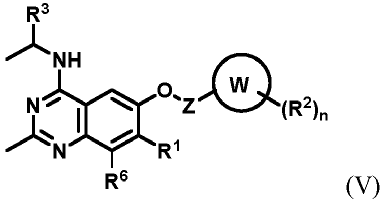
wherein in the formula,
R¹, R², R³, R⁶, Y, Z, W, and n are as defined above.

Preferably, the compound of formula I has a structure of formula (VI): 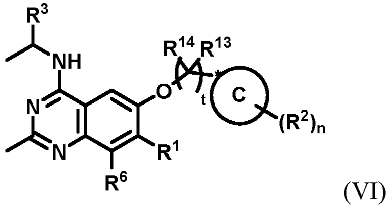 wherein in the formula, R¹, R², R³, R⁶, R¹³, R¹⁴, ring C, t, and n are as defined above.

Preferably, the compound of formula I has a structure of formula (VII): 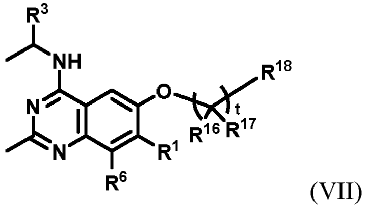
wherein in the formula,
R¹, R³, R⁶, R¹⁶, R¹⁷, R¹⁸, and t are as defined above.

Preferably, the compound of formula I has a structure of formula (VIII): 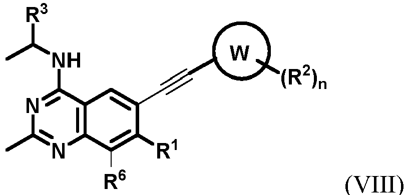
wherein in the formula,
R¹, R², R³, R⁶, and W are as defined above.

Preferably, the compound of formula I has a structure of formula (IX-A) or formula (IX-B): 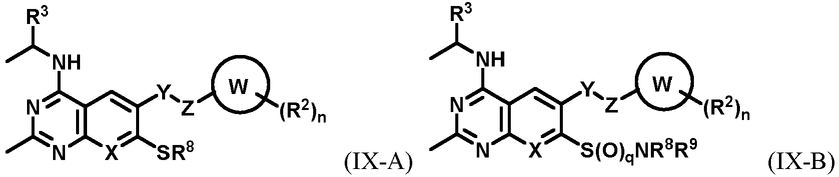 wherein in the formula, R¹, R², R³, R⁸, R⁹, X, Y, Z, W, n, and q are as defined above.

Preferably, the compound of formula I has a structure of formula (X): 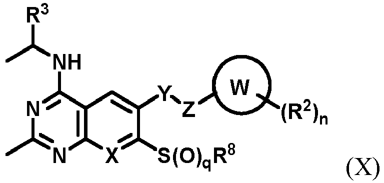 wherein in the formula, R¹, R², R³, X, Y, Z, W, n, and q are as defined above.

Preferably, in formulas I-VIII, R¹ is selected from the group consisting of: hydrogen, deuterium, halogen, cyano, -(CH₂)ₘ₁R⁸, -(CH₂)_{m'1}(CH=CH)R⁸, -(CH₂)_{m'1}(C≡C)R⁸, -(CH₂)ₘ₁O(CH₂)ₚ₁R⁸, -(CH₂)_{m'1}SR⁸, -(CH₂)ₘ₁COR⁸, -(CH₂)ₘ₁C(O)OR⁸, -(CH₂)_{m'1}S(O)_{q1}R⁸, -(CH₂)ₘ₁NR⁸R⁹, -(CH₂)ₘ₁C(O)NR⁸R⁹, -(CH₂)ₘ₁NR⁸C(O)R⁹, -(CH₂)ₘ₁NR⁸C(O)NR⁹R¹⁰, -(CH₂)_{m'1}S(O)_{q1}NR⁸R⁹, -(CH₂)_{m'1}NR⁸S(O)_{q1}R⁹, and -(CH₂)_{m'1}NR⁸S(O)_{q1}NR⁹R¹⁰, wherein H in CH₂ can be optionally substituted; R⁸, R⁹, and R¹⁰ are each independently selected from the group consisting of the following substituted or unsubstituted groups: hydrogen, C₁-C₁₈ alkyl, C₃-C₂₀ cycloalkyl, and 4-to 20-membered heterocyclyl; or in -(CH₂)ₘ₁NR⁸R⁹, -(CH₂)ₘ₁C(O)NR⁸R⁹, and -(CH₂)_{m'1}S(O)_{q1}NR⁸R⁹, R⁸ and R⁹, together with the N atom adjacent thereto, form a substituted or unsubstituted 4- to 8-membered heterocyclyl by cyclization; or in -(CH₂)ₘ₁NR⁸C(O)R⁹, -(CH₂)ₘ₁NR⁸C(O)NR⁹R¹⁰, -(CH₂)_{m'1}NR⁸S(O)_{q1}R⁹, and -(CH₂)_{m'1}NR⁸S(O)_{q1}NR⁹R¹⁰, R⁸ and R⁹, together with the N atom adjacent thereto, form a 4- to 8-membered substituted or unsubstituted heterocyclyl by cyclization, or R⁹ and R¹⁰, together with the atom adjacent thereto, form a substituted or unsubstituted 4- to 8-membered heterocyclyl by cyclization;
Each R² is independently selected from the group consisting of: hydrogen, deuterium, halogen, cyano, -(CH₂)ₘ₂R⁸, -(CH₂)_{m'2}(CH=CH)R⁸, -(CH₂)_{m'2}(C≡C)R⁸, -(CH₂)ₘ₂O(CH₂)ₚ₂R⁸, -(CH₂)_{m'2}SR⁸, -(CH₂)ₘ₂COR⁸, -(CH₂)ₘ₂C(O)OR⁸, -(CH₂)_{m'2}S(O)_{q2}R⁸, -(CH₂)ₘ₂NR⁸R⁹, -(CH₂)ₘ₂C(O)NR⁸R⁹, -(CH₂)ₘ₂NR⁸C(O)R⁹, -(CH₂)ₘ₂NR⁸C(O)NR⁹R¹⁰, -(CH₂)_{m'2}S(O)_{q2}NR⁸R⁹, -(CH₂)_{m'2}NR⁸S(O)_{q2}R⁹, and -(CH₂)_{m'2}NR⁸S(O)_{q2}NR⁹R¹⁰, wherein H in CH₂ can be optionally substituted; R⁸, R⁹, and R¹⁰ are each independently selected from the group consisting of the following substituted or unsubstituted groups: hydrogen, C₁-C₁₈ alkyl, C₃-C₂₀ cycloalkyl, and 4-to 20-membered heterocyclyl; or in -(CH₂)ₘ₂NR⁸R⁹, -(CH₂)ₘ₂C(O)NR⁸R⁹, and -(CH₂)_{m'2}S(O)_{q2}NR⁸R⁹, R⁸ and R⁹, together with the N atom adjacent thereto, form a substituted or unsubstituted 4- to 8-membered heterocyclyl by cyclization; or in -(CH₂)ₘ₂NR⁸C(O)R⁹, -(CH₂)ₘ₂NR⁸C(O)NR⁹R¹⁰, -(CH₂)_{m'2}NR⁸S(O)_{q2}R⁹, and -(CH₂)_{m'2}NR⁸S(O)_{q2}NR⁹R'_{O}, R⁸ and R⁹, together with the N atom adjacent thereto, form a 4- to 8-membered substituted or unsubstituted heterocyclyl by cyclization, or R⁹ and R¹⁰, together with the atom adjacent thereto, form a substituted or unsubstituted 4- to 8-membered heterocyclyl by cyclization;
m1 is 0, 1, 2, 3, 4, or 5;
m'1 is 0, 1, 2, 3, 4, or 5;
p1 is 0, 1, 2, 3, 4, or 5;
q1 is 1 or 2;
m2 is 0, 1, 2, 3, 4, or 5;
m'2 is 0, 1, 2, 3, 4, or 5;
p2 is 0, 1, 2, 3, 4, or 5;
q2 is 1 or 2;
provided that when Y is selected from: O, NH, and NR⁷, and when Z is a bond and W is C₃-C₂₀ cycloalkylene or 4- to 20-membered heterocyclylene, R¹ is not hydrogen, deuterium, halogen, or cyano and m1 is not 0;
wherein the substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, acyl, amido, sulfonyl, sulfonamido, and ureido.

Preferably, in formulas I-X, R³ is selected from the group consisting of the following substituted or unsubstituted groups: C₃-C₁₂ cycloalkyl, 4- to 12-membered heterocyclyl, C₆-C₁₀ aryl, and 5- to 10-membered heteroaryl; preferably, R³ is selected from the group consisting of the following substituted groups: phenyl, pyridyl, pyrimidinyl, and pyridazinyl; more preferably, R³ is selected from: 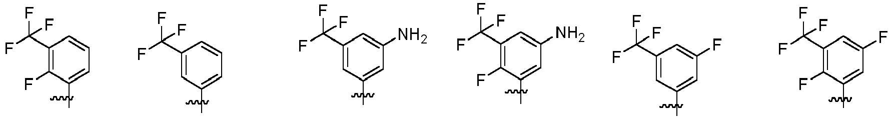 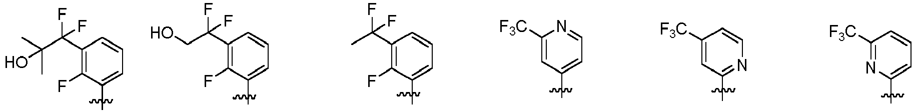 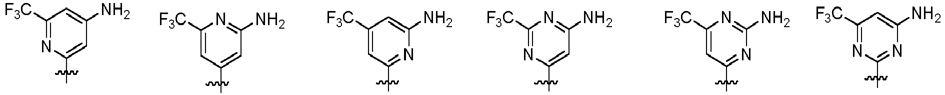 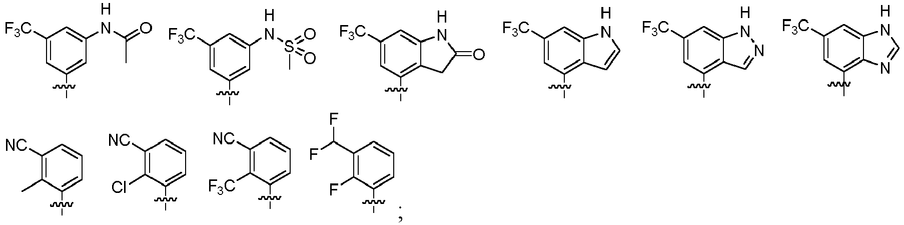 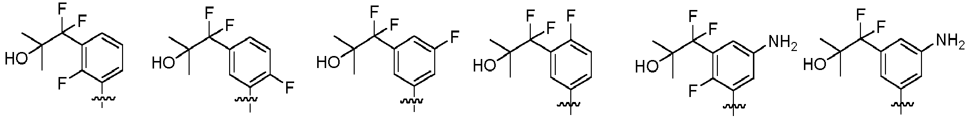 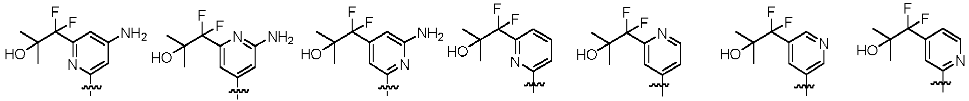 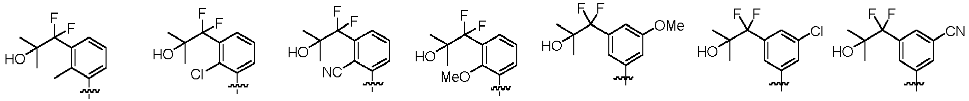 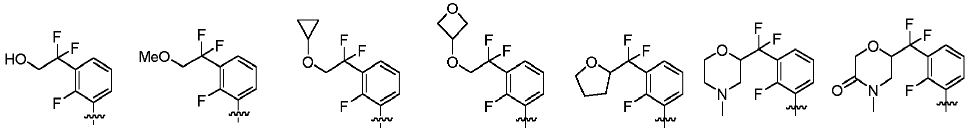 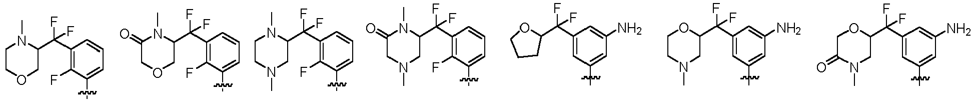 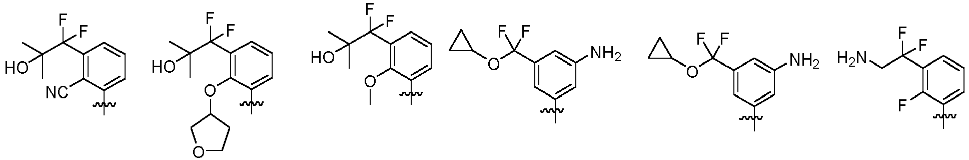 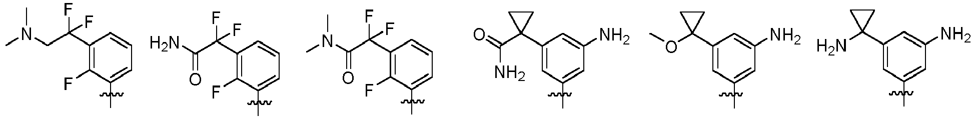 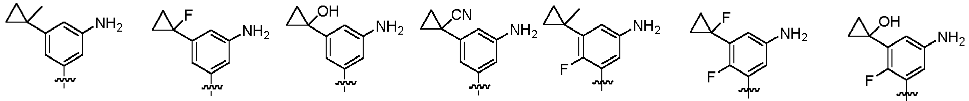 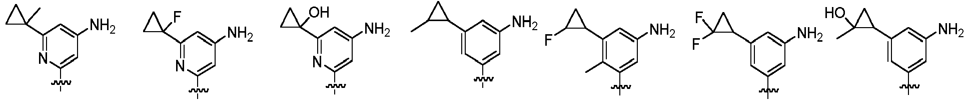 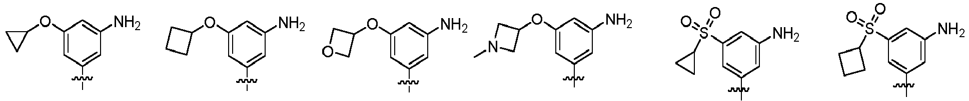 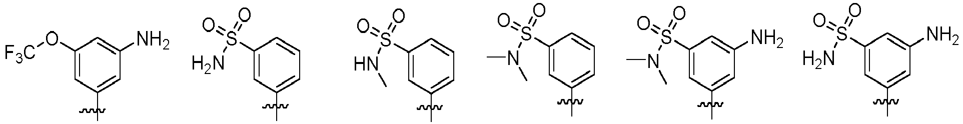 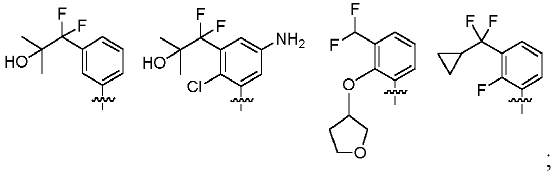 wherein the substitution refers to substitution with one or more groups selected from the group consisting of: hydrogen, deuterium, C₁-C₆ alkyl, deuterated C₁-C₆ alkyl, halogenated C₁-C₆ alkyl, halogenated C₁-C₆ alkylhydroxy, C₃-C₁₂ cycloalkyl, C₁-C₆ alkoxy, deuterated C₁-C₆ alkoxy, halogenated C₁-C₆ alkoxy, C₆-C₁₄ aryl, 5- to 14-membered heteroaryl, 4- to 12-membered heterocyclyl, halogen, oxo, nitro, hydroxy, cyano, ester group, amino, acyl, amido, sulfonyl, sulfonamido, and ureido.

Preferably, in formula I, R⁴ and R⁵ are independently selected from the group consisting of the following substituted or unsubstituted groups: C₁-C₆ alkyl, C₃-C₆ cycloalkyl, and 4- to 6-membered heterocyclyl;
wherein the substitution refers to substitution with one or more groups selected from the group consisting of: hydrogen, deuterium, C₁-C₆ alkyl, deuterated C₁-C₆ alkyl, halogenated C₁-C₆ alkyl, halogenated C₁-C₆ alkylhydroxy, C₃-C₁₂ cycloalkyl, C₁-C₆ alkoxy, deuterated C₁-C₆ alkoxy, halogenated C₁-C₆ alkoxy, C₆-C₁₄ aryl, 5- to 14-membered heteroaryl, 4- to 12-membered heterocyclyl, halogen, oxo, nitro, hydroxy, cyano, ester group, amino, amido, sulfonamido, and ureido.

Preferably, R⁶ is selected from: hydrogen, deuterium, halogen, cyano, and C₁-C₆ alkyl.

Preferably, in the present invention, the substitution refers to substitution with one or more groups selected from the group consisting of: hydrogen, deuterium, C₁-C₆ alkyl, deuterated C₁-C₆ alkyl, halogenated C₁-C₆ alkyl, halogenated C₁-C₆ alkylhydroxy, C₃-C₆ cycloalkyl, C₃-C₆ cycloalkyl-O-, C₁-C₆ alkoxy, deuterated C₁-C₆ alkoxy, halogenated C₁-C₆ alkoxy, C₆-C₁₄ aryl, 5- to 14-membered heteroaryl, 4- to 6-membered heterocyclyl, 4- to 6-membered heterocyclyl-O-, halogen, oxo, nitro, hydroxy, cyano, ester group, amino, amido, sulfonamido, and ureido; wherein the C₁-C₆ alkyl, deuterated C₁-C₆ alkyl, halogenated C₁-C₆ alkyl, halogenated C₁-C₆ alkylhydroxy, C₃-C₆ cycloalkyl, C₃-C₆ cycloalkyl-O-, C₁-C₆ alkoxy, deuterated C₁-C₆ alkoxy, halogenated C₁-C₆ alkoxy, C₆-C₁₄ aryl, 5- to 14-membered heteroaryl, 4- to 6-membered heterocyclyl, or 4- to 6-membered heterocyclyl-O- may be further substituted with one or more R^{a}, wherein R^{a} is selected from: C₁-C₆ alkyl, deuterated C₁-C₆ alkyl, halogenated C₁-C₆ alkyl, halogenated C₁-C₆ alkylhydroxy, C₃-C₆ cycloalkyl, C₃-C₆ cycloalkyl-O-, C₁-C₆ alkoxy, deuterated C₁-C₆ alkoxy, halogenated C₁-C₆ alkoxy, C₆-C₁₄ aryl, 5- to 14-membered heteroaryl, 4- to 6-membered heterocyclyl, 4- to 6-membered heterocyclyl-O-, halogen, oxo, nitro, hydroxy, cyano, ester group, amino, acyl, amido, sulfonyl, sulfonamido, and ureido.

The salts which the compound of the present invention may form are also within the scope of the present invention. Unless otherwise stated, the compound of the present invention is understood to include salts thereof. As used herein, the term "salt" refers to a salt in either an acid or a base form formed with an inorganic or organic acid and a base. In addition, when the compound of the present invention contains a basic moiety, the basis moiety includes, but is not limited to, pyridine or imidazole; when the compound of the present invention contains an acidic moiety, the acidic moiety includes, but is not limited to, carboxylic acid. The zwitterion ("inner salt") that may be formed is encompassed within the scope of the term "salt". Pharmaceutically acceptable (i.e., non-toxic and physiologically acceptable) salts are preferred, although other salts are useful, e.g., in isolation or purification steps of the preparation. The salt can be formed with the compound of the present invention, for example, by reacting compound I with an amount, e.g., an equivalent amount, of acid or base and then salting out from a medium, or by lyophilization in an aqueous solution.

The compound of the present invention contains a basic moiety, including but not limited to amine or a pyridine or imidazole ring, which may form salts with organic or inorganic acids. Typical acids which may form salts include acetate (e.g., formed with acetic acid or trihaloacetic acid such as trifluoroacetic acid), adipate, alginate, ascorbate, aspartate, benzoate, benzenesulfonate, bisulfate, borate, butyrate, citrate, camphorate, camphorsulfonate, cyclopentanepropionate, diglycolate, laurylsulfate, ethanesulfonate, fumarate, glucoheptonate, glycerophosphate, hemisulfate, heptanoate, caproate, hydrochloride, hydrobromide, hydroiodide, isethionate (e.g., 2-hydroxyethanesulfonate), lactate, maleate, methanesulfonate, naphthalenesulfonate (e.g., 2-naphthalenesulfonate), nicotinate, nitrate, oxalate, pectinate, persulfate, phenylpropionate (e.g., 3-phenylpropionate), phosphate, picrate, pivalate, propionate, salicylate, succinate, sulfate (e.g., formed with sulfuric acid), sulfonate, tartrate, thiocyanate, tosylate (e.g., *p*-toluenesulfonate), dodecanoate and the like.

Certain compounds of the present invention may contain an acidic moiety, including but not limited to carboxylic acid, which may form salts with various organic or inorganic bases. Typical salts formed with bases include ammonium salts; alkali metal salts such as sodium, lithium, or potassium salts; alkaline earth metal salts such as calcium or magnesium salts; salts formed with organic bases (such as organic amines) such as benzathine, dicyclohexylamine, hydrabamine (a salt formed with *N,N-*bis(dehydroabietyl)ethylenediamine), *N*-methyl-D-glucamine, *N*-methyl-D-glucamide, *t*-butylamine; salts with amino acids such as arginine and lysine. The basic nitrogen-containing groups may form quaternary ammonium salts with halides such as lower alkyl halides (e.g., methyl, ethyl, propyl, and butyl chlorides, bromides, and iodides), dialkyl sulfates (e.g., dimethyl, diethyl, dibutyl, and diamyl sulfates), long chain halides (e.g., decyl, dodecyl, tetradecyl, and tetradecyl chlorides, bromides, and iodides), and aralkyl halides (e.g., benzyl and phenyl bromides).

The prodrug and solvate of the compound of the present invention are also encompassed with the scope. As used herein, the term "prodrug" refers to a compound that undergoes a chemical conversion via a metabolic or chemical process to yield a compound, salt or solvate of the present invention when used in the treatment of a related disease. The compound of the present invention includes a solvate, such as a hydrate.

The compound, salt or solvate of the present invention may be present in a tautomeric form (e.g., amide and imine ether). All of these tautomers are part of the present invention.

Stereoisomers of all compounds (e.g., those asymmetric carbon atoms which may exist due to various substitutions), including enantiomeric and diastereoisomeric forms thereof, are contemplated within the scope of the present invention. The separate stereoisomer of the compound of the present invention may not be present simultaneously with the other isomers (e.g., as a pure or substantially pure optical isomer having specific activity), or may be present as a mixture, such as a racemate, or as a mixture with all or a portion of the other stereoisomers. The chiral center of the present invention has two configurations, S and R, and is defined by the International Union of Pure and Applied Chemistry (IUPAC) proposed in 1974. The racemic forms can be resolved by physical methods such as fractional crystallization, or separated and crystallized by derivation into diastereoisomers, or separated by chiral column chromatography. The individual optical isomer can be obtained from the racemate by any suitable methods, including but not limited to conventional methods, such as salt formation with optically active acids followed by crystallization.

The content, by weight, of the compound of the present invention, which is obtained by preparation, separation and then purification, is equal to or greater than 90%, e.g., is equal to or greater than 95%, or is equal to or greater than 99% ("very pure" compound), as listed in the text description. Herein, such "very pure" compounds of the present invention are also part of the present invention.

All configurational isomers of the compound of the present invention are encompassed with the scope, whether in admixture, pure or very pure form. The definition of the compound of the present invention includes both cis (*Z*) and trans (*E*) olefin isomers, as well as cis and trans isomers of carbocycle and heterocycle.

Throughout the specification, the groups and substituents may be selected to provide stable moieties and compounds.

The definitions for specific functional groups and chemical terms are described in detail below. For purposes of the present invention, the chemical elements are in accordance with those defined in the Periodic Table of the Elements, CAS version, *Handbook of Chemistry and Physics,* 75^{th} Ed. The definitions for specific functional groups are also described therein. In addition, the basic principles of organic chemistry, as well as specific functional groups and the reactivity thereof are also described in "Organic Chemistry", Thomas Sorrell, University Science Books, Sausaltito: 1999, which is incorporated by reference in its entirety.

Certain compounds of the present invention may be in the form of a specific geometric isomer or stereoisomer. The present invention encompasses all compounds, including cis and trans isomers, R and S enantiomers, diastereoisomers, (D) isomer, (L) isomer, racemic mixtures, and other mixtures. Further, the asymmetric carbon atom may represent a substituent such as an alkyl. All isomers and mixtures thereof are encompassed by the present invention.

According to the present invention, the mixture of isomers may contain the isomers in a variety of ratios. For example, the mixture of only two isomers may have the isomers in the following ratios: 50:50, 60:40, 70:30, 80:20, 90:10, 95:5, 96:4, 97:3, 98:2, 99:1, or 100:0, and all ratios of the isomers are encompassed within the scope of the present invention. Similar ratios, as well as more complex ratios of isomers of the mixtures, which are readily understood by those of ordinary skill in the art are also encompassed within the scope of the invention.

The present invention also includes isotopically-labeled compounds, equivalent to the original compounds disclosed herein. However, in fact, the substitution of one or more atoms with an atom with a different atomic weight or mass number usually occurs. Examples of isotopes that may be listed as the isotopes of the compound of the present invention include isotopes of hydrogen, carbon, nitrogen, oxygen, phosphorus, sulfur, fluorine, and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl. The compounds, or enantiomers, diastereoisomers, isomers, pharmaceutically acceptable salts or solvates of the present invention containing the above isotopes or other isotopic atoms are encompassed within the scope of the present invention. Certain isotopically-labeled compounds of the present invention, such as those labeled with radioisotopes of ³H and ¹⁴C, are also encompassed and useful in the drug and substrate tissue distribution assays. Tritium (i.e., ³H) and carbon-14 (i.e., ¹⁴C) are relatively easy to prepare and detect. They are preferred among isotopes. In addition, substitution with heavier isotopes such as deuterium, i.e., ²H, has advantages in certain therapies due to the good metabolic stability of the isotopes, such as increased half-life *in vivo* or reduced dosage, and thus may be preferred in certain situations. Isotopically-labeled compounds can be prepared by general methods by substituting a readily available isotopically labeled reagent for a non-isotopically labeled reagent using the protocols disclosed in the examples.

If the synthesis of a specific enantiomer of the compound of the present invention is to be designed, the enantiomer can be prepared by asymmetric synthesis, or by derivatization with a chiral auxiliary reagent, wherein the resulting diastereoisomeric mixture is resolved and the chiral auxiliary reagent is removed to obtain a pure enantiomer. Alternatively, if the molecule contains a basic functional group (e.g., an amino) or an acidic functional group (e.g., carboxyl), the molecule forms a diastereoisomeric salt with an appropriate optically active acid or base, and the resulting diastereomeric salt is resolved through a conventional means such as fractional crystallization or chromatography to obtain a pure enantiomer.

As described herein, the compound of the present invention can be substituted with any number of substituents or functional groups to expand their inclusion range. In general, whether the term "substituted" appears before or after the term "optional", a general formula including a substituent in the formula of the present invention means that the hydrogen radical is replaced with a substituent with the indicated structure. When a plurality of positions in a specific structure are substituted with a plurality of specific substituents, the substituents at each of the positions may be identical or different. As used herein, the term "substitution" includes all permissible substitutions of organic compounds. In a broad sense, permissible substituents include acyclic, cyclic, branched unbranched, carbocyclic and heterocyclic, and aromatic and nonaromatic organic compounds. In the present invention, for example, the heteroatom nitrogen may have a hydrogen substituent or any permissible organic compound described above to supplement its valence. Furthermore, the present invention is not intended to limit the permissible substitution of organic compounds in any way. According to the present invention, the combination of substituents and variable groups in the form of stable compounds is excellent in the treatment of diseases, such as infectious diseases or proliferative diseases. As used herein for the purpose described above, the term "stable" means that a compound is stable enough to maintain the structural integrity of the compound when tested over a sufficient period of time, and preferably is effective over a sufficient period of time.

Metabolites of the compounds and pharmaceutically acceptable salts thereof involved in the present application, as well as prodrugs that are convertible *in vivo* into the structures of the compounds and pharmaceutically acceptable salts thereof involved in the present application, are also encompassed by the claims of the present application.

### Preparation method

The preparation methods for the compound having the structure of formula (I) of the present invention is more specifically described below, but these specific methods do not limit the present invention in any way. The compounds of the present invention can also be conveniently prepared by optionally combining various synthetic methods described herein or known in the art, and such combinations can be easily determined by those skilled in the art to which the present invention pertains.

Typically, the compounds of the present invention are prepared by the following procedures, wherein the starting materials and reagents used are commercially available unless otherwise stated. 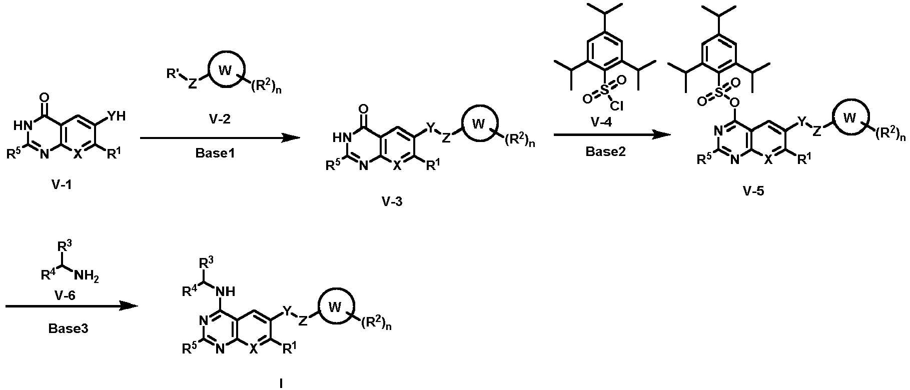
(i) reacting a compound of formula V-1 with a compound of formula V-2 (wherein R' is a leaving group, e.g., halogen, OTs, OMs, or the like) in the presence of a first base (e.g., potassium carbonate, cesium carbonate, or the like) to generate a compound of formula V-3;
(ii) protecting the compound of formula V-3 with sulfonyl chloride (V-4) in the presence of a second base (e.g., TEA, DIPEA, or the like) and a catalyst (e.g., DMAP) to generate a compound of formula V-5;
(iii) reacting the compound of formula V-5 with an amine (formula V-6) in the presence of a third base (e.g., TEA, DIPEA, or the like) to generate the compound of formula (I);

wherein in the formula,
R¹, R², R³, R⁴, R⁵, X, Y, Z, W, and n are as defined above.

### Pharmaceutical composition and mode of administration

The pharmaceutical composition described herein is used to prevent and/or treat the following diseases: inflammation, cancer, cardiovascular disease, infection, immunological disease, and metabolic disease.

The compound of general formula (I) can be used in combination with other drugs known to treat or ameliorate similar conditions. When administered in combination, the mode and dose of administration of the original drug can remain unchanged, while the compound of formula I is administered simultaneously or subsequently. When the compound of formula I is administered in combination with one or more other drugs, a pharmaceutical composition comprising one or more known drugs and the compound of formula I can be preferred. The drug combination also includes administering the compound of formula I and one or more other known drugs over an overlapping period of time. When the compound of formula I is used in combination with one or more other drugs, the dose of the compound of formula I or the known drugs can be lower than that of their administration alone.

The drugs or active ingredients that can be used in combination with the compound of general formula (I) include, but are not limited to: PD-1 inhibitors (such as nivolumab, pembrolizumab, pidilizumab, cemiplimab, JS-001, SHR-120, BGB-A317, IBI-308, GLS-010, GB-226, STW204, HX008, HLX10, BAT 1306, AK105, LZM 009, or a biosimilar thereof), PD-L1 inhibitors (such as durvalumab, atezolizumab, avelumab, CS1001, KN035, HLX20, SHR-1316, BGB-A333, JS003, CS1003, KL -A167, F 520, GR1405, MSB2311, or a biosimilar thereof), CD20 antibodies (such as rituximab, obinutuzumab, ofatumumab, veltuzumab, tositumomab, 1311-tositumomab, ibritumomab, 90Y-ibritumomab, 90In- ibritumomab, ibritumomab tiuxetan, etc.), CD47 antibodies (such as Hu5F9-G4, CC-90002, TTI-621, TTI-622, OSE-172, SRF-231, ALX-148, NI-1701, SHR-1603, IBI188, or IMM01), ALK inhibitors (such as ceritinib, alectinib, brigatinib, lorlatinib, or ocatinib), PI3K inhibitors (such as idelalisib, duvelisib, dactolisib, taselisib, bimiralisib, omipalisib, buparlisib, etc.), BTK inhibitors (such as ibrutinib, tirabrutinib, acalabrutinib, zanubrutinib, vecabrutinib, etc.), EGFR inhibitors (such as afatinib, gefitinib, erlotinib, lapatinib, dacomitinib, icotinib, canertinib, sapitinib, naquotinib, pyrotinib, rociletinib, osimertinib, etc.), VEGFR inhibitors (such as sorafenib, pazopanib, regorafenib, sitravatinib, ningetinib, cabozantinib, sunitinib, donafenib, etc.), HDAC inhibitors (such as givinostat, tucidinostat, vorinostat, fimepinostat, droxinostat, entinostat, dacinostat, quisinostat, tacedinaline, etc.), CDK inhibitors (such as palbociclib, ribociclib, abemaciclib, milciclib, trilaciclib, lerociclib, etc.), MEK inhibitors (such as selumetinib (AZD6244), trametinib (GSK1120212), PD0325901, U0126, pimasertib (AS-703026), PD184352 (CI-1040), etc.), mTOR inhibitors (such as Vistusertib), SHP2 inhibitors (such as RMC-4630, JAB-3068, TNO155, etc.), or a combination thereof.

Dosage forms of the pharmaceutical composition of the present invention include (but are not limited to): an injection, a tablet, a capsule, an aerosol, a suppository, a film, a dropping pill, a liniment for external use, or a controlled-released or sustained-release or nano formulation.

The pharmaceutical composition of the present invention comprises a safe and effective amount of the compound of the present invention or a pharmaceutically acceptable salt thereof and a pharmaceutically acceptable excipient or carrier, wherein the "safe and effective amount" means that the amount of the compound is sufficient to significantly improve the condition without causing serious side effects. Typically, the pharmaceutical composition comprises 1-2000 mg of the compound of the present invention per dose, and more preferably, 10-1000 mg of the compound of the present invention per dose. Preferably, the "dose" is a capsule or a tablet.

The "pharmaceutically acceptable carrier" refers to one or more compatible solid or liquid fillers or gel substances that are suitable for human use and must be of sufficient purity and sufficiently low toxicity. "Compatible" herein means that the components of the composition are capable of intermixing with the compound of the present invention and with each other, without significantly diminishing the pharmaceutical efficacy of the compound. Examples of the pharmaceutically acceptable carrier include cellulose and derivatives thereof (e.g., sodium carboxymethylcellulose, sodium ethylcellulose, cellulose acetate, etc.), gelatin, talc, solid lubricants (e.g., stearic acid or magnesium stearate), calcium sulfate, vegetable oil (e.g., soybean oil, sesame oil, peanut oil, olive oil, etc.), polyols (e.g., propylene glycol, glycerol, mannitol, sorbitol, etc.), emulsifiers (e.g., Tween^{®}), wetting agents (e.g., sodium lauryl sulfate), colorants, flavoring agents, stabilizers, antioxidants, preservatives, pyrogen-free water, etc.

The mode of administration of the compound or the pharmaceutical composition of the present invention is not particularly limited, and representative modes of administration include (but are not limited to): oral, intratumoral, rectal, parenteral (intravenous, intramuscular or subcutaneous), and topical administration.

Solid dosage forms for oral administration include capsules, tablets, pills, pulvises and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol and silicic acid; (b) binders, such as hydroxymethyl cellulose, alginate, gelatin, polyvinylpyrrolidone, sucrose and acacia; (c) humectants, such as glycerol; (d) disintegrants, such as agar, calcium carbonate, potato or tapioca starch, alginic acid, certain complex silicates and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol and sodium lauryl sulfate, or mixtures thereof. In the case of capsules, tablets and pills, the dosage forms may also comprise buffers.

Solid dosage forms such as tablets, dragees, capsules, pills and granules can be prepared using coatings and shells such as enteric coatings and other materials well known in the art. They may comprise opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the above-mentioned excipients.

Liquid dosage forms for oral administration include pharmaceutically acceptable emulsions, solutions, suspensions, syrups and elixirs. In addition to the active compound, the liquid dosage form may comprise inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances.

Besides such inert diluents, the composition may also comprise adjuvants, such as wetting agents, emulsifiers, suspending agents, sweeteners, flavoring agents, and perfuming agents.

Suspensions, in addition to the active compound, may comprise suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may comprise physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents or excipients include water, ethanol, polyols and suitable mixtures thereof.

Dosage forms for topical administration of the compound of the present invention include ointments, pulvises, patches, sprays and inhalants. The active ingredient is mixed under sterile conditions with a physiologically acceptable carrier and any preservatives, buffers or propellants that may be required if necessary.

The treatment method of the present invention can be used alone or in combination with other therapeutic means or drugs.

When the pharmaceutical composition is used, a safe and effective amount of the compound of the present invention is administered to a mammal (such as a human) to be treated, wherein the administration dose is a pharmaceutically effective administration dose. For a human weighing 60 kg, the daily dose of administration is usually 1-2000 mg, preferably 50-1000 mg. In determining a specific dose, such factors as the route of administration, the health condition of the patient and the like will also be considered, which are well known to skilled physicians.

The present invention further provides a method for preparing the pharmaceutical composition, which comprises the step of mixing a pharmaceutically acceptable carrier with the compound of general formula (I), or the crystalline form, the pharmaceutically acceptable salt, the hydrate or the solvate thereof of the present invention to form the pharmaceutical composition.

The present invention further provides a treatment method, which comprises the step of administering to a subject in need of treatment the compound of general formula (I), or the crystalline form, the pharmaceutically acceptable salt, the hydrate or the solvate thereof of the present invention, or the pharmaceutical composition of the present invention to selectively inhibit SOS1.

### Compared to the prior art, the present invention has the following main advantages:

(1) The compound has a good selective inhibitory effect on SOS1;
(2) The compound has better *in vivo* and *in vitro* pharmacodynamic and pharmacokinetic properties and smaller toxic and side effects.

The present invention will be further illustrated with reference to the following specific examples. It should be understood that these examples are merely intended to illustrate the present invention rather than limit the scope of the present invention. Where specific conditions are not indicated in experimental method in the following examples, conventional conditions such as those described in Sambrook et al., Molecular Cloning: A Laboratory Manual (New York: Cold Spring Harbor Laboratory Press, 1989) or conditions recommended by the manufacturer are followed. Unless otherwise indicated, percentages and parts are by weight.

Unless otherwise defined, all technical and scientific terms used herein have the same meaning as commonly understood by those of ordinary skill in the art. In addition, any methods and materials similar or equivalent to those described herein can be used in the methods of the present invention. The preferred embodiments and materials described herein are for illustrative purposes only.

The compound structure of the present invention is determined by nuclear magnetic resonance (NMR) and liquid chromatography-mass spectrometry (LC-MS).

NMR is detected using a Bruker AVANCE-400 nuclear magnetic resonance instrument, and the measuring solvents include deuterated dimethyl sulfoxide (DMSO-d₆), deuterated acetone (CD₃COCD₃), deuterated chloroform (CDCl₃), deuterated methanol (CD₃OD), and the like. The internal standard is tetramethylsilane (TMS), and the chemical shift is measured in parts per million (ppm).

The liquid chromatography-mass spectrometry (LC-MS) is detected using a Waters SQD2 mass spectrometer. HPLC is determined using an Agilent 1100 high pressure chromatograph (Microsorb 5 micron C18 100 × 3.0 mm column).

Qingdao GF254 silica gel plate is used for thin layer chromatography. The specification for TLC is 0.15-0.20 mm, and the specification for preparative thin-layer chromatography is 0.4-0.5 mm. Qingdao 200-300 mesh silica gel is generally used as the carrier in column chromatography.

Starting materials in the examples of the present invention are known and commercially available, or may be synthesized by using or according to the literature reported in the art.

Unless otherwise stated, all reactions in the present invention are carried out in a dry inert gas atmosphere (e.g., nitrogen or argon) with continuous magnetic stirring, and the reaction temperature is Celsius (°C).

### Examples

### Intermediate-1: Preparation of (R)-1-(3-amino-5-(1-aminoethyl)phenyl)-1,1-difluoro-2-methylpropan-2-ol hydrochloride

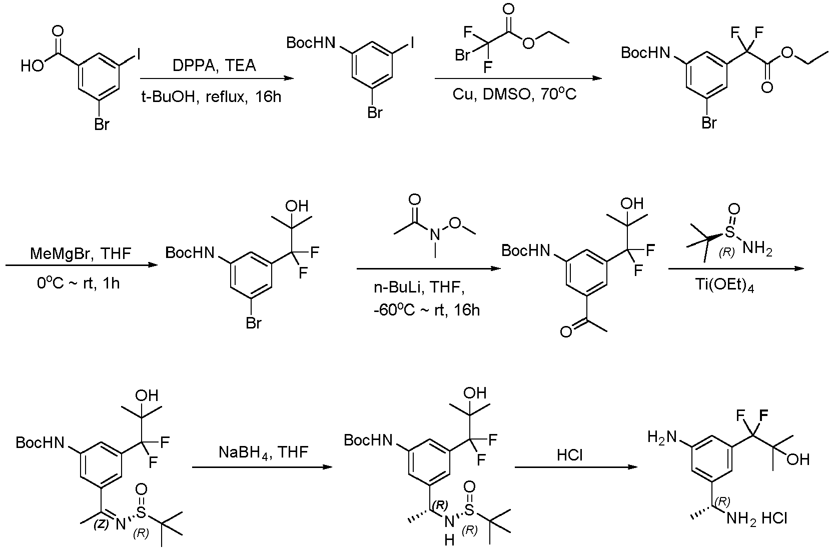

### Step 1: Preparation of tert-butyl (3-bromo-5-iodophenyl)carbamate

3-Bromo-5-iodobenzoic acid (4 g, 12.2 mmol) was added to *tert*-butanol (50 mL), and triethylamine (1.85 g, 18.3 mmol) and triphenylphosphoryl azide (3.7 g, 13.5 mmol) were added. The mixture was refluxed overnight, concentrated to dryness by rotary evaporation, and separated by silica gel column chromatography to give the target product (3.4 g, yield: 71%). LC-MS: m/z 398 (M+H)⁺.

### Step 2: Preparation of ethyl 2-(3-bromo-5-((tert-butoxycarbonyl)amino)phenyl)-2,2-difluoroacetate

*tert*-Butyl (3-bromo-5-iodophenyl)carbamate (3.97 g, 10 mmol) was added to dimethyl sulfoxide (30 mL), and ethyl 2-bromo-2,2-difluoroacetate (5.1 g, 25 mmol) and copper powder (1.6 g, 25 mmol) were added. The mixture was then heated to 70 °C and stirred overnight, poured into water (100 mL), and extracted twice with ethyl acetate (300 mL). The organic phases were combined, dried, concentrated to dryness by rotary evaporation, and separated by silica gel column chromatography to give the target product (3.4 g, yield: 79%).

LC-MS: m/z 394 (M+H)⁺.

### Step 3: Preparation of tert-butyl (3-bromo-5-(1,1-difluoro-2-hydroxy-2-methylpropyl)phenyl)carbamate

Ethyl 2-(3-bromo-5-((tert-butoxycarbonyl)amino)phenyl)-2,2-difluoroacetate (3 g, 7.6 mmol) was added to tetrahydrofuran (30 mL), and methylmagnesium bromide (10.2 mL, 30.5 mmol) was added at 0 °C. After being reacted at room temperature for 1 h, the mixture was poured into ice (50 g), and extracted twice with ethyl acetate (300 mL). The organic phases were combined, dried, concentrated to dryness by rotary evaporation, and separated by silica gel column chromatography to give the target product (2.8 g).

LC-MS: m/z 380 (M+H)⁺.

### Step 4: Preparation of tert-butyl (3-acetyl-5-(1,1-difluoro-2-hydroxy-2-methylpropyl)phenyl)carbamate

*tert*-Butyl (3-bromo-5-(1,1-difluoro-2-hydroxy-2-methylpropyl)phenyl)carbamate (3 g, 7.9 mmol) was added to tetrahydrofuran (30 mL), and *n*-butyllithium (2.5 M, 12.6 mL, 31.6 mmol) was added at -60 °C. After the addition was complete, the mixture was stirred for 0.5 h with the temperature maintained, and then *N*-methoxy-*N*-methylacetamide (3.3 g, 31.6 mmol) was added. The mixture was slowly heated to room temperature, stirred overnight, poured into ice (50 g), and extracted twice with ethyl acetate (300 mL). The organic phases were combined, dried, concentrated to dryness by rotary evaporation, and separated by silica gel column chromatography to give the target product (1.1 g).

LC-MS: m/z 344 (M+H)⁺.

### Step 5: Preparation of (R,Z)-tert-butyl(3-(1-((tert-butylsulfinyl)imino)ethyl)-5-(1,1-difluoro-2-hydroxy-2-methylpr opyl)phenyl)carbamate

*tert*-Butyl (3-acetyl-5-(1,1-difluoro-2-hydroxy-2-methylpropyl)phenyl)carbamate (1 g, 2.9 mmol) was added to tetrahydrofuran (20 mL), and then (*R*)-2-methylpropane-2-sulfinamide (0.53 g, 4.4 mmol) and tetraethyl titanate (2.6 g, 12 mmol) were added. Then, the reaction solution was stirred under reflux overnight, cooled, concentrated, and separated by silica gel column chromatography to give the target product (0.68 g, yield: 52%).

LC-MS: m/z 447 (M+H)⁺.

### Step 6: Preparation of tert-butyl (3-(1,1-difluoro-2-hydroxy-2-methylpropyl)-5-((R)-1-((R)-1,1-dimethylethylsulfinylamino) ethyl)phenyl)carbamate

(*R*,Z)-*tert*-Butyl(3-(1-((*tert*-butylsulfinyl)imino)ethyl)-5-(1,1-difluoro-2-hydroxy-2-methylprop yl)phenyl)carbamate (0.68 g, 1.5 mmol) was added to tetrahydrofuran/water (8 mL/0.16 mL), and sodium borohydride (116 mg, 3.0 mmol) was added at 0 °C. The reaction solution was stirred at room temperature for 0.5 h, and then a saturated ammonium chloride solution (20 mL) was added. The mixture was extracted 2 times with ethyl acetate (60 mL), and the organic phases were combined, dried, concentrated, and separated by silica gel column chromatography to give the target product (600 mg, yield: 88%).

LC-MS: m/z 449 (M+H)⁺.

### Step 7: Preparation of (R)-1-(3-amino-5-(1-aminoethyl)phenyl)-1,1-difluoro-2-methylpropan-2-ol hydrochloride tert-Butyl

(3-(1,1-difluoro-2-hydroxy-2-methylpropyl)-5-((*R*)-1-((*R*)-1,1-dimethylethyl sulfinylamino)ethy l)phenyl)carbamate (600 mg, 1.34 mmol) was added to methanol (3 mL), and a solution of 4 N hydrogen chloride in dioxane (6 mL) was added. Then the reaction solution was stirred at room temperature for 16 h, and concentrated, and the crude product was separated by preparative liquid chromatography to give the target product (188 mg, yield: 50%).

LC-MS: m/z 245 (M+H)⁺. ¹H NMR (400 MHz,DMSO) *δ* 8.37 (brs, 3H), 6.82-6.66 (m, 3H), 5.74-4.94 (m, 2H), 4.30-4.16 (m, 1H), 1.46 (d, *J* = 6.8 Hz, 3H), 1.17 (s, 6H).

### Intermediate-2: Preparation of (R)-1-(2-(1-aminoethyl)pyridin-4-yl)-1,1-difluoro-2-methylpropan-2-ol hydrochloride

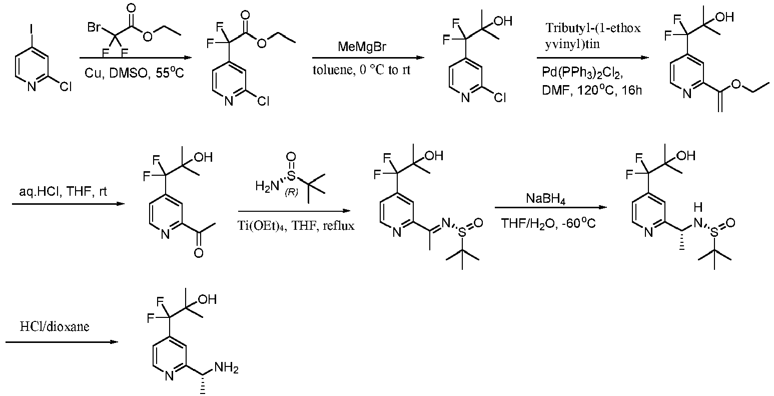

### Step 1: Preparation of ethyl 2-(2-chloropyridin-4-yl)-2,2-difluoroacetate

2-Chloro-4-iodopyridine (4.0 g, 16.7 mmol), ethyl 2-bromo-2,2-difluoroacetate (8.5 g, 41.8 mmol) and active copper powder (2.7 g, 41.8 mmol) were added to dimethyl sulfoxide (30 mL). The mixture was stirred at 55 °C for 16 h in nitrogen atmosphere, cooled to room temperature, diluted with water/ethyl acetate (150 mL/100 mL), stirred, and filtered to remove insoluble solids. The filtrate was separated. The aqueous phase was extracted with ethyl acetate (100 mL). The ethyl acetate layers were combined, washed three times with saturated brine (50 mL), dried, concentrated to dryness by rotary evaporation, and separated by silica gel column chromatography to give the target product (3.5 g, yield: 86%).

LC-MS: m/z 236 (M+H)⁺.

### Step 2: Preparation of 1-(2-chloropyridin-4-yl)-1,1-difluoro-2-methylpropan-2-ol

Ethyl 2-(2-chloropyridin-4-yl)-2,2-difluoroacetate (3.06 g, 13.0 mmol) was added to anhydrous toluene (30 mL). The system was purged 3 times with nitrogen, and methylmagnesium bromide (3 M, 10 mL, 30.0 mmol) was added dropwise in an ice bath. The reaction solution was then stirred at room temperature for 1 h. A saturated ammonium chloride solution (100 mL) was added. The mixture was extracted 2 times with ethyl acetate (50 mL). The organic phases were combined, dried, concentrated, and separated by silica gel column chromatography to give the target product (1.8 g, purity: about 80%, yield: 58%).

LC-MS: m/z 222 (M+H)⁺.

### Step 3: Preparation of 1-(2-(1-ethoxyvinyl)pyridin-4-yl)-1,1-difluoro-2-methylpropan-2-ol

1-(2-Chloropyridin-4-yl)-1,1-difluoro-2-methylpropan-2-ol (1.68 g, 7.6 mmol) was added to *N,N-*dimethylformamide (15 mL), and then tributyl(1-ethoxyvinyl)stannane (3.29 g, 9.1 mmol) and bis(triphenylphosphine)palladium(II) dichloride (266 mg, 0.38 mmol) were added. The system was purged with nitrogen. The reaction solution was stirred at 120 °C overnight, then cooled to room temperature, poured into water/ethyl acetate (50 mL/50 mL), and filtered under reduced pressure through celite to remove flocculent black solids. The filtrate was separated, and the aqueous phase was extracted twice with ethyl acetate (30 mL). The organic phases were combined, washed three times with saturated brine (30 mL), dried, and concentrated to give the target product, which was used directly in the next step without purification.

LC-MS: m/z 258 (M+H)⁺.

### Step 4: Preparation of 1-(4-(1,1-difluoro-2-hydroxy-2-methylpropyl)pyridin-2-yl)ethanone

1-(2-(1-Ethoxyvinyl)pyridin-4-yl)-1,1-difluoro-2-methylpropan-2-ol (crude) obtained in the last step was dissolved in tetrahydrofuran (30 mL), and then a 2 M aqueous hydrochloric acid solution (15 mL) was added. The reaction solution was then stirred at room temperature for 1 h, adjusted to pH = 8 with saturated sodium bicarbonate, and extracted twice with ethyl acetate (50 mL). The organic phases were combined, dried and concentrated. The residue was separated by silica gel column chromatography to give the target product (1.29 g, yield over two steps: 70%).

LC-MS: m/z 230 (M+H)⁺.

### Step 5: Preparation of (R,E)-N-(1-(4-(1,1-difluoro-2-hydroxy-2-methyl-2-methylpropyl)pyridin-2-yl)ethylidene)-2 -methylpropane-2-sulfinamide

1-(4-(1,1-Difluoro-2-hydroxy-2-methylpropyl)pyridin-2-yl)ethanone (1.07 g, 4.69 mmol) was added to tetrahydrofuran (20 mL), and then (R)-2-methylpropane-2-sulfinamide (850 mg, 7.03 mmol) and tetraethyl titanate (4.3 g, 18.76 mmol) were added. Then, the reaction solution was stirred under reflux for 1.5 h, cooled, concentrated, and separated by silica gel column chromatography to give the target product (455 mg, yield: 29%).

LC-MS: m/z 333 (M+H)⁺.

### Step 6: Preparation of (R)-N-((R)-1-(4-(1,1-difluoro-2-hydroxy-2-methylpropyl)pyridin-2-yl)ethyl)-2-methylprop ane-2-sulfinamide

(*R,E*)*-N-*(1*-*(4-(1,1-Difluoro-2-hydroxy-2-methyl-2-methylpropyl)pyridin-2-yl)ethylidene)-2-m ethylpropane-2-sulfinamide (455 mg, 1.37 mmol) was dissolved in tetrahydrofuran/water (7 mL/0.14 mL), and sodium borohydride (78 mg, 2.06 mmol) was added in batches at -50 °C. The reaction solution was slowly heated to room temperature. Half-saturated brine (30 mL) was added. The mixture was extracted 3 times with ethyl acetate (20 mL). The organic phases were combined, dried, and concentrated. The residue was separated by silica gel column chromatography to give the target product (290 mg, yield: 56%).

LC-MS: m/z 335 (M+H)⁺.

### Step 7: Preparation of (R)-1-(2-(1-aminoethyl)pyridin-4-yl)-1,1-difluoro-2-methylpropan-2-ol hydrochloride

(*R*)-*N-*((*R*)-1-(4-(1,1-Difluoro-2-hydroxy-2-methylpropyl)pyridin-2-yl)ethyl)-2-methylpropane-2-sulfinamide (290 mg, 0.87 mmol) was dissolved in methanol (2 mL), and a solution of hydrogen chloride/dioxane (4 M, 4 mL) was added. The reaction solution was stirred at room temperature for 16 h, and concentrated to dryness, and the crude product was separated by preparative liquid chromatography to give the target product (179 mg, yield: 77%).

LC-MS: 231 m/z (M+H)⁺. ¹H NMR (400 MHz, DMSO) δ 8.73 (d, *J* = 4.8 Hz, 1H), 8.60 (brs, 3H), 7.65 (s, 1H), 7.49 (d, *J* = 5.2 Hz, 1H), 5.52 (s, 1H), 4.60 (q, *J* = 6.4 Hz, 1H), 1.52 (d, *J*= 7.2 Hz, 3H), 1.19 (s, 6H).

**The following compounds were synthesized in the same manner as intermediate-2 with different starting materials:**

### Intermediate-3: Preparation of (R)-1-(6-(1-aminoethyl)pyridin-2-yl)-1,1-difluoro-2-methylpropan-2-ol hydrochloride

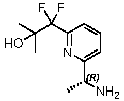

LC-MS: 231 m/z (M+H)⁺. ¹H NMR (400 MHz, DMSO) *δ* 8.61 (s, 3H), 8.04-8.00 (m, 1H), 7.70 (d, *J* = 8.0 Hz, 1H), 7.59 (d, *J* = 7.6 Hz, 1H), 5.28 (s, 1H), 4.54 (m, 1H), 1.56 (d, *J* = 6.8 Hz, 3H), 1.24 (s, 6H).

### Intermediate-4: Preparation of (R)-1-(4-(1-aminoethyl)pyridin-2-yl)-1,1-difluoro-2-methylpropan-2-ol hydrochloride

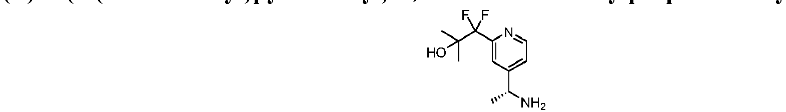

LC-MS: 231 m/z (M+H)⁺. ¹H NMR (400 MHz, DMSO) δ 8.86 (brs, 3H), 8.70 (d, *J* = 5.2 Hz, 1H), 7.77 (s, 1H), 7.73 (d, *J* = 5.2 Hz, 1H), 4.54-4.50 (m, 1H), 1.53 (d, *J* = 6.8 Hz, 3H), 1.23 (s, 6H).

### Intermediate-5: Preparation of (R)-1-(5-(1-aminoethyl)pyridin-3-yl)-1,1-difluoro-2-methylpropan-2-ol hydrochloride

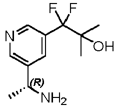

**LC-MS: 231 m/z**

### Intermediate-6: Preparation of (R)-1-(3-(1-aminoethyl)-4-fluorophenyl)-1,1-difluoro-2-methylpropan-2-ol hydrochloride

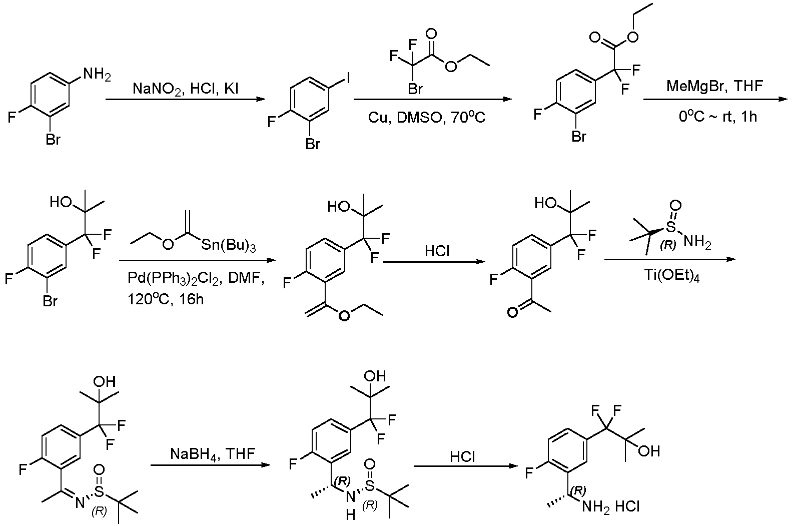

### Step 1: Preparation of 2-bromo-1-fluoro-4-iodobenzene

3-Bromo-4-fluoroaniline (5 g, 26.5 mmol) was added to acetonitrile/water (50 mL/8 mL), and concentrated hydrochloric acid (11 mL) and sodium nitrite (2 g, 29.1 mmol) were added at 0 °C. The mixture was reacted for 0.5 h, and then a solution of potassium iodide (6.6 g, 39.8 mmol)/water (15 mL) was added. The mixture was stirred at room temperature for 3 h, poured into water (100 mL), and extracted twice with ethyl acetate (300 mL). The organic phases were combined, dried, concentrated to dryness by rotary evaporation, and separated by silica gel column chromatography to give the target product (15.8 g, yield: 100%).

### Step 2: Preparation of ethyl 2-(3-bromo-4-fluorophenyl)-2,2-difluoroacetate

2-Bromo-1-fluoro-4-iodobenzene (15.8 g, 52.7 mmol) was added to dimethyl sulfoxide (110 mL), and ethyl 2-bromo-2,2-difluoroacetate (26.8 g, 131.6 mmol) and copper powder (8.4 g, 131.6 mmol) were added. The mixture was then heated to 70 °C and stirred overnight, poured into water (300 mL), and extracted twice with ethyl acetate (800 mL). The organic phases were combined, dried, concentrated to dryness by rotary evaporation, and separated by silica gel column chromatography to give the target product (9.8 g, yield: 63%).

¹H NMR (400 MHz, CDCl₃) *δ* 7.83 (dd, *J* = 6.4 Hz, 2.0 Hz; 1H), 7.61-7.51 (m, 1H), 7.20 (t, *J* = 8.4 Hz; 1H), 4.32 (q, *J* = 7.2 Hz; 2H), 1.32 (t, *J* = 6.8 Hz; 3H).

### Step 3: Preparation of 1-(3-bromo-4-fluorophenyl)-1,1-difluoro-2-methylpropan-2-ol

Ethyl 2-(3-bromo-4-fluorophenyl)-2,2-difluoroacetate (9.8 g, 33 mmol) was added to tetrahydrofuran (150 mL), and methylmagnesium bromide (33 mL, 99 mmol) was added at 0 °C. After being reacted at room temperature for 1 h, the mixture was poured into ice (100 g), and extracted twice with ethyl acetate (400 mL). The organic phases were combined, dried, concentrated to dryness by rotary evaporation, and separated by silica gel column chromatography to give the target product (9 g, yield: 97%).

¹H NMR (400 MHz, CDCl₃) *δ* 7.74 (dd, *J* = 6.4 Hz, 2.0 Hz; 1H), 7.54-7.43 (m, 1H), 7.16 (t, *J* = 8.4 Hz; 1H), 1.32-1.29 (m, 6H). **Step 4: Preparation of 1-(3-(1-ethoxyvinyl)-4-fluorophenyl)-1,1-difluoro-2-methylpropan-2-ol**
1-(3-Bromo-4-fluorophenyl)-1,1-difluoro-2-methylpropan-2-ol (9.3 g, 32.9 mmol) was added to *N,N-*dimethylformamide (100 mL), and tributyl(1-ethoxyvinyl)stannane (14.2 g, 39.4 mmol) and bis(triphenylphosphine)palladium(II) dichloride (1.2 g, 1.65 mmol) were added. After the addition was complete, the mixture was heated to 120 °C and stirred for 16 h in nitrogen atmosphere. After the reaction was complete, the mixture was cooled, poured into water (300 mL), and extracted twice with ethyl acetate (600 mL). The organic phases were combined, dried, and concentrated to dryness by rotary evaporation to give the target product, which was used directly in the next step.

### Step 5: Preparation of 1-(5-(1,1-difluoro-2-hydroxy-2-methylpropyl)-2-fluorophenyl)ethanone

1-(3-(1-Ethoxyvinyl)-4-fluorophenyl)-1,1-difluoro-2-methylpropan-2-ol (crude) was added to tetrahydrofuran (60 mL), and then an aqueous hydrochloric acid solution (20 mL, 4 mol/L) was added. The mixture was stirred at room temperature for 1 h, then adjusted to pH = 7.0-8.0 with a saturated aqueous sodium bicarbonate solution, and extracted twice with ethyl acetate (600 mL). The organic phases were combined, dried, concentrated to dryness by rotary evaporation, and separated by silica gel column chromatography to give the target product (6.6 g, yield over two steps: 81%).

LC-MS: m/z 247 (M+H)⁺.

### Step 6: Preparation of (R,Z)-N-(1-(5-(1,1-difluoro-2-hydroxy-2-methyl-2-methylpropyl)-2-fluorophenyl)ethyliden e)-2-methylpropane-2-sulfinamide

1-(5-(1,1-Difluoro-2-hydroxy-2-methylpropyl)-2-fluorophenyl)ethanone (6.6 g, 26.8 mmol) was added to tetrahydrofuran (70 mL), and then (*R*)-2-methylpropane-2-sulfinamide (4.9 g, 40.2 mmol) and tetraethyl titanate (23.9 g, 107 mmol) were added. Then, the reaction solution was stirred at 60 °C overnight, cooled, concentrated, and separated by silica gel column chromatography to give the target product (6.4 g, yield: 68%).

LC-MS: m/z 350 (M+H)⁺.

### Step 7: Preparation of (R)-N-((R)-1-(5-(1,1-difluoro-2-hydroxy-2-methylpropyl)-2-fluorophenyl)ethyl)-2-methylpropa ne-2-sulfinamide

(*R,Z*)-*N*-(1-(5-(1,1-Difluoro-2-hydroxy-2-methyl-2-methylpropyl)-2-fluorophenyl)ethylidene)-2 -methylpropane-2-sulfinamide (6.3 g, 18.1 mmol) was added to tetrahydrofuran/water (80 mL/1.6 mL), and sodium borohydride (1.4 g, 36.2 mmol) was added at 0 °C. The reaction solution was stirred at room temperature for 0.5 h, and then a saturated ammonium chloride solution (100 mL) was added. The mixture was extracted 2 times with ethyl acetate (300 mL), and the organic phases were combined, dried, concentrated, and separated by silica gel column chromatography to give the target product (4.1 g, yield: 64%).

LC-MS: m/z 352 (M+H)⁺.

### Step 8: Preparation of (R)-1-(3-(1-aminoethyl)-4-fluorophenyl)-1,1-difluoro-2-methylpropan-2-ol hydrochloride

(*R*)-*N*-((*R*)-1-(5-(1,1-Difluoro-2-hydroxy-2-methylpropyl)-2-fluorophenyl)ethyl)-2-methylprop ane-2-sulfinamide (4 g, 11.4 mmol) was added to methanol (20 mL), and a solution of 4 N hydrogen chloride in dioxane (20 mL) was added. The reaction solution was then stirred at room temperature for 16 h, and concentrated, and the residue was separated by preparative liquid chromatography to give the target product (2.32 g, yield: 82%).

LC-MS: m/z 248 (M+H)⁺. ¹H NMR (400 MHz, DMSO) *δ* 8.70 (brs, 3H), 7.79 (dd, *J* = 7.2 Hz, 2.4 Hz; 1H), 7.56-7.49 (m, 1H), 7.37 (t, *J* = 9.6 Hz; 1H), 5.33 (s, 1H), 4.63 (q, *J* = 6.4 Hz; 1H), 1.53 (d, *J* = 6.8 Hz, 3H), 1.18 (s, 6H).

The following compounds were synthesized in the same manner as intermediate-6 with different starting materials:

### Intermediate-7: Preparation of (R)-1-(3-(1-aminoethyl)-5-fluorophenyl)-1,1-difluoro-2-methylpropan-2-ol hydrochloride

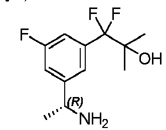

LC-MS: m/z 248 (M+H)⁺. ¹H NMR (400 MHz, DMSO) δ 8.72 (s, 3H), 7.62 (d, *J* = 9.7 Hz, 1H), 7.49 (s, 1H), 7.27 (d, *J* = 9.4 Hz, 1H), 4.52-4.47 (m, 1H), 1.53 (d, *J* = 6.8 Hz, 3H), 1.18 (s, 6H).

### Intermediate-8: Preparation of (R)-1-(5-(1-aminoethyl)-2-fluorophenyl)-1,1-difluoro-2-methylpropan-2-ol

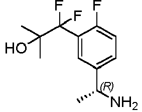

LC-MS: m/z 248 (M+H)⁺. ¹H NMR (400 MHz, DMSO) δ 8.57 (brs, 3H), 7.72 (s, 1H), 7.62-7.60 (m, 1H), 7.35 (dd, *J* = 10.8 Hz, 8.8 Hz, 1H), 5.38 (s, 1H), 4.50-4.44 (m, 1H), 1.51 (d, *J* = 6.8 Hz, 3H), 1.21 (s, 6H).

### Intermediate-9: Preparation of (R)-1-(3-(1-aminoethyl)-5-methoxyphenyl)-1,1-difluoro-2-methylpropan-2-ol hydrochloride

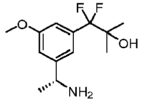

LC-MS: m/z 260 (M+H)⁺. ¹H NMR (400 MHz, DMSO) δ 8.67 (s, 3H), 7.36 (s, 1H), 7.18 (s, 1H), 6.98 (s, 1H), 5.31 (brs, 1H), 4.44-4.40 (m, 1H), 3.81 (s, 3H), 1.53 (d, *J* = 6.8 Hz, 3H), 1.18 (s, 6H).

### Intermediate-10: Preparation of (R)-1-(3-(1-aminoethyl)-2-chlorophenyl)-1,1-difluoro-2-methylpropan-2-ol hydrochloride

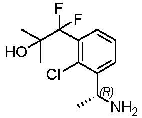

LC-MS: m/z 264 (M+H)⁺. ¹H NMR (400 MHz, DMSO) *δ* 8.74 (brs, 3H), 7.90 (dd, *J* = 8.8 Hz, 3.2 Hz, 1H), 7.65-7.54 (m, 2H), 5.45 (s, 1H), 4.63 (q, *J* = 8.8 Hz, 1H), 1.54 (d, *J* = 8.8 Hz, 3H), 1.27 (s, 6H).

### Intermediate-11: Preparation of (R)-1-(3-(1-aminoethyl)-2-methylphenyl)-1,1-difluoro-2-methylpropan-2-ol

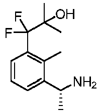

LC-MS: m/z 244 (M+H)⁺. ¹H NMR (400 MHz, DMSO) δ 8.49 (brs, 3H), 7.69 (d, *J=* 7.2 Hz, 1H), 7.41-7.34 (m, 2H), 4.71-4.65 (m, 1H), 3.57 (s, 1H), 2.46 (s, 3H), 1.48 (d, *J=* 6.8 Hz, 3H), 1.22 (s, 6H).

### Intermediate-12: Preparation of (R)-3-(1-aminoethyl)-5-(1,1-difluoro-2-hydroxy-2-methylpropyl)benzonitrile

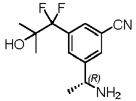

LC-MS: m/z 255 (M+H)⁺. ¹H NMR (400 MHz, DMSO) δ 7.97 (s, 1H), 7.81 (s, 1H), 7.72 (s, 1H), 5.40 (s, 1H), 4.14 (q, *J* = 6.6 Hz, 1H), 1.28 (d,*J*= 6.6 Hz, 3H), 1.17 (s, 6H).

### Intermediate-13: Preparation of 2-(1-aminoethyl)-6-(1-methylcyclopropyl)pyridin-4-amine

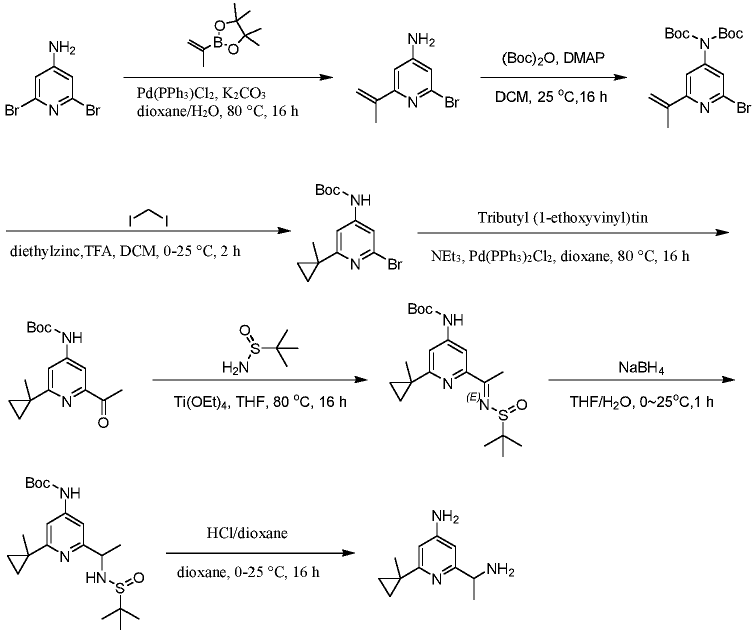

### Step 1: Preparation of 2-bromo-6-(prop-1-en-2-yl)pyridin-4-amine

To a solution of 2,6-dibromopyridin-4-amine (19.0 g, 75.4 mmol, 1.00 eq) and 4,4,5,5-tetramethyl-2-(prop-1-en-2-yl)-1,3,2-dioxaborolane (25.4 g, 151 mmol, 2.00 eq) in dioxane (150 mL) and H₂O (30 mL) were added K₂CO₃ (31.3 g, 226 mmol, 3.00 eq) and Pd(PPh₃)₂Cl₂ (3.71 g, 5.28 mmol, 0.07 eq) in nitrogen atmosphere. The reaction solution was reacted at 80 °C for 16 h. The resulting reaction solution was quenched with water (500 mL) and then extracted with EtOAc (500 mL × 2). The combined organic phase was washed with saturated brine (300 mL), dried over anhydrous Na₂SO₄ and then filtered. The filtrate was concentrated under reduced pressure, and the residue was separated by silica gel column chromatography to give the target product (8.00 g, 37.6 mmol, yield: 49.8%).

LC-MS: m/z 213 (M+H)⁺.

### Step 2: Preparation of tert-butyl (2-bromo-6-(prop-1-en-2-yl)pyridin-4-yl)(tert-butoxycarbonyl)carbamate

To a solution of 2-bromo-6-(prop-1-en-2-yl)pyridin-4-amine (8.00 g, 37.6 mmol, 1.00 eq) in DCM (80 mL) were added (Boc)₂O (32.8 g, 150 mmol, 34.5 mL, 4.00 eq) and DMAP (1.38 g, 11.3 mmol, 0.30 eq). The reaction solution was reacted at 25 °C for 16 h and then concentrated under reduced pressure. The residue was separated by silica gel column chromatography to give the target product (8.00 g, 19.4 mmol, yield: 51.6%).

LC-MS: m/z 413 (M+H)⁺.

### Step 3: Preparation of tert-butyl (2-bromo-6-(1-methylcyclopropyl)pyridin-4-yl)carbamate

To diethyl zinc (1.00 M, 38.7 mL, 4.00 *eq)* in DCM (20 mL) was added TFA (4.41 g, 38.7 mmol, 2.87 mL, 4.00 *eq)* at 0 °C in nitrogen atmosphere. The reaction solution was reacted at 0 °C for 15 min. A solution of CH₂I₂ (10.4 g, 38.7 mmol, 3.12 mL, 4.00 *eq)* in DCM (20 mL) was then added dropwise at 0 °C. The reaction solution was reacted at 0 °C for 20 min. A solution of *tert*-butyl (2-bromo-6-(prop-1-en-2-yl)pyridin-4-yl)(*tert*-butoxycarbonyl)carbamate (4.00 g, 9.68 mmol, 1.00 *eq*) in DCM (20 mL) was then added at 0 °C. The reaction solution was reacted at 20 °C for 5 h. The resulting reaction solution was quenched with water (200 mL) and then extracted with DCM (100 mL × 2). The combined organic phase was washed with saturated brine (100 mL), dried over anhydrous Na₂SO₄ and then filtered. The filtrate was concentrated under reduced pressure, and the residue was separated by silica gel column chromatography to give the target product (1.08 g, 2.64 mmol, yield: 17.0%, purity: 80.0%).

LC-MS: m/z 327 (M+H)⁺.

### Step 4: Preparation of tert-butyl (2-acetyl-6-(1-methylcyclopropyl)pyridin-4-yl)carbamate

To a solution of *tert*-butyl (2-bromo-6-(1-methylcyclopropyl)pyridin-4-yl)carbamate (1.05 g, 3.21 mmol, 1.00 eq) in dioxane (5 mL) were added tributyl(1-ethoxyvinyl)tin (1.43 g, 3.96 mmol, 1.34 mL, 1.24 eq), TEA (649 mg, 6.42 mmol, 893 µL, 2.00 eq) and Pd(PPh₃)₂Cl₂ (113 mg, 160 µmol, 0.05 eq) in nitrogen atmosphere. The reaction solution was reacted at 60 °C for 16 h. The resulting reaction solution was quenched with 1 N HCl (50 mL) and then extracted with EtOAc (200 mL). The combined organic phase was washed with saturated brine (100 mL), dried over anhydrous Na₂SO₄ and then filtered. The filtrate was concentrated under reduced pressure, and the residue was separated by silica gel column chromatography to give the target product (560 mg, 1.93 mmol, yield: 60.1%).

LC-MS: m/z 291 (M+H)⁺.

### Step 5: Preparation of tert-butyl (E)-(2-(1-((tert-butylsulfinyl)imino)ethyl)-6-(1-methylcyclopropyl)pyridin-4-yl)carbamate

To *tert*-butyl (2-acetyl-6-(1-methylcyclopropyl)pyridin-4-yl)carbamate (0.56 g, 1.93 mmol, 1.00 eq) and *tert*-butylsulfinamide (351 mg, 2.90 mmol, 1.50 eq) in THF (5 mL) was added Ti(OEt)₄ (1.10 g, 4.82 mmol, 999 µL, 2.50 eq). The reaction solution was reacted at 80 °C for 16 h and then concentrated under reduced pressure. The residue was separated by silica gel column chromatography to give the target product (440 mg, 1.01 mmol, yield: 52.1%, purity: 90.0%).

LC-MS: m/z 394 (M+H)⁺.

### Step 6: Preparation of tert-butyl (2-(1-((tert-butylsulfinyl)amino)ethyl)-6-(1-methylcyclopropyl)pyridin-4-yl)carbamate

To a solution of *tert*-butyl (*E*)-(2-(1-((*tert*-butylsulfinyl)imino)ethyl)-6-(1-methylcyclopropyl)pyridin-4-yl)carbamate (440 mg, 1.12 mmol, 1.00 eq) in THF (5 mL) and H₂O (0.1 mL) was added NaBH₄ (46.5 mg, 1.23 mmol, 1.10 eq) at 0 °C. The reaction solution was reacted at 25 °C for 1 h. The resulting reaction solution was quenched with water (20 mL) and then extracted with EtOAc (100 mL). The combined organic phase was washed with saturated brine (30 mL), dried over anhydrous Na₂SO₄ and then filtered. The filtrate was concentrated under reduced pressure, and the residue was separated by silica gel column chromatography to give the target product (300 mg, 758 µmol, yield: 67.8%).

LC-MS: m/z 396 (M+H)⁺.

### Step 7: Preparation of 2-(1-aminoethyl)-6-(1-methylcyclopropyl)pyridin-4-amine

To a solution of *tert*-butyl (2-(1-((*tert*-butylsulfinyl)amino)ethyl)-6-(1-methylcyclopropyl)pyridin-4-yl)carbamate (300 mg, 758 µmol, 1.00 eq) in dioxane (1 mL) was added HCl/dioxane (1 mL) at 0 °C. The reaction solution was reacted at 25 °C for 16 h and then filtered. The filter cake was dried *in vacuo* to give the target product as a white solid (165 mg, 647 µmol, yield: 85.4%, purity: 89.3%). LC-MS: m/z 192 (M+H)⁺. ¹H NMR (400MHz, CD₃OD) δ 6.91 (d, *J* = 2.3 Hz, 1H), 6.78 (d, *J* = 2.2 Hz, 1H), 4.78 - 4.65 (m, 1H), 4.78 - 4.65 (m, 1H), 1.75 - 1.71 (m, 1H), 1.75 - 1.71 (m, 1H), 1.73 (d, *J* = 7.0 Hz, 4H), 1.54 - 1.50 (m, 1H), 1.51 (s, 3H), 1.15 - 1.10 (m, 2H), 1.01 - 0.95 (m, 2H).

### Intermediate-14: Preparation of 2-(1-aminoethyl)-6-(1-fluorocyclopropyl)pyridin-4-amine

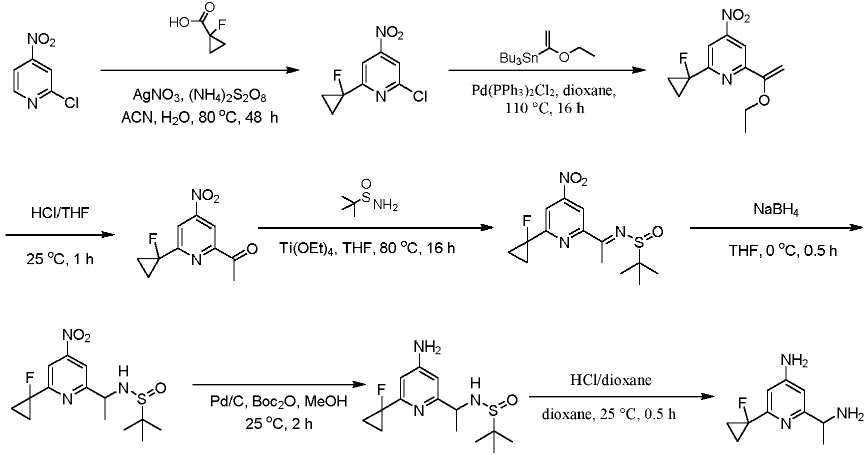

### Step 1: Preparation of 2-chloro-6-(1-fluorocyclopropyl)-4-nitropyridine

A mixture of 2-chloro-4-nitropyridine (16.0 g, 101 mmol, 1.00 *eq),* 1-fluorocyclopropane-1-carboxylic acid (13.7 g, 131 mmol, 1.30 *eq)* and AgNO₃ (3.43 g, 20.2 mmol, 0.200 *eq)* in ACN (50.0 mL) and H₂O (65.0 mL) was heated to 80 °C, followed by addition of a solution of (NH₄)₂S₂O₈ (46.0 g, 202 mmol, 43.9 mL, 2.00 *eq)* in H₂O (65.0 mL). The reaction solution was reacted at 80 °C for 48 h. The resulting reaction solution was quenched with a 2 M NaOH solution (500 mL) and then extracted with EtOAc (500 mL). The combined organic phase was dried over anhydrous MgSO₄ and then filtered. The filtrate was concentrated under reduced pressure, and the residue was separated by silica gel column chromatography to give the target product (2.50 g, 11.5 mmol, yield: 11.4%).

¹H NMR (400MHz, CDCl₃) δ 8.55 - 8.62 (m, 1 H) 7.45 (d, *J* =5.25 Hz, 1 H) 1.48 - 1.55 (m, 2 H) 0.87 - 0.95 (m, 2 H).

### Step 2: Preparation of 2-(1-ethoxyvinyl)-6-(1-fluorocyclopropyl)-4-nitropyridine

A solution of 2-chloro-6-(1-fluorocyclopropyl)-4-nitropyridine (2.37 g, 11.0 mmol, 1.00 *eq),* tributyl(1-ethoxyvinyl)tin (7.00 g, 19.4 mmol, 6.54 mL, 1.75 *eq),* and Pd(PPh₃)₂Cl₂ (778 mg, 1.11 mmol, 0.100 *eq)* in dioxane (25.0 mL) was reacted at 110 °C for 16 h in nitrogen atmosphere. The resulting reaction solution was quenched with H₂O (60 mL) and then extracted with EtOAc (60 mL). The combined organic phase was washed with saturated brine (100 mL), dried over anhydrous Na₂SO₄ and then filtered. The filtrate was concentrated under reduced pressure, and the residue was separated by silica gel column chromatography to give the target product (1.66 g, 6.58 mmol, yield: 59.4%).

¹H NMR (400MHz, CDCl₃) δ 8.77 (dd, *J*=5.13, 1.25 Hz, 1 H) 7.49 (d, *J*=5.13 Hz, 1 H) 4.51 - 4.69 (m, 2 H) 3.94 (q, J=7.00 Hz, 2 H) 1.30 - 1.38 (m, 5 H) 0.77 - 0.85 (m, 2 H).

### Step 3: Preparation of 1-(6-(1-fluorocyclopropyl)-4-nitropyridin-2-yl)ethan-1-one

To a solution of 2-(1-ethoxyvinyl)-6-(1-fluorocyclopropyl)-4-nitropyridine (1.63 g, 6.46 mmol, 1.00 *eq)* in THF (5.00 mL) was added an aqueous HCl solution (2.00 M, 4.85 mL, 1.50 *eq).* The reaction solution was reacted at 25 °C for 1 h. The resulting reaction solution was quenched with H₂O (30 mL) and then extracted with EtOAc (50 mL). The combined organic phase was washed with saturated brine (100 mL), dried over anhydrous Na₂SO₄ and then filtered. The filtrate was concentrated under reduced pressure, and the residue was separated by silica gel column chromatography to give the target product (1.44 g, 6.26 mmol, yield: 96.8%, purity: 97.4%).

¹H NMR (400MHz, CDCl₃) δ (dd, *J*=5.14, 1.38 Hz, 1 H) 7.69 (d, *J*=5.27 Hz, 1 H) 2.74 (s, 3 H) 1.45 - 1.55 (m, 2 H) 0.77 - 0.86 (m, 2 H).

### Step 4: Preparation of (E)-N-(1-(6-(1-fluorocyclopropyl)-4-nitropyridin-2-yl)ethylene)-2-methylpropane-2-sulfoni mide

To 1-(6-(1-fluorocyclopropyl)-4-nitropyridin-2-yl)ethan-1-one (1.24 g, 5.53 mmol, 1.00 *eq*) and *tert*-butylsulfinamide (1.01 g, 8.30 mmol, 1.50 *eq*) in THF (15.0 mL) was added Ti(OEt)₄ (5.05 g, 22.1 mmol, 4.59 mL, 4.00 *eq).* The reaction solution was reacted at 80 °C for 16 h. The resulting reaction solution was quenched with H₂O (200 mL) and then extracted with EtOAc (300 mL). The combined organic phase was dried over anhydrous Na₂SO₄ and then filtered. The filtrate was concentrated under reduced pressure, and the residue was separated by silica gel column chromatography to give the target product (750 mg, 2.44 mmol, yield: 44.1%).

¹H NMR (400MHz, CDCl₃) δ 8.89 (dd, *J*=5.02, 1.00 Hz, 1 H) 7.57 - 7.70 (m, 1 H) 2.65 - 2.87 (m, 3 H) 1.40 - 1.55 (m, 2 H) 1.31 (d, *J*=12.30 Hz, 9 H) 0.84 (dd, *J*=9.03, 2.51 Hz, 2 H).

### Step 5: Preparation of N-(1-(6-(1-fluorocyclopropyl)-4-nitropyridin-2-yl)ethyl)-2-methylpropane-2-sulfinamide

To a solution of (*E*)*-N-*(1-(6-(1-fluorocyclopropyl)-4-nitropyridin-2-yl)ethylene)-2-methylpropane-2-sulfonimid e (730 mg, 2.23 mmol, 1.00 *eq)* in THF (8.00 mL) and H₂O (0.100 mL) was added NaBH₄ (126 mg, 3.34 mmol, 1.50 *eq).* The reaction solution was reacted at 0 °C for 0.5 h. At 0 °C, the resulting reaction solution was quenched with water (20 mL) and then extracted with EtOAc (20 mL). The combined organic phase was dried over anhydrous Na₂SO₄ and then filtered. The filtrate was concentrated under reduced pressure, and the residue was separated by silica gel column chromatography to give the target product as a yellow solid (0.300 g, 911 µmol, yield: 40.8%).

¹H NMR (400MHz, CDCl₃) δ 8.80 (br s, 1 H) 7.40 (br s, 1 H) 5.35 (br d, *J*=6.50 Hz, 1 H) 4.90 (br d, *J*=9.01 Hz, 1 H) 1.62 (br s, 5 H) 1.26 (br s, 9 H) 0.82 - 0.99 (m, 2 H).

### Step 6: Preparation of N (1-(4-amino-6-(1-fluorocyclopropyl)pyridin-2-yl)ethyl)-2-methylpropane-2-sulfinamide

To a solution of *N-*(1-(6-(1-fluorocyclopropyl)-4-nitropyridin-2-yl)ethyl)-2-methylpropane-2-sulfinamide (200 mg, 607 µmol, 1.00 *eq)* and Boc₂O (159 mg, 729 µmol, 167 µL, 1.20 *eq)* in MeOH (2.00 mL) was added Pd/C (607 µmol, 2.00 mL, purity: 10.0%, 1.00 *eq)* in nitrogen atmosphere. The reaction solution was reacted at 25 °C for 2 h in hydrogen atmosphere and then concentrated under reduced pressure. The residue was separated by silica gel column chromatography to give the target product (140 mg, 468 µmol, yield: 77.0%).

LC-MS: m/z 300 (M+H)⁺.

### Step 7: Preparation of 2-(1-aminoethyl)-6-(1-fluorocyclopropyl)pyridin-4-amine

To a solution of N (1-(4-amino-6-(1-fluorocyclopropyl)pyridin-2-yl)ethyl)-2-methylpropane-2-sulfinamide (120 mg, 401 µmol, 1.00 *eq)* in dioxane (1.00 mL) was added HCl/dioxane (1.80 mL). The reaction solution was reacted at 25 °C for 0.5 h, and then concentrated under reduced pressure to give the target product (52.0 mg, 259 µmol, yield: 64.6%, purity: 97.1%).

LC-MS: m/z 196 (M+H)⁺. ¹H NMR (400MHz, DMSO) δ 9.02 - 9.39 (m, 3 H) 8.23 (d, *J*=6.85 Hz, 1 H) 6.99 (d, *J*=6.85 Hz, 1 H) 5.20 (br d, *J*=5.50 Hz, 1 H) 1.68 (br d, *J*=6.85 Hz, 3 H) 1.56 - 1.66 (m, 2 H) 1.27 - 1.39 (m, 1 H) 0.95 (br s, 1 H).

### Intermediate-15: Preparation of (R)-5-(7-bromo-4-((1-(3-(1,1-difluoro-2-hydroxy-2-methylpropyl)-2-fluorophenyl)ethyl)am ino)-8-fluoro-2-methylquinazolin-6-yl)-1-methylpyridin-2(1H)-one

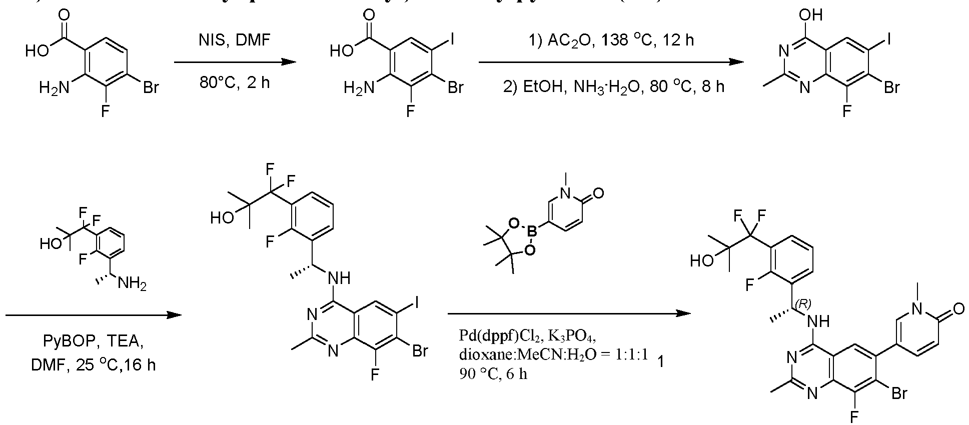

### Step 1: Preparation of 2-amino-4-bromo-3-fluoro-5-iodobenzoic acid

To a solution of 2-amino-4-bromo-3-fluorobenzoic acid (2.00 g, 8.55 mmol, 1.00 eq) in DMF (20.0 mL) was added NIS (2.12 g, 9.40 mmol, 1.10 eq). The resulting mixture was reacted at 80 °C for 2 h, then quenched with water (100 mL) and filtered. The filter cake was collected and dried to give the target product (2.20 g, yield: 67.2%), which was used directly in the next step without purification.

LC-MS: m/z 360 (M+H)⁺.

### Step 2: Preparation of 7-bromo-8-fluoro-6-iodo-2-methylquinazolin-4-ol

A solution of 2-amino-4-bromo-3-fluoro-5-iodobenzoic acid (5.20 g, 14.5 mmol, 1.00 eq) in Ac₂O (50 mL) was reacted at 138 °C for 12 h, and then concentrated under reduced pressure. The residue was reacted with a mixed solvent of EtOH (50 mL) and NH₃.H₂O (50 mL) at 80 °C for 5 h, and then filtered. The filter cake was collected and dried to give the target product (4.00 g, yield: 69.6%), which was used directly in the next step without purification.

LC-MS: m/z 383 (M+H)⁺.

### Step 3: Preparation of (R)-1-(3-(1-((7-bromo-8-fluoro-6-iodo-2-methylquinazolin-4-yl)amino)ethyl)-2-fluorophen yl)-1,1-difluoro-2-methylpropan-2-ol

To a solution of 7-bromo-8-fluoro-6-iodo-2-methylquinazolin-4-ol (1.60 g, 4.18 mmol, 1.00 eq) in DMF (16 mL) were added PyBOP (4.35 g, 8.36 mmol, 2.00 eq) and TEA (2.11 g, 20.9 mmol, 2.91 mL, 5.00 eq). The reaction solution was reacted at room temperature for 0.5 h, followed by addition of (*R*)-1-(3-(1-aminoethyl)-2-fluorophenyl)-1,1-difluoro-2-methylpropan-2-ol hydrochloride (1.54 g, 5.43 mmol, 1.30 eq). The resulting mixture was reacted at 25 °C for 16 h, followed by addition of EtOAc (50 mL) and H₂O (50 mL). The organic phase was separated, then dried over anhydrous MgSO₄ and filtered. The filtrate was concentrated under reduced pressure, and the residue was separated by preparative liquid chromatography to give the target product (1.10 g, yield: 43.2%).

LC-MS: m/z 612 (M+H)⁺. ¹H NMR (400MHz, DMSO) δ8.93 (s, 1 H) 8.78 (br d, *J*=7.21 Hz, 1 H) 7.59 (br t, *J*=6.54 Hz, 1 H) 7.29 - 7.36 (m, 1 H) 7.19 - 7.26 (m, 1 H) 5.76 (t, *J*=7.09 Hz, 1 H) 5.33 (s, 1 H) 2.36 (s, 3 H) 1.58 (d, *J*=7.09 Hz, 3 H) 1.22 (br d, *J*=10.27 Hz, 6 H).

### Step 4: Preparation of (R)-5-(7-bromo-4-((1-(3-(1,1-difluoro-2-hydroxy-2-methylpropyl)-2-fluorophenyl)ethyl)am ino)-8-fluoro-2-methylquinazolin-6-yl)-1-methylpyridin-2(1H)-one

In nitrogen atmosphere, a mixture of (*R*)-1-(3-(1-((7-bromo-8-fluoro-6-iodo-2-methylquinazolin-4-yl)amino)ethyl)-2-fluorophenyl)-1,1-difluoro-2-methylpropan-2-ol (1 g, 1.63 mmol, 1 eq), 1-methyl-5-(4,4,5,5-tetramethyl-1,3,2-dioxaborolan-2-yl)pyridin-2(1*H*)-one (461 mg, 1.96 mmol, 1.2 eq), Pd(dppf)Cl₂ (120 mg, 163 µmol, 0.1 eq) and K₃PO₄ (1.04 g, 4.90 mmol, 3 eq) in dioxane (5 mL), MeCN (5 mL) and H₂O (5 mL) was reacted at 90 °C for 6 h. EtOAc (20 mL) and H₂O (20 mL) were then added. The organic phase was separated, then dried over anhydrous MgSO₄ and filtered. The filtrate was concentrated under reduced pressure, and the residue was separated by preparative liquid chromatography to give the target product (750 mg, yield: 77.4%).

LC-MS: m/z 593 (M+H)⁺. ¹H NMR (400MHz, DMSO) δ 8.71 (br d, *J*=7.28 Hz, 1 H) 8.34 (s, 1 H) 8.00 (d, *J*=2.51 Hz, 1 H) 7.57 - 7.65 (m, 2 H) 7.29 - 7.36 (m, 1 H) 7.18 - 7.26 (m, 1 H) 6.53 (d, *J*=9.29 Hz, 1 H) 5.81 (br t, *J*=7.15 Hz, 1 H) 5.34 (s, 1 H) 3.54 (s, 3 H) 2.40 (s, 3 H) 1.58 (d, *J*=7.03 Hz, 3 H) 1.23 (br d, *J*=10.04 Hz, 6 H).

### Example 1: Preparation of 1,1-difluoro-1-(2-fluoro-3-((R)-1-((7-methoxy-2-methyl-6-(((S)-1-methylpyrrolin-2-yl)meth oxy)quinazolin-4-yl)amino)ethyl)phenyl)-2-methylpropan-2-ol

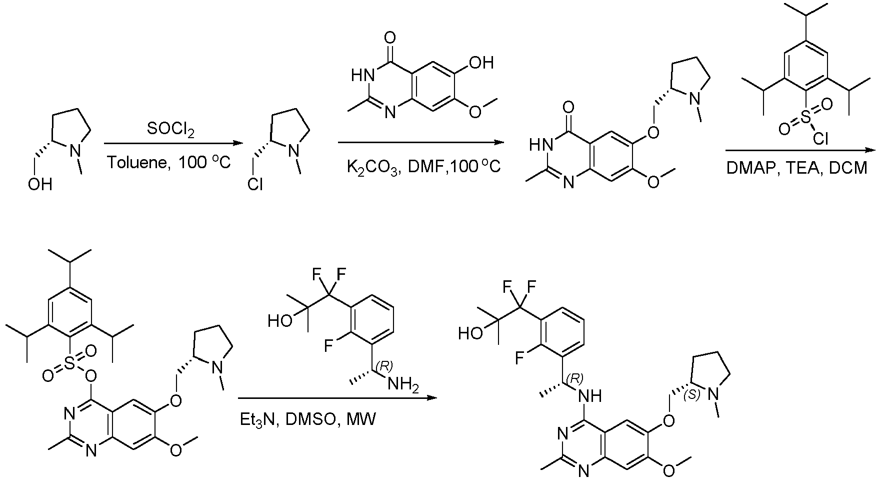

### Step 1: Preparation of (S)-2-(chloromethyl)-1-methylpyrroline

*N*-Methyl-L-prolinol (500.0 mg, 4.34 mmol) was dissolved in toluene (5 mL), and thionyl chloride (2.0 mL) was added. The resulting reaction solution was stirred at 100 °C for 2.0 h, and then concentrated under reduced pressure. The crude product obtained was used directly in the next step without further purification.

### Step 2: Preparation of (S)-7-methoxy-2-methyl-6-((1-methylpyrrolin-2-yl)methoxy)quinazolin-4(3H)-one

6-Hydroxy-7-methoxy-2-methylquinazolin-4(3*H*)-one (120 mg, 0.58 mmol) was added to *N,N-*dimethylformamide (10 mL), followed by addition of (*S*)-2-(chloromethyl)-1-methylpyrroline (77.8 mg, 0.58 mmol) obtained in the last step and potassium carbonate (402.2 mg, 2.91 mmol). The resulting reaction solution was stirred at 100 °C for 3.0 h, and then cooled to room temperature. The mixture was separated by preparative liquid chromatography to give the target product (39 mg, yield: 22%).

LC-MS: m/z 304 (M+H)⁺.

### Step 3: Preparation of (S)-7-methoxy-2-methyl-6-((1-methylpyrrolin-2-yl)methoxy)quinazolin-4-yl-2,4,6-triisopro pylbenzenesulfonate

(*S*)-7-Methoxy-2-methyl-6-((1-methylpyrrolin-2-yl)methoxy)quinazolin-4(3*H*)-one (38.0 mg, 0.13 mmol) was added to dichloromethane (4 mL), followed by addition of 2,4,6-triisopropylsulfonyl chloride (45.5 mg, 0.15 mmol), triethylamine (25.4 mg, 0.25 mmol) and 4-dimethylaminopyridine (1.5 mg, 0.013 mmol). The resulting reaction solution was stirred at room temperature overnight, then poured into water, and extracted with dichloromethane (10 mL). The organic phase was dried and concentrated under reduced pressure, and the resulting residue was purified by silica gel column chromatography (PE: EA= 5: 1) to give the target product (14.0 mg, yield: 20%).

LC-MS: m/z 570(M+H)⁺.

### Step 4: Preparation of 1,1-difluoro-1-(2-fluoro-3-((R)-1-((7-methoxy-2-methyl-6-(((S)-1-methylpyrrolin-2-yl)meth oxy)quinazolin-4-yl)amino)ethyl)phenyl)-2-methylpropan-2-ol

(*S*)-7-Methoxy-2-methyl-6-((1-methylpyrrolin-2-yl)methoxy)quinazolin-4-yl-2,4,6-triisopropyl benzenesulfonate (31.0 mg, 0.054 mmol) was added to dimethyl sulfoxide (2 mL), and then (R)-1-(3-(1-aminoethyl)-2-fluorophenyl)-1,1-difluoro-2-methylpropan-2-ol (20.2 mg, 0.082 mmol) and triethylamine (0.4 mL) were added. The resulting reaction solution was reacted at 120 °C for 2.0 h in microwave, then cooled to room temperature, quenched with water, and then extracted with ethyl acetate (10 mL). The organic phase was dried and concentrated under reduced pressure, and the resulting residue was separated by preparative liquid chromatography to give the target compound (4 mg, yield: 13.9%).

LC-MS: m/z 533 (M+H)⁺. ¹H NMR (400 MHz, DMSO) δ 8.07 (m, 1H), 7.81 (m, 1H), 7.57 (d, *J* = 6.3 Hz, 1H), 7.30 (t, *J* = 7.1 Hz, 1H), 7.25 - 7.17 (m, 1H), 7.02 (d, *J* = 3.0 Hz, 1H), 5.79 (m, 1H), 5.34 (m, 1H), 4.10 - 3.97 (m, 1H), 3.87 (m, 3H), 2.43 (m, 2H), 2.34 - 2.17 (m, 5H), 2.13 - 1.94 (m, 2H), 1.82 - 1.66 (m, 2H), 1.58 (m, 3H), 1.24 (m, 9H).

**The following compounds were synthesized in the same manner as in Example 1 with different starting materials:**

### Example 2: Preparation of N-((R)-1-(3-amino-5-(trifluoromethyl)phenyl)ethyl)-7-methoxy-2-methyl-6-(((S)-1-methylp yrrolin-2-yl)methoxy)quinazolin-4-amine

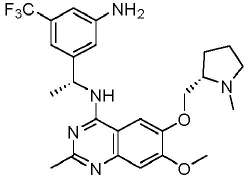

LC-MS: m/z 490 (M+H)⁺.

### Example 3: Preparation of N-((R)-1-(3-(difluoromethyl)-2-methylphenyl)ethyl)-7-methoxy-2-methyl-6-(((S)-1-methyl pyrrolin-2-yl)methoxy)quinazolin-4-amine

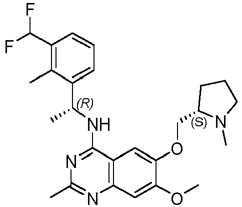

LC-MS: m/z 471 (M+H)⁺.

### Example 4: Preparation of N-((R)-1-(3-amino-5-(trifluoromethyl)phenyl)ethyl)-2-methyl-6-(((S)-1-methylpyrrolin-2-y 1)methoxy)quinazolin-4-amine

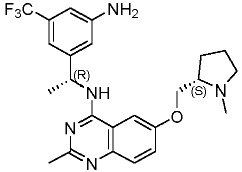

LC-MS: m/z 460 (M+H)⁺.

### Example 5: Preparation of N-((R)-1-(3-amino-5-(trifluoromethyl)phenyl)ethyl)-2,7-dimethyl-6-(((S)-1-methylpyrrolin -2-yl)methoxy))quinazolin-4-amine

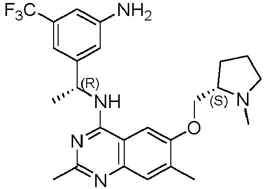

LC-MS: m/z 474 (M+H)⁺.

### Example 6: Preparation of (S)-5-(((4-(((R)-1-(3-amino-5-(trifluoromethyl)phenyl)ethyl)amino)-7-methoxy-2-methylqu inazolin-6-yl)oxy)methyl)-1-methylpyrrolin-2-one

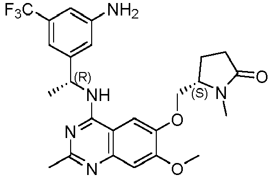

LC-MS: m/z 504 (M+H)⁺. ¹H NMR (400 MHz, DMSO) δ 14.28 (brs, 1H), 9.71 (d, *J* = 7.6 Hz, 1H), 8.04 (s, 1H), 7.17 (m, 3H), 6.88 (d, *J* = 4.3 Hz, 2H), 6.76 (s, 1H), 5.81 - 5.61 (m, 1H), 4.33 (m, 1H), 4.15 (m, 1H), 3.97 (m, 4H), 2.80 (s, 3H), 2.59 (s, 3H), 2.45 (m, 1H), 2.27 - 2.13 (m, 2H), 1.91 (m, 1H), 1.64 (d, *J* = 7.0 Hz, 3H).

### Example 7: Preparation of (R)-N-(1-(3-amino-5-(trifluoromethyl)phenyl)ethyl)-6-(2-cyclopropoxy)-7-methoxy-2-meth ylquinazolin-4-amine

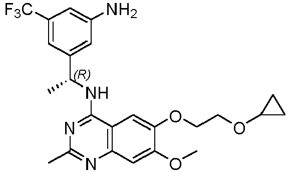

LC-MS: m/z 477 (M+H)⁺. ¹H NMR (400 MHz, DMSO-d₆) δ 7.93 (d, 1H), 7.71 (s, 1H), 7.03(s, 1H), 6.86 (d, 1H), 6.69 (s, 1H), 5.57-5.53 (m, 3H), 4.22-4.18 (m, 2H), 3.91-3.84 (m, 5H), 3.44-3.39 (m, 1H), 2.35 (s, 3H), 1.55-1.53 (d, 3H), 0.54-0.44 (m, 4H).

### Example 8: Preparation of (R)-N-(1-(3-amino-5-(trifluoromethyl)phenyl)ethyl)-6-(2-(cyclopropyl(methyl)amino)ethox y)-7-methoxy-2-methylquinolin-4-amine

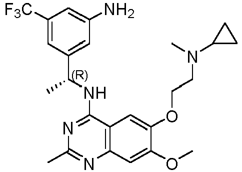

LC-MS: m/z 490 (M+H)⁺. ¹H NMR (400 MHz, DMSO) δ 7.94 (d, *J* = 8.0 Hz, 1H), 7.69 (s, 1H), 7.02 (s, 1H), 6.87 (d, *J* = 11.5 Hz, 2H), 6.70 (s, 1H), 5.64 - 5.44 (m, 3H), 4.23 - 4.07 (m, 2H), 3.86 (s, 3H), 2.96 (t, *J* = 5.9 Hz, 2H), 2.39 (s, 3H), 2.35 (s, 3H), 1.87 - 1.74 (m, 1H), 1.52 (d, *J* = 8.0 Hz, 3H), 0.51 - 0.40 (m, 2H), 0.37 - 0.24 (m, 2H).

### Example 9: Preparation of (R)-1-(3-(1-((6-(2-((cyclobutylmethyl)(methyl)amino)ethoxy)-7-methoxy-2-methylquinolin-4-yl)amino)ethyl)-2-fluorophenyl)-1,1-difluoro-2-methylpropan-2-ol

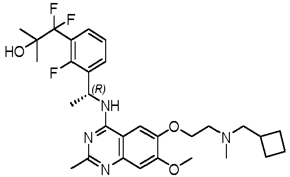

LC-MS: m/z 561 (M+H)⁺. ¹H NMR (400 MHz, DMSO) δ 8.03 (d, *J* = 7.4 Hz, 1H), 7.74 (s, 1H), 7.58 (t, *J* = 6.7 Hz, 1H), 7.30 (t, *J* = 6.7 Hz, 1H), 7.20 (t, *J* = 7.7 Hz, 1H), 7.02 (s, 1H), 5.80 (p, *J* = 7.0 Hz, 1H), 5.31 (s, 1H), 4.16 (m, 2H), 3.86 (s, 3H), 2.78 (t, *J* = 6.0 Hz, 2H), 2.27 (d, *J* = 10.1 Hz, 6H), 2.08 - 1.95 (m, 2H), 1.93 - 1.73 (m, 3H), 1.66 (m, 2H), 1.58 (d, *J* = 7.0 Hz, 3H), 1.22 (m, 8H).

### Example 10: Preparation of (R)-N-(1-(3-amino-5-(trifluoromethyl)phenyl)ethyl)-6-(2-((cyclobutylmethyl)(methyl)amin o)ethoxy)-7-methoxy-2-methylquinolin-4-amine

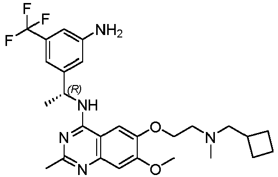

LC-MS: m/z 518 (M+H)⁺. ¹H NMR (400 MHz, DMSO) δ 7.93 (d, *J* = 8.0 Hz, 1H), 7.69 (s, 1H), 7.02 (s, 1H), 6.87 (d, *J* = 11.0 Hz, 2H), 6.69 (s, 1H), 5.63 - 5.46 (m, 3H), 4.13 (m, 2H), 3.86 (s, 3H), 2.76 (t, *J* = 6.0 Hz, 2H), 2.47 (m, 2H), 2.34 (s, 3H), 2.25 (s, 3H), 2.00 (m, 2H), 1.90 - 1.71 (m, 3H), 1.70 - 1.59 (m, 2H), 1.55 (d, *J* = 7.0 Hz, 3H).

### Example 11: Preparation of (R)-N-(1-(3-amino-5-(trifluoromethyl)phenyl)ethyl)-6-((1-((dimethylamino)methyl)cyclopr opyl)methoxy)-7-methoxy-2-methylquinazolin-4-amine

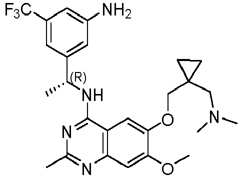

LC-MS: m/z 504 (M+H)⁺. ¹H NMR (400 MHz, DMSO) δ 7.95 (d, *J* = 8.0 Hz, 1H), 7.64 (d, *J* = 9.6 Hz, 1H), 7.02 (s, 1H), 6.86 (d, *J* = 10.4 Hz, 2H), 6.69 (s, 1H), 5.62 - 5.46 (m, 3H), 3.96 (m, 2H), 3.88 (s, 3H), 2.39 - 2.10 (m, 11H), 1.54 (d, *J* = 7.1 Hz, 3H), 0.67 (s, 2H), 0.48 (s, 2H).

### Example 12: (R)-1-(3-(1-((6-((1-((Dimethylamino)methyl)cyclopropyl)methoxy)-7-methoxy-2-methylqui nolin-4-yl)amino)ethyl)-2-fluorophenyl)-1,1-difluoro-2-methylpropan-2-ol

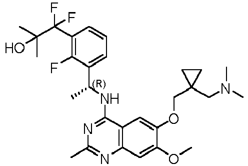

LC-MS: m/z 507 (M+H)⁺. ¹H NMR (400 MHz, DMSO) δ 8.04 (d, *J* = 7.4 Hz, 1H), 7.67 (s, 1H), 7.58 (t, *J* = 6.6 Hz, 1H), 7.30 (t, *J* = 6.6 Hz, 1H), 7.21 (m, 1H), 7.01 (s, 1H), 5.79 (m, 1H), 5.31 (s, 1H), 3.98 (m, 2H), 3.89 (s, 3H), 2.26 (m, 8H), 1.58 (d, *J* = 7.0 Hz, 3H), 1.32 - 1.12 (m, 9H), 0.68 (m, 2H), 0.48 (m, 2H).

### Example 13: Preparation of (R)-N-(2-((4-((1-(3-amino-5-(trifluoromethyl)phenyl)ethyl)amino)-7-methoxy-2-methylqui nolin-6-yl)oxy)ethyl)-N-methylmethanesulfonamide

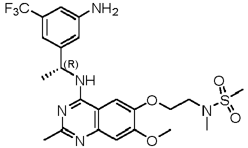

LC-MS: m/z 528 (M+H)⁺. ¹H NMR (400 MHz, DMSO) δ 7.99 (d, *J* = 7.7 Hz, 1H), 7.74 (s, 1H), 7.05 (s, 1H), 6.86 (d, *J* = 10.6 Hz, 2H), 6.69 (s, 1H), 5.61 - 5.43 (m, 3H), 4.20 (t, *J* = 19.3 Hz, 2H), 3.87 (s, 3H), 3.59 (t, *J* = 5.5 Hz, 2H), 2.99 (s, 3H), 2.92 (s, 3H), 2.34 (s, 3H), 1.52 (t, *J* = 17.4 Hz, 3H).

### Example 14: Preparation of 1-((S)-2-(((4-(((R)-1-(3-amino-5-(trifluoromethyl)phenyl)ethyl)amino)-7-methoxy-2-methyl quinolin-6-yl)oxy)methyl)pyrrol-1-yl)ethan-1-one

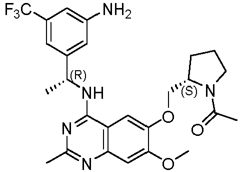

LC-MS: m/z 518 (M+H)⁺. ¹H NMR (400 MHz, DMSO) δ 7.92 (d, *J* = 7.4 Hz, 1H), 7.79 (s, 1H), 7.02 (s, 1H), 6.87 (d, *J* = 14.3 Hz, 2H), 6.69 (s, 1H), 5.53 (m, 3H), 4.32 (m, 2H), 4.05 (m, 3H), 3.88 (s, 3H), 2.34 (m, 3H), 2.17 (m, 2H), 1.99 (m, 5H), 1.54 (d, *J* = 7.0 Hz, 3H).

### Example 15: Preparation of (R)-N-(1-(3-amino-5-(trifluoromethyl)phenyl)ethyl)-7-methoxy-2-methyl-6-((1-(morpholin ylmethyl)cyclopropyl)methoxy)quinazolin-4-amine

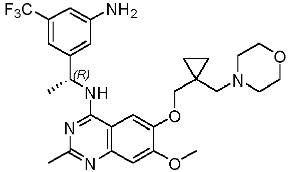

LC-MS: m/z 546 (M+H)⁺. ¹H NMR (400 MHz, DMSO) δ 7.92 (d, *J* = 7.9 Hz, 1H), 7.64 (s, 1H), 7.01 (s, 1H), 6.87 (m, 2H), 6.70 (s, 1H), 5.66 - 5.44 (m, 3H), 4.28 - 3.93 (m, 2H), 3.87 (s, 3H), 3.60 - 3.48 (m, 4H), 2.66 - 2.56 (m, 1H), 2.50 (s, 3H), 2.35 (m, 4H), 2.07 - 1.95 (m, 1H), 1.56 (t, *J* = 8.6 Hz, 3H), 0.63 (m, 2H), 0.50 (m, 2H).

### Example 16: tert-Butyl (S)-2-(((4-(((R)-1-(2-fluoro-3-(trifluoromethyl)phenyl)ethyl)amino)-7-methoxy-2-methylqu inolin-6-yl)oxy)methyl)pyrroline-1-carboxylate

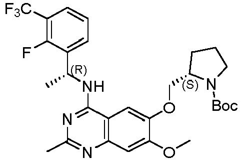

LC-MS: m/z 579 (M+H)⁺. ¹H NMR (400 MHz, DMSO) δ 8.10 (m, 1H), 7.77 (m, 2H), 7.63 (t, *J* = 7.1 Hz, 1H), 7.34 (t, *J* = 7.8 Hz, 1H), 7.03 (s, 1H), 5.75 (m, 1H), 4.07 (m, 3H), 3.87 (s, 3H), 2.30 (s, 3H), 1.91 (m, 4H), 1.62 (d, *J* = 7.1 Hz, 3H), 1.40 (m, 11H).

### Example 17: N-((R)-1-(2-Fluoro-3-(trifluoromethyl)phenyl)ethyl)-7-methoxy-2-methyl-6-(((S)-pyrrolin-2-yl)methoxy)quinazolin-4-amine

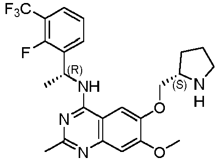

LC-MS: m/z 479 (M+H)⁺. ¹H NMR (400 MHz, DMSO) δ 8.14 (t, *J* = 8.6 Hz, 1H), 7.84 - 7.70 (m, 2H), 7.62 (t, *J* = 6.9 Hz, 1H), 7.35 (t, *J* = 7.8 Hz, 1H), 7.03 (s, 1H), 5.85 - 5.67 (m, 1H), 4.23 - 3.92 (m, 2H), 3.87 (s, 3H), 3.56 (m, 1H), 2.91 (m, 2H), 2.30 (s, 3H), 2.12 - 1.67 (m, 4H), 1.62 (d, *J* = 7.1 Hz, 3H).

### Example 18: Preparation of N-((R)-1-(2-fluoro-3-(trifluoromethyl)phenyl)ethyl)-7-methoxy-2-methyl-6-(((S)-1-methylp yrrolin-2-yl)methoxy)quinazolin-4-amine

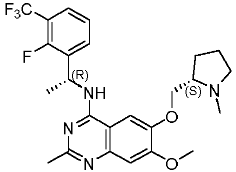

LC-MS: m/z 493 (M+H)⁺. ¹H NMR (400 MHz, DMSO) δ 14.61 (brs, 1H), 10.00 (s, 1H), 8.21 (d, *J* = 19.4 Hz, 1H), 7.92 (t, *J* = 7.2 Hz, 1H), 7.72 (t, *J* = 7.0 Hz, 1H), 7.43 (t, *J* = 7.8 Hz, 1H), 7.28 (s, 1H), 5.93 (m, 1H), 4.66 (d, *J* = 9.6 Hz, 1H), 4.54 - 4.37 (m, 1H), 4.23 (m, 1H), 3.98 (s, 3H), 3.71 (m, 2H), 3.03 (s, 3H), 2.54 (s, 3H), 2.37 (m, 1H), 2.25 - 1.90 (m, 3H), 1.73 (t, *J* = 10.3 Hz, 3H).

### Example 19: Preparation of (R)-1-(3-(1-((6-(2-(((3,3-difluorocyclobutyl)methyl)(methyl)amino)ethoxy)-7-methoxy-2-m ethylquinolin-4-yl)amino)ethyl)-2-fluorophenyl)-1,1-difluoro-2-methylpropan-2-ol

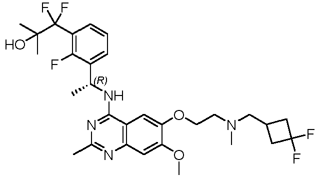

LC-MS: m/z 597 (M+H)⁺. ¹H NMR (400 MHz, DMSO) δ 8.04 (d, *J* = 7.4 Hz, 1H), 7.74 (s, 1H), 7.58 (t, *J* = 6.7 Hz, 1H), 7.31 (m, 1H), 7.20 (t, *J* = 7.7 Hz, 1H), 7.02 (s, 1H), 5.79 (m, 1H), 5.34 (s, 1H), 4.36 (t, *J* = 5.1 Hz, 2H), 3.83 (s, 3H), 2.83 (t, *J* = 5.7 Hz, 2H), 2.75 (d, *J* = 10.4 Hz, 2H), 2.68 - 2.56 (m, 4H), 2.39 - 2.15 (m, 7H), 1.58 (d, *J* = 7.0 Hz, 3H), 1.20 (m, 6H).

### Example 20: Preparation of N-((R)-1-(2-fluoro-3-(trifluoromethyl)phenyl)ethyl)-7-methoxy-2-methyl-6-(((S)-1-(methyl sulfonyl)pyrrolidin-2-yl)methoxy)quinazolin-4-amine

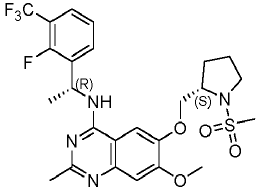

LC-MS: m/z 557 (M+H)⁺. ¹H NMR (400 MHz, DMSO) δ 8.15 (d, *J* = 6.9 Hz, 1H), 7.79 (t, *J* = 7.1 Hz, 1H), 7.74 (s, 1H), 7.63 (t, *J* = 7.0 Hz, 1H), 7.35 (t, *J* = 7.8 Hz, 1H), 7.04 (s, 1H), 5.75 (p, *J* = 6.9 Hz, 1H), 4.15 (d, *J* = 10.8 Hz, 2H), 4.08 - 3.95 (m, 1H), 3.87 (s, 3H), 3.32 - 3.24 (m, 2H), 3.02 (s, 3H), 2.28 (s, 3H), 1.99 (m, 4H), 1.62 (d, *J* = 7.0 Hz, 3H).

### Example 21: Preparation of (R)-N-(1-(3-amino-5-(trifluoromethyl)phenyl)ethyl)-6-(2-(((1-fluorocyclopropyl)methyl)(m ethyl)amino)ethoxy)-7-methoxy-2-methylquinazolin-4-amine

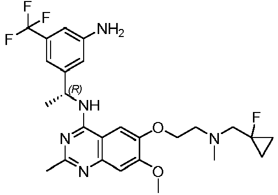

LC-MS: m/z 522 (M+H)⁺. ¹H NMR (400 MHz, DMSO) δ 8.21 - 7.98 (brs, 1H), 7.75 (s, 1H), 7.04 (s, 1H), 6.88 (m, 2H), 6.71 (s, 1H), 5.64 - 5.49 (m, 3H), 4.21 (m, 2H), 3.88 (s, 3H), 2.91 (m, 4H), 2.46 (s, 3H), 2.36 (s, 3H), 1.57 (d, *J* = 7.0 Hz, 3H), 0.99 (m 2H), 0.70 (m, 2H).

### Example 22: Preparation of (R)-N-(1-(3-amino-5-(trifluoromethyl)phenyl)ethyl)-6-(2-(((3,3-difluorocyclobutyl)methyl)( methyl)amino)ethoxy)-7-methoxy-2-methylquinazolin-4-amine

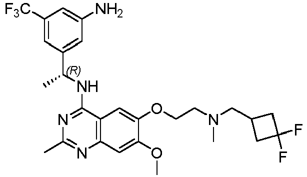

LC-MS: m/z 554 (M+H)⁺. ¹H NMR (400 MHz, DMSO) δ 7.94 (d, *J* = 7.9 Hz, 1H), 7.70 (s, 1H), 7.02 (s, 1H), 6.86 (m, 2H), 6.69 (s, 1H), 5.62 - 5.48 (m, 3H), 4.14 (m, 2H), 3.86 (s, 3H), 2.80 (m, 2H), 2.75 (m, 1H), 2.69 - 2.59 (m, 3H), 2.58 - 2.54 (m, 2H), 2.35 (s, 3H), 2.27 (s, 3H), 2.20 (m, 1H), 1.55 (d, *J* = 7.0 Hz, 3H).

### Example 23: 1,1-Difluoro-1-(2-fluoro-3-((R)-1-((7-methoxy-2-methyl-6-(((R)-morpholin-2-yl)methoxy)q uinazolin-4-yl)amino)ethoxy)phenyl)-2-methylpropan-2-ol

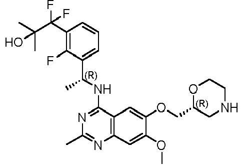

LC-MS: m/z 535 (M+H)⁺. ¹H NMR (400 MHz, DMSO) δ 9.02 (brs, 1H), 7.96 (s, 1H), 7.63 (t, *J* = 6.9 Hz, 1H), 7.36 (t, *J* = 6.9 Hz, 1H), 7.26 (t, *J* = 7.8 Hz, 1H), 7.11 (s, 1H), 5.96 - 5.81 (m, 1H), 5.35 (s, 1H), 4.32 - 4.10 (m, 3H), 4.06 (m, 1H), 3.94 (s, 3H), 3.80 (m, 1H), 3.50 - 3.38 (m, 2H), 3.26 (m, 1H), 3.05 (m, 2H), 2.45 (s, 3H), 1.64 (d, *J =* 6.9 Hz, 3H), 1.22 (d, *J* = 7.5 Hz, 6H).

### Example 24: 1,1-Difluoro-1-(2-fluoro-3-((R)-1-((7-methoxy-2-methyl-6-(((S)-morpholin-2-yl)methoxy)q uinazolin-4-yl)amino)ethoxy)phenyl)-2-methylpropan-2-ol

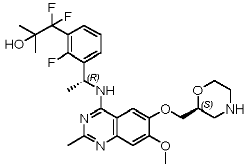

LC-MS: m/z 535 (M+H)⁺. ¹H NMR (400 MHz, DMSO) δ 8.04 (d, *J* = 7.4 Hz, 1H), 7.73 (s, 1H), 7.65 - 7.51 (m, 1H), 7.30 (t, *J* = 6.7 Hz, 1H), 7.20 (t, *J* = 7.7 Hz, 1H), 7.02 (s, 1H), 5.80 (m, 1H), 5.33 (s, 1H), 4.14 - 3.94 (m, 2H), 3.87 (s, 3H), 3.78 (m, 2H), 3.51 (m, 1H), 2.97 (m, 1H), 2.68 (m, 2H), 2.60 - 2.52 (m, 2H), 2.28(s, 3H), 1.58 (d, *J* = 7.0 Hz, 3H), 1.20 (t, *J* = 17.2 Hz, 6H).

### Example 25: 1,1-Difluoro-1-(2-fluoro-3-((R)-1-((7-methoxy-2-methyl-6-(((S)-4-methylmorpholin-2-yl)m ethoxy)quinazolin-4-yl)amino)ethoxy)phenyl)-2-methylpropan-2-ol

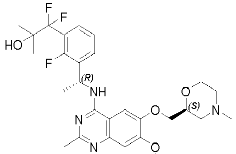

LC-MS: m/z 549 (M+H)⁺. ¹H NMR (400 MHz, DMSO) δ 8.04 (d, *J* = 7.4 Hz, 1H), 7.74 (s, 1H), 7.58 (t, *J* = 6.6 Hz, 1H), 7.30 (t, *J* = 6.6 Hz, 1H), 7.20 (t, *J* = 7.7 Hz, 1H), 7.03 (s, 1H), 5.80 (t, *J* = 7.1 Hz, 1H), 5.32 (s, 1H), 4.25 - 3.98 (m, 2H), 3.85 (m, 5H), 3.59 (m, 1H), 2.87 (m, 1H), 2.72 - 2.56 (m, 1H), 2.28 (s, 3H), 2.22 (s, 3H), 2.04 (m, 1H), 1.94 (m, 1H), 1.58 (d, *J* = 7.0 Hz, 3H), 1.22 (d, *J* = 11.3 Hz, 6H).

### Example 26: 1-(3-((R)-1-((6-(((S)-1,4-Dioxan-2-yl)methoxy)-7-methoxy-2-methylquinazolin-4-yl)amino) ethyl)-2-fluorophenyl)-1,1-difluoro-2-methylpropan-2-ol

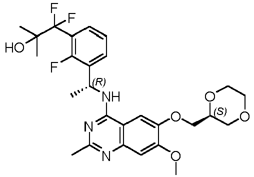

LC-MS: m/z 536 (M+H)⁺. ¹H NMR (400 MHz, DMSO) δ 8.04 (d, *J* = 7.3 Hz, 1H), 7.74 (s, 1H), 7.58 (t, *J* = 6.9 Hz, 1H), 7.30 (t, *J* = 6.7 Hz, 1H), 7.20 (t, *J* = 7.7 Hz, 1H), 7.03 (s, 1H), 5.88 - 5.70 (m, 1H), 5.32 (s, 1H), 4.17 - 4.02 (m, 2H), 4.01 - 3.84 (m, 5H), 3.80 (m, 1H), 3.74 - 3.62 (m, 2H), 3.60 - 3.41 (m, 2H), 2.28 (s, 3H), 1.58 (d, *J* = 7.0 Hz, 3H), 1.20 (t, *J* = 17.3 Hz, 6H).

### Example 27: 1-(3-((R)-1-((6-(((R)-1,4-Dioxan-2-yl)methoxy)-7-methoxy-2-methylquinazolin-4-yl)amino) ethyl)-2-fluorophenyl)-1,1-difluoro-2-methylpropan-2-ol

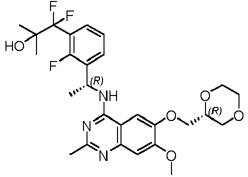

LC-MS: m/z 536 (M+H)⁺. ¹H NMR (400 MHz, DMSO) δ 8.05 (d, *J* = 7.2 Hz, 1H), 7.76 (d, *J* = 10.9 Hz, 1H), 7.58 (t, *J* = 6.8 Hz, 1H), 7.30 (t, *J* = 6.7 Hz, 1H), 7.20 (t, *J* = 7.7 Hz, 1H), 7.03 (s, 1H), 5.88 - 5.72 (m, 1H), 5.33 (s, 1H), 4.09 (m, 2H), 4.01 - 3.84 (m, 5H), 3.80 (m, 1H), 3.76 - 3.63 (m, 2H), 3.50 (m, 2H), 2.29 (s, 3H), 1.58 (d, *J* = 7.0 Hz, 3H), 1.22 (d, *J* = 11.0 Hz, 7H).

### Example 28: 6-(((S)-1,4-Dioxan-2-yl)methoxy)-N-((R)-1-(3-amino-5-(trifluoromethyl)phenyl)ethyl)-7-m ethoxy-2-methylquinazolin-4-amine

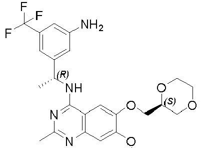

LC-MS: m/z 493 (M+H)⁺. ¹H NMR (400 MHz, DMSO) δ 8.05 (d, *J* = 7.1 Hz, 1H), 7.73 (s, 1H), 7.04 (s, 1H), 6.87 (d, *J* = 10.7 Hz, 2H), 6.70 (s, 1H), 5.56 (brs, 2H), 4.15 - 4.01 (m, 2H), 4.01 - 3.84 (m, 5H), 3.79 (m, 1H), 3.74 - 3.60 (m, 2H), 3.58 - 3.44 (m, 3H), 2.36 (s, 3H), 1.56 (d, *J* = 6.9 Hz, 3H).

### Example 29: 1,1-Difluoro-1-(2-fluoro-3-((R)-1-((7-methoxy-2-methyl-6-(((S)-4-(methylsulfonyl)morphol in-2-yl)methoxy)quinazolin-4-yl)amino)ethoxy)phenyl)-2-methylpropan-2-ol

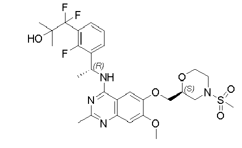

LC-MS: m/z 613 (M+H)⁺. ¹H NMR (400 MHz, DMSO) δ 8.13 (s, 1H), 7.79 (s, 1H), 7.58 (t, *J* = 6.8 Hz, 1H), 7.30 (t, *J* = 6.7 Hz, 1H), 7.21 (t, *J* = 7.7 Hz, 1H), 7.04 (s, 1H), 5.81 (t, *J* = 7.1 Hz, 1H), 5.33 (s, 1H), 4.30 - 4.08 (m, 2H), 4.09 - 3.94 (m, 2H), 3.88 (s, 3H), 3.76 - 3.56 (m, 2H), 3.41 (m, 1H), 3.06 - 2.72 (m, 5H), 2.29 (s, 3H), 1.58 (d, *J* = 7.0 Hz, 3H), 1.20 (t, *J* = 14.5 Hz, 6H).

### Example 30: N-((R)-1-(3-Amino-5-(trifluoromethyl)phenyl)ethyl)-7-methoxy-2-methyl-6-(((S)-5-methyl-5-azaspiro[2.4]heptan-6-yl)methoxy)quinazolin-4-amine

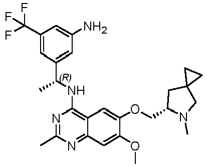

LC-MS: m/z 516 (M+H)⁺. ¹H NMR (400 MHz, DMSO) δ 7.98 (d, *J* = 7.9 Hz, 1H), 7.71 (s, 1H), 7.02 (s, 1H), 6.85 (t, *J* = 11.2 Hz, 2H), 6.69 (s, 1H), 5.64 - 5.45 (m, 3H), 4.14 - 3.98 (m, 2H), 3.86 (s, 3H), 2.91 (m, 1H), 2.61 (m, 1H), 2.53 (m, 1H), 2.42 (s, 3H), 2.35 (s, 3H), 2.13 (dd, *J* = 12.6, 8.2 Hz, 1H), 1.67 - 1.44 (m, 4H), 0.63 - 0.39 (m, 4H).

### Example 31: N-((R)-1-(3-Amino-5-(trifluoromethyl)phenyl)ethyl)-6-(((S)-4,4-difluoro-1-methylpyrrolidi n-2-yl)methoxy)-7-methoxy-2-methylquinazolin-4-amine

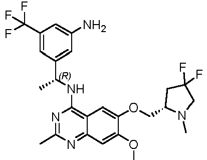

LC-MS: m/z 526 (M+H)⁺. ¹H NMR (400 MHz, DMSO) δ 7.95 (d, *J* = 8.0 Hz, 1H), 7.73 (s, 1H), 7.04 (s, 1H), 6.86 (d, *J* = 10.1 Hz, 2H), 6.69 (s, 1H), 5.66 - 5.46 (m, 3H), 4.11 (d, *J* = 5.2 Hz, 2H), 3.87 (s, 3H), 3.45 - 3.34 (m, 1H), 3.05 (t, *J* = 5.3 Hz, 1H), 2.80 - 2.56 (m, 2H), 2.43 (s, 3H), 2.35 (s, 3H), 2.30 - 2.12 (m, 1H), 1.56 (t, *J* = 7.2 Hz, 3H).

### Example 32: Preparation of N-((R)-1-(3-amino-5-(trifluoromethyl)phenyl)ethyl)-7-methoxy-2-methyl-6-(((S)-1-(methyl sulfonyl)pyrrolidin-2-yl)methoxy)quinazolin-4-amine

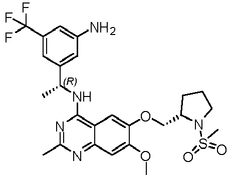

LC-MS: m/z 554 (M+H)⁺. ¹H NMR (400 MHz, DMSO) δ 7.99 (t, *J* = 13.1 Hz, 1H), 7.71 (s, 1H), 7.06 (d, *J* = 10.1 Hz, 1H), 6.87 (d, *J* = 9.9 Hz, 2H), 6.70 (s, 1H), 5.64 - 5.45 (m, 3H), 4.09 (t, *J* = 12.0 Hz, 2H), 3.99 (m, 1H), 3.88 (s, 3H), 2.99 (s, 3H), 2.36 (s, 3H), 2.02 (m, 4H), 1.56 (d, *J* = 7.0 Hz, 3H).

### Example 33: (R)-N-(2-((4-((1-(3-(1,1-Difluoro-2-hydroxy-2-methylpropyl)-2-fluorophenyl)ethyl)amino)-7-methoxy-2-methylquinazolin-6-yl)oxy)ethyl)-N-methylmethanesulfonamide

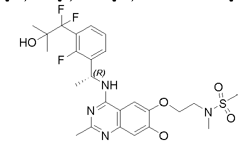

LC-MS: m/z 571 (M+H)⁺. ¹H NMR (400 MHz, DMSO) δ 8.13 (d, *J* = 6.8 Hz, 1H), 7.79 (d, *J* = 15.0 Hz, 1H), 7.60 (t, *J* = 6.7 Hz, 1H), 7.38 - 7.25 (m, 1H), 7.21 (t, *J* = 7.7 Hz, 1H), 7.04 (d, *J* = 10.1 Hz, 1H), 5.81 (p, *J* = 6.8 Hz, 1H), 5.33 (s, 1H), 4.27 (t, *J* = 5.0 Hz, 2H), 3.87 (s, 3H), 3.61 (t, *J* = 5.4 Hz, 2H), 3.01 (s, 3H), 2.95 (s, 3H), 2.29 (s, 3H), 1.59 (d, *J* = 7.0 Hz, 3H), 1.22 (d, *J=* 11.1 Hz, 6H).

### Example 34: (R)-N-(1-(3-Amino-5-(trifluoromethyl)phenyl)ethyl)-6-((1-(dimethylamino)cyclopropyl)me thoxy)-7-methoxy-2-methylquinazolin-4-amine

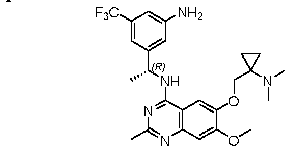

LC-MS: m/z 490 (M+H)⁺. ¹H NMR (400 MHz, DMSO) δ 7.93 (d, *J* = 8.0 Hz, 1H), 7.65 (s, 1H), 7.03 (s, 1H), 6.86 (d, *J* = 10.5 Hz, 2H), 6.69 (s, 1H), 5.57 (d, *J* = 11.2 Hz, 3H), 4.19 - 4.02 (m, 2H), 3.87 (s, 3H), 2.42 (s, 6H), 2.35 (s, 3H), 1.55 (d, *J* = 7.0 Hz, 3H), 0.70 (q, *J* = 6.8 Hz, 4H).

### Example 35: (R)-N-(1-(5-Amino-2-fluoro-3-(trifluoromethyl)phenyl)ethyl)-6-(2-cyclopropoxyethyloxy)-7-methoxy-2-methylquinazolin-4-amine

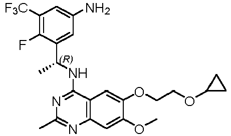

LC-MS: m/z 495 (M+H)⁺. ¹H NMR (400 MHz, DMSO) δ 7.96 (d, *J* = 7.2 Hz, 1H), 7.77 (s, 1H), 7.04 (s, 1H), 6.89 - 6.79 (m, 1H), 6.70 (dd, *J* = 5.4, 2.7 Hz, 1H), 5.67 (p, *J* = 6.9 Hz, 1H), 5.33 (s, 2H), 4.35 - 4.11 (m, 2H), 3.87 (m, 5H), 3.43 (m, 1H), 2.31 (s, 3H), 1.55 (d, *J* = 7.0 Hz, 3H), 0.62 - 0.35 (m, 4H).

### Example 36: (R)-6-(2-Cyclopropoxyethyloxy)-7-methoxy-2-methyl-N-(1-(4-(trifluoromethyl)pyridin-2-y l)ethyl)quinazolin-4-amine

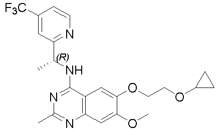

LC-MS: m/z 463 (M+H)⁺. ¹H NMR (400 MHz, DMSO) δ 8.81 (d, *J* = 4.8 Hz, 1H), 8.14 (d, *J* = 7.1 Hz, 1H), 7.78 (d, *J* = 18.9 Hz, 2H), 7.63 (d, *J* = 4.3 Hz, 1H), 7.04 (s, 1H), 5.68 (m, 1H), 4.22 (d, *J* = 4.4 Hz, 2H), 3.87 (m, 5H), 3.46 - 3.40 (m, 1H), 2.30 (s, 3H), 1.65 (d, *J* = 6.9 Hz, 3H), 0.49 (m, 4H).

### Example 37: (R)-6-(2-Cyclopropoxyethyloxy)-7-methoxy-2-methyl-N-(1-(2-(trifluoromethyl)pyridin-2-y l)ethyl)quinazolin-4-amine

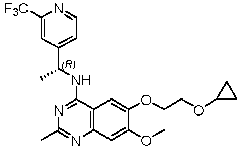

LC-MS: m/z 463 (M+H)⁺. ¹H NMR (400 MHz, DMSO) δ 8.70 (d, *J* = 4.6 Hz, 1H), 8.12 (d, *J* = 6.9 Hz, 1H), 7.95 (s, 1H), 7.73 (s, 2H), 7.05 (s, 1H), 5.69 - 5.51 (m, 1H), 4.23 (d, *J* = 3.8 Hz, 2H), 3.88 (s, 5H), 3.50 - 3.39 (m, 1H), 2.30 (s, 3H), 1.63 (d, *J* = 6.9 Hz, 3H), 0.64 - 0.30 (m, 4H).

### Example 38: (R)-N-(1-(6-Amino-4-(trifluoromethyl)pyridin-2-yl)ethyl)-6-(2-cyclopropoxyethyloxy)-7-m ethoxy-2-methylquinazolin-4-amine

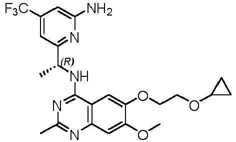

LC-MS: m/z 478 (M+H)⁺. ¹H NMR (400 MHz, DMSO) δ 7.95 (d, *J* = 7.7 Hz, 1H), 7.73 (s, 1H), 7.04 (s, 1H), 6.76 (s, 1H), 6.56 (s, 1H), 6.50 (s, 2H), 5.46 (m, 1H), 4.29 - 4.13 (m, 2H), 3.95 - 3.80 (m, 5H), 3.42 (m, 1H), 2.32 (s, 3H), 1.55 (d, *J* = 7.1 Hz, 3H), 0.50 (m, 4H).

### Example 39: N-((R)-1-(3-Amino-5-(trifluoromethyl)phenyl)ethyl)-7-methoxy-2-methyl-6-(((S)-tetrahydr ofuran-2-yl)methoxuinazolin-4-amine

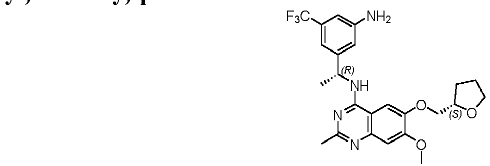

LC-MS: m/z 477 (M+H)⁺. ¹H NMR (400 MHz, DMSO) δ 7.94 (d, *J* = 8.0 Hz, 1H), 7.70 (s, 1H), 7.03 (s, 1H), 6.86 (d, *J* = 10.1 Hz, 2H), 6.69 (s, 1H), 5.57 (m, 3H), 4.31 - 4.16 (m, 1H), 4.04 (d, *J* = 5.3 Hz, 2H), 3.93 - 3.76 (m, 4H), 3.71 (m, 1H), 2.35 (s, 3H), 2.13 - 2.00 (m, 1H), 2.00 - 1.81 (m, 2H), 1.79 - 1.67 (m, 1H), 1.55 (d, *J* = 7.0 Hz, 3H).

### Example 40: N-((R)-1-(3-Amino-5-(trifluoromethyl)phenyl)ethyl)-7-methoxy-2-methyl-6-(((R)-tetrahyd rofuran-2-yl)methoxy)quinazolin-4-amine

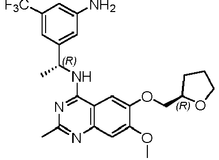

LC-MS: m/z 477 (M+H)⁺. ¹H NMR (400 MHz, DMSO) δ 7.94 (d, *J* = 8.0 Hz, 1H), 7.70 (s, 1H), 7.03 (s, 1H), 6.86 (d, *J* = 10.2 Hz, 2H), 6.69 (s, 1H), 5.65 - 5.44 (m, 3H), 4.33 - 4.18 (m, 1H), 4.04 (m, 2H), 3.94 - 3.78 (m, 4H), 3.71 (m, 1H), 2.35 (s, 3H), 2.13 - 2.04 (m, 1H), 2.02 - 1.81 (m, 2H), 1.81 - 1.67 (m, 1H), 1.55 (d, *J* = 7.0 Hz, 3H).

### Example 41: (R)-N-(1-(2-Amino-6-(trifluoromethyl)pyridin-4-yl)ethyl)-6-(2-cyclopropoxyethoxy)-7-met hoxy-2-methylauinazolin-4-amine

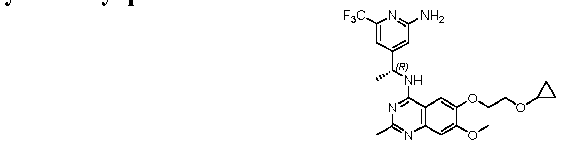

LC-MS: m/z 478 (M+H)⁺. ¹H NMR (400 MHz, DMSO) δ 7.94 (d, *J* = 8.0 Hz, 1H), 7.72 (s, 1H), 7.05 (s, 1H), 6.99 (s, 1H), 6.66 (s, 1H), 6.49 (s, 2H), 5.48 (m, 1H), 4.29 - 4.16 (m, 2H), 3.92 - 3.77 (m, 5H), 3.41 (m, 1H), 2.34 (s, 3H), 1.55 (d, *J* = 7.1 Hz, 3H), 0.64 - 0.40 (m, 4H).

### Example 42: (R)-N-(1-(3-Amino-5-(trifluoromethyl)phenyl)ethyl)-7-methoxy-6-(2-methoxyethoxy)-2-me thylauinazolin-4-amine

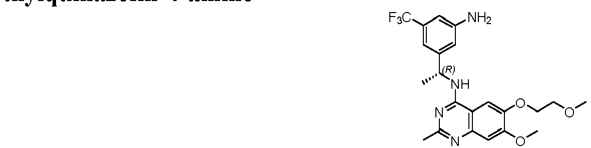

LC-MS: m/z 451 (M+H)⁺. ¹H NMR (400 MHz, DMSO) δ 7.95 (d, *J* = 7.9 Hz, 1H), 7.72 (s, 1H), 7.03 (s, 1H), 6.87 (d, *J* = 10.9 Hz, 2H), 6.69 (s, 1H), 5.56 (m, 3H), 4.30 - 4.14 (m, 2H), 3.89 (s, 3H), 3.73 (m, 2H), 3.32 (s, 3H), 2.35 (s, 3H), 1.55 (d, *J* = 7.0 Hz, 3H).

### Example 43: (R)-N-(1-(3-Amino-5-(trifluoromethyl)phenyl)ethyl)-7-methoxy-6-((3-(methoxymethyl)oxe tan-3-yl)methoxy)-2-methylauinazolin-4-amine

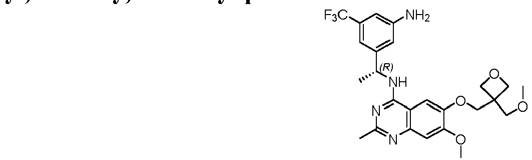

LC-MS: m/z 507 (M+H)⁺. ¹H NMR (400 MHz, DMSO) δ 7.99 (d, *J* = 7.9 Hz, 1H), 7.81 (s, 1H), 7.06 (s, 1H), 6.88 (d, *J* = 8.1 Hz, 2H), 6.71 (s, 1H), 5.71 - 5.43 (m, 3H), 4.50 (m, 4H), 4.28 (m, 2H), 3.89 (s, 3H), 3.71 (s, 2H), 3.34 (s, 3H), 2.37 (s, 3H), 1.57 (d, *J* = 7.0 Hz, 3H).

### Example 44: (R)-N-(1-(3-Amino-5-(trifluoromethyl)phenyl)ethyl)-7-methoxy-2-methyl-6-(2-(oxetan-3-yl oxy)ethoxy)quinazolin-4-amine

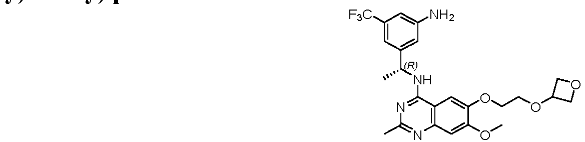

LC-MS: m/z 493 (M+H)⁺. ¹H NMR (400 MHz, DMSO) δ 7.94 (d, *J* = 7.9 Hz, 1H), 7.72 (s, 1H), 7.04 (s, 1H), 6.86 (d, *J* = 10.5 Hz, 2H), 6.69 (s, 1H), 5.65 - 5.43 (m, 3H), 4.75 - 4.60 (m, 3H), 4.44 (m, 2H), 4.28 - 4.14 (m, 2H), 3.88 (s, 3H), 3.80 (t, *J* = 4.5 Hz, 2H), 2.35 (s, 3H), 1.55 (d, *J* = 7.0 Hz, 3H).

### Example 45: (R)-N-(1-(3-Amino-5-(trifluoromethyl)phenyl)ethyl)-6-(2-cyclopropoxyethoxy)-2-methyl-7 -(trifluoromethyl)quinazolin-4-amine

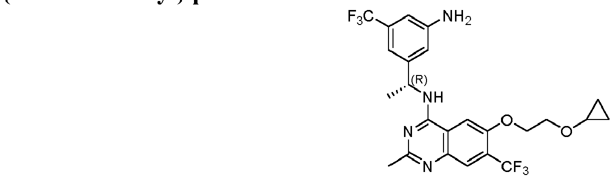

LC-MS: m/z 515 (M+H)⁺. ¹H NMR (400 MHz, DMSO) δ 8.61 (d, *J* = 6.7 Hz, 1H), 8.06 (s, 1H), 7.85 (s, 1H), 7.80 (t, *J* = 7.0 Hz, 1H), 7.69 - 7.59 (m, 1H), 7.35 (t, *J* = 7.7 Hz, 1H), 5.76 (m, 1H), 4.34 (t, *J* = 14.1 Hz, 2H), 3.87 (m, 2H), 3.44 (m, 1H), 2.33 (s, 3H), 1.66 (d, *J* = 7.1 Hz, 3H), 0.56 - 0.40 (m, 4H).

### Example 46: (R)-N-(1-(3-Amino-5-(trifluoromethyl)phenyl)ethyl)-7-methoxy-6-((1-methoxycyclopropyl )methoxy)-2-methylquinazolin-4-amine

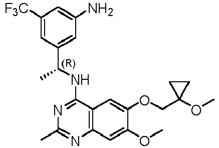

LC-MS: m/z 477 (M+H)⁺. ¹H NMR (400 MHz, DMSO) δ 7.94 (d, *J* = 7.9 Hz, 1H), 7.71 (s, 1H), 7.04 (s, 1H), 6.88 (d, *J* = 8.6 Hz, 2H), 6.70 (s, 1H), 5.66 - 5.43 (m, 3H), 4.20 (q, *J* = 11.1 Hz, 2H), 3.89 (s, 3H), 3.34 (s, 3H), 2.36 (s, 3H), 1.56 (d, *J* = 7.0 Hz, 3H), 0.95 - 0.85 (m, 2H), 0.75 (t, *J* = 5.4 Hz, 2H).

### Example 47: N-((R)-1-(3-Amino-5-(trifluoromethyl)phenyl)ethyl)-7-methoxy-6-((S)-2-methoxypropyl)-2 -methylquinazolin-4-amine

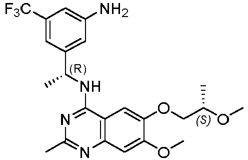

LC-MS: m/z 465 (M+H)⁺. ¹H NMR (400 MHz, DMSO) δ 7.96 (d, *J* = 7.7 Hz, 1H), 7.70 (s, 1H), 7.03 (s, 1H), 6.86 (d, *J* = 10.4 Hz, 2H), 6.69 (s, 1H), 5.56 (m, 3H), 4.04 (m, 2H), 3.87 (s, 3H), 3.75 (m, 1H), 3.36 (s, 3H), 2.35 (s, 3H), 1.55 (d, *J* = 7.0 Hz, 3H), 1.24 (d, *J* = 6.3 Hz, 3H).

### Example 48: N-((R)-1-(3-Amino-5-(trifluoromethyl)phenyl)ethyl)-7-methoxy-6-((R)-2-methoxypropyl)-2-methylquinazolin-4-amine

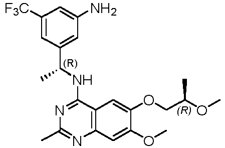

LC-MS: m/z 465 (M+H)⁺. ¹H NMR (400 MHz, DMSO) δ 7.96 (d, *J* = 7.9 Hz, 1H), 7.70 (s, 1H), 7.03 (s, 1H), 6.86 (d, *J* = 10.3 Hz, 2H), 6.69 (s, 1H), 5.69 - 5.42 (m, 3H), 4.09 (m, 1H), 3.99 (m, 1H), 3.87 (s, 3H), 3.75 (m, 1H), 3.36 (s, 3H), 2.35 (s, 3H), 1.55 (d, *J* = 7.0 Hz, 3H), 1.24 (d, *J* = 6.3 Hz, 3H).

### Example 49: (R)-N-(1-(3-Amino-5-(trifluoromethyl)phenyl)ethyl)-6-(2-cyclobutoxyethoxy)-7-methoxy-2 -methylquinazolin-4-amine

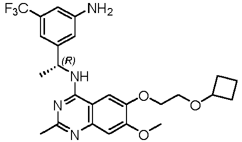

LC-MS: m/z 491 (M+H)⁺. ¹H NMR (400 MHz, DMSO) δ 7.97 (d, *J* = 7.9 Hz, 1H), 7.73 (s, 1H), 7.04 (s, 1H), 6.88 (d, *J* = 10.2 Hz, 2H), 6.71 (s, 1H), 5.68 - 5.40 (m, 3H), 4.32 - 4.13 (m, 2H), 4.08 - 3.96 (m, 1H), 3.88 (s, 3H), 3.70 (t, *J* = 4.8 Hz, 2H), 2.36 (s, 3H), 2.24 - 2.10 (m, 2H), 1.95 - 1.78 (m, 2H), 1.69 - 1.35 (m, 5H).

### Example 50: (R)-N-(1-(3-Amino-5-(trifluoromethyl)phenyl)ethyl)-7-methoxy-6-(2-(2-methoxyethoxy)et hoxy)-2-methylquinazolin-4-amine

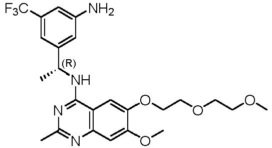

LC-MS: m/z 495 (M+H)⁺. ¹H NMR (400 MHz, DMSO) δ 7.95 (d, *J* = 7.9 Hz, 1H), 7.72 (s, 1H), 7.03 (s, 1H), 6.86 (d, *J* = 10.9 Hz, 2H), 6.69 (s, 1H), 5.64 - 5.38 (m, 3H), 4.28 - 4.14 (m, 2H), 3.94 - 3.77 (m, 5H), 3.69 - 3.57 (m, 2H), 3.48 (m, 2H), 3.25 (s, 3H), 2.35 (s, 3H), 1.55 (d, *J* = 7.0 Hz, 3H).

### Example 51: (R)-N-(1-(6-Amino-4-(trifluoromethyl)pyridin-2-yl)ethyl)-7-methoxy-6-(2-methoxyethoxy) -2-methylquinazolin-4-amine

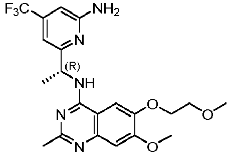

LC-MS: m/z 452 (M+H)⁺. ¹H NMR (400 MHz, DMSO) δ 7.95 (d, *J* = 7.7 Hz, 1H), 7.74 (s, 1H), 7.04 (s, 1H), 6.77 (s, 1H), 6.55 (m, 1H), 6.49 (s, 2H), 5.48 (m, 1H), 4.30 - 4.14 (m, 2H), 3.88 (s, 3H), 3.75 (t, *J* = 4.6 Hz, 2H), 3.35 (d, *J* = 3.1 Hz, 3H), 2.33 (s, 3H), 1.56 (d, *J* = 7.1 Hz, 3H).

### Example 52: (R)-N-(1-(4-Amino-6-(trifluoromethyl)pyridin-2-yl)ethyl)-7-methoxy-6-(2-methoxyethoxy) -2-methylquinazolin-4-amine

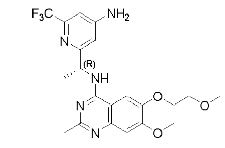

LC-MS: m/z 452 (M+H)⁺. ¹H NMR (400 MHz, DMSO) δ 7.98 (d, *J* = 7.8 Hz, 1H), 7.77 (s, 1H), 7.05 (s, 1H), 6.76 (d, *J=* 1.7 Hz, 1H), 6.70 (s, 1H), 6.49 (s, 2H), 5.49 (p, *J* = 7.0 Hz, 1H), 4.29 - 4.17 (m, 2H), 3.88 (s, 3H), 3.76 (t, *J=* 4.6 Hz, 2H), 3.35 (s, 3H), 2.34 (s, 3H), 1.56 (d, *J* = 7.1 Hz, 3H).

### Example 53: (R)-N-(1-(3-Amino-5-(trifluoromethyl)phenyl)ethyl)-6-(3-cyclopropoxypropoxy)-7-methox y-2-methylquinazolin-4-amine

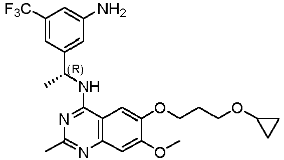

LC-MS: m/z 491 (M+H)⁺. ¹H NMR (400 MHz, DMSO) δ 7.99 (d, *J* = 7.8 Hz, 1H), 7.71 (s, 1H), 7.03 (s, 1H), 6.87 (d, *J* = 11.3 Hz, 2H), 6.69 (s, 1H), 5.57 (m, 3H), 4.23 - 4.05 (m, 2H), 3.87 (s, 3H), 3.70 - 3.58 (m, 2H), 3.29 (m, 1H), 2.35 (s, 3H), 2.02 (p, *J=* 6.1 Hz, 2H), 1.55 (d, *J* = 7.0 Hz, 3H), 0.55 - 0.36 (m, 4H).

### Example 54: (R)-N(1-(2-Fluoro-3-(trifluoromethyl)phenyl)ethyl)-7-methoxy-2-methyl-6-((1-((methyla mino)methyl)cyclopropyl)methoxy)quinazolin-4-amine

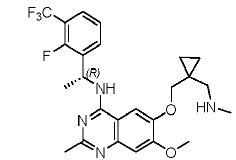

LC-MS: m/z 493 (M+H)⁺. ¹H NMR (400 MHz, DMSO) δ 8.00 (s, 1H), 7.90 (t, *J=* 7.2 Hz, 1H), 7.72 (t, *J* = 7.1 Hz, 1H), 7.48 (d, *J* = 8.0 Hz, 1H), 7.24 (s, 1H), 7.11 (d, *J* = 7.9 Hz, 1H), 5.93 (t, *J* = 7.0 Hz, 1H), 4.09 (m, 2H), 3.98 (s, 3H), 3.08 (m, 2H), 2.63 (s, 3H), 2.54 (s, 3H), 1.70 (d, *J=* 7.0 Hz, 3H), 0.91 - 0.73 (m, 4H).

### Example 55: (R)-(1-(((4-((1-(2-Fluoro-3-(trifluoromethyl)phenyl)ethyl)amino)-7-methoxy-2-methylquin azolin-6-yl)oxy)methyl)cyclopropyl)methanol

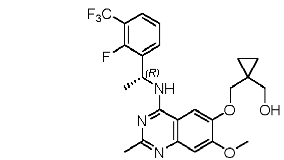

LC-MS: m/z 480 (M+H)⁺. ¹H NMR (400 MHz, DMSO) δ 8.10 (d, *J* = 6.9 Hz, 1H), 7.83 - 7.73 (m, 1H), 7.70 (s, 1H), 7.62 (t, *J* = 6.9 Hz, 1H), 7.35 (t, *J* = 7.7 Hz, 1H), 7.03 (d, *J* = 8.2 Hz, 1H), 5.83 - 5.68 (m, 1H), 4.68 (brs, 1H), 4.03 (m, 2H), 3.87 (s, 3H), 3.45 (m, 2H), 2.26 (s, 3H), 1.61 (d, *J=* 7.0 Hz, 3H), 0.58 (s, 4H).

### Example 56: (R)-N-(1-(3-Amino-5-(trifluoromethyl)phenyl)ethyl)-7-methoxy-6-((1-(methoxymethyl)cycl opropyl)methoxy)-2-methylquinazolin-4-amine

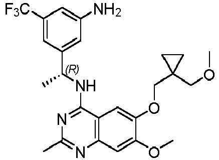

LC-MS: m/z 491 (M+H)⁺. ¹H NMR (400 MHz, DMSO) δ 7.94 (d, *J* = 7.9 Hz, 1H), 7.67 (s, 1H), 7.02 (s, 1H), 6.86 (d, *J* = 10.2 Hz, 2H), 6.69 (s, 1H), 5.64 - 5.46 (m, 3H), 3.97 (dd, *J* = 36.2, 9.9 Hz, 2H), 3.88 (s, 3H), 3.36 (dd, *J* = 19.9, 6.9 Hz, 2H), 3.26 (s, 3H), 2.35 (s, 3H), 1.54 (d, *J=* 7.0 Hz, 3H), 0.63 (t, *J=* 12.3 Hz, 4H).

### Example 57: (R)-N-(1-(3-Amino-5-(trifluoromethyl)phenyl)ethyl)-7-methoxy-6-(2-(methoxy-d3)ethoxy)-2-methylquinazolin-4-amine

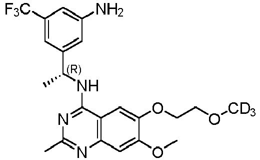

LC-MS: m/z 454 (M+H)⁺. ¹H NMR (400 MHz, DMSO) δ 7.95 (d, *J* = 7.8 Hz, 1H), 7.72 (s, 1H), 7.03 (s, 1H), 6.87 (d, *J* = 10.5 Hz, 2H), 6.70 (s, 1H), 5.67 - 5.40 (m, 3H), 4.27 - 4.09 (m, 2H), 3.87 (s, 3H), 3.79 - 3.68 (m, 2H), 2.36 (s, 3H), 1.55 (d, *J=* 7.0 Hz, 3H).

### Example 58: (R)-N-(1-(3-Amino-5-(trifluoromethyl)phenyl)ethyl)-6-(2-(cyclopropylmethoxy)ethoxy)-7-methoxy-2-methylquinazolin-4-amine

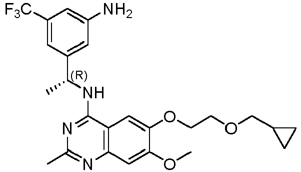

LC-MS: m/z 491 (M+H)⁺. ¹H NMR (400 MHz, DMSO) δ 7.75 (d, *J* = 7.9 Hz, 1H), 7.54 (s, 1H), 6.84 (s, 1H), 6.68 (d, *J* = 10.3 Hz, 2H), 6.51 (s, 1H), 5.45 - 5.21 (m, 3H), 4.09 - 3.94 (m, 2H), 3.68 (s, 3H), 3.62 (t, *J=* 4.8 Hz, 2H), 3.16 (m, 2H), 2.17 (s, 3H), 1.36 (d, *J=* 7.0 Hz, 3H), 0.37 - 0.20 (m, 2H), 0.01 (m, 2H).

### Example 59: (R)-N-(1-(3-Amino-5-(trifluoromethyl)phenyl)ethyl)-6-((1,3-dimethoxypropan-2-yl)oxy)-7-methoxy-2-methylquinazolin-4-amine

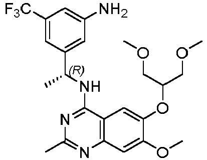

LC-MS: m/z 495 (M+H)⁺. ¹H NMR (400 MHz, DMSO) δ 8.07 (s, 1H), 7.85 (s, 1H), 7.04 (s, 1H), 6.86 (d, *J* = 12.9 Hz, 2H), 6.70 (s, 1H), 5.67 - 5.46 (m, 3H), 4.98 - 4.77 (m, 1H), 3.87 (s, 3H), 3.67 - 3.49 (m, 4H), 3.32 (s, 6H), 2.37 (s, 3H), 1.55 (d, *J=* 7.0 Hz, 3H).

### Example 60: (R)-N(1-(6-Amino-4-(trifluoromethyl)pyridin-2-yl)ethyl)-7-methoxy-2-methyl-6-(2-(oxeta n-3-yloxy)ethoxy)quinazolin-4-amine

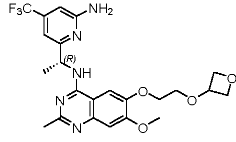

LC-MS: m/z 494 (M+H)⁺. ¹H NMR (400 MHz, DMSO) δ 7.96 (d, *J* = 7.7 Hz, 1H), 7.74 (s, 1H), 7.05 (s, 1H), 6.77 (s, 1H), 6.57 (s, 1H), 6.48 (s, 2H), 5.57 - 5.40 (m, 1H), 4.70 (d, *J* = 4.5 Hz, 3H), 4.46 (d, *J* = 5.4 Hz, 2H), 4.23 (d, *J* = 5.0 Hz, 2H), 3.89 (s, 3H), 3.80 (t, *J* = 4.6 Hz, 2H), 2.33 (s, 3H), 1.56 (d, *J=* 7.1 Hz, 3H).

### Example 61: (R)-N-(1-(3-Amino-5-(trifluoromethyl)phenyl)ethyl)-7-methoxy-2-methyl-6-(2-((3-methylo xetan-3-yl)oxy)ethoxy)quinazolin-4-amine

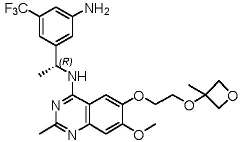

LC-MS: m/z 507 (M+H)⁺. ¹H NMR (400 MHz, DMSO) δ 7.96 (d, *J* = 7.9 Hz, 1H), 7.73 (s, 1H), 7.04 (s, 1H), 6.87 (d, *J* = 10.6 Hz, 2H), 6.70 (s, 1H), 5.56 (m, 3H), 4.58 (d, *J* = 6.5 Hz, 2H), 4.32 (d, *J* = 6.6 Hz, 2H), 4.22 (dd, *J* = 10.0, 5.0 Hz, 2H), 3.87 (s, 3H), 3.79 (t, *J* = 5.0 Hz, 2H), 2.36 (s, 3H), 1.62 - 1.41 (m, 6H).

### Example 62: (R)-N-(1-(3-Amino-5-(trifluoromethyl)phenyl)ethyl)-7-methoxy-2-methyl-6-(2-(pyridin-3-y loxy)ethoxy)quinazolin-4-amine

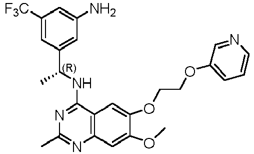

LC-MS: m/z 514 (M+H)⁺. ¹H NMR (400 MHz, DMSO) δ 8.37 (s, 1H), 8.21 (d, *J =* 3.5 Hz, 1H), 8.12 (s, 1H), 7.82 (s, 1H), 7.50 (dd, *J* = 8.4, 1.8 Hz, 1H), 7.37 (dd, *J* = 8.3, 4.6 Hz, 1H), 7.06 (s, 1H), 6.87 (d, *J=* 10.1 Hz, 2H), 6.70 (s, 1H), 5.76 - 5.47 (m, 3H), 4.60 - 4.34 (m, 4H), 3.87 (s, 3H), 2.38 (s, 3H), 1.56 (d, *J=* 7.0 Hz, 3H).

### Example 63: N-((R)-1-(3-Amino-5-(trifluoromethyl)phenyl)ethyl)-7-methoxy-6-(1-(1-(methoxymethyl)c yclopropyl)ethoxy)-2-methylquinazolin-4-amine

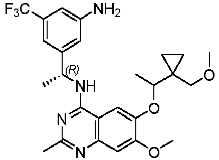

LC-MS: m/z 505 (M+H)⁺. ¹H NMR (400 MHz, DMSO) δ 8.01 (s, 1H), 7.76 (d, *J* = 3.3 Hz, 1H), 7.02 (s, 1H), 6.97 - 6.77 (m, 2H), 6.70 (s, 1H), 5.68 - 5.48 (m, 3H), 4.57 (dd, *J* = 19.7, 6.3 Hz, 1H), 3.86 (s, 3H), 3.52 (t, *J* = 9.7 Hz, 1H), 3.24 (m, 4H), 2.35 (s, 3H), 1.55 (d, *J* = 7.0 Hz, 3H), 1.26 (m, 3H), 0.62 (m, 2H), 0.47 (m, 2H).

### Example 64: (R)-N-(1-(3-Amino-5-(trifluoromethyl)phenyl)ethyl)-7-methoxy-2-methyl-6-(2-(1-methylcy clobutoxy)ethoxy)quinazolin-4-amine

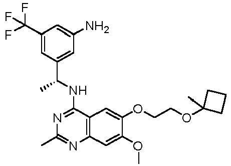

LC-MS: m/z 505 (M+H)⁺. ¹H NMR (400 MHz, DMSO) δ 7.96 (d, *J* = 8.0 Hz, 1H), 7.72 (s, 1H), 7.03 (s, 1H), 6.86 (d, *J* = 10.9 Hz, 2H), 6.69 (s, 1H), 5.56 (d, *J* = 11.3 Hz, 3H), 4.18 (dd, *J* = 10.6, 5.2 Hz, 2H), 3.87 (s, 3H), 3.67 (t, *J* = 5.2 Hz, 2H), 2.35 (s, 3H), 2.17 - 2.03 (m, 2H), 1.82 (t, *J=* 8.9 Hz, 2H), 1.71 - 1.47 (m, 5H), 1.33 (s, 3H).

### Example 65: N((R)-1-(3-Amino-5-(trifluoromethyl)phenyl)ethyl)-7-methoxy-6-((1-(1-methoxyethyl)cycl opropyl)methoxy)-2-methylquinazolin-4-amine

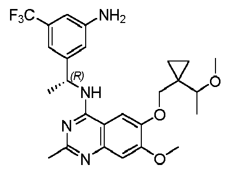

LC-MS: m/z 505 (M+H)⁺.

### Examples 65A and 65B: Two isomers, N-((R)-1-(3-amino-5-(trifluoromethyl)phenyl)ethyl)-7-methoxy-6-((1-((R)-1-methoxyethyl) cyclopropyl)methoxy)-2-methylquinazolin-4-carbamate and N((R)-1-(3-amino-5-(trifluoromethyl)phenyl)ethyl)-7-methoxy-6-((1-((S)-1-methoxyethyl) cyclopropyl)methoxy)-2-methylquinazolin-4-carbamate, obtained by chiral separation

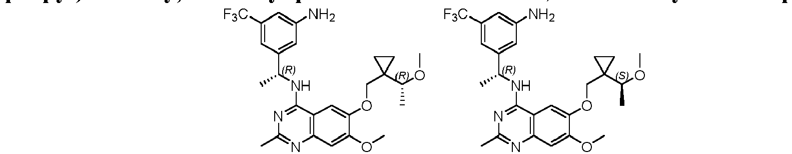

### Example 65A

LC-MS: m/z 505 (M+H)⁺. ¹H NMR (400 MHz, DMSO) δ 8.28 (s, 1H), 7.94 (d, *J* = 7.9 Hz, 1H), 7.64 (s, 1H), 7.02 (s, 1H), 6.86 (d, *J* = 9.7 Hz, 2H), 6.69 (s, 1H), 5.66 - 5.40 (m, 3H), 4.11 (m, 1H), 3.88 (m, 4H), 3.29 (s, 3H), 3.13 (m, 2H), 2.35 (s, 3H), 1.55 (d, *J* = 7.0 Hz, 3H), 1.20 (d, *J=* 6.4 Hz, 3H), 0.76 - 0.42 (m, 4H).

### Example 65B

LC-MS: m/z 505 (M+H)⁺. ¹H NMR (400 MHz, DMSO) δ 8.23 (s, 1H), 7.95 (d, *J* = 7.8 Hz, 1H), 7.64 (s, 1H), 7.02 (s, 1H), 6.86 (d, *J* = 10.9 Hz, 2H), 6.69 (s, 1H), 5.65 - 5.44 (m, 3H), 4.17 (d, *J=* 10.2 Hz, 1H), 3.87 (s, 3H), 3.81 (d, *J* = 10.2 Hz, 1H), 3.28 (s, 3H), 3.11 (m, 2H), 2.33 (s, 3H), 1.54 (d, *J=* 7.0 Hz, 3H), 1.21 (d, *J=* 6.4 Hz, 3H), 0.73 - 0.46 (m, 4H).

### Example 66: N-((R)-1-(3-Amino-5-(trifluoromethyl)phenyl)ethyl)-7-methoxy-2-methyl-6-(2-(((S)-tetrah ydrofuran-3-yl)oxy)ethoxy)quinazolin-4-amine

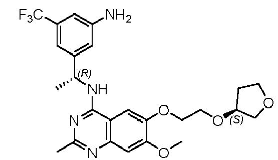

LC-MS: m/z 507 (M+H)⁺. ¹H NMR (400 MHz, DMSO) δ 7.95 (d, *J* = 8.0 Hz, 1H), 7.72 (s, 1H), 7.03 (s, 1H), 6.86 (d, *J* = 11.3 Hz, 2H), 6.69 (s, 1H), 5.56 (d, *J* = 10.7 Hz, 3H), 4.32 - 4.09 (m, 3H), 3.87 (s, 3H), 3.81 (d, *J=* 4.3 Hz, 2H), 3.77 - 3.61 (m, 4H), 2.35 (s, 3H), 1.95 (m, 2H), 1.54 (d, *J* = 7.0 Hz, 3H).

### Example 67: N-((R)-1-(3-Amino-5-(trifluoromethyl)phenyl)ethyl)-7-methoxy-2-methyl-6-(2-(((R)-tetrah ydrofuran-3-yl)oxy)ethoxy)quinazolin-4-amine

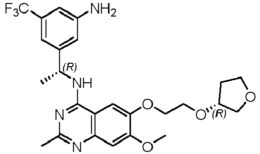

LC-MS: m/z 507 (M+H)⁺. ¹H NMR (400 MHz, DMSO) δ 7.95 (d, *J* = 8.0 Hz, 1H), 7.72 (s, 1H), 7.03 (s, 1H), 6.86 (d, *J=* 10.8 Hz, 2H), 6.69 (s, 1H), 5.55 (d, *J=* 13.1 Hz, 3H), 4.33 - 4.08 (m, 3H), 3.87 (s, 3H), 3.81 (d, *J* = 4.7 Hz, 2H), 3.70 (dt, *J* = 9.9, 6.4 Hz, 4H), 1.95 (m, 2H), 1.54 (d, *J* = 7.0 Hz, 3H).

### Example 68: N-((R)-1-(3-Amino-5-(trifluoromethyl)phenyl)ethyl)-7-methoxy-2-methyl-6-((R)-2-(oxetan-3-yloxy)propoxy)quinazolin-4-amine

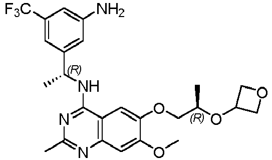

LC-MS: m/z 507 (M+H)⁺. ¹H NMR (400 MHz, DMSO) δ 7.95 (d, *J* = 7.9 Hz, 1H), 7.69 (s, 1H), 7.04 (s, 1H), 6.86 (d, *J* = 10.1 Hz, 2H), 6.70 (s, 1H), 5.68 - 5.44 (m, 3H), 4.79 (d, *J* = 5.9 Hz, 1H), 4.75 - 4.62 (m, 2H), 4.44 (dd, *J* = 9.5, 5.9 Hz, 2H), 4.02 (dd, *J =* 8.9, 2.6 Hz, 2H), 3.88 (s, 4H), 2.35 (s, 3H), 1.55 (d, *J=* 7.0 Hz, 3H), 1.23 (d, *J=* 6.3 Hz, 3H).

### Example 69: N((R)-1-(3-Amino-5-(trifluoromethyl)phenyl)ethyl)-7-methoxy-2-methyl-6-((S)-2-(oxetan-3-yloxy)propoxy)quinazolin-4-amine

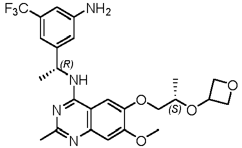

LC-MS: m/z 507 (M+H)⁺. ¹H NMR (400 MHz, DMSO) δ 7.94 (d, *J* = 7.9 Hz, 1H), 7.68 (s, 1H), 7.04 (s, 1H), 6.87 (d, *J* = 9.8 Hz, 2H), 6.70 (s, 1H), 5.56 (dd, *J* = 14.5, 6.7 Hz, 3H), 4.86 - 4.75 (m, 1H), 4.69 (dd, *J* = 14.7, 6.7 Hz, 2H), 4.44 (dd, *J* = 9.5, 5.9 Hz, 2H), 4.01 (dd, *J* = 9.5, 5.6 Hz, 2H), 3.89 (d, *J* = 7.1 Hz, 4H), 2.35 (s, 3H), 1.55 (d, *J* = 7.0 Hz, 3H), 1.23 (d, *J* = 6.3 Hz, 3H).

### Example 70: (R)-N(1-(4-Amino-6-(trifluoromethyl)pyridin-2-yl)ethyl)-7-methoxy-2-methyl-6-(2-(oxeta n-3-yloxy)ethoxy)quinazolin-4-amine

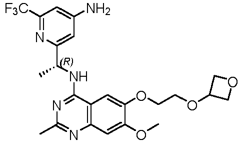

LC-MS: m/z 494 (M+H)⁺. ¹H NMR (400 MHz, DMSO) δ 8.10 (s, 1H), 7.79 (s, 1H), 7.06 (s, 1H), 6.84 - 6.65 (m, 2H), 6.49 (s, 2H), 5.58 - 5.38 (m, 1H), 4.77 - 4.65 (m, 3H), 4.45 (d, *J* = 5.5 Hz, 2H), 4.22 (d, *J* = 2.6 Hz, 2H), 3.90 (s, 3H), 3.80 (t, *J* = 4.5 Hz, 2H), 2.36 (s, 3H), 1.57 (d, *J* = 7.1 Hz, 3H).

### Example 71: (S)-1,1-Difluoro-1-(2-fluoro-3-(1-((7-methoxy-6-(2-methoxyethoxy)-2-methylquinazolin-4-yl)amino)ethyl)phenyl)-2-methylpropan-2-ol formate

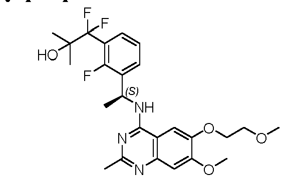

LC-MS: m/z 494 (M+H)⁺. ¹H NMR (400 MHz, DMSO) δ 8.23 (s, 1H), 8.05 (d, *J* = 7.4 Hz, 1H), 7.76 (s, 1H), 7.58 (t, *J* = 6.6 Hz, 1H), 7.30 (t, *J* = 6.6 Hz, 1H), 7.22 (m, 1H), 7.03 (s, 1H), 5.80 (p, *J* = 6.9 Hz, 1H), 5.34 (s, 1H), 4.24 (m, 2H), 3.87 (s, 3H), 3.77 (t, *J=* 4.6 Hz, 2H), 3.36 (s, 3H), 2.28 (s, 3H), 1.58 (d, *J=* 7.0 Hz, 3H), 1.22 (d, *J=* 11.3 Hz, 6H).

### Example 72: (R)-1,1-Difluoro-1-(2-fluoro-3-(1-((7-methoxy-6-(2-methoxyethoxy)-2-methylquinazolin-4-yl)amino)ethyl)phenyl)-2-methylpropan-2-ol

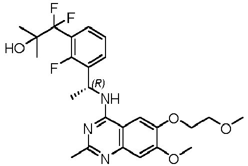

LC-MS: m/z 494 (M+H)⁺. ¹H NMR (400 MHz, DMSO) δ 8.10 (d, *J* = 7.2 Hz, 1H), 7.81 (s, 1H), 7.64 (t, *J* = 6.5 Hz, 1H), 7.36 (t, *J* = 6.8 Hz, 1H), 7.28 (m, 1H), 7.09 (s, 1H), 6.00 - 5.77 (m, 1H), 5.39 (s, 1H), 4.30 (d, *J* = 3.4 Hz, 2H), 3.93 (s, 3H), 3.82 (t, *J* = 4.2 Hz, 2H), 3.41 (s, 3H), 2.34 (s, 3H), 1.64 (d, *J* = 6.9 Hz, 3H), 1.28 (d, *J* = 11.1 Hz, 6H).

### Example 73: (R)-N-(1-(4-Amino-6-(trifluoromethyl)pyridin-2-yl)ethyl)-7-methoxy-2-methyl-6-(2-(1-met hylcyclobutyloxy)ethoxy)quinazolin-4-amine

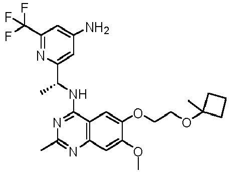

LC-MS: m/z 506 (M+H)⁺. ¹H NMR (400 MHz, DMSO) δ 7.99 (d, *J* = 7.5 Hz, 1H), 7.79 (s, 1H), 7.06 (s, 1H), 6.73 (d, *J* = 22.6 Hz, 2H), 6.49 (s, 2H), 5.57 - 5.37 (m, 1H), 4.58 (d, *J* = 6.2 Hz, 2H), 4.32 (d, *J* = 6.3 Hz, 2H), 4.23 (s, 2H), 3.88 (s, 3H), 3.80 (s, 2H), 2.34 (s, 3H), 1.56 (d, *J* = 6.9 Hz, 3H), 1.51 (s, 3H).

### Example 74: (R)-1-(3-Amino-5-(1-((7-methoxy-6-(2-methoxyethoxy)-2-methylquinazolin-4-yl)amino)eth yl)phenyl)-2-methylpropan-2-ol

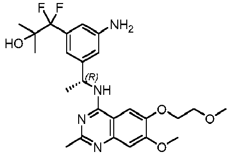

LC-MS: m/z 491 (M+H)⁺. ¹H NMR (400 MHz, DMSO) δ 8.14 (s, 1H), 7.77 (s, 1H), 7.04 (s, 1H), 6.70 (d, *J* = 10.8 Hz, 2H), 6.56 (s, 1H), 5.67 - 5.46 (m, 1H), 5.19 (s, 2H), 5.08 (s, 1H), 4.30 - 4.14 (m, 2H), 3.88 (s, 3H), 3.81 - 3.69 (m, 2H), 3.35 (s, 3H), 2.38 (s, 3H), 1.55 (d, *J* = 7.0 Hz, 3H), 1.12 (s, 6H).

### Example 75: (R)-1,1-Difluoro-1-(2-(1-((7-methoxy-6-(2-methoxyethoxy)-2-methylquinazolin-4-yl)amino )ethyl)phenyl)-2-methylpropan-2-ol

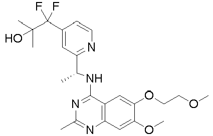

LC-MS: m/z 477 (M+H)⁺. ¹H NMR (400 MHz, DMSO) δ 8.63 (d, *J* = 5.0 Hz, 1H), 8.09 (d, *J* = 7.5 Hz, 1H), 7.75 (s, 1H), 7.51 (s, 1H), 7.31 (d, *J* = 4.0 Hz, 1H), 7.02 (s, 1H), 5.61 (t, *J=* 7.2 Hz, 1H), 5.38 (s, 1H), 4.21 (d, *J* = 2.7 Hz, 2H), 3.87 (s, 3H), 3.75 (t, *J* = 4.6 Hz, 2H), 3.35 (s, 3H), 2.29 (s, 3H), 1.64 (d, *J* = 7.1 Hz, 3H), 1.10 (d, *J* = 6.5 Hz, 6H).

### Example 76: (R)-1,1-Difluoro-1-(6-(1-((7-methoxy-6-(2-methoxyethoxy)-2-methylquinazolin-4-yl)amino )ethyl)phenyl)-2-methylpropan-2-ol

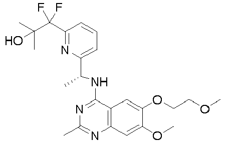

LC-MS: m/z 477 (M+H)⁺. ¹H NMR (400 MHz, DMSO) δ 8.04 (d, *J* = 7.4 Hz, 1H), 7.86 (t, *J* = 7.8 Hz, 1H), 7.74 (s, 1H), 7.52 (d, *J* = 7.8 Hz, 1H), 7.45 (d, *J* = 7.7 Hz, 1H), 7.04 (s, 1H), 5.62 (t, *J=* 7.2 Hz, 1H), 5.28 (s, 1H), 4.29 - 4.19 (m, 2H), 3.88 (s, 3H), 3.82 - 3.69 (m, 2H), 3.36 (s, 3H), 2.29 (s, 3H), 1.62 (d, *J* = 7.1 Hz, 3H), 1.18 (d, *J* = 7.7 Hz, 6H).

### Example 77: (R)-1,1-Difluoro-1-(5-(1-((7-methoxy-6-(2-methoxyethoxy)-2-methylquinazolin-4-yl)amino )ethyl)phenyl)-2-methylpropan-2-ol

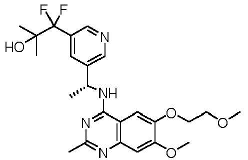

LC-MS: m/z 477 (M+H)⁺. ¹H NMR (400 MHz, DMSO) δ 8.77 (s, 1H), 8.51 (d, *J* = 1.6 Hz, 1H), 8.15 (s, 1H), 7.92 (s, 1H), 7.73 (s, 1H), 7.03 (s, 1H), 5.69 - 5.51 (m, 1H), 5.40 (s, 1H), 4.28 - 4.10 (m, 2H), 3.87 (s, 3H), 3.80 - 3.68 (m, 2H), 3.35 (s, 3H), 2.33 (s, 3H), 1.66 (d, *J* = 7.1 Hz, 3H), 1.14 (d, *J=* 9.2 Hz, 6H).

### Example 78: (R)-1,1-Difluoro-1-(4-(1-((7-methoxy-6-(2-methoxyethoxy)-2-methylquinazolin-4-yl)amino )ethyl)phenyl)-2-methylpropan-2-ol

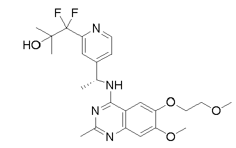

**LC-MS: m/z 477 (M+H)⁺.** ¹H NMR (400 MHz, DMSO) δ 8.56 (d, *J* = 5.0 Hz, 1H), 8.06 (d, *J* = 7.4 Hz, 1H), 7.72 (s, 1H), 7.65 (s, 1H), 7.52 (d, *J=* 4.3 Hz, 1H), 7.04 (s, 1H), 5.58 (t, *J* = 7.2 Hz, 1H), 5.26 (s, 1H), 4.22 (d, *J* = 2.8 Hz, 2H), 3.88 (s, 3H), 3.76 (t, *J* = 4.7 Hz, 2H), 3.36 (s, 3H), 2.30 (s, 3H), 1.61 (d, *J* = 7.1 Hz, 3H), 1.18 (d, *J* = 5.4 Hz, 6H).

### Example 79: (R)-1,1-Difluoro-1-(4-fluoro-3-(1-((7-methoxy-6-(2-methoxyethoxy)-2-methylquinazolin-4-yl)amino)ethyl)phenyl)-2-methylpropan-2-ol

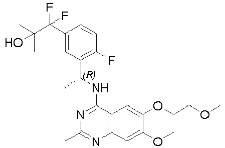

LC-MS: m/z 494 (M+H)⁺. ¹H NMR (400 MHz, DMSO) δ 8.04 (d, *J* = 7.3 Hz, 1H), 7.75 (s, 1H), 7.65 - 7.52 (m, 1H), 7.37 - 7.31 (m, 1H), 7.23 (t, *J=* 9.4 Hz, 1H), 7.02 (s, 1H), 5.75 (t, *J=* 7.1 Hz, 1H), 5.20 (s, 1H), 4.29 - 4.15 (m, 2H), 3.87 (s, 3H), 3.80 - 3.71 (m, 2H), 3.36 (s, 3H), 2.29 (s, 3H), 1.61 (d, *J=* 7.0 Hz, 3H), 1.08 (s, 3H), 0.99 (s, 3H).

### Example 80: (R)-1,1-Difluoro-1-(3-fluoro-5-(1-((7-methoxy-6-(2-methoxyethoxy)-2-methylquinazolin-4-yl)amino)ethyl)phenyl)-2-methylpropan-2-ol

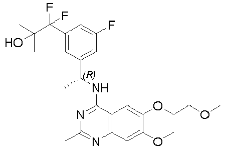

LC-MS: m/z 494 (M+H)⁺. ¹H NMR (400 MHz, DMSO) δ 7.98 (d, *J* = 7.7 Hz, 1H), 7.69 (s, 1H), 7.44 - 7.32 (m, 2H), 7.10 (d, *J* = 9.3 Hz, 1H), 7.03 (s, 1H), 5.60 (t, *J* = 7.2 Hz, 1H), 5.30 (s, 1H), 4.22 (dd, *J* = 5.3, 3.5 Hz, 2H), 3.87 (s, 3H), 3.82 - 3.73 (m, 2H), 3.35 (s, 3H), 2.32 (s, 3H), 1.60 (d, *J=* 7.0 Hz, 3H), 1.12 (d, *J=* 5.5 Hz, 6H).

### Example 81: (R)-1,1-Difluoro-1-(2-fluoro-5-(1-((7-methoxy-6-(2-methoxyethoxy)-2-methylquinazolin-4-yl)amino)ethyl)phenyl)-2-methylpropan-2-ol

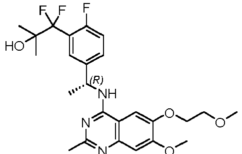

LC-MS: m/z 494 (M+H)⁺. ¹H NMR (400 MHz, DMSO) δ 7.97 (d, *J* = 7.7 Hz, 1H), 7.68 (s, 1H), 7.55 (m, 2H), 7.21 (dd, *J=* 11.1, 8.6 Hz, 1H), 7.02 (s, 1H), 5.59 (t, *J* = 7.2 Hz, 1H), 5.27 (s, 1H), 4.24 - 4.16 (m, 2H), 3.87 (s, 3H), 3.81 - 3.72 (m, 2H), 3.35 (s, 3H), 2.34 (s, 3H), 1.59 (d, *J=* 7.1 Hz, 3H), 1.15 (s, 6H).

### Example 82: N-((R)-1-(3-Amino-5-(trifluoromethyl)phenyl)ethyl)-6-(2-(2,2-difluorocyclopropyloxy)etho xy)-7-methoxy-2-methylquinazolin-4-amine

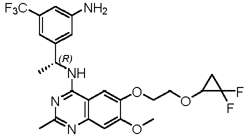

LC-MS: m/z 513 (M+H)⁺. ¹H NMR (400 MHz, DMSO) δ 7.94 (t, *J* = 18.5 Hz, 1H), 7.75 (s, 1H), 7.04 (s, 1H), 6.85 (t, *J* = 16.3 Hz, 2H), 6.70 (s, 1H), 5.56 (dd, *J=* 16.5, 9.2 Hz, 3H), 4.35 - 4.19 (m, 2H), 4.08 - 3.92 (m, 3H), 3.88 (s, 3H), 2.36 (s, 3H), 1.82 - 1.47 (m, 5H).

### Example 83: (R)-N-(1-(3-Amino-5-(trifluoromethoxy)phenyl)ethyl)-7-methoxy-6-(2-methoxyethoxy)-2-methylquinazolin-4-amine

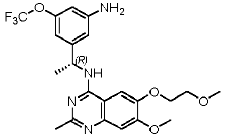

LC-MS: m/z 467 (M+H)⁺. ¹H NMR (400 MHz, DMSO) δ 7.88 (d, *J* = 8.0 Hz, 1H), 7.71 (s, 1H), 7.03 (s, 1H), 6.60 (s, 1H), 6.47 (d, *J* = 16.9 Hz, 1H), 6.32 (d, *J* = 11.2 Hz, 1H), 5.59 - 5.40 (m, 3H), 4.28 - 4.14 (m, 2H), 3.87 (s, 3H), 3.75 (t, *J* = 4.7 Hz, 2H), 3.34 (d, *J* = 12.2 Hz, 3H), 2.35 (s, 3H), 1.51 (t, *J=* 11.5 Hz, 3H).

### Example 84: (R)-3-(1-((7-Methoxy-6-(2-methoxyethoxy)-2-methylquinazolin-4-yl)amino)ethyl)benzenes ulfonamide

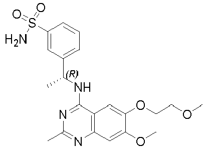

LC-MS: m/z 447 (M+H)⁺. ¹H NMR (400 MHz, DMSO) δ 8.05 (d, *J* = 7.7 Hz, 1H), 7.93 (s, 1H), 7.79 - 7.61 (m, 3H), 7.52 (t, *J=* 7.7 Hz, 1H), 7.32 (s, 2H), 7.03 (s, 1H), 5.67 (m, 1H), 4.22 (dd, *J* = 7.1, 4.2 Hz, 2H), 3.87 (s, 3H), 3.75 (t, *J* = 4.7 Hz, 2H), 3.35 (s, 3H), 2.35 (s, 3H), 1.61 (d, *J* = 7.1 Hz, 3H).

### Example 85: (R)-2,2-Difluoro-2-(3-(1-((7-methoxy-6-(2-methoxyethoxy)-2-methylquinazolin-4-yl)amino )ethyl)phenyl)-2-methylpropan-2-ol

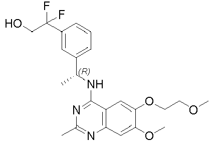

LC-MS: m/z 448 (M+H)⁺. ¹H NMR (400 MHz, DMSO) δ 8.01 (d, *J* = 7.9 Hz, 1H), 7.72 (s, 1H), 7.66 - 7.52 (m, 2H), 7.51 - 7.33 (m, 2H), 7.03 (s, 1H), 5.63 (m, 2H), 4.22 (d, *J=* 2.5 Hz, 2H), 3.94 - 3.71 (m, 7H), 3.35 (s, 3H), 2.35 (s, 3H), 1.60 (d, *J* = 7.1 Hz, 3H).

### Example 86: (R)-1-(3-(1-((6,7-Bis(2-methoxyethoxy)-2-methylquinazolin-4-yl)amino)ethyl)-2-fluorophe nyl)-1,1-difluoro-2-methylpropan-2-ol

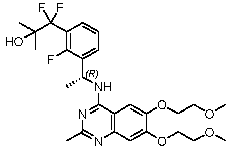

LC-MS: m/z 538 (M+H)⁺. ¹H NMR (400 MHz, DMSO) δ 8.31 (s, 1H), 7.85 (s, 1H), 7.62 (t, *J* = 6.8 Hz, 1H), 7.27 (dt, *J* = 15.5, 7.3 Hz, 2H), 7.06 (s, 1H), 5.91 - 5.77 (m, 1H), 5.32 (s, 1H), 4.31 - 4.17 (m, 4H), 3.83 - 3.66 (m, 4H), 3.38 (m, 6H), 2.32 (s, 3H), 1.60 (d, *J* = 7.0 Hz, 3H), 1.23 (d, *J=* 10.7 Hz, 6H).

### Example 87: (R)-1,1-Difluoro-1-(3-methoxy-5-(1-((7-methoxy-6-(2-methoxyethoxy)-2-methylquinazolin-4-yl)amino)ethyl)phenyl)-2-methylpropan-2-ol

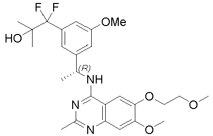

LC-MS: m/z 506 (M+H)⁺. ¹H NMR (400 MHz, DMSO) δ 7.97 (d, *J* = 7.9 Hz, 1H), 7.72 (s, 1H), 7.15 (d, *J* = 13.3 Hz, 2H), 7.04 (s, 1H), 6.87 (s, 1H), 5.68 - 5.53 (m, 1H), 5.22 (s, 1H), 4.29 - 4.12 (m, 2H), 3.88 (s, 3H), 3.75 (d, *J* = 7.3 Hz, 5H), 3.36 (s, 3H), 2.35 (s, 3H), 1.61 (d, *J* = 7.0 Hz, 3H), 1.14 (s, 6H).

### Example 88: (R)-1-(2-Chloro-3-(1-((7-methoxy-6-(2-methoxyethoxy)-2-methylquinazolin-4-yl)amino)et hyl)phenyl)-1,1-difluoro-2-methylpropan-2-ol

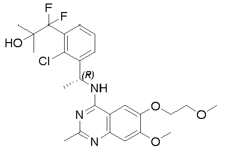

LC-MS: m/z 510 (M+H)⁺. H NMR (400 MHz, DMSO) δ 8.13 (d, *J* = 7.1 Hz, 1H), 7.79 (s, 1H), 7.63 (d, *J* = 6.6 Hz, 1H), 7.43 - 7.27 (m, 2H), 7.02 (s, 1H), 5.95 (t, *J* = 7.0 Hz, 1H), 5.30 (s, 1H), 4.26 (dd, *J* = 9.7, 4.7 Hz, 2H), 3.87 (s, 3H), 3.77 (t, *J* = 4.6 Hz, 2H), 3.37 (s, 3H), 2.25 (s, 3H), 1.55 (d, *J=* 7.0 Hz, 3H), 1.26 (d, *J=* 13.6 Hz, 6H).

### Example 89: (R)-4-((1-(3-(1,1-Difluoro-2-hydroxy-2-methylpropyl)-2-fluorophenyl)ethyl)amino)-6-(2-m ethoxyethoxy)-2-methylquinazoline-7-carbonitrile

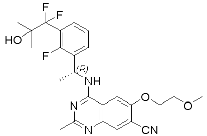

LC-MS: m/z 489 (M+H)⁺. ¹H NMR (400 MHz, DMSO) δ 8.52 (d, *J =* 7.1 Hz, 1H), 8.07 (s, 1H), 8.04 (s, 1H), 7.59 (t, *J* = 6.6 Hz, 1H), 7.32 (t, *J* = 6.7 Hz, 1H), 7.22 (t, *J* = 7.7 Hz, 1H), 5.79 (p, *J* = 6.8 Hz, 1H), 5.30 (s, 1H), 4.40 (dd, *J* = 5.0, 2.9 Hz, 2H), 3.89 - 3.75 (m, 2H), 3.39 (s, 3H), 2.33 (s, 3H), 1.62 (d, *J* = 7.0 Hz, 3H), 1.22 (d, *J* = 10.4 Hz, 6H).

### Example 90: (R)-1,1-Difluoro-1-(3-(1-((7-methoxy-6-(2-methoxyethoxy)-2-methylquinazolin-4-yl)amino )ethyl)-2-methylphenyl)-2-methylpropan-2-ol

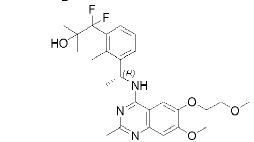

LC-MS: m/z 490 (M+H)⁺. ¹H NMR (400 MHz, DMSO) δ 8.07 (d, *J* = 7.4 Hz, 1H), 7.75 (s, 1H), 7.60 (d, *J* = 7.2 Hz, 1H), 7.30 - 7.14 (m, 2H), 7.01 (s, 1H), 5.79 (t, *J* = 7.1 Hz, 1H), 5.24 (s, 1H), 4.32 - 4.18 (m, 2H), 3.86 (s, 3H), 3.76 (t, *J* = 4.6 Hz, 2H), 3.36 (s, 3H), 2.63 (s, 3H), 2.30 (s, 3H), 1.52 (d, *J* = 6.9 Hz, 3H), 1.22 (d, *J* = 10.1 Hz, 6H).

### Example 91: (R)-3-(1,1-Difluoro-2-hydroxy-2-methylpropane)-5-(1-((7-methoxy-6-(2-methoxyethoxy)-2 -methylquinazolin-4-yl)amino)ethyl)benzonitrile

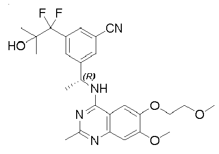

LC-MS: m/z 501 (M+H)⁺. ¹H NMR (400 MHz, DMSO) δ 8.03 (d, *J* = 10.3 Hz, 2H), 7.87 (s, 1H), 7.70 (d, *J=* 11.1 Hz, 2H), 7.03 (s, 1H), 5.58 (dd, *J* = 14.1, 7.0 Hz, 1H), 5.41 (s, 1H), 4.22 (dd, *J=* 5.5, 3.6 Hz, 2H), 3.87 (s, 3H), 3.82 - 3.71 (m, 2H), 3.36 (s, 3H), 2.31 (s, 3H), 1.62 (d, *J* = 7.1 Hz, 3H), 1.12 (d, *J* = 10.2 Hz, 6H).

### Example 92: (R)-1,1-Difluoro-1-(2-fluoro-3-(1-((7-methoxy-2-methyl-6-(2-(oxetan-3-yloxy)ethoxy)quina zolin-4-yl)amino)ethyl)phenyl)-2-methylpropan-2-ol

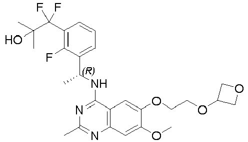

LC-MS: m/z 536 (M+H)⁺. ¹H NMR (400 MHz, DMSO) δ 8.04 (d, *J* = 7.4 Hz, 1H), 7.76 (s, 1H), 7.59 (s, 1H), 7.29 (d, *J* = 6.7 Hz, 1H), 7.21 (d, *J* = 7.7 Hz, 1H), 7.04 (s, 1H), 5.80 (s, 1H), 5.33 (s, 1H), 4.76 - 4.62 (m, 3H), 4.46 (dd, *J* = 5.7, 3.6 Hz, 2H), 4.24 (dd, *J* = 8.0, 4.2 Hz, 2H), 3.88 (s, 3H), 3.81 (t, *J=* 4.6 Hz, 2H), 2.29 (s, 3H), 1.58 (d, *J* = 7.0 z, 3H), 1.22 (d, *J=* 11.2 Hz, 6H).

### Example 93: (R)-1-(3-(1-((6-(2-Cyclopropyloxyethoxy)-7-methoxy-2-methylquinazolin-4-yl)amino)ethyl )-2-fluorophenyl)-1,1-difluoro-2-methylpropan-2-ol

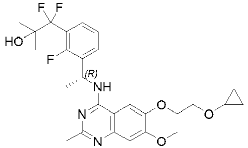

LC-MS: m/z 520 (M+H)⁺. ¹H NMR (400 MHz, DMSO) δ 8.02 (d, *J* = 7.4 Hz, 1H), 7.75 (s, 1H), 7.58 (s, 1H), 7.29 (d, *J=* 6.7 Hz, 1H), 7.21 (d, *J=* 7.7 Hz, 1H), 7.03 (s, 1H), 5.81 (d, *J* = 7.2 Hz, 1H), 5.32 (s, 1H), 4.23 (dd, *J* = 7.5, 4.4 Hz, 2H), 3.86 (d, *J* = 6.5 Hz, 5H), 3.43 (td, *J* = 5.9, 3.0 Hz, 1H), 2.28 (s, 3H), 1.57 (d, *J* = 7.0 Hz, 3H), 1.22 (d, *J* = 11.0 Hz, 6H).

### Example 94: (R)-1,1-Difluoro-1-(2-fluoro-3-(1-((6-(2-methoxyethoxy)-2-methylquinazolin-4-yl)amino)et hyl)phenyl)-2-methylpropan-2-ol

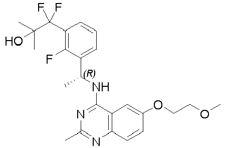

LC-MS: m/z 464 (M+H)⁺. ¹H NMR (400 MHz, DMSO) δ 8.21 (d, *J* = 7.3 Hz, 1H), 7.84 (d, *J* = 2.4 Hz, 1H), 7.59 (t, *J* = 6.6 Hz, 1H), 7.53 (d, *J* = 9.1 Hz, 1H), 7.38 (dd, *J* = 9.1, 2.5 Hz, 1H), 7.30 (t, *J=* 6.6 Hz, 1H), 7.21 (d, *J=* 7.7 Hz, 1H), 5.80 (t, *J=* 7.1 Hz, 1H), 5.32 (s, 1H), 4.25 (d, *J* = 3.1 Hz, 2H), 3.75 (t, *J* = 4.5 Hz, 2H), 3.36 (s, 3H), 2.30 (s, 3H), 1.59 (d, *J* = 7.0 Hz, 3H), 1.22 (d, *J=* 11.3 Hz, 6H).

### Example 95: (R)-1-(3-(1-((7-Bromo-6-(2-methoxyethoxy)-2-methylquinazolin-4-yl)amino)ethyl)-2-fluor ophenyl)-1,1-difluoro-2-methylpropan-2-ol

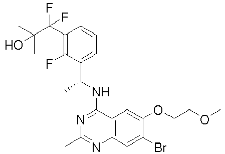

LC-MS: m/z 542 (M+H)⁺. ¹H NMR (400 MHz, DMSO) δ 8.39 (d, *J =* 7.0 Hz, 1H), 7.93 (s, 1H), 7.86 (s, 1H), 7.59 (t, *J* = 6.6 Hz, 1H), 7.31 (t, *J* = 6.6 Hz, 1H), 7.21 (t, *J* = 7.7 Hz, 1H), 5.80 (t, *J* = 7.1 Hz, 1H), 5.31 (s, 1H), 4.34 (dd, J = 7.2, 4.1 Hz, 2H), 3.81 (t, *J* = 4.6 Hz, 2H), 3.40 (s, 3H), 2.31 (s, 3H), 1.60 (d, *J* = 7.0 Hz, 3H), 1.22 (d, *J=* 10.6 Hz, 6H).

### Example 96: (R)-4-((1-(3-(1,1-Difluoro-2-hydroxy-2-methylpropyl)-2-fluorophenyl)ethyl)amino)-6-(2-m ethoxyethoxy)-2-methylquinazolin-7-ol

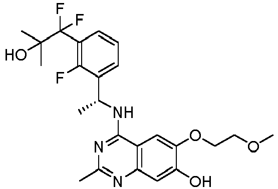

LC-MS: m/z 480 (M+H)⁺. ¹H NMR (400 MHz, DMSO) δ 7.71 (d, *J* = 7.1 Hz, 1H), 7.59 (d, *J* = 11.6 Hz, 2H), 7.28 (t, *J =* 6.7 Hz, 1H), 7.18 (t, *J* = 7.7 Hz, 1H), 6.67 (s, 1H), 5.91 - 5.60 (m, 1H), 4.20 (d, *J=* 4.0 Hz, 2H), 3.75 (t, *J=* 4.5 Hz, 2H), 3.35 - 3.19 (m, 3H), 2.19 (s, 3H), 1.54 (d, *J=* 6.9 Hz, 3H), 1.22 (d, *J=* 11.8 Hz, 6H).

### Example 97: (R)-1,1-Difluoro-1-(2-fluoro-3-(1-((7-deuterated methoxy-6-(2-methoxyethoxy)-2-methylquinazolin-4-yl)amino)ethyl)phenyl)-2-methylpro pan-2-ol

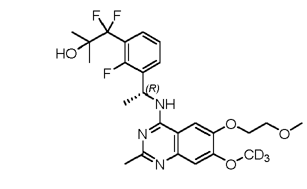

LC-MS: m/z 497 (M+H)⁺. ¹H NMR (400 MHz, DMSO) δ 8.03 (d, *J =* 7.4 Hz, 1H), 7.75 (s, 1H), 7.58 (s, 1H), 7.29 (d, *J* = 6.8 Hz, 1H), 7.21 (d, *J* = 7.7 Hz, 1H), 7.02 (s, 1H), 5.84 - 5.76 (m, 1H), 5.42 - 5.20 (m, 1H), 4.24 (dd, *J* = 7.6, 4.3 Hz, 2H), 3.76 (t, *J* = 4.6 Hz, 2H), 3.36 (s, 3H), 2.28 (s, 3H), 1.58 (d, *J=* 7.0 Hz, 3H), 1.22 (d, *J=* 11.0 Hz, 6H).

### Example 98: (R)-1-(3-(1-((7-Ethoxy-6-(2-methoxyethoxy)-2-methylquinazolin-4-yl)amino)ethyl)-2-fluor ophenyl)-1,1-difluoro-2-methylpropan-2-ol

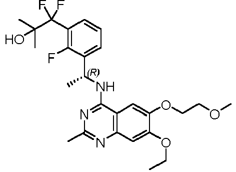

LC-MS: m/z 508 (M+H)⁺. ¹H NMR (400 MHz, DMSO) δ 8.03 (d, *J =* 7.4 Hz, 1H), 7.76 (s, 1H), 7.58 (t, *J* = 6.5 Hz, 1H), 7.30 (t, *J* = 6.6 Hz, 1H), 7.20 (t, *J* = 7.7 Hz, 1H), 7.00 (s, 1H), 5.79 (t, *J* = 7.1 Hz, 1H), 5.32 (s, 1H), 4.28 - 4.21 (m, 2H), 4.14 (q, *J* = 6.9 Hz, 2H), 3.77 (t, J = 4.7 Hz, 2H), 3.38 (s, 3H), 2.28 (s, 3H), 1.58 (d, *J* = 7.0 Hz, 3H), 1.38 (t, *J* = 6.9 Hz, 3H), 1.22 (d, *J=* 11.2 Hz, 6H).

### Example 99: (R)-1,1-Difluoro-1-(2-fluoro-3-(1-((7-isopropoxy-6-(2-methoxyethoxy)-2-methylquinazolin-4-yl)amino)ethyl)phenyl)-2-methylpropan-2-ol

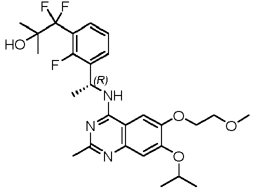

LC-MS: m/z 522 (M+H)⁺. ¹H NMR (400 MHz, DMSO) δ 8.03 (d, *J* = 7.4 Hz, 1H), 7.77 (s, 1H), 7.58 (t, *J* = 6.5 Hz, 1H), 7.30 (t, *J* = 6.6 Hz, 1H), 7.20 (t, *J* = 7.7 Hz, 1H), 7.01 (s, 1H), 5.79 (t, *J* = 7.2 Hz, 1H), 5.31 (s, 1H), 4.79 - 4.67 (m, 1H), 4.24 (dd, *J* = 6.0, 3.4 Hz, 2H), 3.76 (t, *J=* 4.7 Hz, 2H), 3.38 (s, 3H), 2.28 (s, 3H), 1.57 (d, *J* = 7.0 Hz, 3H), 1.32 (dd, *J* = 5.6, 4.9 Hz, 6H), 1.22 (d, *J=* 11.3 Hz, 6H).

### Example 100: (R)-1,1-Difluoro-1-(2-fluoro-3-(1-((7-cyclopropoxy-6-(2-methoxyethoxy)-2-methylquinazol in-4-yl)amino)ethyl)phenyl)-2-methylpropan-2-ol

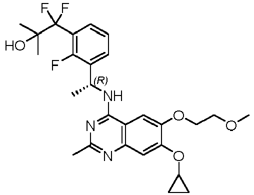

LC-MS: m/z 520 (M+H)⁺.

### Example 101: (R)-1,1-Difluoro-1-(2-fluoro-3-(1-((6-(2-methoxyethoxy)-2-methyl-7-(pyridin-3-yl)quinazol in-4-yl)amino)ethyl)phenyl)-2-methylpropyl-2-ol

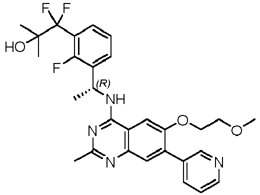

LC-MS: m/z 541 (M+H)⁺. ¹H NMR (400 MHz, DMSO) δ 8.82 (d, *J* = 2.3 Hz, 1H), 8.61 - 8.56 (m, 1H), 8.32 (d, *J* = 7.4 Hz, 1H), 8.08 - 8.01 (m, 1H), 7.95 (s, 1H), 7.65 - 7.58 (m, 2H), 7.49 (dd, *J* = 7.9, 4.8 Hz, 1H), 7.32 (t, *J* = 6.9 Hz, 1H), 7.22 (t, *J* = 7.7 Hz, 1H), 5.87 - 5.79 (m, 1H), 5.32 (s, 1H), 4.38 - 4.27 (m, 2H), 3.72 (t, *J=* 4.7 Hz, 2H), 3.30 (s, 3H), 2.33 (s, 3H), 1.62 (d, *J* = 7.0 Hz, 3H), 1.24 (s, 3H), 1.22 (s, 3H).

### Example 102: (R)-1,1-Difluoro-1-(2-fluoro-3-(1-((6-(2-methoxyethoxy)-2-methyl-7-(1-methyl-1H pyrazol-4-yl)quinazolin-4-yl)amino)ethyl)phenyl)-2-methylpropyl-2-ol

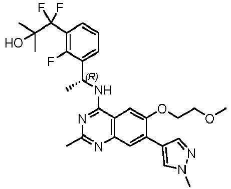

LC-MS: m/z 544 (M+H)⁺. ¹H NMR (400 MHz, DMSO) δ 8.30 (s, 1H), 8.21 (d, *J* = 7.4 Hz, 1H), 8.10 (s, 1H), 7.85 (s, 2H), 7.64 - 7.57 (m, 1H), 7.35 - 7.27 (m, 1H), 7.21 (t, *J* = 7.7 Hz, 1H), 5.88 - 5.76 (m, 1H), 5.32 (s, 1H), 4.42 - 4.29 (m, 2H), 3.92 - 3.85 (m, 5H), 3.43 (s, 3H), 2.31 (s, 3H), 1.61 (d, *J=* 7.1 Hz, 3H), 1.24 (s, 3H), 1.22 (s, 3H).

### Example 103: (R)-1,1-Difluoro-1-(2-fluoro-3-(1-(6-(2-methoxyethoxy)-2-methyl-7-(oxyethyl-3-amino)qui nazolin-4-yl)aminoethyl)phenyl)-2-methylpropan-2-ol

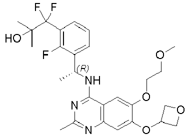

LC-MS: m/z 536 (M+H)⁺. ¹H NMR (400 MHz, DMSO) δ 8.13 (s, 1H), 7.83 (s, 1H), 7.60 (s, 1H), 7.28 (d, *J* = 6.5 Hz, 1H), 7.19 (t, *J* = 7.7 Hz, 1H), 6.63 (s, 1H), 5.78 (d, *J* = 7.0 Hz, 1H), 5.42 - 5.30 (m, 1H), 4.99 (t, *J* = 6.4 Hz, 2H), 4.58 (dd, *J* = 12.2, 5.1 Hz, 2H), 4.27 (d, *J* = 2.5 Hz, 2H), 3.79 (t, *J* = 4.7 Hz, 2H), 3.39 (s, 4H), 2.26 (s, 3H), 1.57 (d, *J* = 6.9 Hz, 3H), 1.22 (d, *J* = 11.0 Hz, 6H).

### Example 104: tert-Butyl (R)-3-(4-(1-(3-(1,1-difluoro-2-hydroxy-2-methylpropyl)-2-fluorophenyl)ethyl)-6-(2-methox yethoxy)-2-methylquinazolin-7-yl)azetidine-1-carboxylate

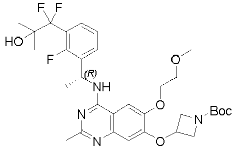

LC-MS: m/z 635 (M+H)⁺. ¹H NMR (400 MHz, DMSO) δ 8.09 (d, *J* = 7.4 Hz, 1H), 7.84 (s, 1H), 7.59 (t, *J* = 6.6 Hz, 1H), 7.31 (d, *J* = 6.9 Hz, 1H), 7.21 (t, *J* = 7.7 Hz, 1H), 6.77 (s, 1H), 5.88 - 5.72 (m, 1H), 5.10 (s, 1H), 4.63 (d, *J=* 6.1 Hz, 1H), 4.44 - 4.21 (m, 4H), 3.81 (dd, *J* = 13.2, 8.4 Hz, 4H), 3.40 (s, 3H), 2.30 (s, 3H), 1.59 (d, *J* = 7.0 Hz, 3H), 1.40 (d, *J* = 5.9 Hz, 9H), 1.23 (d, *J=* 11.2 Hz, 6H).

### Example 105: (R)-1-(3-(1-((7-(Azetidin-3-yloxy)-6-(2-methoxyethoxy)-2-methylquinazolin-4-yl)amino)eth yl)-2-fluorohenl-1,1-difluoro-2-methylpropan-2-ol

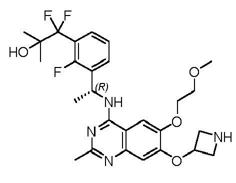

LC-MS: m/z 535 (M+H)⁺.

### Example 106: (R)-1,1-Difluoro-1-(2-fluoro-3-(1-((6-(2-methoxyethoxy)-2-methyl-7-((1-methylazetidin-3-y l)oxy)quinazoline-4-quinazoline)amino)ethyl)phenyl)-2-methylpropan-2-ol

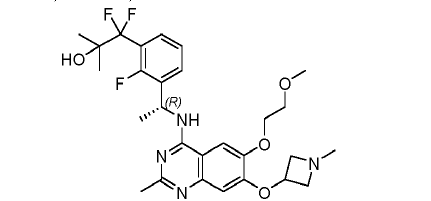

LC-MS: m/z 549 (M+H)⁺.

### Example 107: (R)-1-(3-((4-((1-(3-(1,1-Difluoro-2-hydroxy-2-methylpropyl)-2-fluorophenyl)ethyl)amino)-6-(2-methoxyethoxy)-2-methylquinazolin-7-yl)oxy)azetidin-1-yl)ethan-1-one

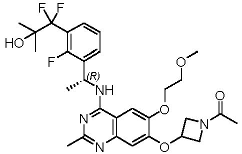

LC-MS: m/z 577 (M+H)⁺.

### Example 108: (R)-1,1-Difluoro-1-(2-fluoro-3-(1-(6-(2-methoxyethoxy)-2-methyl-7-(pyridin-3-yloxy)quina zolin-4-yl)aminoethyl)phenyl)-2-methylpropan-2-ol

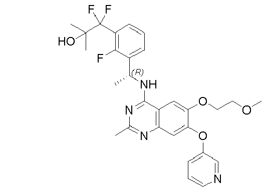

LC-MS: m/z 557 (M+H)⁺. ¹H NMR (400 MHz, DMSO) δ 8.52 - 8.37 (m, 1H), 8.24 (d, *J* = 16.4 Hz, 1H), 8.02 (s, 1H), 7.60 (s,1H), 7.45 (s, 2H), 7.31 (s, 1H), 7.23 (s, 1H), 7.05 (s, 1H), 5.79 (s, 1H), 5.32 (s, 1H), 4.26 (s, 2H), 3.62 (s,2H), 3.22 (s, 3H), 2.28 (s, 3H), 1.59 (s, 3H), 1.21 - 1.14 (m, 6H).

### Example 109: (R)-1,1-Difluoro-1-(2-fluoro-3-(1-((7-fluoro-6-(2-methoxyethoxy)-2-methylquinazolin-4-yl) amino)ethyl)phenyl)-2-methylpropan-2-ol

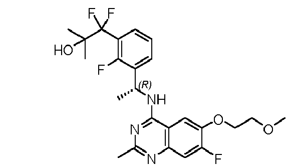

LC-MS: m/z 482 (M+H)⁺. ¹H NMR (400 MHz, DMSO) δ 8.28 (d, *J* = 8.0 Hz, 1H), 8.02 (d, *J* = 8.0 Hz, 1H), 7.59 (t, *J* = 8.0 Hz, 1H), 7.39 (d, *J* = 8.0 Hz, 1H), 7.33-7.30 (m, 1H), 7.21 (d, *J* = 8.0 Hz, 1H), 5.80 (m, 1H), 5.33 (s, 1H), 4.36-4.33 (m, 2H), 3.79 (t, *J* = 4.0 Hz, 2H), 3.34 (s, 3H), 2.31 (s, 3H), 1.60 (d, *J=* 8.0 Hz, 3H), 1.24 (s, 3H), 1.21 (s, 3H).

### Example 110: (R)-1-(3-(1-((7-Chloro-6-(2-methoxyethoxy)-2-methylquinazolin-4-yl)amino)ethyl)-2-fluor ophenyl)-1,1-difluoro-2-methylpropan-2-ol

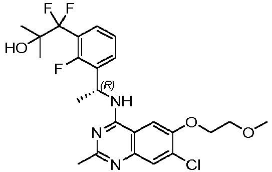

LC-MS: m/z 498 (M+H)⁺. ¹H NMR (400 MHz, DMSO) δ 8.41 (d, *J* = 8.0 Hz, 1H), 7.99 (s, 1H), 7.69 (s, 1H), 7.60 (d, *J* = 6.0 Hz, 1H), 7.33-7.30 (m, 1H), 7.24-7.20 (m, 1H), 5.80 (m, 1H), 5.34 (s, 1H), 4.36-4.33 (m, 2H), 3.81 (t, *J=* 4.0 Hz, 2H), 3.39 (s, 3H), 2.51 (s, 3H), 1.61 (d, *J* = 7.2 Hz, 3H), 1.24 (s, 3H), 1.21 (s, 3H).

### Example 111: (R)-1,1-Difluoro-1-(2-fluoro-3-(1-((6-(2-methoxyethoxy)-2-methyl-7-(trifluoromethyl)quin azolin-4-yl)amino)ethyl)phenyl)-2-methylpropan-2-ol

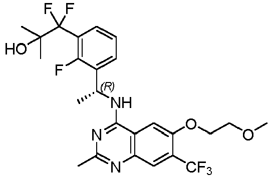

LC-MS: m/z 532 (M+H)⁺.

### Example 112: (R)-1,1-Difluoro-1-(2-fluoro-3-(1-((fluoro-6-(2-methoxyethoxy)-2-methyl-7-(trifluorometho xy)quinazolin-4-yl)amino)ethyl)phenyl)-2-methylpropan-2-ol

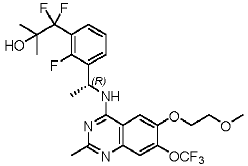

LC-MS: m/z 548 (M+H)⁺.

### Example 113: (R)-1-(3-Amino-5-(1-((6,7-bis(2-methoxyethoxy)-2-methylquinazolin-4-yl)amino)ethyl)phe nyl)-1,1-difluoro-2-methylpropan-2-ol

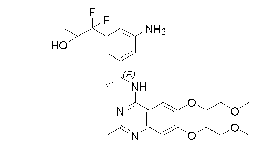

LC-MS: m/z 535 (M+H)⁺. ¹H NMR (400 MHz, DMSO) δ 7.89 (d, *J* = 8.0 Hz, 1H), 7.73 (s, 1H), 7.03 (s, 1H), 6.70 (d, *J* = 12.1 Hz, 2H), 6.56 (d, *J=* 4.9 Hz, 1H), 5.61 - 5.47 (m, 1H), 5.15 (s, 3H), 4.27 - 4.14 (m, 4H), 3.83 - 3.62 (m, 4H), 3.36 (s, 3H), 3.34 (s, 3H), 2.34 (s, 3H), 1.53 (d, *J=* 7.0 Hz,3H), 1.12 (s, 6H).

### Example 114: (R)-N-(1-(6-Amino-4-(trifluoromethyl)pyridin-2-yl)ethyl)-6,7-bis(2-methoxyethoxy)-2-met hylquinazolin-4-amine

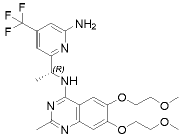

LC-MS: m/z 496 (M+H)⁺. ¹H NMR (400 MHz, DMSO) δ 7.95 (d, *J* = 7.8 Hz, 1H), 7.76 (s, 1H), 7.05 (s, 1H), 6.77 (s, 1H), 6.57 (s, 1H), 6.46 (s, 2H), 5.54 - 5.36 (m, 1H), 4.23 (dt, *J* = 10.2, 5.2 Hz, 4H), 3.74 (dt, *J* = 11.1, 4.5 Hz, 4H), 3.36 (s, 3H), 3.34 (s,3H), 2.32 (s, 3H), 1.56 (d, *J* = 7.1 Hz, 3H).

### Example 115: (R)-1-(3-Amino-5-(1-(7-methoxy-2-methyl-6-(2-(pyridine-3-oxy)ethoxy)quinazolin-4-yl)am ino)ethyl)phenyl-1,1-difluoro-2-methylpropan-2-ol

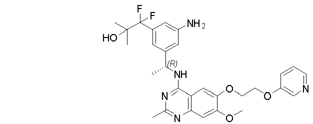

LC-MS: m/z 554 (M+H)⁺. ¹H NMR (400 MHz, DMSO) δ 8.37 (d, *J* = 2.9 Hz, 1H), 8.20 (dd, *J* = 4.6, 1.1 Hz, 1H), 7.93 (d, *J =* 8.0 Hz, 1H), 7.80 (s, 1H), 7.50 (dd, *J* = 8.4, 1.7 Hz, 1H), 7.36 (dd, *J* = 8.4, 4.6 Hz, 1H), 7.04 (s, 1H), 6.70 (d, *J* = 10.3 Hz, 2H), 6.55 (s, 1H), 5.60 - 5.48 (m, 1H), 5.18 (s, 2H), 4.47 (dd, *J* = 20.2, 3.9 Hz, 4H), 3.86 (s, 3H), 2.35 (s, 3H), 1.54 (d, *J* = 7.0 Hz, 3H), 1.12 (s, 6H).

### Example 116: (R)-1-(3-Amino-5-(1-((6-(2-cyclopropoxyethoxy)-7-methoxy-2-methylquinazolin-4-yl)amin o)ethyl)phenyl)-1,1-difluoro-2-methylpropyl-2-ol

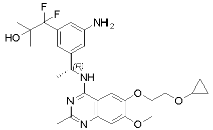

LC-MS: m/z 517 (M+H)⁺. 1H NMR (400 MHz, DMSO) δ 7.89 (d, *J* = 8.0 Hz, 1H), 7.71 (s, 1H), 7.02 (s, 1H), 6.74 - 6.66 (m, 2H), 6.56 (t, *J* = 1.9 Hz, 1H), 5.59 - 5.47 (m, 1H), 5.16 (s, 2H), 5.08 (s, 1H), 4.19 (dd, *J* = 5.9, 4.0 Hz, 2H), 3.87 (s, 3H), 3.92 - 3.81 (m, 2H), 3.46 - 3.38 (m, 1H), 2.35 (s, 3H), 1.54 (d, *J=* 7.0 Hz, 3H), 1.12 (s, 6H), 0.56 - 0.41 (m, 4H).

### Example 117: (R)-1-(3-Amino-5-(1-((7-methoxy-2-methyl-6-(2-(oxetan-3-yloxy)ethoxy)quinazolin-4-yl)a mino)ethyl)phenyl)-1,1-difluoro-2-methylpropyl-2-ol

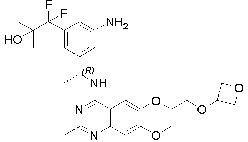

LC-MS: m/z 533 (M+H)⁺. 1H NMR (400 MHz, DMSO) δ 7.90 (d, *J =* 8.1 Hz, 1H), 7.72 (s, 1H), 7.03 (s, 1H), 6.74 - 6.66 (m, 2H), 6.56 (t, *J* = 1.9 Hz, 1H), 5.59 - 5.47 (m, 1H), 5.17 (s, 2H), 5.09 (s, 1H), 4.76 - 4.62 (m, 3H), 4.50 - 4.40 (m, 2H), 4.20 (dd, *J=* 5.8, 3.8 Hz, 2H), 3.88 (s, 3H), 3.79 (dd, *J* = 5.8, 3.7 Hz, 2H), 2.35 (s, 3H), 1.54 (d, *J* = 7.0 Hz, 3H), 1.12 (s, 6H).

### Example 118: 1-(3-Amino-5-(1R)-1-(6-(2-(2,2-difluorocyclopropoxy)ethoxy)-7-methoxy-2-methylquinazo lin-4-yl)aminoethyl)phenyl)-1,1-difluoro-2-methylpropan-2-ol

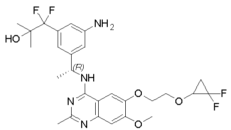

LC-MS: m/z 553 (M+H)⁺. ¹H NMR (400 MHz, DMSO) δ 7.89 (d, *J* = 8.0 Hz, 1H), 7.72 (s, 1H), 7.03 (s, 1H), 6.69 (d, *J* = 11.5 Hz, 2H), 6.55 (s, 1H), 5.62 - 5.46 (m, 1H), 5.16 (s, 2H), 5.07 (s, 1H), 4.23 (d, *J* = 4.5 Hz, 2H), 4.11 - 3.95 (m, 3H), 3.87 (s, 3H), 2.34 (s, 3H), 1.81 - 1.44 (m, 5H), 1.19 - 1.07 (m, 6H).

### Example 119: (R)-1-(3-Amino-5-(1-(7-methoxy-6-(2-(2-methoxyethoxy)ethoxy)-2-methylquinazolin-4-yl) amino)ethylphenyl)-1,1-difluoro-2-methylpropan-2-ol

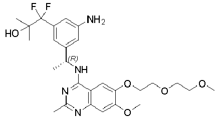

LC-MS: m/z 535 (M+H)⁺. ¹H NMR (400 MHz, DMSO) δ 7.89 (d, *J* = 8.0 Hz, 1H), 7.72 (s, 1H), 7.02 (s, 1H), 6.70 (d, *J* = 11.6 Hz, 2H), 6.55 (s, 1H), 5.59 - 5.42 (m, 1H), 5.16 (s, 2H), 4.20 (t, *J* = 4.8 Hz, 2H), 3.92 - 3.77 (m, 5H), 3.63 (dd, *J* = 5.6, 3.9 Hz, 2H), 3.48 (dd, *J* = 5.6, 3.8 Hz, 2H), 3.26 (s, 3H), 2.34 (s, 4H), 1.53 (d, *J* = 7.0 Hz, 3H), 1.10 (d, *J* = 10.9 Hz, 6H).

### Example 120: (R)-N-(3-(1,1-Difluoro-2-hydroxy-2-methylpropyl)-5-(1-(7-methoxy-6-(2-methoxyethoxy)-2-methylquinazolin-4-ylamino)ethyl)phenylacetamide

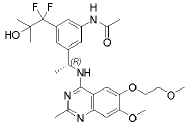

LC-MS: m/z 533 (M+H)⁺. ¹H NMR (400 MHz, DMSO) δ 10.01 (s, 1H), 8.03 (s, 1H), 7.75 (d, *J* = 12.1 Hz, 2H), 7.61 (s, 1H), 7.26 (s, 1H), 7.04 (s, 1H), 5.65 - 5.51 (m, 1H), 5.19 (s, 1H), 4.27 - 4.15 (m, 2H), 3.88 (s, 3H), 3.81 - 3.70 (m, 2H), 3.37 (s, 3H), 2.35 (s, 3H), 2.02 (s, 3H), 1.60 (d, *J=* 7.1 Hz, 3H), 1.14 (s, 6H).

### Example 121: Methyl (R)-(3-(1,1-difluoro-2-hydroxy-2-methylpropyl)-5-(1-(7-methoxy-6-(2-methoxyethoxy)-2-m ethylquinazolin-4-ylamino)ethyl)phenylcarbamate

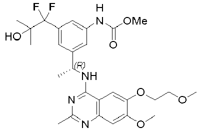

LC-MS: m/z 549 (M+H)⁺. ¹H NMR (400 MHz, DMSO) δ 9.70 (s, 1H), 7.98 (d, *J* = 7.7 Hz, 1H), 7.72 (s, 1H), 7.64 (s, 1H), 7.50 (s, 1H), 7.21 (s, 1H), 7.02 (s, 1H), 5.65 - 5.51 (m, 1H), 5.17 (s, 1H), 4.30 - 4.15 (m, 2H), 3.87 (s, 3H), 3.79 - 3.71 (m, 2H), 3.64 (s, 3H), 2.34 (s, 3H), 1.58 (d, *J=* 7.0 Hz, 3H), 1.12 (s, 6H).

### Example 122: (R)-1-(3-Amino-5-(1-(7-methoxy-6-(2-(2-methoxyethoxy)ethoxy)-2-methylquinazolin-4-yl) amino)ethylphenyl)-1,1-difluoro-2-methylpropan-2-ol

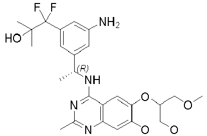

LC-MS: m/z 535 (M+H)⁺. ¹H NMR (400 MHz, DMSO) δ 7.90 (d, *J* = 8.0 Hz, 1H), 7.84 (s, 1H), 7.03 (s, 1H), 6.72 (d, *J* = 12.9 Hz, 2H), 6.58 (s, 1H), 5.56 (d, *J* = 7.3 Hz, 1H), 5.16 (s, 2H), 5.06 (s, 1H), 4.96 - 4.83 (m, 1H), 3.88 (s, 3H), 3.69 - 3.52 (m, 4H), 3.30 (s, 6H), 2.36 (s, 3H), 1.55 (d, *J* = 7.0 Hz, 3H), 1.13 (d, *J* = 2.7 Hz, 6H).

### Example 123: (R)-1-(3-Amino-5-(1-((6-(2-methoxyethoxy)-2-methyl-7-(1H-pyrazolyl-3-yl)quinazolin-4-yl )amino)ethyl)phenyl)-1,1-difluoro-2-methylpropan-2-ol

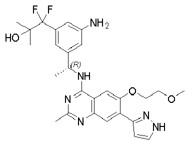

LC-MS: m/z 527 (M+H)⁺. ¹H NMR (400 MHz, DMSO) δ 13.05 (s, 1H), 8.14 (s, 2H), 7.82 (d, *J* = 23.4 Hz, 2H), 6.98 (s, 1H), 6.73 (d, *J* = 10.4 Hz, 2H), 6.57 (s, 1H), 5.64 - 5.50 (m, 1H), 5.16 (s, 2H), 5.08 (s, 1H), 4.34 (s, 2H), 3.85 (s, 2H), 3.39 (s, 3H), 2.39 (s, 3H), 1.58 (d, *J* = 6.9 Hz, 3H), 1.13 (s, 6H).

### Example 124: (R)-4-((1-(3-Amino-5-(1,1-difluoro-2-hydroxy-2-methylpropyl)phenyl)ethyl)amino)-6-(2-m ethoxyethoxy)-2-methylquinazoline-7-cyano

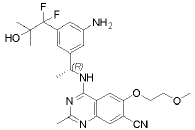

LC-MS: m/z 486 (M+H)⁺. ¹H NMR (400 MHz, DMSO) δ 8.41 (d, *J* = 7.9 Hz, 1H), 8.06 (s, 1H), 8.00 (s, 1H), 6.74 - 6.66 (m, 2H), 6.57 (t, *J* = 1.9 Hz, 1H), 5.60 - 5.50 (m, 1H), 5.19 (s, 2H), 5.10 (s, 1H), 4.41 - 4.33 (m, 2H), 3.83 - 3.76 (m, 2H), 3.38 (s, 3H), 2.39 (s, 3H), 1.57 (d, *J* = 7.0 Hz, 3H), 1.12 (s, 6H).

### Example 125: (R)-1-(5-Amino-2-fluoro-3-(1-((7-methoxy-6-(2-methoxyethoxy)-2-methylquinazolin-4-yl)a mino)ethyl)-2-fluorophenyl)-1,1-difluoro-2-methylpropan-2-ol

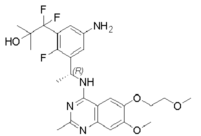

LC-MS: m/z 509 (M+H)⁺.

### Example 126: (R)-1-(5-Amino-3-(1-((6-(2-cyclopropoxyethoxy)-7-methoxy-2-methylquinazolin-4-yl)amin o)ethyl)-2-fluorophenyl)-1,1-difluoro-2-methylpropan-2-ol

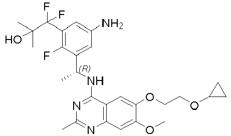

LC-MS: m/z 535 (M+H)⁺.

### Example 127: (R)-1-(5-Amino-2-fluoro-3-(1-((7-methoxy-2-methyl-6-(2-(oxetan-3-yloxy)ethoxy)quinazoli n-4-yl)amino)ethyl)phenyl)-1,1-difluoro-2-methylpropan-2-ol

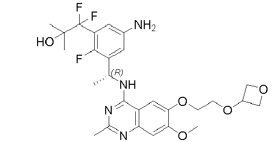

LC-MS: m/z 551 (M+H)⁺.

### Example 128: (R)-1-(5-Amino-2-chloro-3-(1-((7-methoxy-2-methyl-6-(2-methoxyethoxy)2-methylquinazo lin-4-yl)amino)ethyl)phenyl)-1,1-difluoro-2-methylpropan-2-ol

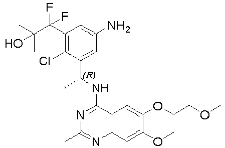

LC-MS: m/z 525 (M+H)⁺.

### Example 129: (R)-1-(5-Amino-2-chloro-3-(1-((6-(2-cyclopropoxyethoxy)-7-methoxy-2-methylquinazolin-4-yl)amino)ethyl)phenyl)-1,1-difluoro-2-methylpropan-2-ol

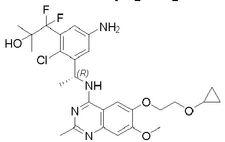

LC-MS: m/z 535 (M+H)⁺.

### Example 130: (R)-1-(5-Amino-2-chloro-3-(1-((7-methoxy-2-methyl-6-(2-(oxetan-3-yloxy)ethoxy)quinazoli n-4-yl)amino)ethyl)phenyl)-1,1-difluoro-2-methylpropan-2-ol

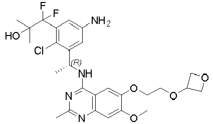

LC-MS: m/z 567 (M+H)⁺.

### Example 131: (R)-1,1-Difluoro-1-(3-(1-((7-methoxy-6-(2-methoxyethoxy)-2-methylquinazolin-4-yl)amino )ethyl)phenyl)-1,1-difluoro-2-methylpropan-2-ol

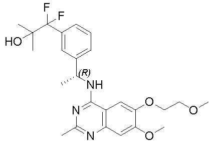

LC-MS: m/z 476 (M+H)⁺.

### Example 132: (R)-1-(3-(1-((6-(2-Cyclopropoxyethoxy)-7-methoxy-2-methylquinazolin-4-yl)amino)ethyl)p henyl)-1,1-difluoro-2-methylpropan-2-ol

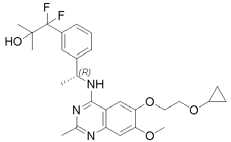

LC-MS: m/z 502 (M+H)⁺.

### Example 133: (R)-1,1-Difluoro-1-(3-(1-((7-methoxy-2-methyl-6-(2-(oxetan-3-yloxy)ethoxy)quinazolin-4-yl )amino)ethyl)phenyl)-1,1-difluoro-2-methylpropan-2-ol

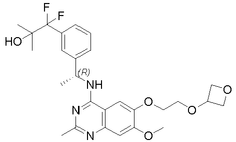

LC-MS: m/z 518 (M+H)⁺.

### Example 134: N-(1-(4-Amino-6-(1-methylcyclopropyl)pyridin-2-yl)ethyl)-7-methoxy-6-(2-methoxyethoxy )-2-methylquinazolin-4-amine

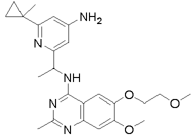

LC-MS: m/z 438 (M+H)⁺. ¹H NMR (400 MHz, DMSO) δ 7.86 (d, *J* = 7.9 Hz, 1H), 7.63 (s, 1H), 7.04 (s, 1H), 6.37 (s, 1H), 6.29 (s, 1H), 5.79 (s, 2H), 5.38 - 5.34 (m, 1H), 4.23 - 4.21 (m, 2H), 3.88 (s, 3H), 3.76 - 3.74 (m, 2H), 3.35 (s, 3H), 2.35 (s, 3H), 1.50 (d, *J* = 6.9 Hz, 3H), 1.38 (s, 3H), 1.24-1.12 (m, 2H), 0.65 (m, 2H).

### Example 135: N-(1-(4-Amino-6-(1-methylcyclopropyl)pyridin-2-yl)ethyl)-6-(2-cyclopropoxyethoxy)-7-me thoxy-2-methylquinazolin-4-amine

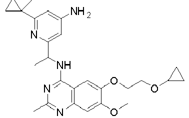

LC-MS: m/z 464 (M+H)⁺. ¹H NMR (400 MHz, DMSO) δ 7.86 (d, *J* = 7.9 Hz, 1H), 7.63 (s, 1H), 7.04 (s, 1H), 6.37 (d, *J=* 1.6 Hz, 1H), 6.29 (s, 1H), 5.79 (s, 2H), 5.46 - 5.17 (m, 1H), 4.28 - 4.13 (m, 2H), 3.95 - 3.78 (m, 5H), 3.46 - 3.35 (m, 1H), 2.35 (s, 3H), 1.50 (d, *J=* 6.9 Hz, 3H), 1.38 (s, 3H), 1.15 (m 2H), 0.65 (m, 2H), 0.56 - 0.36 (m, 4H).

### Example 136: N-(1-(4-Amino-6-(1-methylcyclopropyl)pyridin-2-yl)ethyl)-7-methoxy-2-methyl-6-(2-(oxet an-3-yloxy)ethoxy)quinazolin-4-amine

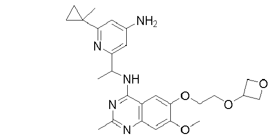

LC-MS: m/z 480 (M+H)⁺. ¹H NMR (400 MHz, DMSO) δ 7.88 (m, 1H), 7.64 (s, 1H), 7.05 (s, 1H), 6.37 (s, 1H), 6.29 (s, 1H), 5.79 (s, 2H), 5.37 (m, 1H), 4.72 - 4.69 (m, 3H), 4.46 - 4.42 (m, 2H), 4.22 (m, 2H), 3.89 (s, 3H), 3.81 - 3.79 (m, 2H), 2.35 (s, 3H), 1.49 (d, *J=* 8.0 Hz, 3H), 1.38 (s, 3H), 1.19 (m, 2H), 0.66 (m, 2H).

### Example 137: N-(1-(4-Amino-6-(1-fluorocyclopropyl)pyridin-2-yl)ethyl)-7-methoxy-6-(2-methoxyethoxy) -2-methylquinazolin-4-amine

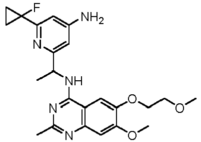

LC-MS: m/z 442 (M+H)⁺. ¹H NMR (400 MHz, DMSO) δ 7.96-7.94 (m, 2H), 7.74 (s, 1H), 6.97 (s, 1H), 6.52 (d, *J* = 8.0 Hz, 1H), 6.24 (d, *J* = 8.0 Hz, 1H), 6.14 (s, 2H), 4.19 (m, 2H), 3.85 (s, 3H), 3.74 - 3.72 (m, 2H), 3.35 (s, 3H), 2.28 (s, 3H), 1.88 (m, 1H), 1.62-1.57 (m, 2H), 1.48 (d, *J* = 8.0 Hz, 3H), 0.93 (m, 1H).

### Example 138: (R)-N-(1-(3-(1,1-Difluoro-2-methoxyethyl)-2-fluorophenyl)ethyl)-7-methoxy-6-(2-methoxy ethoxy)-2-methylquinazolin-4-amine

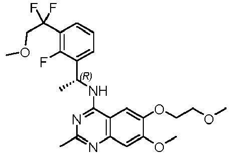

LC-MS: m/z 480 (M+H)⁺.

### Example 139: (R)-N-(1-(3-(2-Cyclopropyloxy-1,1-difluoroethyl)-2-fluorophenyl)ethyl)-7-methoxy-6-(2-m ethoxyethoxy)-2-methylquinazolin-4-amine

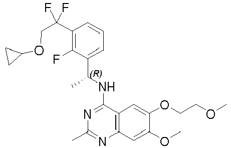

LC-MS: m/z 506 (M+H)⁺.

### Example 140: (R)-N-(1-(3-(1,1-Difluoro-2-(oxetan-3-yloxy)ethyl)-2-fluorophenyl)ethyl)-7-methoxy-6-(2-methoxyethoxy)-2-methylquinazolin-4-amine

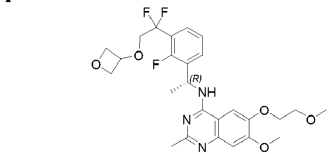

LC-MS: m/z 522 (M+H)⁺.

### Example 141: N-((1R)-1-(3-(Difluoro(tetrahydrofuran-2-yl)methyl)-2-fluorophenyl)ethyl)-7-methoxy-6-( 2-methoxyethoxy)-2-methylquinazolin-4-amine isomer A and isomer B)

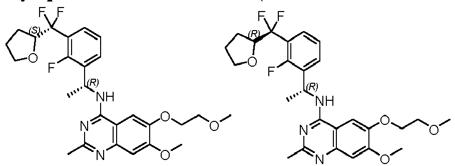

### Isomer A

LC-MS: m/z 506 (M+H)⁺. ¹H NMR (400 MHz, DMSO) δ 8.04-8.01 (m, 1H), 7.75 (s, 1H), 7.64-7.60 (m, 1H), 7.40-7.37 (m, 1H), 7.25-7.22 (m, 1H), 7.03 (s, 1H), 5.81-5.75 (m, 1H), 4.54-4.48 (m, 1H), 4.26-4.23 (m, 2H), 3.87 (s, 3H), 3.78-3.74 (m, 4H), 3.36 (s, 3H), 2.29 (s, 3H), 2.03-1.94 (m, 2H), 1.85-1.82 (m, 2H), 1.59 (d, *J=* 4 Hz, 2H)

### Isomer B

LC-MS: m/z 506 (M+H)⁺. ¹H NMR (400 MHz, DMSO) δ 8.03-8.01 (m, 1H), 7.75 (s, 1H), 7.64-7.60 (m, 1H), 7.40-7.37 (m, 1H), 7.25-7.22 (m, 1H), 7.03 (s, 1H), 5.81-5.75 (m, 1H), 4.54-4.48 (m, 1H), 4.26-4.23 (m, 2H), 3.87 (s, 3H), 3.76-3.74 (m, 4H), 3.36 (s, 3H), 2.29 (s, 3H), 2.03-1.94 (m, 2H), 1.86-1.82 (m, 2H), 1.59 (d, *J=* 4 Hz, 2H)

### Example 142: N-((1R)-1-(3-(Difluoro(4-methylmorpholin-2-yl)methyl)-2-fluorophenyl)ethyl)-7-methoxy-6-(2-methoxyethoxy)-2-methylquinazolin-4-amine

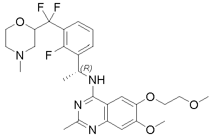

LC-MS: m/z 536 (M+H)⁺.

### Example 143: (R)-1,1-Difluoro-1-(2-fluoro-3-(1-((8-fluoro-7-methoxy-6-(2-methoxyethoxy)-2-methylquin azolin-4-yl)amino)ethyl)phenyl)-2-methylpropan-2-ol

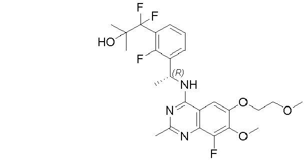

LC-MS: m/z 512 (M+H)⁺.

### Example 144: (R)-1-(3-(1-((6-(2-Cyclopropoxyethoxy)-8-fluoro-7-methoxy-2-methylquinazolin-4-yl)amin o)ethyl)-2-fluorophenyl)-1,1-difluoro-2-methylpropan-2-ol

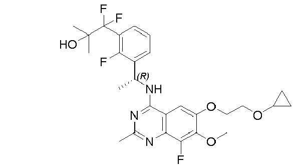

LC-MS: m/z 538 (M+H)⁺.

### Example 145: (R)-1,1-Difluoro-1-(2-fluoro-3-(1-((8-fluoro-7-methoxy-2-methyl-6-(2-(oxetan-3-yloxy)etho xy)quinazolin-4-yl)amino)ethyl)phenyl)-2-methylpropan-2-ol

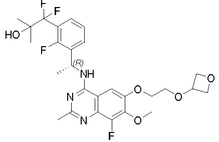

LC-MS: m/z 554 (M+H)⁺.

### Preparation of Examples 146-148

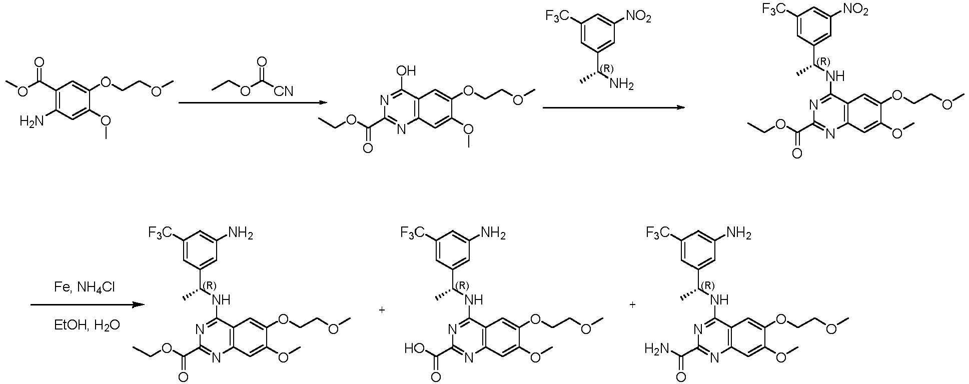

Step 1: Methyl 2-amino-4-methoxy-5-(2-methoxyethoxy)benzoate (1.2 g), ethyl cyanoformate (0.932 g) and a 2 M solution of HCl in dioxane (20 mL) were heated in a sealed tube for 20 h, and then concentrated under reduced pressure. DMF and DBU were added to the residue to dissolve the solid. The mixture was then separated by preparative liquid chromatography to give the target product (230 mg, yield: 15%). LC-MS: m/z 323 (M+H)⁺.

Step 2: Ethyl 4-hydroxy-7-methoxy-6-(2-methoxyethoxy)quinazoline-2-carboxylate (230 mg) was dissolved in DMF (5 mL), DBU (540 mg) was added, and BOP (628 mg) was added slowly in an ice-water bath. The mixture was stirred at room temperature for 1 h, and (R)-1-(3-nitro-5-(trifluoromethyl)phenyl)ethan-1-amine (201 mg) was then added. The mixture was stirred at 100 °C for 16 h, and then separated by preparative liquid chromatography to give the target product (150 mg, yield: 39%). LC-MS: m/z 540 (M+H)⁺.

Step 3: Ethyl 4-hydroxy-7-methoxy-6-(2-methoxyethoxy)quinazoline-2-carboxylate (20 mg, 0.04 mmol) was added to ethanol (5 mL) and water (2 mL), and ammonium chloride (42 mg, 0.80 mmol) and iron powder (23 mg, 0.40 mmol) were added successively. The mixture was heated to 80 °C, stirred for 3 h, and then separated by preparative liquid chromatography to give the target product.

### Example 146: Ethyl (R)-4-((1-(3-amino-5-(trifluoromethyl)phenyl)ethyl)amino)-7-methoxy-6-(2-methoxyethox y)quinazoline-2-carboxylate

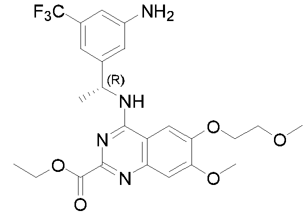

LC-MS: m/z 495 (M+H)⁺. ¹H NMR (400 MHz, DMSO) δ 8.29 (d, *J* = 7.5 Hz, 1H), 7.81 (s, 1H), 7.27 (s, 1H), 6.88 (m, 2H), 6.71 (s, 1H), 5.56 (m, 3H), 4.28 (d, *J* = 6.6 Hz, 4H), 3.93 (s, 3H), 3.77 (m, 2H), 3.36 (s, 3H), 1.58 (d, *J* = 6.6 Hz, 3H), 1.30 (t, *J* = 6.9 Hz, 3H).

### Example 147: (R)-4-((1-(3-Amino-5-(trifluoromethyl)phenyl)ethyl)amino)-7-methoxy-6-(2-methoxyethox y)quinazoline-2-carboxylic acid

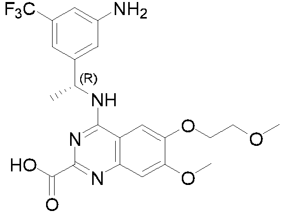

LC-MS: m/z 467 (M+H)⁺. ¹H NMR (400 MHz, DMSO) δ 8.39 (d, *J* = 7.4 Hz, 1H), 7.83 (s, 1H), 7.29 (s, 1H), 6.90 (s, 1H), 6.86 (s, 1H), 6.71 (s, 1H), 5.80 - 5.67 (m, 1H), 5.54 (s, 2H), 4.27 (d, *J* = 4.5 Hz, 2H), 3.92 (s, 3H), 3.77 (t, *J* = 4.4 Hz, 2H), 3.51 (s, 3H), 1.58 (d, *J* = 7.0 Hz, 3H).

### Example 148: (R)-4-((1-(3-Amino-5-(trifluoromethyl)phenyl)ethyl)amino)-7-methoxy-6-(2-methoxyethox y)quinazoline-2-carboxamide

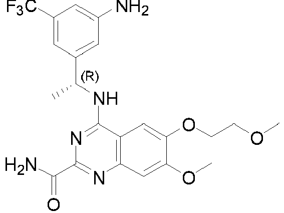

LC-MS: m/z 466 (M+H)⁺. ¹H NMR (400 MHz, DMSO) δ 8.22 (d, *J* = 8.0 Hz, 1H), 7.82 (s, 2H), 7.46 (s, 1H), 7.23 (s, 1H), 6.91 (s, 1H), 6.86 (s, 1H), 6.70 (s, 1H), 5.73 - 5.58 (m, 1H), 5.51 (s, 2H), 4.27 (d, *J* = 4.1 Hz, 2H), 3.92 (s, 3H), 3.77 (t, *J=* 4.5 Hz, 2H), 3.36 (s, 3H), 1.57 (d, *J* = 7.1 Hz, 3H).

### Example 149: (R)-N-(1-(3-Amino-5-(trifluoromethyl)phenyl)ethyl)-2-cyclopropyl-6,7-dimethoxyquinazol in-4-amine

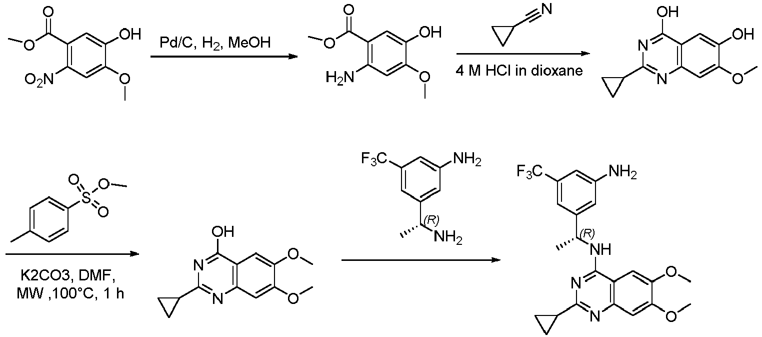

### Step 1: Preparation of methyl 2-amino-5-hydroxy-4-methylbenzoate

Methyl 5-hydroxy-4-methoxy-2-nitrobenzoate (10 g) was dispersed in MeOH (100 mL), and palladium on carbon (10% wt, 1.5 g) was added. The system was purged three times with hydrogen. The mixture was stirred at room temperature overnight. THF was added to the reaction solution to dissolve the solid, the mixture was filtered through celite, and the filtrate was concentrated under reduced pressure to give the target product (8.0 g, yield: 92%). which was used directly in the next step without purification. LC-MS: m/z 198 (M+H)⁺.

### Step 2: Preparation of 2-cyclopropyl-7-methoxyquinazoline-4,6-diol

A mixture of methyl 2-amino-5-hydroxy-4-methylbenzoate (6 g), cyclopropanecarbonitrile (6.1 g) and a 4 M solution of HCl in dioxane (100 mL) was heated to 110 °C and stirred overnight. The resulting reaction solution was cooled and filtered. The filter cake was collected and air-dried to give the target product (6.0 g, yield: 85%). which was used directly in the next step without purification. LC-MS: m/z 233 (M+H)⁺.

### Step 3: Preparation of 2-cyclopropyl-6,7-dimethoxyquinazolin-4-ol

A mixture of 2-cyclopropyl-7-methoxyquinazoline-4,6-diol (400 mg), methyl p-toluenesulfonate (500 mg), potassium carbonate (476 mg) and DMF (5 mL) was heated to 100 °C and stirred overnight. The resulting mixture was separated by medium-pressure column chromatography to give the target product (50 mg, yield: 12%). LC-MS: m/z 247 (M+H)⁺.

### Step 4: Preparation of (R)-N (1-(3-amino-5-(trifluoromethyl)phenyl)ethyl)-2-cyclopropyl-6,7-dimethoxyquinazolin-4-amine

2-Cyclopropyl-6,7-dimethoxyquinazolin-4-ol (50 mg) was dissolved in DMF (5 mL), DBU (152 mg) was added, and then BOP (177 mg) was added slowly with cooling in an ice-water bath. The resulting mixture was stirred at room temperature for 1 h, and (R)-3-(1-aminoethyl)-5-(trifluoromethyl)aniline (49 mg) was then added. The reaction mixture was stirred at 100 °C for 16 h, then cooled, and separated by preparative chromatography to give the target product (21 mg, yield: 26%). LC-MS: m/z 433 (M+H)⁺. ¹H NMR (400 MHz, DMSO) δ 7.96 (d, *J=* 6.9 Hz, 1H), 7.68 (s, 1H), 6.98 (s, 1H), 6.83 (s, 1H), 6.79 - 6.76 (m, 1H), 6.68 (s, 1H), 5.49 (s, 2H), 5.39 - 5.22 (m, 1H), 3.90 (s, 3H), 3.86 (s, 3H), 1.86 (dd, *J* = 8.2, 3.6 Hz, 1H), 1.54 (d, *J=* 7.0 Hz, 3H), 0.98 (d, *J=* 4.6 Hz, 1H), 0.85 - 0.55 (m, 3H).

The following compounds were synthesized in the same manner as the compound of Example **149** with different starting materials:

### Example 150: (R)-N-(1-(3-Amino-5-(trifluoromethyl)phenyl)ethyl)-2-cyclopropyl-7-methoxy-6-(2-metho xyethoxy)quinazolin-4-amine

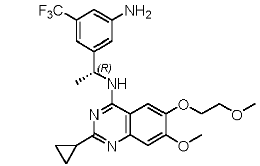

LC-MS: m/z 477 (M+H)⁺. ¹H NMR (400 MHz, DMSO) δ 7.93 (d, *J* = 7.1 Hz, 1H), 7.70 (s, 1H), 6.99 (s, 1H), 6.83 (s, 1H), 6.79 (s, 1H), 6.68 (s, 1H), 5.49 (s, 2H), 5.30 (t, *J* = 7.0 Hz, 1H), 4.21 (dd, *J* = 8.5, 4.3 Hz, 2H), 3.87 (s, 3H), 3.75 (t, *J* = 4.6 Hz, 2H), 1.86 (m, 1H), 1.53 (d, *J* = 7.0 Hz, 3H), 0.98 (m, 1H), 0.84 - 0.57 (m, 3H).

### Example 151: (R)-N-(1-(3-Amino-5-(trifluoromethyl)phenyl)ethyl)-2-(3,3-difluorocyclobutyl)-7-methoxy-6-(2-methoxyethoxy)quinazolin-4-amine

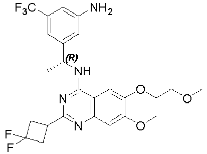

LC-MS: m/z 527 (M+H)⁺. ¹H NMR (400 MHz, DMSO) δ 8.38 (s, 1H), 7.90 (s, 1H), 7.09 (s, 1H), 6.87 (d, *J=* 14.5 Hz, 2H), 6.69 (s, 1H), 5.59 - 5.35 (m, 3H), 4.27 (dd, J = 9.9, 4.8 Hz, 2H), 3.89 (s, 3H), 3.76 (t, *J* = 4.6 Hz, 2H), 3.35 (s, 4H), 2.91 - 2.72 (m, 4H), 1.60 (d, *J* = 7.0 Hz, 3H).

### Example 152: (R)-1-(3-Amino-5-(1-((2-(3,3-difluorocyclobutyl)-7-methoxy-6-(2-methoxyethoxy)quinazoli n-4-yl)amino)ethyl)phenyl)-1,1-difluoro-2-methylpropan-2-ol

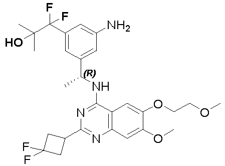

LC-MS: m/z 567 (M+H)⁺. ¹H NMR (400 MHz, DMSO) δ 8.06 (d, *J* = 7.3 Hz, 1H), 7.75 (s, 1H), 7.07 (s, 1H), 6.69 (s, 2H), 6.56 (s, 1H), 5.58 - 5.38 (m, 1H), 5.13 (s, 2H), 5.05 (s, 1H), 4.22 (d, *J=* 5.3 Hz, 2H), 3.89 (s, 3H), 3.81 - 3.70 (m, 2H), 3.37 (s, 3H), 3.26 - 3.20 (m, 1H), 2.94 - 2.72 (m, 4H), 1.56 (d, *J* = 7.0 Hz, 3H), 1.09 (s, 6H).

### Example 153: Preparation of (R)-1-(3-(1-(7-ethynyl-6-(2-methoxyethoxy)-2-methylquinazoline-4-amino)ethyl)-2-fluorop henyl)-1,1-difluoro-2-methylpropan-2-ol

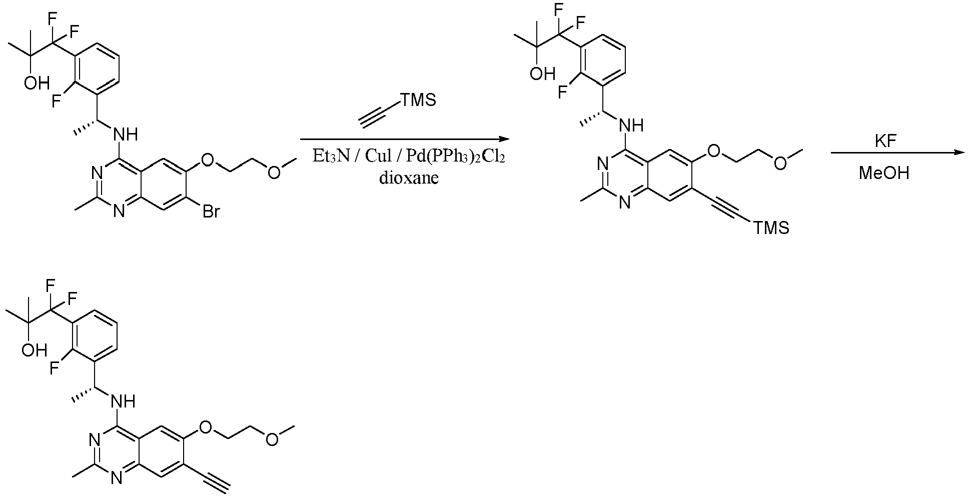

### Step 1: Preparation of (R)-1,1-difluoro-1-(2-fluoro-3-(1-((6-(2-methoxyethoxy)-2-methyl-7-((trimethylsilyl)ethyny l)quinazolin-4-yl)amino)ethyl)phenyl)-2-methylpropan-2-ol

In an argon atmosphere, (*R*)-1-(3-(1-(7-bromo-6-(2-methoxyethoxy)-2-methylquinazoline-4-amino)ethyl)-2-fluoropheny 1)-1,1-difluoro-2-methylpropan-2-ol (100 mg, 0.18 mmol) was added to dioxane (5 mL), and triethylamine (56 mg, 0.55 mmol), cuprous iodide (20 mg), Pd(PPh₃)₂Cl₂ (20 mg), and ethynyltrimethylsilane (54 mg, 0.55 mmol) were added at room temperature. The reaction solution was microwaved to 80 °C, reacted for 2 h, and then concentrated under reduced pressure to give the target product (120 mg). which was used directly in the next step without purification.

### Step 2: Preparation of (R)-1-(3-(1-(7-ethynyl-6-(2-methoxyethoxy)-2-methylquinazoline-4-amino)ethyl)-2-fluorop henyl)-1,1-difluoro-2-methylpropan-2-ol

(*R*)-1,1-difluoro-1-(2-fluoro-3-(1-((6-(2-methoxyethoxy)-2-methyl-7-((trimethylsilyl)ethynyl)q uinazolin-4-yl)amino)ethyl)phenyl)-2-methylpropan-2-ol (120 mg, 0.21 mmol) obtained in the last step was added to methanol (5 mL) at room temperature, and potassium fluoride (38 mg, 0.64 mmol) was then added. The resulting reaction solution was stirred at room temperature for 1 h, and then concentrated under reduced pressure. The residue was separated by preparative liquid chromatography to give the target product (65 mg, yield: 64%).

LC-MS: m/z 488 (M+H)⁺. ¹H NMR (400 MHz, DMSO) δ 8.32 (d, *J =* 7.2 Hz, 1H), 7.85 (s, 1H), 7.72 - 7.52 (m, 3H), 7.31 (t, *J=* 6.6 Hz, 1H), 7.23 (dd, *J=* 24.6, 16.9 Hz, 1H), 5.79 (t, *J* = 7.1 Hz, 1H), 5.31 (s, 1H), 4.48 (s, 1H), 4.38 - 4.22 (m, 2H), 3.79 (t, *J* = 4.7 Hz, 2H), 3.39 (s, 3H), 2.30 (s, 3H), 1.60 (d, *J=* 7.0 Hz, 3H), 1.22 (d, *J=* 10.9 Hz, 6H).

The following compounds were synthesized in the same manner as the compound of Example **153** with different starting materials:

### Example 154: (R)-1,1-Difluoro-1-(2-fluoro-3-(1-((6-(2-methoxyethoxy)-2-methyl-7-(prop-1-yn-1-yl)quina zolin-4-yl)amino)ethyl)phenyl)-2-methylpropan-2-ol

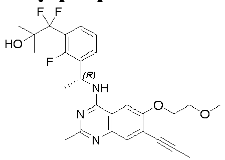

LC-MS: m/z 502 (M+H)⁺.

### Example 155: (R)-1-(3-(1-((7-(Cyclopropylethynyl)-6-(2-methoxyethoxy)-2-methylquinazoline-4-amino)e thyl)-2-fluorophenyl)-1,1-difluoro-2-methylpropan-2-ol

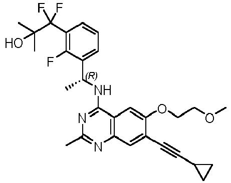

LC-MS: m/z 528 (M+H)⁺.

### Example 156: (R)-1-(3-(1-((6-(2-Cyclopropoxyethoxy)-7-ethynyl-2-methylquinazolin-4-yl)amino)ethyl)-2-fluorophenyl)-1,1-difluoro-2-methylpropan-2-ol

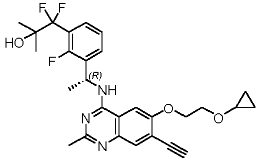

LC-MS: m/z 514 (M+H)⁺. ¹H NMR (400 MHz, DMSO) δ 8.32 (d, *J* = 7.2 Hz, 1H), 7.84 (s, 1H), 7.64 (s, 1H), 7.59 (t, *J* = 6.6 Hz, 1H), 7.31 (t, *J* = 6.7 Hz, 1H), 7.21 (t, *J* = 7.7 Hz, 1H), 5.78 (dd, *J* = 14.1, 7.0 Hz, 1H), 5.30 (s, 1H), 4.48 (s, 1H), 4.28 (t, *J* = 6.7 Hz, 2H), 3.89 (t, *J* = 4.6 Hz, 2H), 3.49 (m, 1H), 2.30 (s, 3H), 1.60 (d, *J* = 7.0 Hz, 3H), 1.22 (d, *J* = 10.8 Hz, 6H), 0.65 - 0.39 (m, 4H).

### Example 157: (R)-1-(3-(1-((7-Ethynyl-2-methyl-6-(2-(oxetan-3-yloxy)ethoxy)quinazolin-4-yl)amino)ethyl) -2-fluorophenyl)-1,1-difluoro-2-methylpropan-2-ol

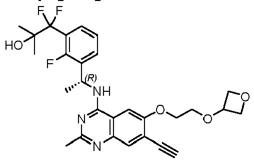

LC-MS: m/z 530 (M+H)⁺. ¹H NMR (400 MHz, DMSO) δ 8.34 (d, *J* = 8 Hz, 1H), 7.85 (s, 1H), 7.65 (s, 1H), 7.59 (m, 1H), 7.31 (m, 1H), 7.23-7.21 (m, 1H), 5.79 (m, 1H), 5.37 (brs, 1H), 4.74-4.72 (m, 3H), 4.53 (s, 1H), 4.48 - 4.46 (m, 2H), 4.31 - 4.30 (m, 2H), 3.84 (t, *J* = 4.0 Hz, 2H), 2.30 (s, 3H), 1.60 (d, *J=* 8.0 Hz, 3H), 1.22 (d, *J=* 8.0 Hz, 6H).

### Example 158: 1-(3-((R)-1-((7-Ethynyl-2-methyl-6-(((S)-tetrahydrofuran-3-yl)oxy)quinazolin-4-yl)amino) ethyl)-2-fluorophenyl)-1,1-difluoro-2-methylpropan-2-ol

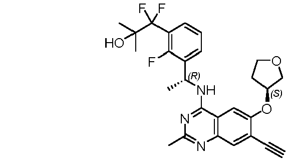

LC-MS: m/z 500 (M+H)⁺. ¹H NMR (400 MHz, DMSO) δ 8.32 (d, *J* = 6.5 Hz, 1H), 7.83 (s, 1H), 7.66 (s, 1H), 7.58 (t, *J* = 6.6 Hz, 1H), 7.31 (t, *J* = 6.6 Hz, 1H), 7.22 (t, *J* = 7.7 Hz, 1H), 5.80 (t, *J=* 7.1 Hz, 1H), 5.30 (d, *J=* 8.2 Hz, 1H), 5.28 (m, 1H), 4.49 (s, 1H), 4.04 (dd, *J=* 10.2, 4.6 Hz, 1H), 3.97 - 3.77 (m, 3H), 2.43 - 2.24 (m, 4H), 2.11 - 1.97 (m, 1H), 1.61 (d, *J* = 7.0 Hz, 3H), 1.22 (d, *J=* 10.5 Hz, 6H).

### Example 159: (R)-1-(3-(1-((7-Ethynyl-2-methyl-6-(2-(pyridin-3-yloxy)ethoxy)quinazolin-4-yl)amino)ethyl )-2-fluorophenyl)-1,1-difluoro-2-methylpropan-2-ol

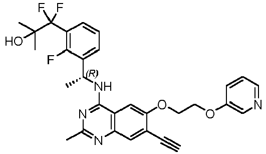

LC-MS: m/z 551 (M+H)⁺.

### Example 160: (R)-N-(2-((4-((1-(3-(1,1-Difluoro-2-hydroxy-2-methylpropyl)-2-fluorophenyl)ethyl)amino)-7-ethynyl-2-methylquinazolin-6-yl)oxy)ethyl)-N-methylmethanesulfonamide

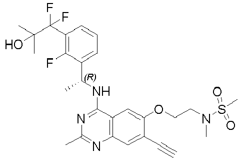

LC-MS: m/z 565 (M+H)⁺.

### Example 161: (R)-5-(4-((1-(3-(1,1-Difluoro-2-hydroxy-2-methylpropyl)-2-fluorophenyl)ethyl)amino)-7-et hynyl-2-methylquinazolin-6-yl)-1-methylpyridin-2(1H)-one

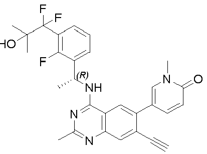

LC-MS: m/z 521 (M+H)⁺.

### Example 162: (R)-1-(4-(4-((1-(3-(1,1-Difluoro-2-hydroxy-2-methylpropyl)-2-fluorophenyl)ethyl)amino)-7 -ethynyl-2-methylquinazolin-6-yl)-4-methoxypiperidin-1-yl)ethan-1-one

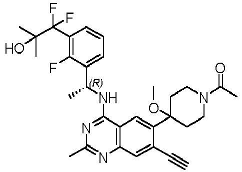

LC-MS: m/z 569 (M+H)⁺.

### Example 163: (R)-1-(3-Amino-5-(1-((7-ethynyl-6-(2-methoxyethoxy)-2-methylquinazolin-4-yl)amino)ethy l)phenyl)-1,1-difluoro-2-methylpropan-2-ol

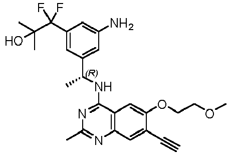

LC-MS: m/z 485 (M+H)⁺. ¹H NMR (400 MHz, DMSO) δ 8.18 (d, *J* = 8 Hz, 1H), 7.81 (s, 1H), 7.63 (s, 1H), 6.71-6.68 (m, 2H), 6.56 (s, 1H), 5.56-5.52 (m, 1H), 5.17 (s, 2H), 5.06 (s, 1H), 4.45 (s, 1H), 4.28 - 4.26 (m, 2H), 3.78 - 3.76 (m, 2H), 3.38 (s, 3H), 2.36(s, 3H), 1.56 (d, *J=* 4.0 Hz, 3H), 1.12 (s, 6H).

### Example 164: 1-(3-Amino-5-((R)-1-((7-ethynyl-2-methyl-6-(((S)-tetrahydrofuran-3-yl)oxy)quinazolin-4-y l)amino)ethyl)phenyl)-1,1-difluoro-2-methylpropan-2-ol

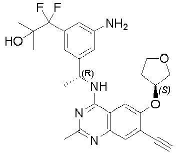

LC-MS: m/z 497 (M+H)⁺. ¹H NMR (400 MHz, DMSO) δ 8.22 (m, 1H), 7.79 (s, 1H), 7.64 (s, 1H), 6.71 (s, 1H), 6.68 (s, 1H), 6.56 (s, 1H), 5.57 (m, 1H), 5.23-5.20 (m, 3H), 5.08 (s, 1H), 4.00 (m, 1H), 3.92-3.82 (m, 3H), 2.37 (s, 3H), 2.32-2.30 (m, 1H), 1.56 (d, *J* = 8.0 Hz, 3H), 1.11 (s, 6H).

### Example 165: (R)-1-(3-(1-((7-Ethynyl-8-fluoro-6-(2-methoxyethoxy)-2-methylquinazolin-4-yl)amino)ethy l)-2-fluorophenyl)-1,1-difluoro-2-methylpropan-2-ol

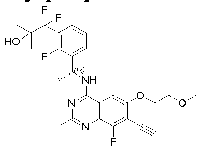

LC-MS: m/z 506 (M+H)⁺.

### Example 166: (R)-N-(1-(3-Amino-5-(trifluoromethyl)phenyl)ethyl)-7-ethynyl-6-(2-methoxyethoxy)-2-met hylquinazolin-4-amine

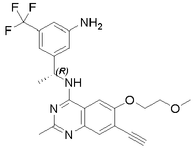

LC-MS: m/z 445 (M+H)⁺.

### Example 167: N-((R)-1-(3-Amino-5-(trifluoromethyl)phenyl)ethyl)-7-ethynyl-2-methyl-6-(((S)-tetrahydro furan-3-loxuinazolin-4-amine

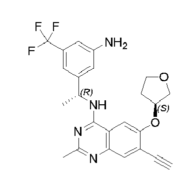

LC-MS: m/z 457 (M+H)⁺.

### Example 168: (R)-N-(1-(3-Amino-5-(trifluoromethyl)phenyl)ethyl)-6-(2-cyclopropoxyethoxy)-7-ethynyl-2 -methylquinazolin-4-amine

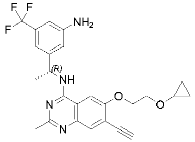

LC-MS: m/z 471 (M+H)⁺.

### Example 169: (R)-N-(1-(3-Amino-5-(trifluoromethyl)phenyl)ethyl)-7-ethynyl-2-methyl-6-(2-(oxetan-3-ylo xy)ethoxy)quinazolin-4-amine

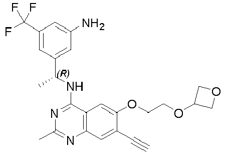

LC-MS: m/z 487 (M+H)⁺.

### Example 170: (R)-5-(4-((1-(3-(1,1-Difluoro-2-hydroxy-2-methylpropyl)-2-fluorophenyl)ethyl)amino)-7-et hynyl-8-fluoro-2-methylquinazolin-6-yl)-1-methylpyridin-2(1H)-one

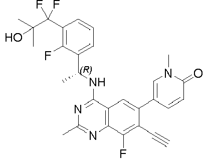

LC-MS: m/z 539 (M+H)⁺. ¹H NMR (400 MHz, DMSO) δ 8.67 (d, *J =* 7.3 Hz, 1H), 8.27 (s, 1H), 8.07 (d, *J* = 2.4 Hz, 1H), 7.74 (dd, *J* = 9.4, 2.6 Hz, 1H), 7.60 (t, *J* = 6.7 Hz, 1H), 7.32 (t, *J* = 6.8 Hz, 1H), 7.21 (t, *J* = 7.7 Hz, 1H), 6.52 (d, *J=* 9.4 Hz, 1H), 5.81 (t, *J* = 7.1 Hz, 1H), 5.32 (s, 1H), 4.81 (s, 1H), 3.54 (s, 3H), 2.39 (s, 3H), 1.58 (d, *J* = 7.0 Hz, 3H), 1.22 (d, *J* = 10.0 Hz, 6H).

### Example 171: Biological Test Evaluation

The following biological test examples are further described to explain the present invention, but these examples are not intended to limit the scope of the present invention.

### Inhibition of the binding of KRAS ^{G12C} to SOS1 by the compounds.

### Procedure

(1) Gradient dilution of the test compounds: 10 mM mother solution (dissolved in 100% DMSO) was added to a 384-well test plate, the final content of DMSO being 0.25%.
(2) 5 µL of Tag1-SOS1 solution was added to the test plate, and 5 µL of dilution buffer was added to the control group.
(3) 5 µL of Tag2-KRAS^{G12C} solution was added to the test plate.
(4) 10 µL of Anti-Tag1-Tb3+ and Anti-Tag2-XL665 detection solution were added to the test plate. The plate was centrifuged at 1000 rpm for 1 min and incubated at room temperature for 2 h.
(5) The plate was read.
(6) The IC₅₀ values for the compounds were finally calculated using GraphPad Prism software, and a curve was fit and plotted.

The inhibitory activity of the compounds of the examples of the present invention on the binding of KRAS ^{G12C} enzyme to SOS1 is shown in Table 1.

**Table 1. Inhibitory activity of compounds of examples of the present invention**

| **IC₅₀** | (nM) |
|---|---|
| **Example 1** | 4.6 |
| **Example 2** | 8.5 |
| **Example 6** | 13 |
| **Example 7** | 3.3 |
| **Example 8** | 1.9 |
| **Example 9** | 3.4 |
| **Example 10** | 2.9 |
| **Example 11** | 3.6 |
| **Example 12** | 8.3 |
| **Example 13** | 2.5 |
| **Example 14** | 16 |
| **Example 15** | 6.9 |
| **Example 16** | 1089 |
| **Example 17** | 70 |
| **Example 18** | 36 |
| **Example 19** | 9.4 |
| **Example 20** | 121 |
| **Example 21** | 4.7 |
| **Example 22** | 7.0 |
| **Example 23** | 7.9 |
| **Example 24** | 7.7 |
| **Example 25** | 3.9 |
| **Example 26** | 9.2 |
| **Example 27** | 8.5 |
| **Example 28** | 7.9 |
| **Example 29** | 4.7 |
| **Example 30** | / |
| **Example 31** | / |
| **Example 32** | 31 |
| **Example 33** | 7.5 |
| **Example 34** | 5.4 |
| **Example 35** | 17 |
| **Example 36** | >1000 |
| **Example 37** | >1000 |
| **Example 38** | 33 |
| **Example 39** | 21 |
| **Example 40** | 8.2 |
| **Example 41** | 252 |
| **Example 42** | 5.6 |
| **Example 43** | 5.2 |
| **Example 44** | 4.4 |
| **Example 45** | 167 |
| **Example 46** | 10 |
| **Example 47** | 7.4 |
| **Example 49** | 4.6 |
| **Example 50** | 4.2 |
| **Example 51** | 24 |
| **Example 52** | 31 |
| **Example 53** | 3.7 |
| **Example 54** | 15 |
| **Example 55** | 16 |
| **Example 56** | 9.6 |
| **Example 57** | 5.2 |
| **Example 58** | 5.1 |
| **Example 59** | 4.7 |
| **Example 60** | 18 |
| **Example 61** | 3.7 |
| **Example 62** | 5.3 |
| **Example 63** | 12 |
| **Example 64** | 8.7 |
| **Example 65** | 16 |
| **Example 65A** | 18 |
| **Example 65B** | 7.3 |
| **Example 66** | 7.0 |
| **Example 67** | 4.2 |
| **Example 68** | 6.2 |
| **Example 69** | 6.2 |
| **Example 70** | 24 |
| **Example 71** | 314 |
| **Example 72** | 3.2 |
| **Example 73** | 15 |
| **Example 74** | 3.1 |
| **Example 75** | 40 |
| **Example 76** | 188 |
| **Example 77** | 924 |
| **Example 78** | / |
| **Example 79** | 19 |
| **Example 80** | 6.2 |
| **Example 81** | / |
| **Example 82** | 4.5 |
| **Example 83** | 15 |
| **Example 84** | 18 |
| **Example 85** | 4.1 |
| **Example 86** | 4.4 |
| **Example 87** | 170 |
| **Example 88** | 20 |
| **Example 89** | 6.7 |

As can be seen from Table 1:
The compounds of the examples of the present invention showed good inhibitory activity against the binding of KRAS^{G12C} to SOS1.

### Example 172: Inhibition of H358 Cell Proliferation by Compounds

### Procedure

### 1. Cell culturing

(a) The cells were thawed in T75 cell culture flasks:

**Table 2. Culture f H358 cells**

| No. | Cell line | Medium | Passage # T75 |
|---|---|---|---|
| 1 | NCI-H358 | RPMI 1640+10%FBS+1%PS | 2∼3x10⁶ |

(b) When 80-90% cell confluence was achieved, the cells were passaged.

### 2. Cell proliferation assay

### Procedure

The diluted test compounds were added to a 384-well cell culture plate using a nanoliter pipetting system, and duplicate wells were set. An equal volume of medium was added to the positive control group; an equal volume of DMSO was added to the negative control group. The plate was centrifuged at 1000 rpm at room temperature for 1 min.

The cells were inoculated into a) 384-well culture plate, an equal volume of cells was added to the negative control group, and only an equal volume of medium was added to the positive control group. The plate was centrifuged at 1000 rpm at room temperature for 1 min. The final concentration of DMSO in the final compounds was 0.5%. The plate was incubated in a thermostatic incubator at 37 °C with 5% CO₂ for 7 days.

CellTiter-Glo^{®} 3D was added to b) 384-well cell culture plate at 20 µL/well. The plate was shaken at 320 rpm in the dark for 20 min, and incubated at room temperature in the dark for 2 h. The luminescence values were read using an Envision multi-mode microplate reader.

### 3. Data analysis

The inhibition rates (IRs) of the test compounds were calculated according to the following formula: IR (%) = (1 - (RLU compound - RLU blank control) / (RLU vehicle control - RLU blank control)) × 100%. The inhibition rates of the compounds at different concentrations were calculated in Excel, and then inhibition curves were plotted and relevant parameters including minimum inhibition rate, maximum inhibition rate and IC₅₀ were calculated using GraphPad Prism software. The experimental results are shown in Table 3.

**Table 3. Inhibitory activity of compounds of examples of the present invention on H358 cell proliferation**

| **IC₅₀** | (nM) |
|---|---|
| **Example 44** | 25 |
| **Example 72** | 16.6 |
| **Example 74** | 6.5 |
| **Example 79** | 419 |
| **Example 80** | 53 |
| **Example 82** | 33 |
| **Example 83** | 187 |
| **Example 85** | 39 |
| **Example 86** | 11.8 |
| **Example 89** | 130 |
| **Example 90** | 48.6 |
| **Example 91** | >10000 |
| **Example 92** | 18.24 |
| **Example 93** | 40.93 |
| **Example 97** | 15.5 |
| **Example 98** | 12.8 |
| **Example 99** | 19.97 |
| **Example 101** | 15.48 |
| **Example 102** | 13.26 |
| **Example 103** | 23.5 |
| **Example 113** | 10.40 |
| **Example 115** | 9.92 |
| **Example 116** | 4.29 |
| **Example 117** | 2.65 |
| **Example 118** | 2.65 |
| **Example 119** | 11.47 |
| **Example 122** | 15.63 |
| **Example 123** | 32.83 |
| **Example 153** | 34.71 |
| **Example 157** | 15.85 |
| **Example 163** | 14.5 |

### Pharmacokinetic test evaluation

Male SD rats weighing about 220 g were fasted overnight and then intragastrically administered solutions of the compounds of the present invention [CMC/TW80 as a carrier] at 10 mg/kg. Blood was collected at 0.5, 1.0, 2.0, 4.0, 6.0, 8.0, 12, 24, 36, and 48 h after administration of the compounds of the present invention, and the plasma concentrations of the compounds of the present invention were determined by LC/MS/MS.

As can be seen from the results, the compounds of the present invention have good pharmacokinetic properties.

### Anti-tumor pharmacodynamic evaluation

100 µL of a suspension containing 5 × 10⁶ MIA PaCa-2 tumor cells was subcutaneously inoculated into the right posterior abdomens of nude mice. The health of the mice was monitored daily, and measurements were started when tumors grew to be palpable. The tumor volume was calculated as follows: 0.5 × L × W², where L and W represent the length and width of the tumor, respectively. When the tumors grew to about 100 mm³, the mice were randomized into groups. The mice were intragastrically administered a corresponding dose (50 mg/kg) of compound suspension in CMC-Na twice daily, and their general states were monitored at the same time. The tumors were measured 3 times a week, and the body weight was measured twice a week. The test results are shown in Table 4.

The structure of the reference compound BI3406:

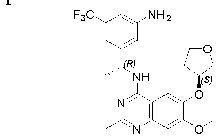

**Table 4. Pharmacodynamic test evaluation of anti-tumor activity**

| **Group** | **TV (mm³) (tumor volume) Day 0** | **TV (mm³) (tumor volume) Day 21** | **%TGI (tumor growth inhibition)** |
|---|---|---|---|
| Blank control group | 103 | 601 | / |
| **Example 72** | 103 | 175 | 84.9% |
| BI3406 | 103 | 223 | 75.7% |

As can be seen from the results, the compounds of the present invention have better anti-tumor effects than the reference compound BI3406.

All documents mentioned in the present invention are incorporated as references, just as each document is individually cited as a reference. In addition, it should be understood that various modifications or changes may be made by those skilled in the art after reading the above teachings of the present invention, and these equivalent forms also fall within the scope defined by the claims appended hereto.

## Claims

1. A substituted benzo- or pyrido-pyrimidinamine compound having a structure of general formula (I), or a stereoisomer, a tautomer, a crystalline form, a pharmaceutically acceptable salt, a hydrate, a solvate, or a prodrug thereof: 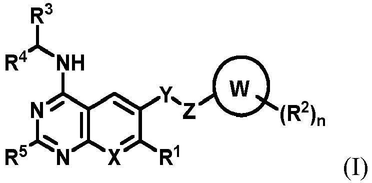
wherein in the formula,
X is selected from: CR⁶ and N, wherein R⁶ is selected from: hydrogen, deuterium, halogen, cyano, C₁-C₆ alkyl, C₃-C₆ cycloalkyl, and 4- to 6-membered heterocyclyl;
Y is selected from the group consisting of: O, NH, NR⁷, S, SO, SO₂, C=C, substituted or unsubstituted 4- to 20-membered heterocyclyl, substituted or unsubstituted C₆-C₁₄ aryl, and 5-to 14-membered heteroaryl, wherein R⁷ is selected from the group consisting of the following substituted or unsubstituted groups: C₁-C₆ alkyl, C₃-C₆ cycloalkyl, and 4- to 6-membered heterocyclyl;
Z is selected from the group consisting of the following substituted or unsubstituted groups: a bond and substituted or unsubstituted C₁-C₁₈ alkylene;
W is selected from the group consisting of the following substituted or unsubstituted groups: a bond, C₃-C₂₀ cycloalkylene, 4- to 20-membered heterocyclylene, OR¹¹, NR¹¹R¹², SO₂, NR¹²SO₂, CO, and NR¹²CO; R¹¹ is independently selected from the group consisting of the following substituted or unsubstituted groups: C₃-C₂₀ cycloalkylene, 4- to 20-membered heterocyclylene, C₃-C₂₀ cycloalkylene Ci-Cis alkylene, 4- to 20-membered heterocyclylene C₁-C₁₈ alkylene, C₆-C₁₄ aryl, and 5- to 14-membered heteroaryl; R¹² is independently selected from the group consisting of the following substituted or unsubstituted groups: hydrogen, deuterium, C₁-C₆ alkyl, and C₃-C₆ cycloalkyl;
R¹ and R² are each independently selected from the group consisting of: hydrogen, deuterium, halogen, cyano, -(CH₂)ₘR⁸, -(CH₂)ₘ(CH=CH)R⁸, -(CH₂)ₘ(C≡C)R⁸, -(CH₂)ₘO(CH₂)ₚR⁸, -(CH₂)ₘSR⁸, -(CH₂)ₘCOR⁸, -(CH₂)ₘC(O)OR⁸, -(CH₂)ₘS(O)_{q}R⁸, -(CH₂)ₘNR⁸R⁹, -(CH₂)ₘC(O)NR⁸R⁹, -(CH₂)ₘNR⁸C(O)R⁹, -(CH₂)ₘNR⁸C(O)NR⁹R¹⁰, -(CH₂)ₘS(O)_{q}NR⁸R⁹, -(CH₂)ₘNR⁸S(O)_{q}R⁹, and -(CH₂)ₘNR⁸S(O)_{q}NR⁹R¹⁰, wherein H in CH₂ can be optionally substituted; R⁸, R⁹, and R¹⁰ are each independently selected from the group consisting of the following substituted or unsubstituted groups: hydrogen, Ci-Cis alkyl, Ci-Cis alkoxy, C₃-C₂₀ cycloalkyl, 4- to 20-membered heterocyclyl, C₆-C₁₄ aryl, and 5- to 14-membered heteroaryl; or in -(CH₂)ₘNR⁸R⁹, -(CH₂)ₘC(O)NR⁸R⁹, or -(CH₂)ₘS(O)_{q}NR⁸R⁹, R⁸ and R⁹, together with the N atom adjacent thereto, form a substituted or unsubstituted 4- to 8-membered heterocyclyl by cyclization; or in -(CH₂)ₘNR⁸C(O)R⁹, -(CH₂)ₘNR⁸C(O)NR⁹R¹⁰, -(CH₂)ₘNR⁸S(O)_{q}R⁹, or -(CH₂)ₘNR⁸S(O)_{q}NR⁹R¹⁰, R⁸ and R⁹, together with the N atom adjacent thereto, form a substituted or unsubstituted 4- to 8-membered heterocyclyl by cyclization, or R⁹ and R¹⁰, together with the atom adjacent thereto, form a substituted or unsubstituted 4- to 8-membered heterocyclyl by cyclization;
R³ is selected from the group consisting of the following substituted or unsubstituted groups: C₃-C₁₈ cycloalkyl, 4- to 20-membered heterocyclyl, C₆-C₁₄ aryl, and 5- to 14-membered heteroaryl;
R⁴ and R⁵ are each independently selected from the group consisting of the following substituted or unsubstituted groups: C₁-C₆ alkyl, C₃-C₆ cycloalkyl, 4- to 6-membered heterocyclyl, ester group, COOH, CONH₂, C₂-C₆ alkenyl, and C₂-C₆ alkynyl;
wherein the above substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₃-C₂₀ cycloalkyl-O-, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, 4- to 20-membered heterocyclyl-O-, halogen, oxo C₁-C₆ alkyl, nitro, hydroxy, cyano, C₂-C₆ ester group, C₁-C₆ amino, C₂-C₆ acyl, C₁-C₆ amido, C₁-C₆ sulfonyl, C₁-C₆ sulfonamido, and C₁-C₆ ureido; wherein the C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₃-C₂₀ cycloalkyl-O-, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, or 4- to 20-membered heterocyclyl-O- can be further substituted with one or more R^{a}, wherein R^{a} is selected from: C₁-C₆ alkyl, deuterated C₁-C₆ alkyl, halogenated C₁-C₆ alkyl, halogenated C₁-C₆ alkylhydroxy, C₃-C₆ cycloalkyl, C₃-C₆ cycloalkyl-O-, C₁-C₆ alkoxy, deuterated C₁-C₆ alkoxy, halogenated C₁-C₆ alkoxy, C₆-C₁₄ aryl, 5- to 14-membered heteroaryl, 4- to 6-membered heterocyclyl, 4- to 6-membered heterocyclyl-O-, halogen, oxo C₁-C₆ alkyl, nitro, hydroxy, cyano, C₂-C₆ ester group, C₁-C₆ amino, C₂-C₆ amido, C₁-C₆ sulfonamido, and C₁-C₆ ureido; or two substituents on the same carbon atom together form -(CH₂)ₙ- or =O;
m and n are each independently 0, 1, 2, 3, 4, or 5;
p is 0, 1, 2, 3, 4, or 5;
q is 1 or 2;
provided that when Y is selected from the group consisting of: O, NH, and NR⁷, and when Z is a bond and W is C₃-C₂₀ cycloalkylene or 4- to 20-membered heterocyclylene, R¹ is not hydrogen, deuterium, halogen, cyano, R⁸, O(CH₂)ₚR⁸, COR⁸, -C(O)OR⁸, NR⁸R⁹, C(O)NR⁸R⁹, -NR⁸C(O)R⁹, or -NR⁸C(O)NR⁹R¹⁰.

2. The substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof according to claim 1, having a structure represented by general formula (III): 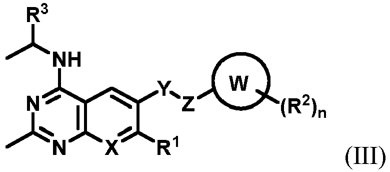 wherein in the formula, R¹, R², R³, X, Y, Z, W and n are as defined in claim 1.

3. The substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof according to claim 1, having a structure represented by general formula (IV): 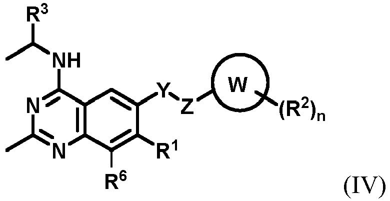 wherein in the formula, R¹, R², R³, R⁶, X, Y, Z, W and n are as defined in claim 1.

4. The substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof according to claim 1, having a structure represented by formula (VI): 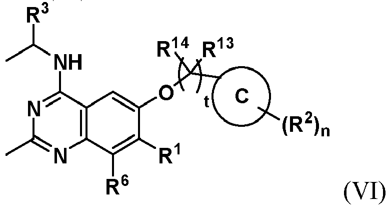
wherein in the formula,
R¹³ and R¹⁴ are each independently selected from: H, C₁-C₆ alkyl, deuterated C₁-C₆ alkyl, halogenated C₁-C₆ alkyl, halogen, oxo, nitro, hydroxy, cyano, ester group, amino, amido, sulfonamido, and ureido;
ring C is selected from the group consisting of the following substituted or unsubstituted groups: C₃-C₁₂ cycloalkylene and 4- to 12-membered heterocyclylene;
each R² is identical or different and is independently selected from the group consisting of: -(CH₂)ₘO(CH₂)ₚR⁸, -(CH₂)ₘ(CH=CH)ₚR⁸, (CH₂)ₘ(C≡C)ₚR⁸, -(CH₂)ₘSR⁸, -(CH₂)ₘCOR⁸, -(CH₂)ₘC(O)OR⁸, -(CH₂)ₘS(O)_{q}R⁸, -(CH₂)ₘNR⁸R⁹, -(CH₂)ₘC(O)NR⁸R⁹, -(CH₂)ₘNR⁸C(O)R⁹, -(CH₂)ₘNR⁸C(O)NR⁹R¹⁰, -(CH₂)ₘS(O)_{q}NR⁸R⁹, -(CH₂)ₘNR⁸S(O)_{q}R⁹, and -(CH₂)ₘNR⁸S(O)_{q}NR⁹R¹⁰, wherein H in CH₂ can be optionally substituted;
m is 1, 2, 3, 4, or 5;
t is 1, 2, 3, 4, 5, or 6;
wherein the substitution refers to substitution with one or more groups selected from the group consisting of: hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido;
R¹, R³, R⁶, R⁸, R⁹, R¹⁰, p, q and n are as defined in claim 1.

5. The substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof according to claim 1, having a structure represented by formula (VII): 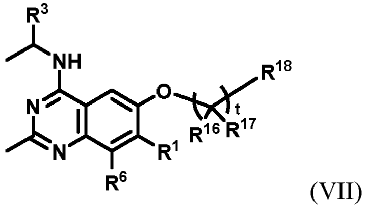
wherein in the formula,
R¹⁶ and R¹⁷ are each independently selected from: H, C₁-C₆ alkyl, deuterated C₁-C₆ alkyl, halogenated C₁-C₆ alkyl, halogen, oxo, nitro, hydroxy, cyano, ester group, amino, amido, sulfonamido, and ureido;
R¹⁸ is selected from: OR¹¹, NR¹¹R¹², NR¹²SO₂R², COR², and NR¹²COR²; R¹¹ is independently selected from: substituted C₃-C₁₂ cycloalkyl, substituted or unsubstituted 4- to 12-membered heterocyclyl, substituted or unsubstituted C₃-C₁₂ cycloalkylene C₁-C₆ alkylene, substituted or unsubstituted 4- to 12-membered heterocyclylene C₁-C₆ alkylene, substituted or unsubstituted C₆-C₁₄ aryl, and substituted or unsubstituted 5- to 14-membered heteroaryl; R¹² is independently selected from the group consisting of the following substituted or unsubstituted groups: hydrogen, deuterium, C₁-C₆ alkyl, and C₃-C₆ cycloalkyl;
or R¹⁸ is selected from: -(CH₂)ₘ(CH=CH)R⁸, -(CH₂)ₘ(C≡C)R⁸, -(CH₂)ₘO(CH₂)ₚR⁸, -(CH₂)ₘSR⁸, -(CH₂)ₘCOR⁸, -(CH₂)ₘC(O)OR⁸, -(CH₂)ₘS(O)_{q}R⁸, -(CH₂)ₘNR⁸R⁹, -(CH₂)ₘC(O)NR⁸R⁹, -(CH₂)ₘNR⁸C(O)R⁹, -(CH₂)ₘNR⁸C(O)NR⁹R¹⁰, -(CH₂)ₘS(O)_{q}NR⁸R⁹, -(CH₂)ₘNR⁸S(O)_{q}R⁹, and -(CH₂)ₘNR⁸S(O)_{q}NR⁹R¹⁰, wherein H in CH₂ can be optionally substituted; R⁸, R⁹, and R¹⁰ are each independently selected from: substituted or unsubstituted C₁-C₁₈ alkyl, substituted or unsubstituted C₃-C₂₀ cycloalkyl, and substituted or unsubstituted 4-to 20-membered heterocyclyl;
wherein the substitution in R⁸, R⁹, and R¹⁰ refers to substitution with one or more groups selected from the group consisting of: C₃-C₂₀ cycloalkyl, C₁-C₁₈ alkoxy, deuterated Ci-Cis alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido;
t is 1, 2, 3, 4, 5, or 6;
wherein unless otherwise stated, the above substitution refers to substitution with one or more groups selected from the group consisting of: hydrogen, deuterium, Ci-Cis alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido;
R¹, R², R³, R⁶, m, p and q are as defined in claim 1.

6. The compound, or the stereoisomer, the tautomer, the crystalline form, the pharmaceutically acceptable salt, the hydrate, the solvate or the prodrug thereof according to claim 5, wherein R¹⁸ is selected from: OR¹¹, NR¹¹R¹² and NR¹²SO₂R²; wherein R¹¹ is independently selected from: substituted C₃-C₁₂ cycloalkyl, substituted or unsubstituted 4- to 12-membered heterocyclyl, substituted or unsubstituted C₃-C₁₂ cycloalkylene C₁-C₆ alkylene, substituted or unsubstituted 4- to 12-membered heterocyclylene C₁-C₆ alkylene, substituted or unsubstituted C₆-C₁₄ aryl, and substituted or unsubstituted 5- to 14-membered heteroaryl; R¹² is independently selected from the group consisting of: hydrogen, deuterium, substituted or unsubstituted C₁-C₆ alkyl, and substituted or unsubstituted C₃-C₆ cycloalkyl;
or R¹⁸ is selected from: -(CH₂)ₘO(CH₂)ₚR⁸; wherein H in CH₂ can be optionally substituted; R⁸ is selected from: substituted or unsubstituted Ci-Cis alkyl, substituted or unsubstituted C₃-C₂₀ cycloalkyl, and substituted or unsubstituted 4- to 20-membered heterocyclyl; preferably, R⁸ is substituted or unsubstituted Ci-Cis alkyl (preferably C₁-C₆ alkyl);
the above substitution refers to substitution with one or more groups selected from the group consisting of: C₁-C₆ alkyl, deuterated C₁-C₆ alkyl, halogenated C₁-C₆ alkyl, halogenated C₁-C₆ alkylhydroxy, C₃-C₆ cycloalkyl, C₁-C₆ alkoxy, deuterated C₁-C₆ alkoxy, halogenated C₁-C₆ alkoxy, C₆-C₁₀ aryl, 5- to 10-membered heteroaryl, 4- to 6-membered heterocyclyl, halogen, oxo, nitro, hydroxy, cyano, ester group, amino, amido, sulfonamido, and ureido;
in another preferred embodiment, R³ is selected from: substituted C₆-C₁₄ aryl and substituted 5-to 14-membered heteroaryl; the substitution refers to substitution with one or more groups selected from the group consisting of: R^{3a}, hydrogen, deuterium, C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₃-C₂₀ cycloalkyl-O-, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, 4- to 20-membered heterocyclyl-O-, halogen, oxo, nitro, hydroxy, cyano, ester group, amino, amido, sulfonamido, and ureido; wherein the C₁-C₁₈ alkyl, deuterated C₁-C₁₈ alkyl, halogenated C₁-C₁₈ alkyl, halogenated C₁-C₁₈ alkylhydroxy, C₃-C₂₀ cycloalkyl, C₃-C₂₀ cycloalkyl-O-, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, or 4- to 20-membered heterocyclyl-O- can be further substituted with one or more R^{a}, wherein R^{a} is selected from: C₁-C₆ alkyl, deuterated C₁-C₆ alkyl, halogenated C₁-C₆ alkyl, halogenated C₁-C₆ alkylhydroxy, C₃-C₆ cycloalkyl, C₃-C₆ cycloalkyl-O-, C₁-C₆ alkoxy, deuterated C₁-C₆ alkoxy, halogenated C₁-C₆ alkoxy, C₆-C₁₄ aryl, 5- to 14-membered heteroaryl, 4- to 6-membered heterocyclyl, 4- to 6-membered heterocyclyl-O-, halogen, oxo, nitro, hydroxy, cyano, ester group, amino, amido, sulfonamido, and ureido; provided that at least one R^{3a} substituent is present;
wherein R^{3a} is selected from: C₁-C₁₈ alkyl substituted with hydroxy, C₁-C₁₈ haloalkyl substituted with hydroxy, Ci-Cis deuteroalkyl substituted with hydroxy, Ci-Cis alkyl substituted with alkoxy, Ci-Cis haloalkyl substituted with alkoxy, Ci-Cis deuteroalkyl substituted with alkoxy, Ci-Cis alkyl substituted with cycloalkyloxy, Ci-Cis haloalkyl substituted with cycloalkyloxy, Ci-Cis deuteroalkyl substituted with cycloalkyloxy, Ci-Cis alkyl substituted with heterocyclyloxy, C₁-C₁₈ haloalkyl substituted with heterocyclyloxy, C₁-C₁₈ deuteroalkyl substituted with heterocyclyloxy, Ci-Cis haloalkyl substituted with cycloalkyl, C₁-C₁₈ haloalkyl substituted with heterocyclyl, Ci-Cis haloalkyl substituted with amino, C₁-C₁₈ haloalkyl substituted with cyano, C₁-C₁₈ haloalkyl substituted with amido, substituted C₃-C₁₂ cycloalkyl, substituted 4- to 12-membered heterocyclyl, substituted or unsubstituted haloalkyloxy, substituted or unsubstituted cycloalkyloxy, substituted or unsubstituted heterocyclyloxy, substituted or unsubstituted sulfonamido, and substituted or unsubstituted cycloalkylsulfonyl; the above substitution refers to substitution with one or more groups selected from the group consisting of: C₁-C₆ alkyl, deuterated C₁-C₆ alkyl, halogenated C₁-C₆ alkyl, halogenated C₁-C₆ alkylhydroxy, C₃-C₆ cycloalkyl, C₁-C₆ alkoxy, deuterated C₁-C₆ alkoxy, halogenated C₁-C₆ alkoxy, C₆-C₁₀ aryl, 5- to 10-membered heteroaryl, 4- to 6-membered heterocyclyl, halogen, oxo, nitro, hydroxy, cyano, ester group, amino, amido, sulfonamido, and ureido.

7. The substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof according to claim 1, having a structure represented by formula (VIII): 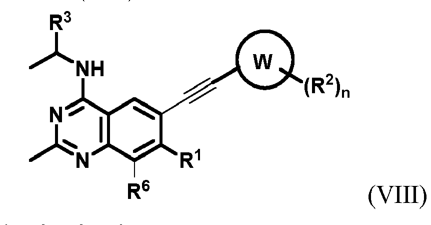 wherein in the formula, R¹, R², R³, R⁶ and Ware as defined in claim 1.

8. The substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof according to claim 1, having a structure represented by formula (IX-A) or formula (IX-B): 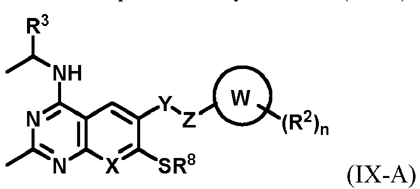 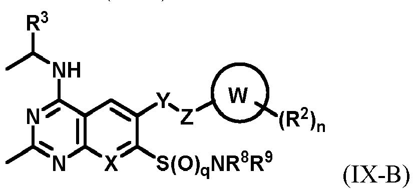 wherein in the formula, R², R³, R⁸, R⁹, X, Y, Z, W, n and q are as defined in claim 1.

9. The substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof according to claim 1, having a structure represented by formula (X): 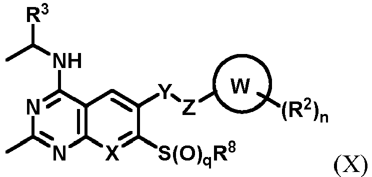
wherein in the formula, R⁸ is selected from the group consisting of the following substituted or unsubstituted groups: C₃-C₂₀ cycloalkyl and 4- to 20-membered heterocyclyl;
wherein the substitution refers to substitution with one or more groups selected from the group consisting of: hydrogen, deuterium, Ci-Cis alkyl, deuterated C₁-C₁₈ alkyl, halogenated C₁-C₁₈ alkyl, halogenated C₁-C₁₈ alkylhydroxy, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5- to 14-membered heteroaryl, 4- to 20-membered heterocyclyl, halogen, oxo, nitro, hydroxy, cyano, ester group, amino, amido, sulfonamido, and ureido;
R², R³, X, Y, Z, W, n and q are as defined in claim 1.

10. The substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof according to claim 1, wherein R³ is selected from:

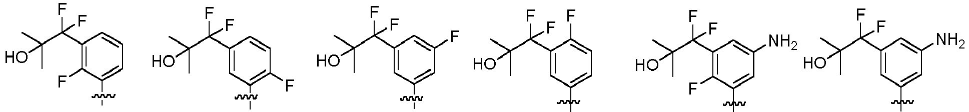

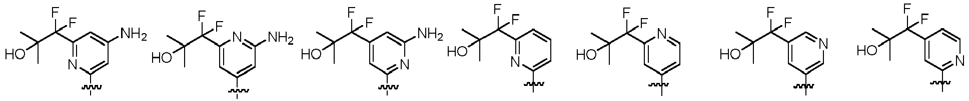

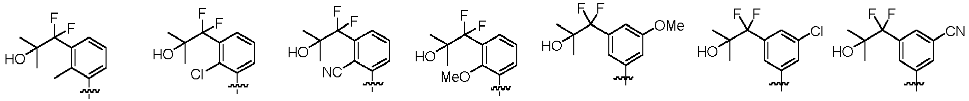

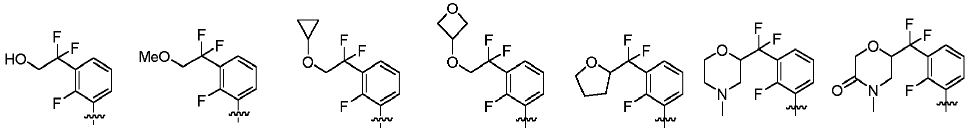

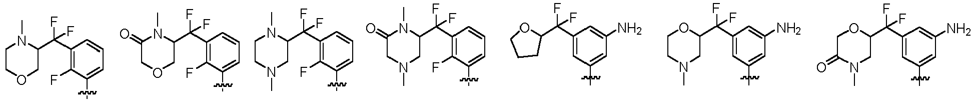

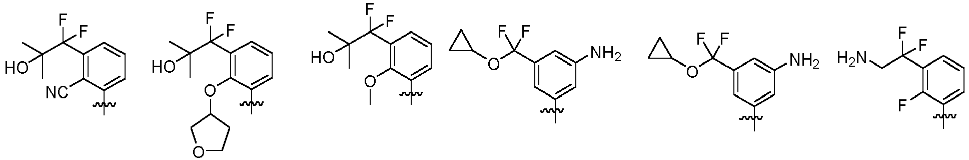

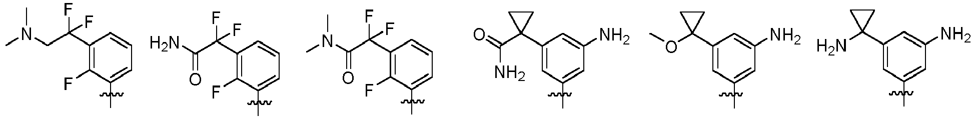

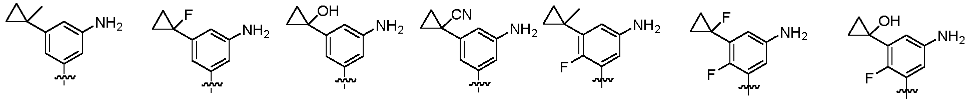

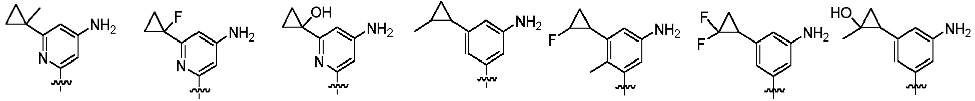

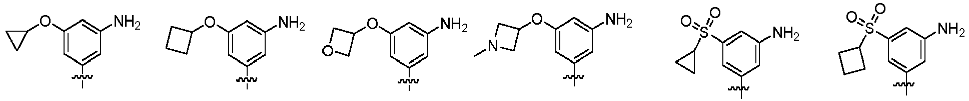

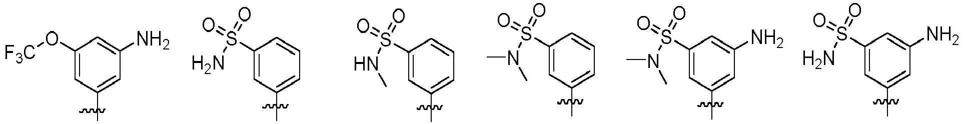

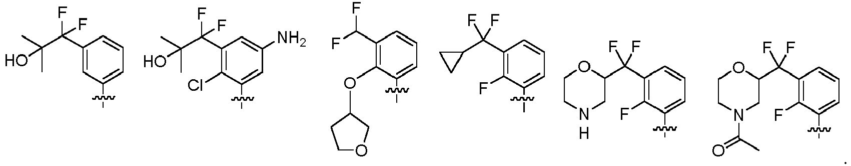

11. The substituted benzo- or pyrido-pyrimidinamine compound having the structure of general formula (I), or the stereoisomer, the tautomer, the crystalline form, the pharmaceutically acceptable salt, the hydrate, the solvate, or the prodrug thereof according to any one of claims 1-10, wherein the compound is selected from the group consisting of:

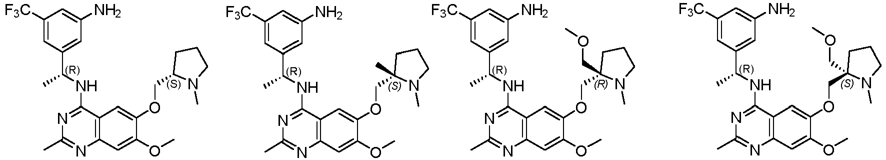

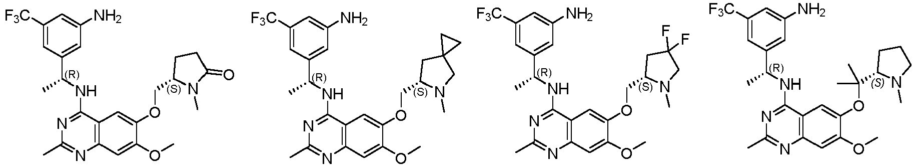

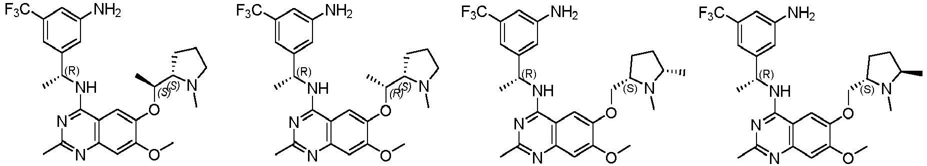

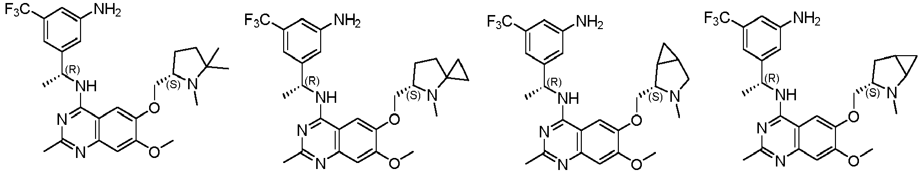

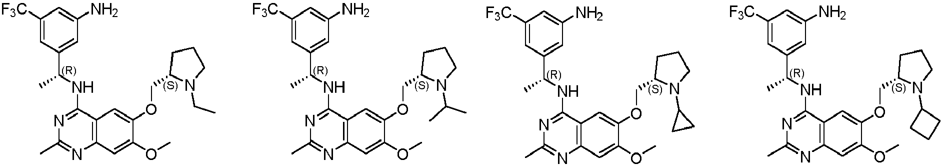

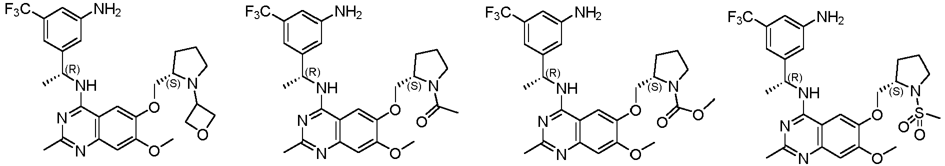

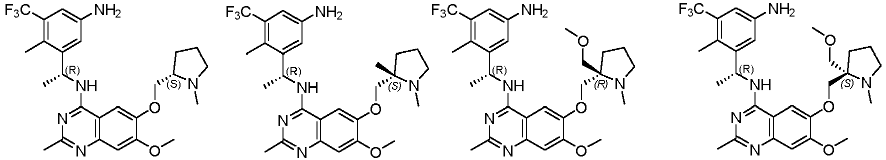

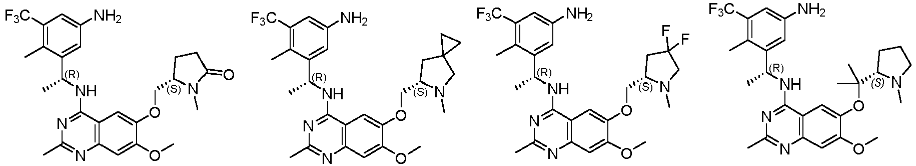

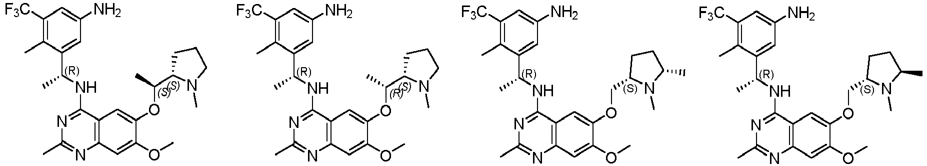

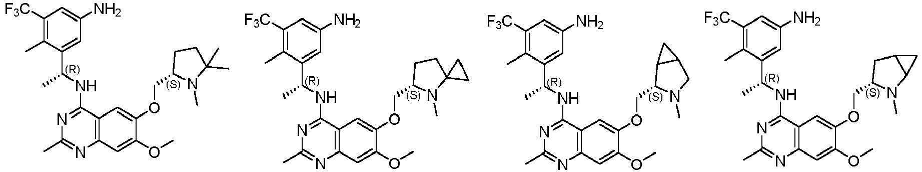

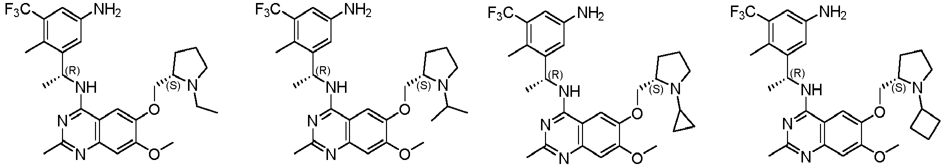

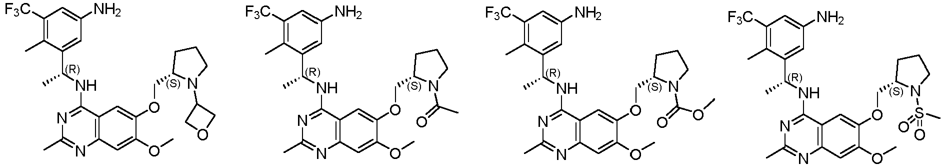

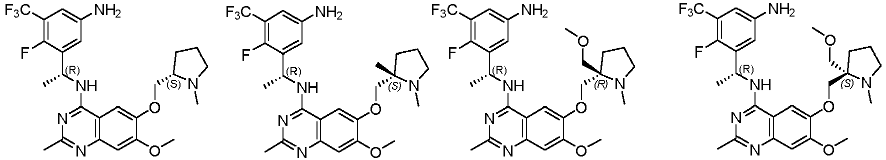

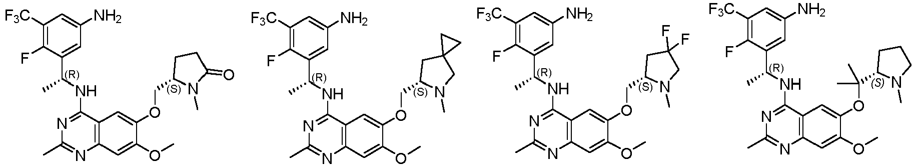

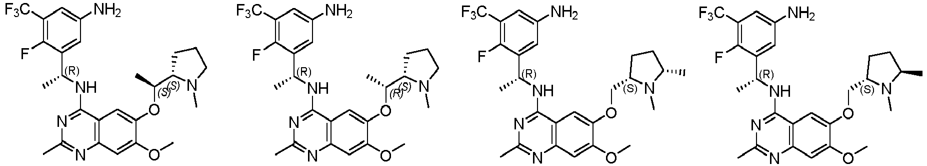

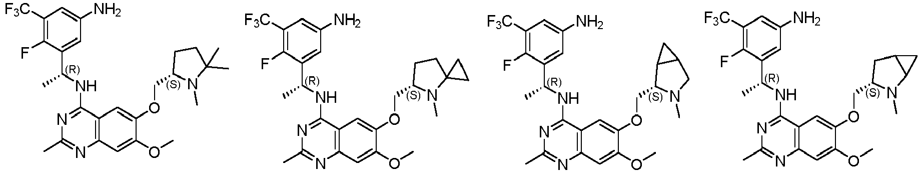

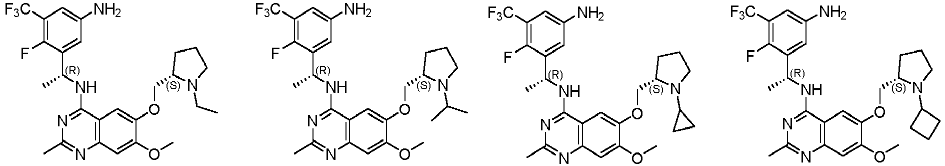

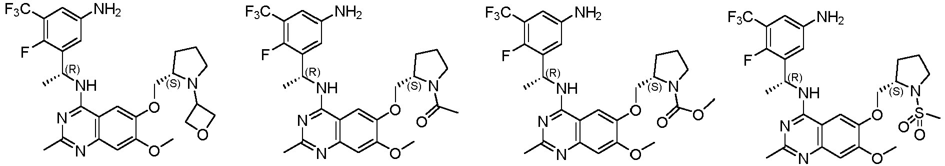

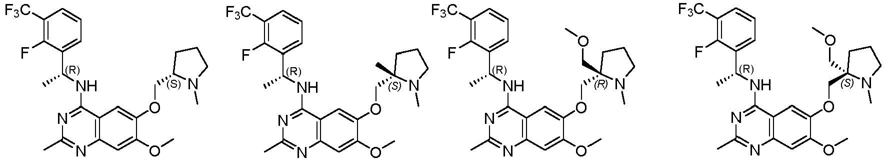

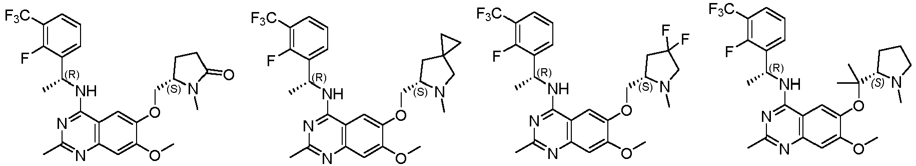

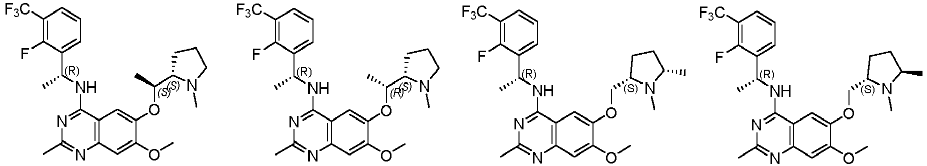

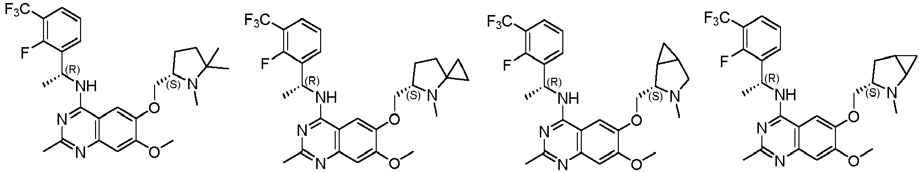

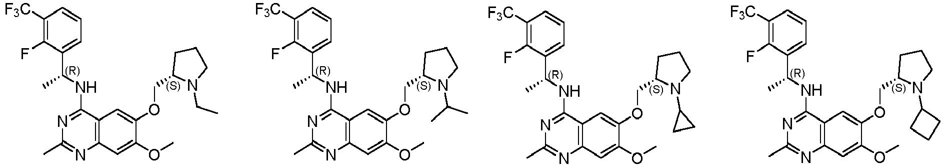

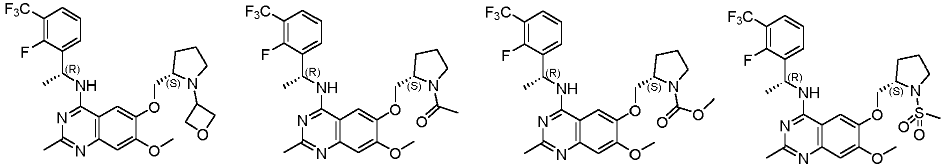

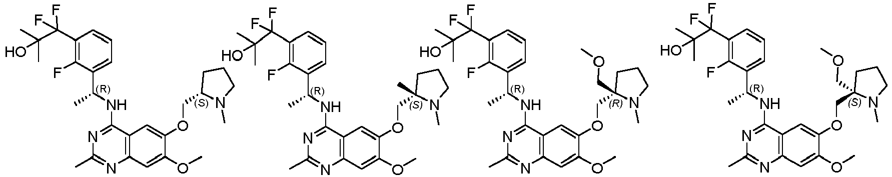

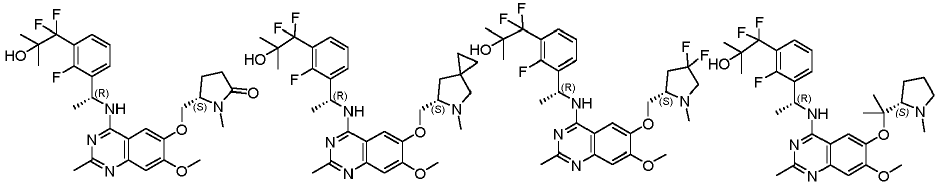

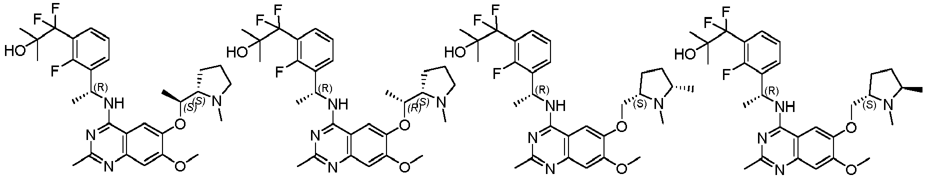

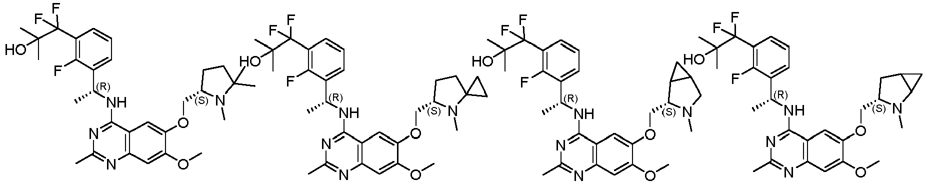

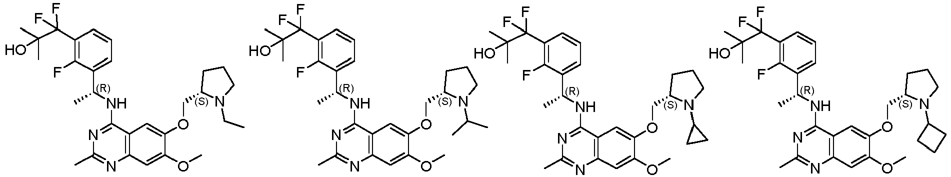

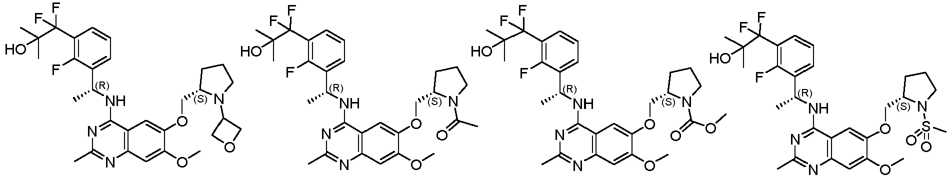

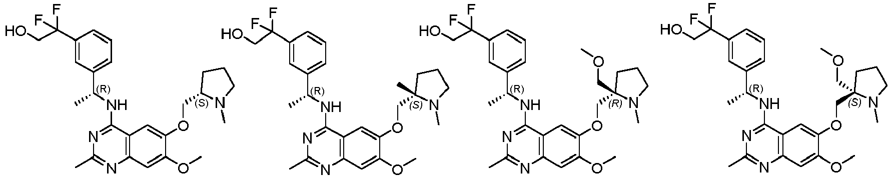

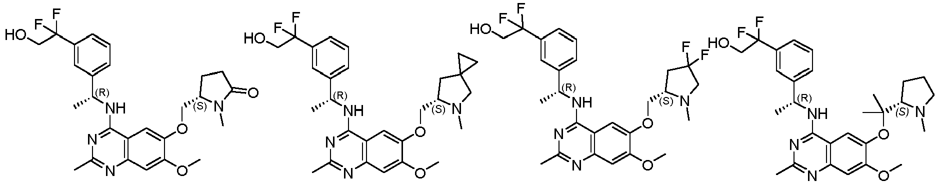

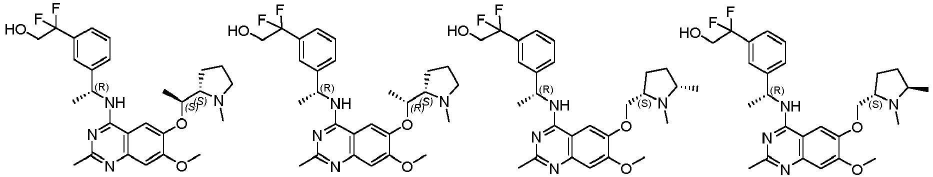

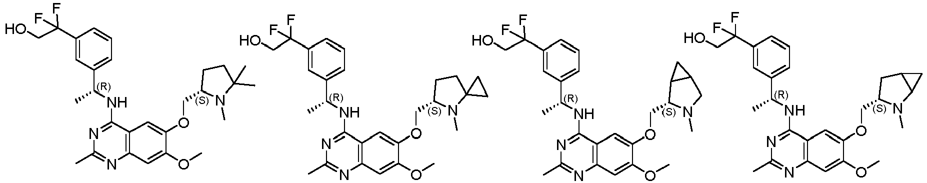

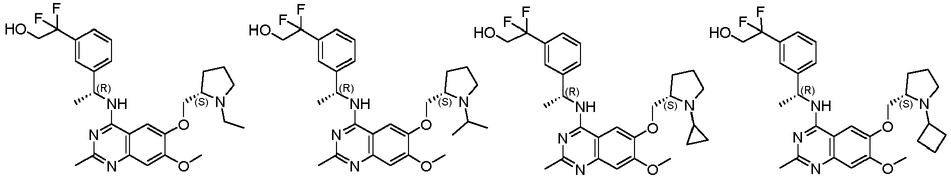

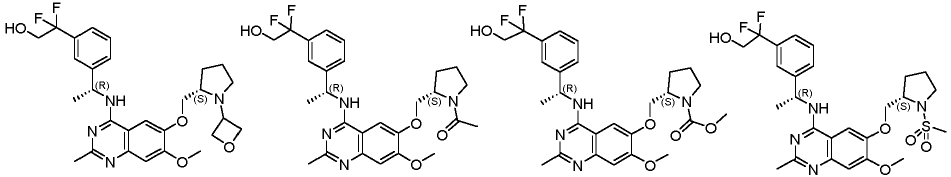

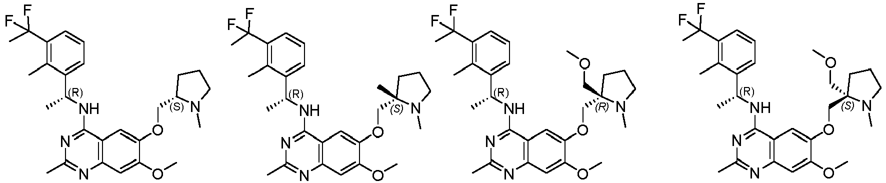

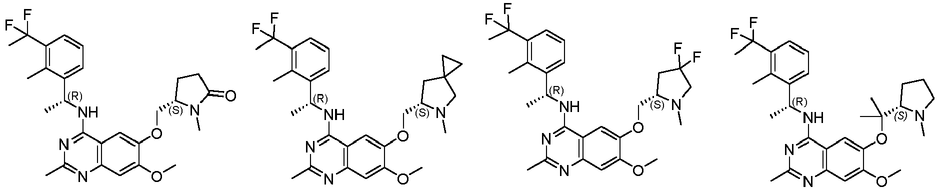

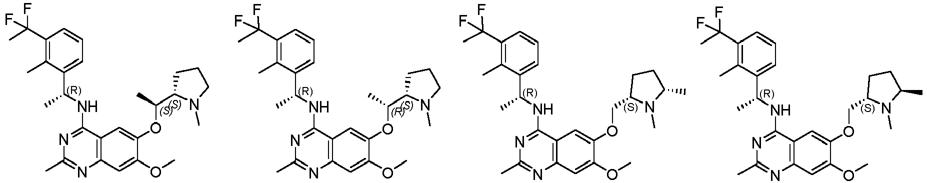

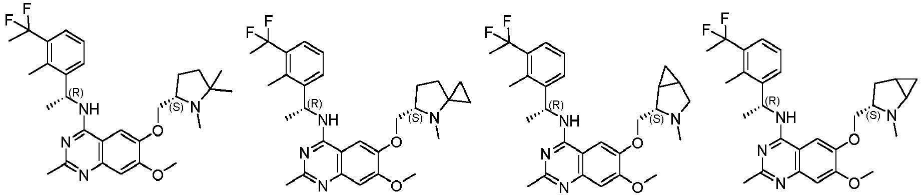

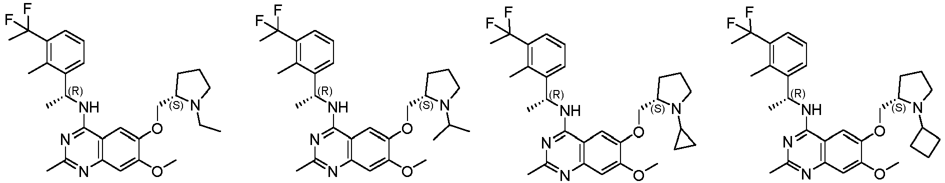

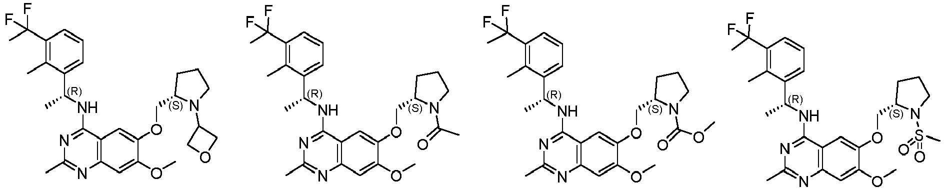

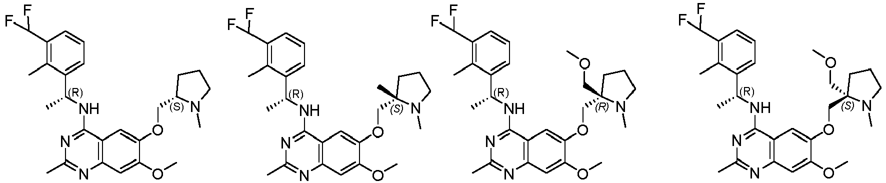

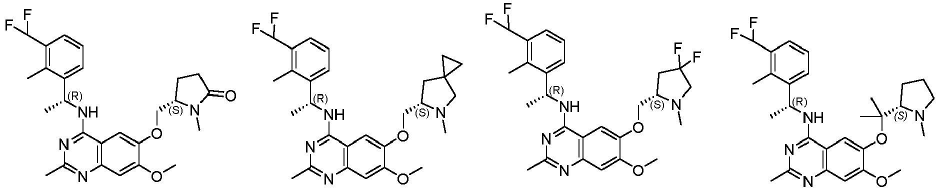

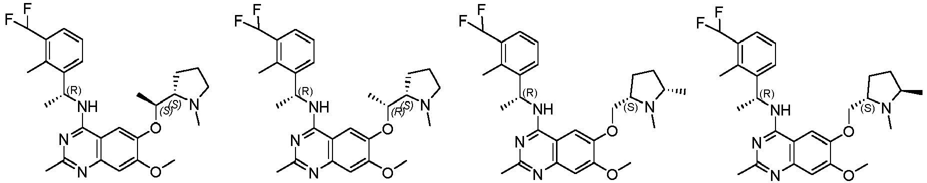

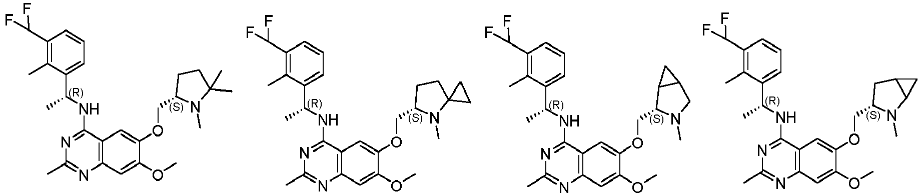

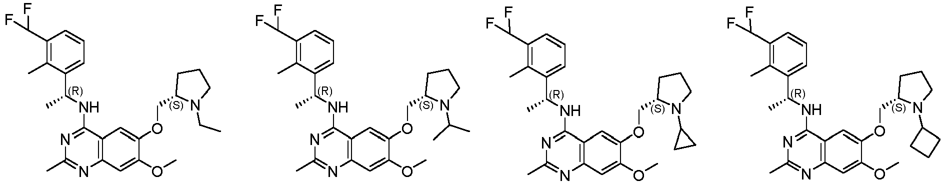

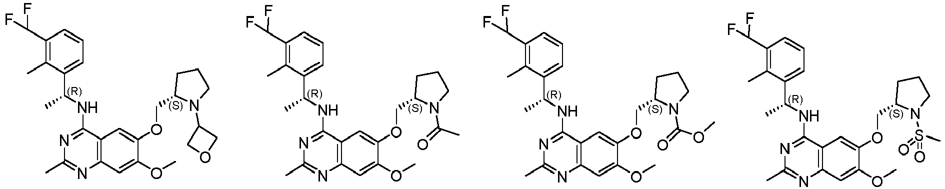

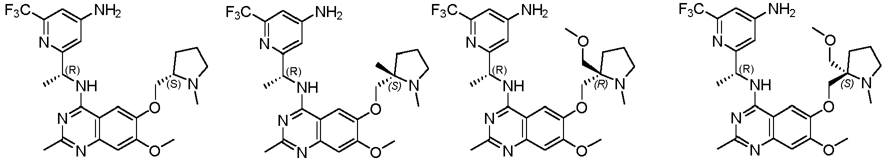

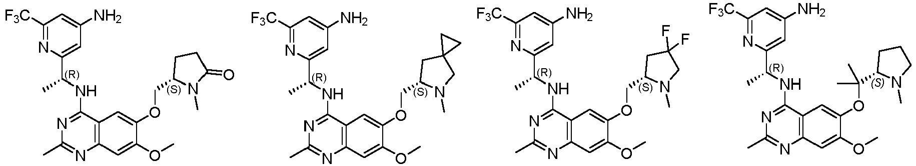

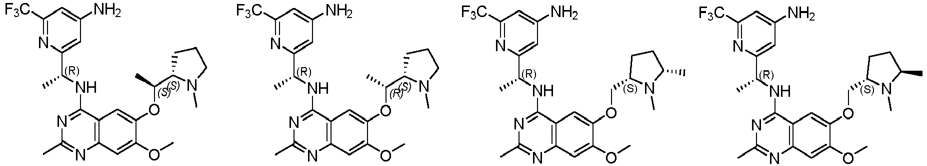

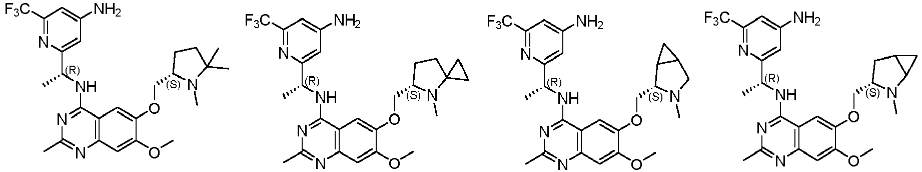

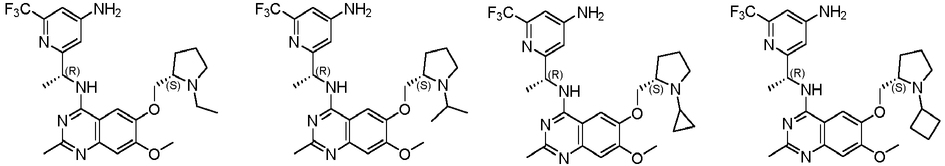

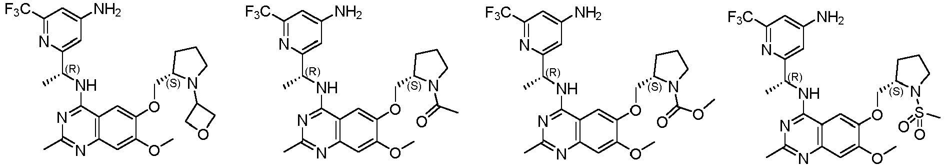

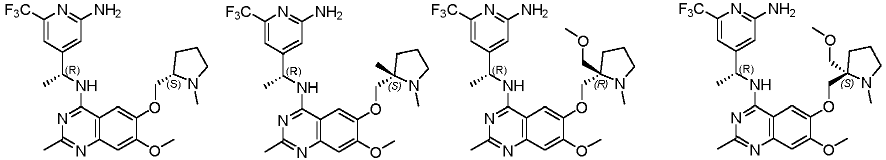

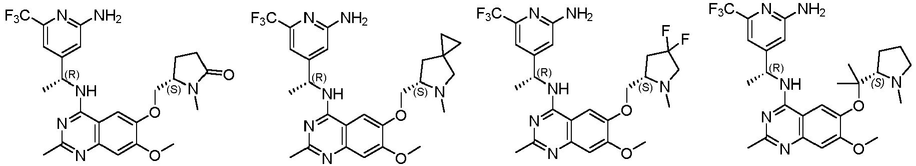

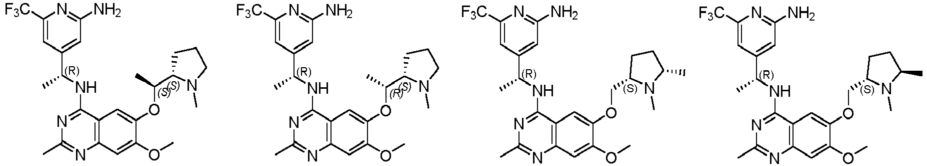

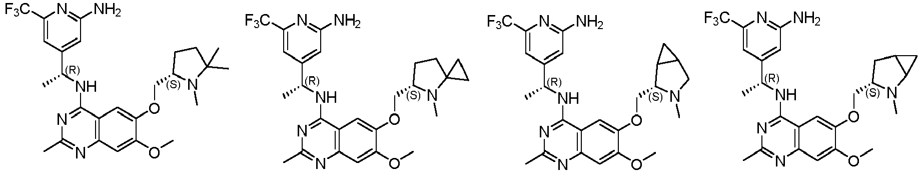

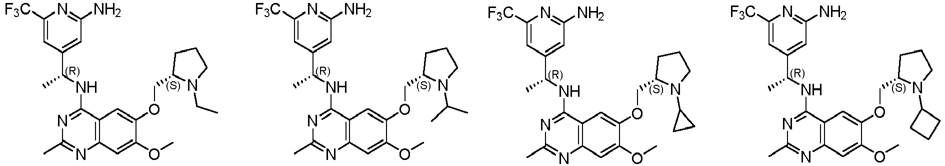

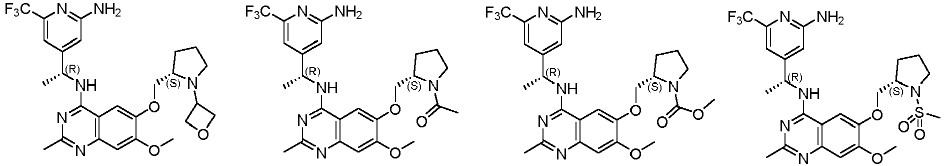

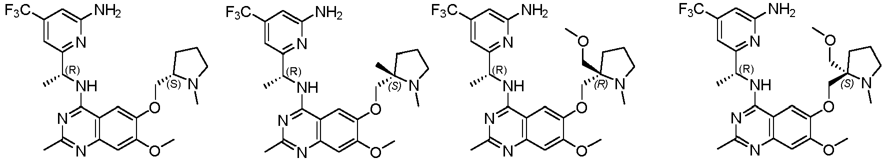

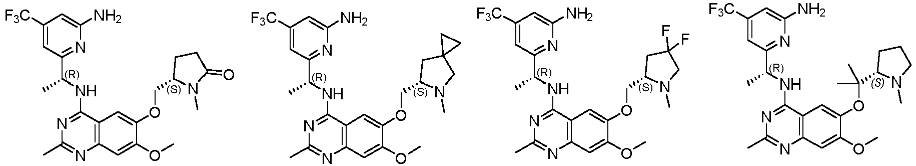

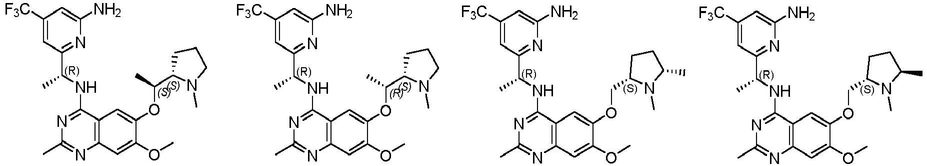

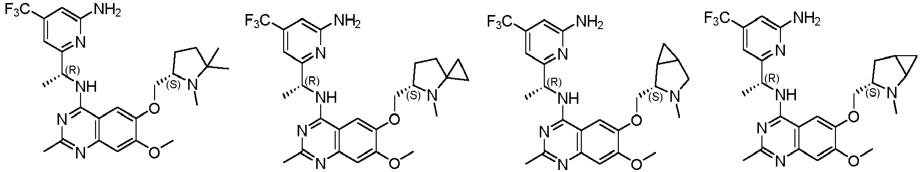

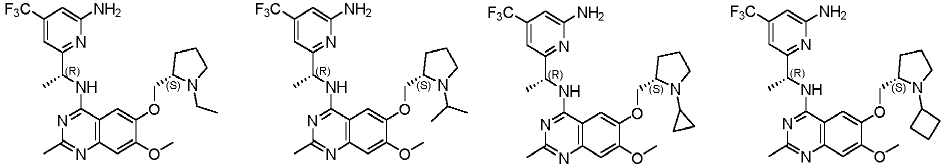

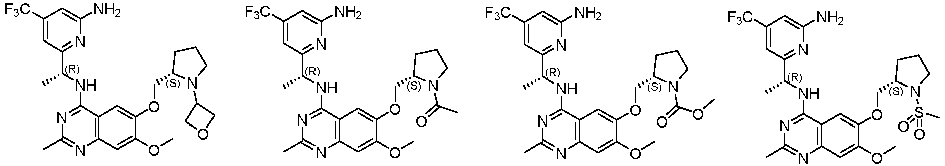

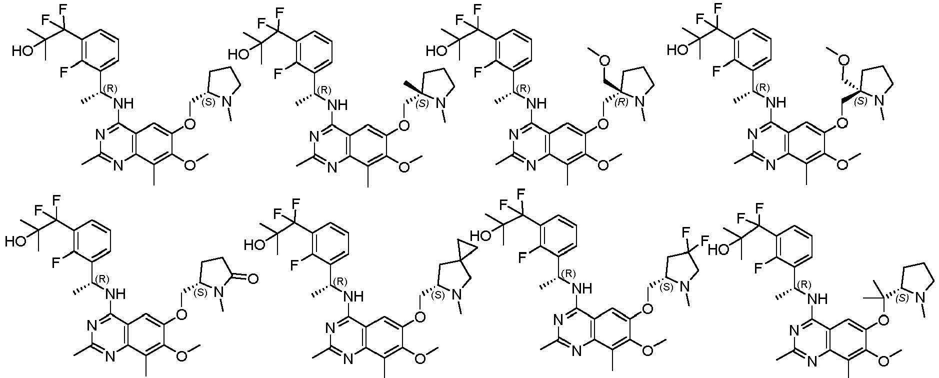

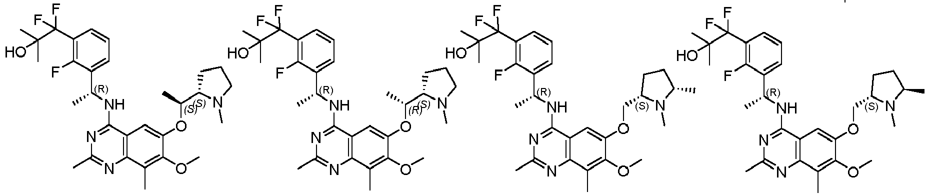

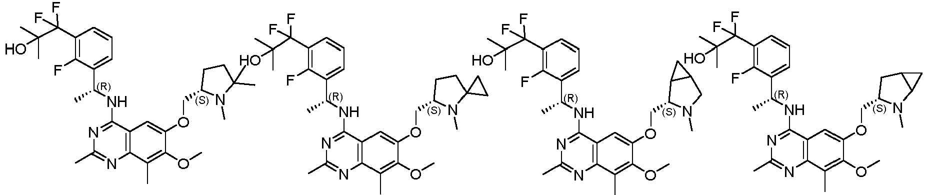

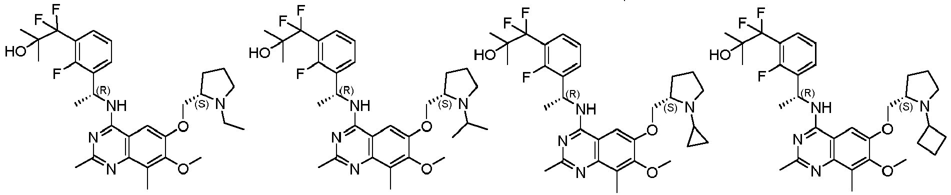

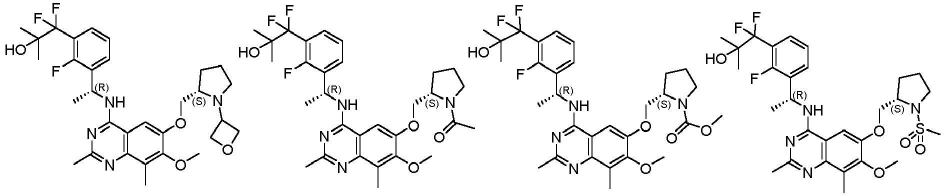

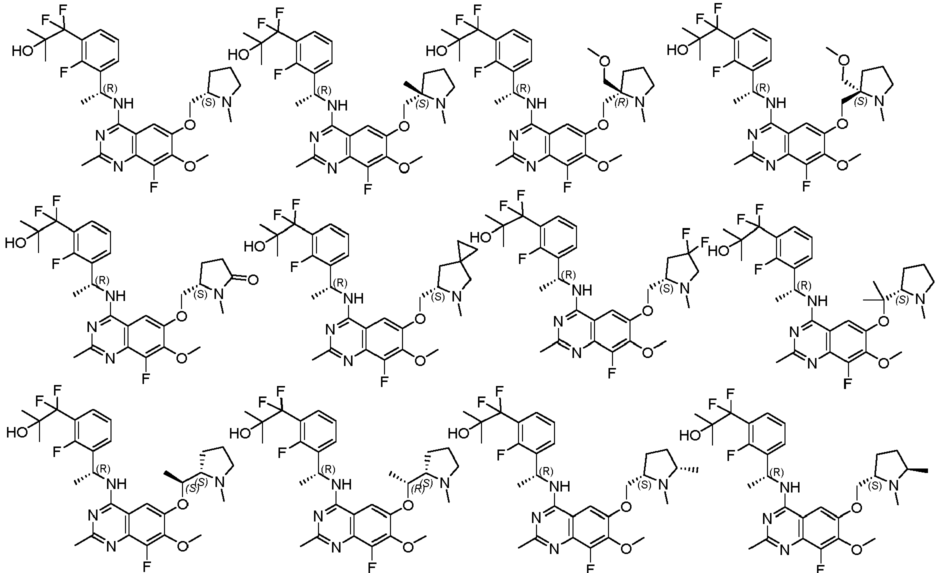

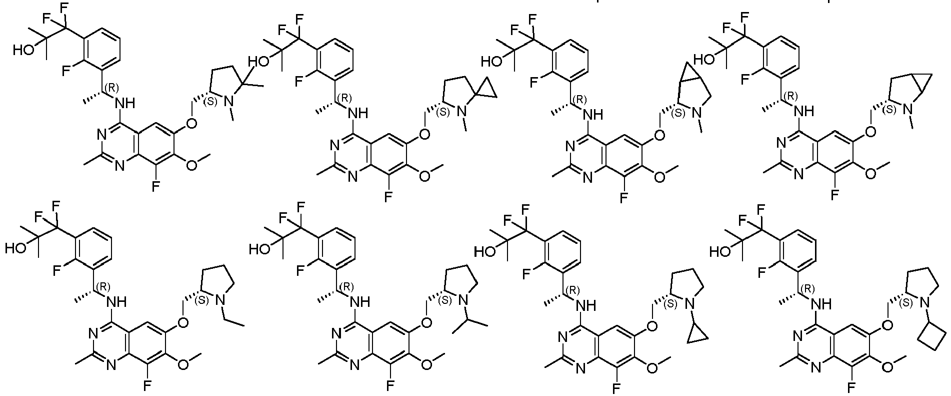

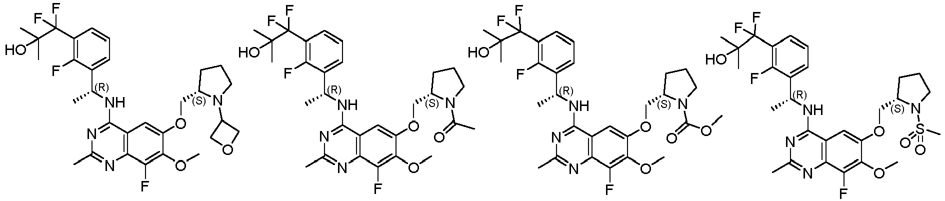

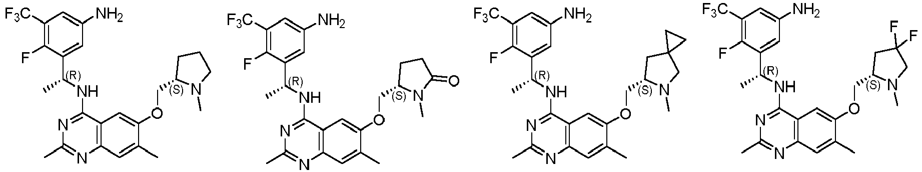

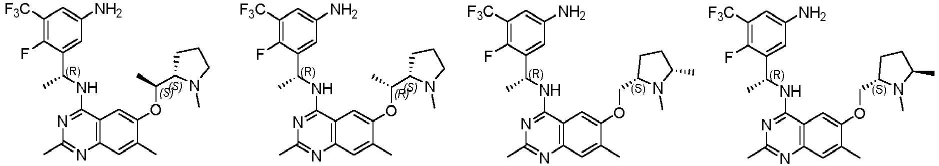

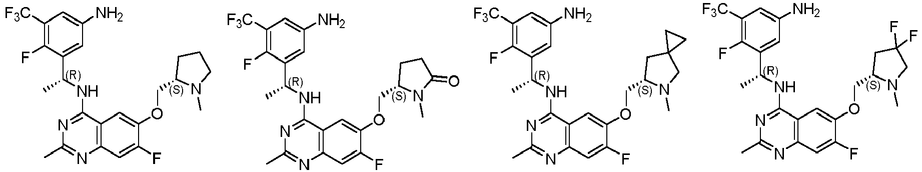

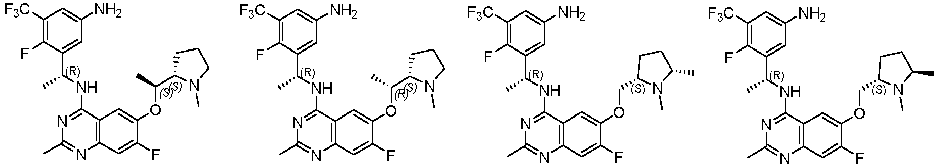

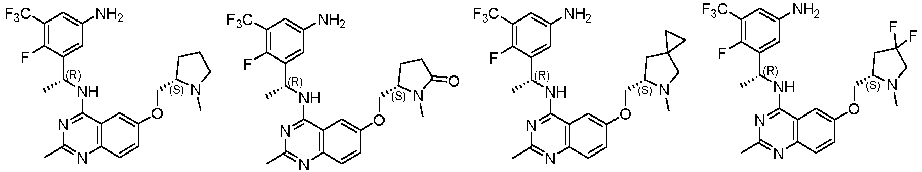

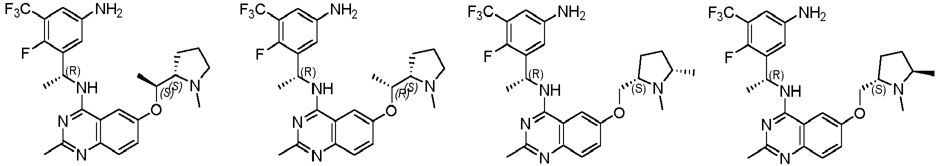

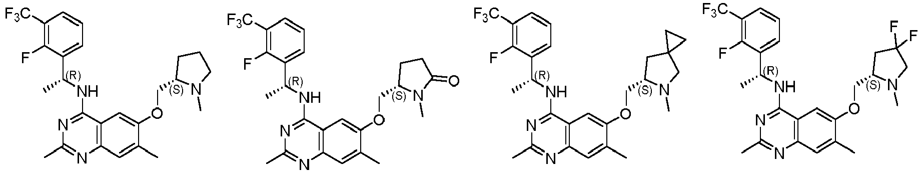

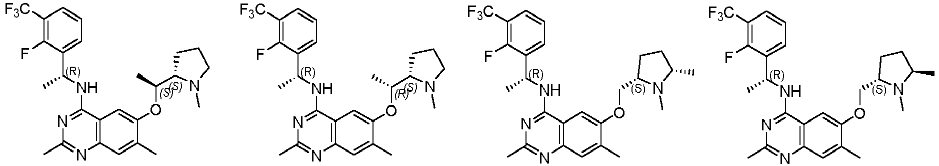

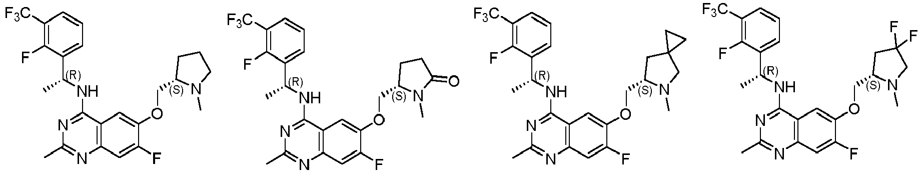

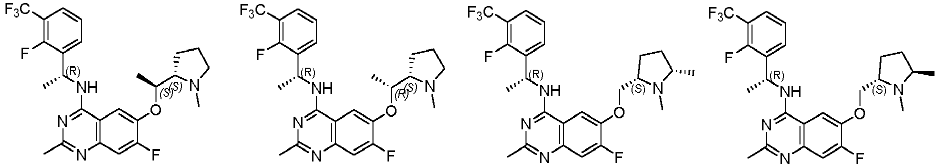

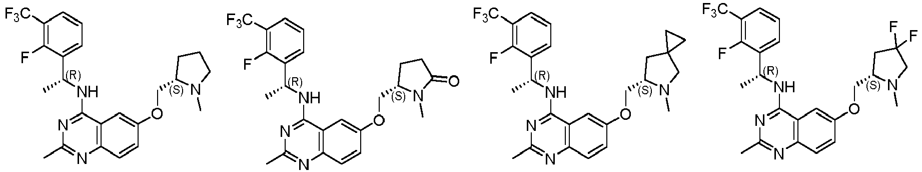

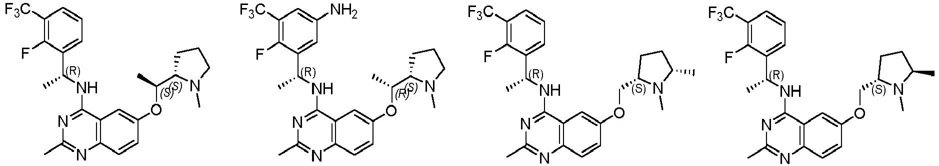

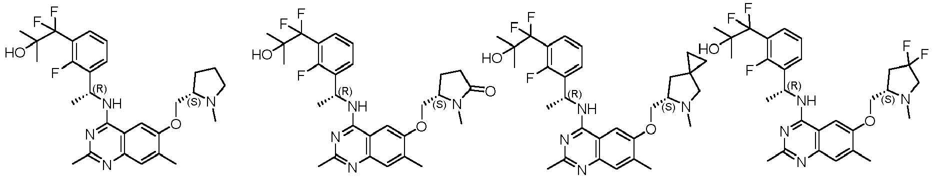

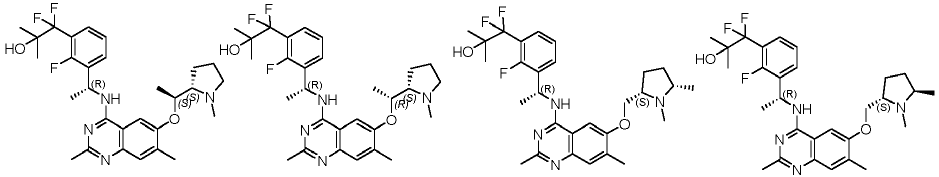

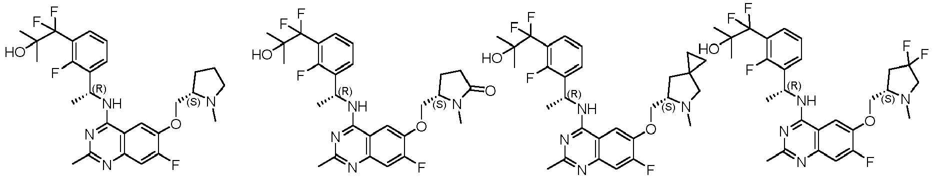

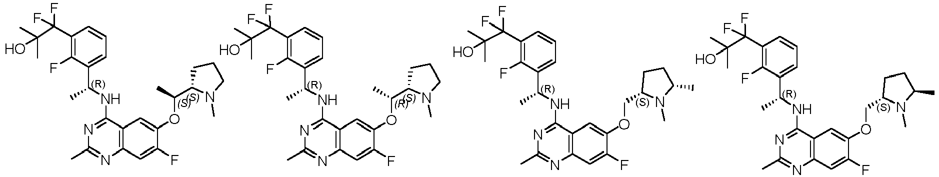

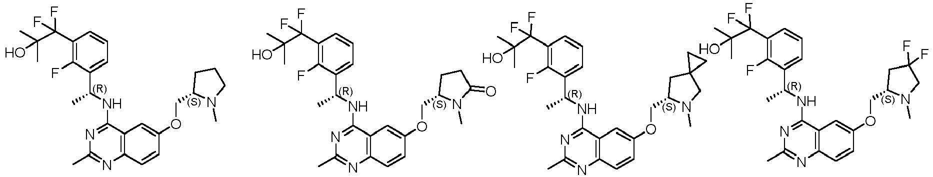

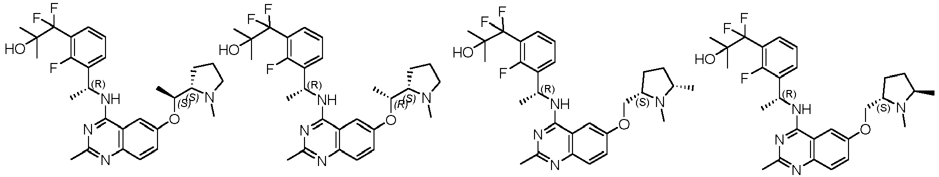

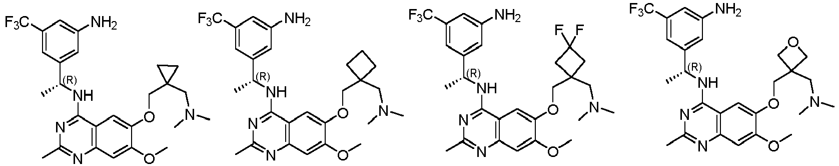

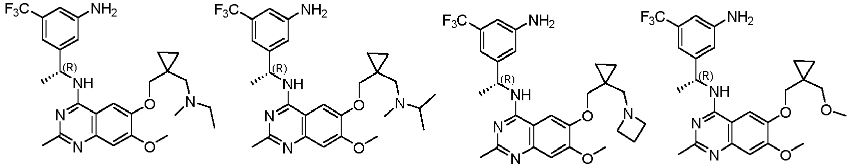

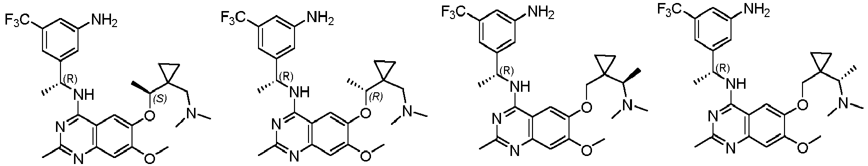

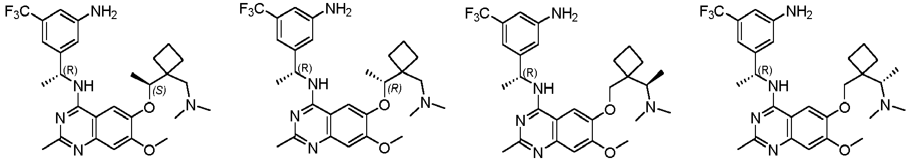

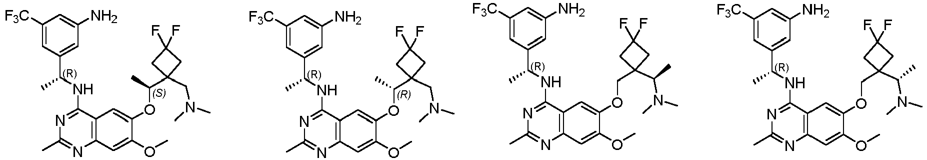

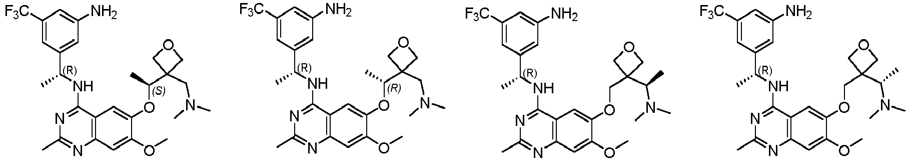

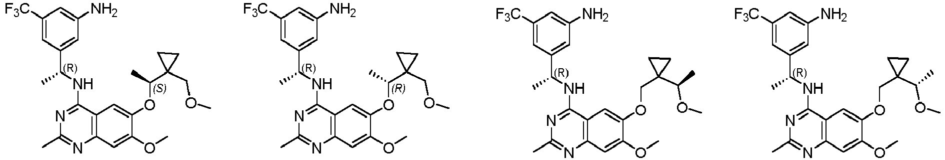

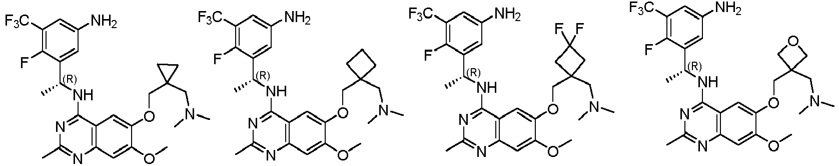

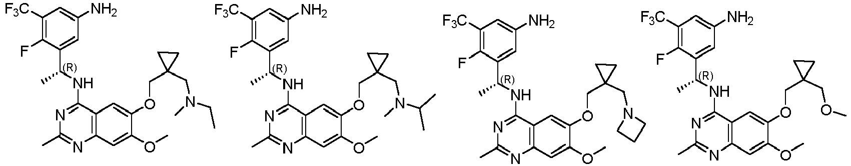

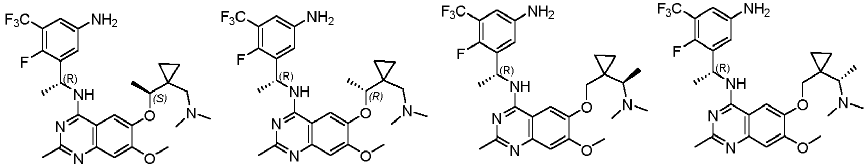

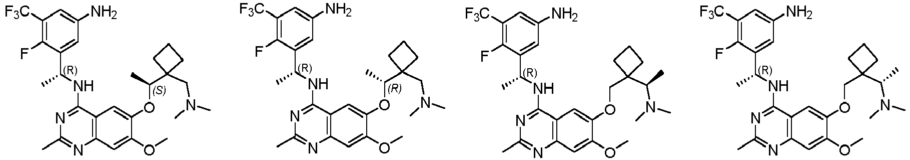

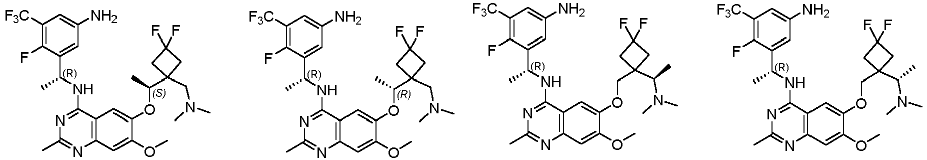

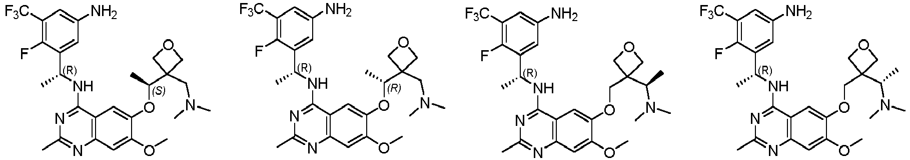

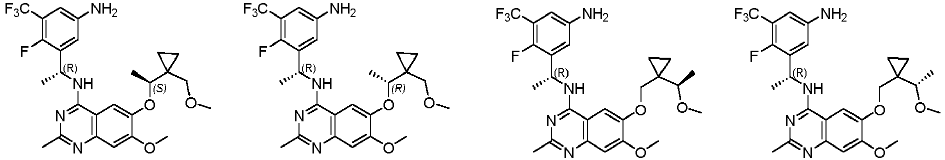

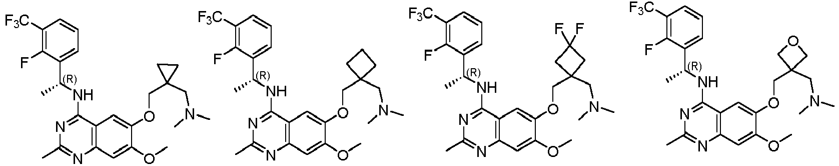

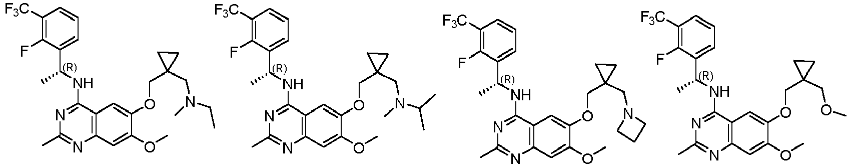

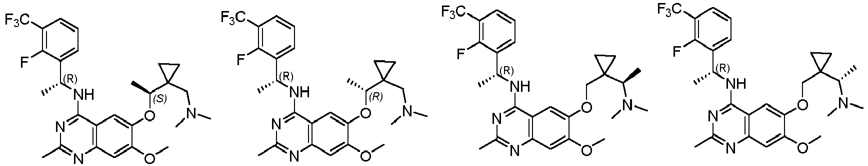

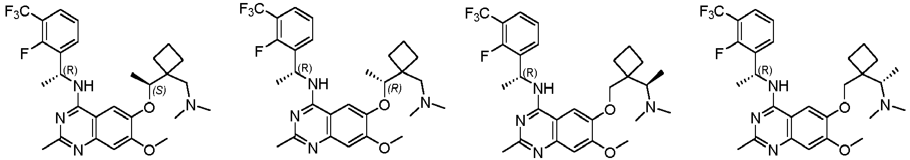

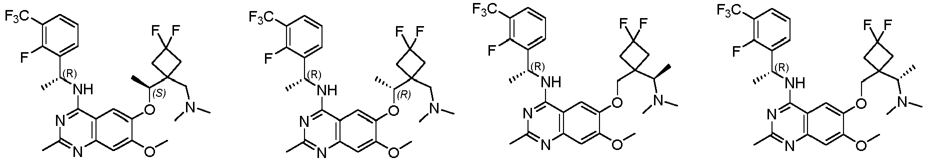

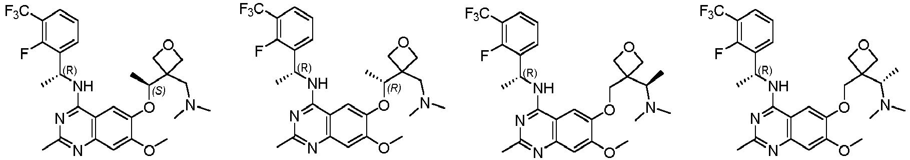

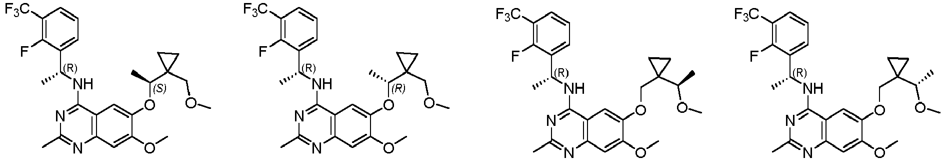

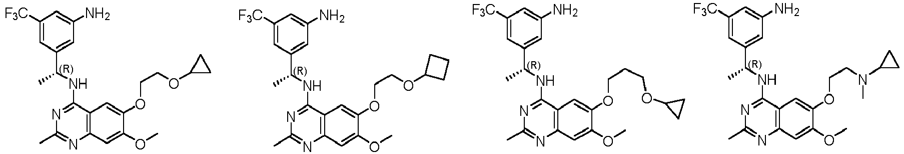

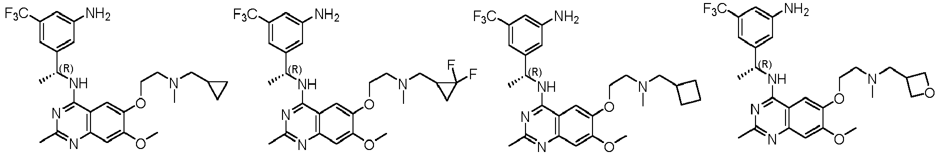

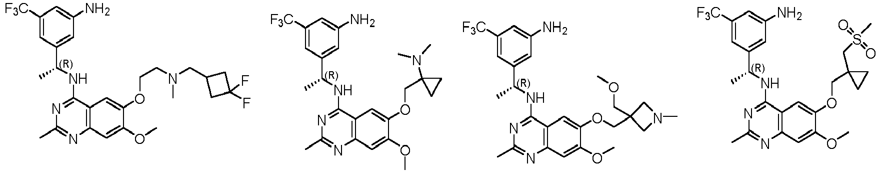

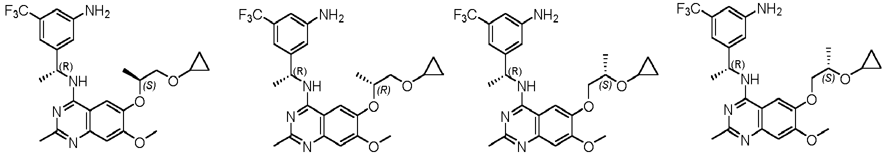

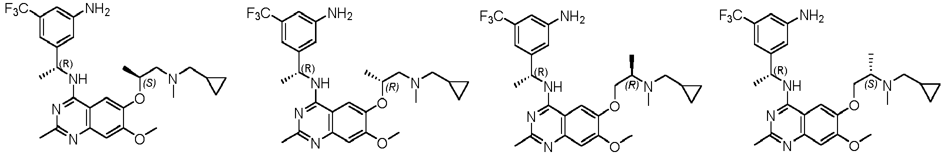

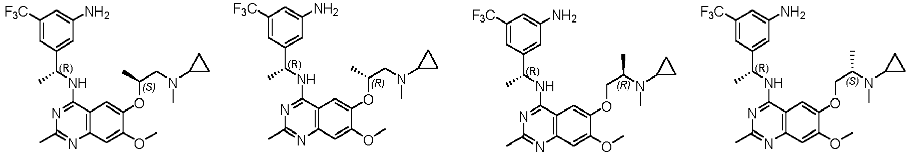

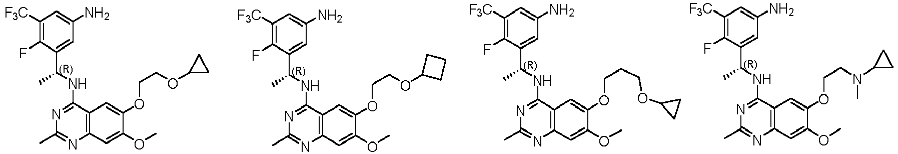

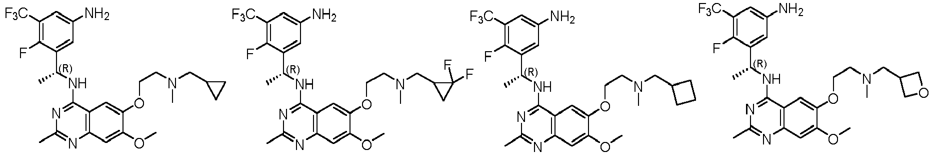

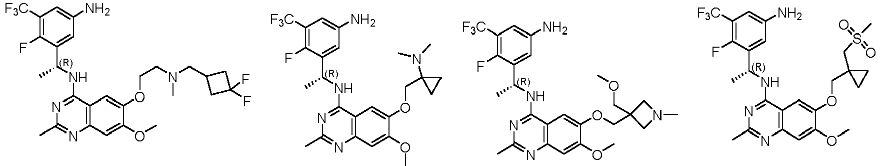

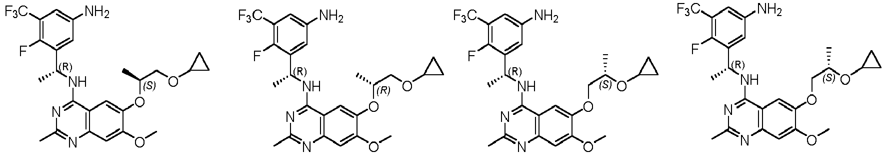

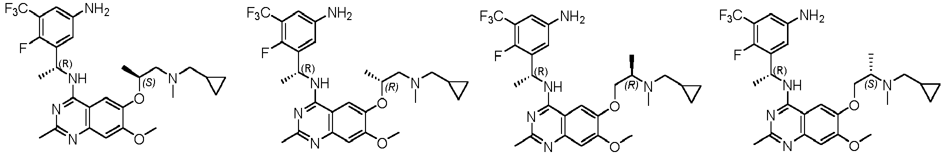

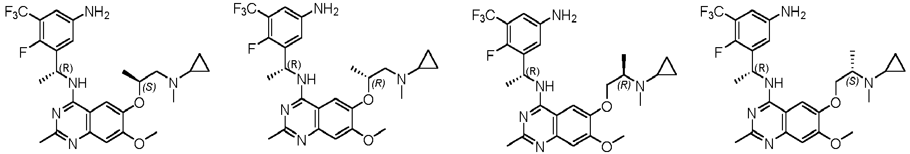

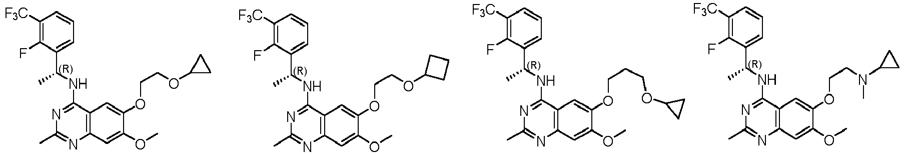

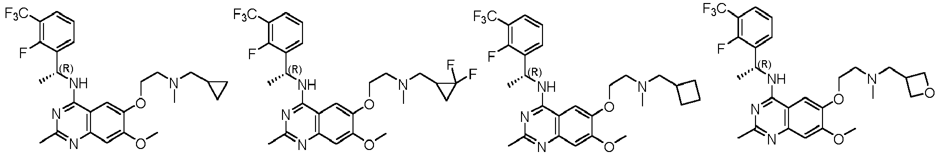

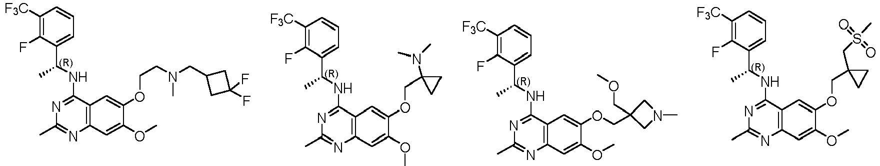

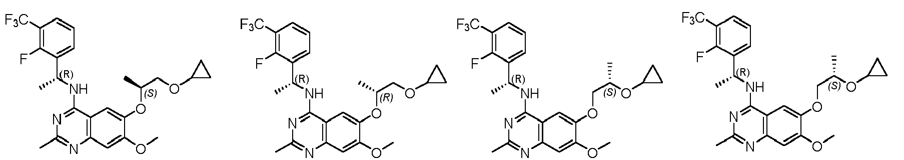

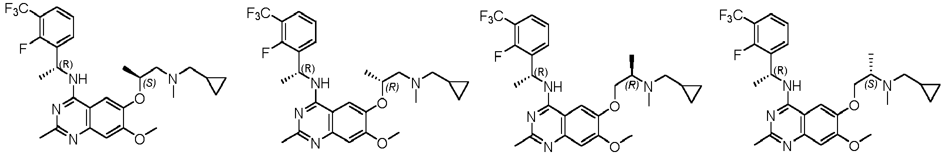

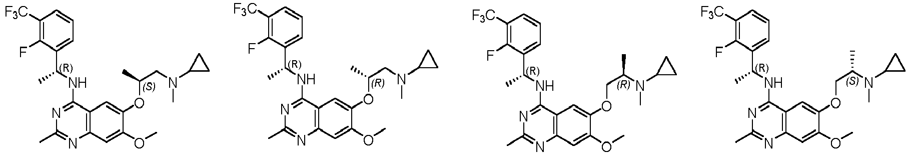

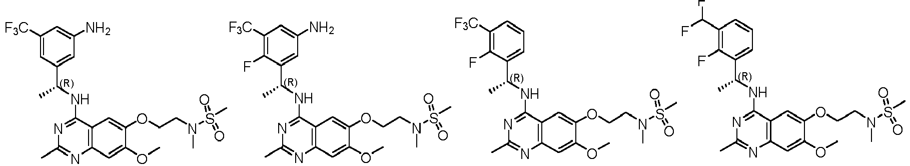

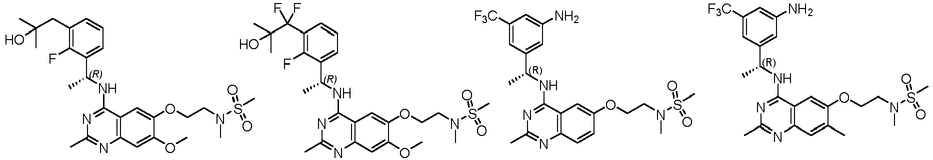

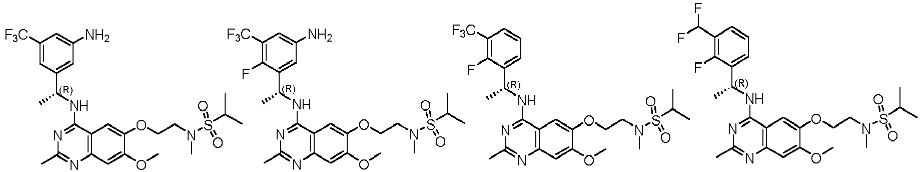

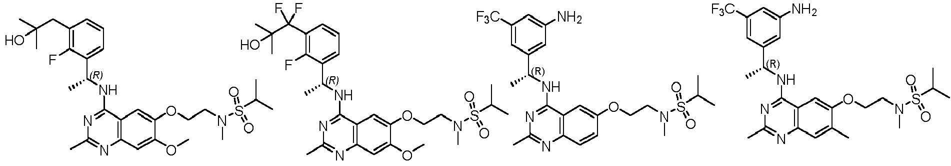

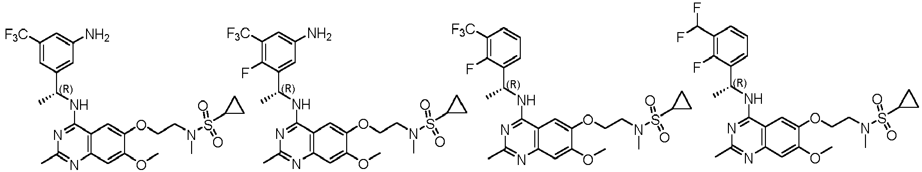

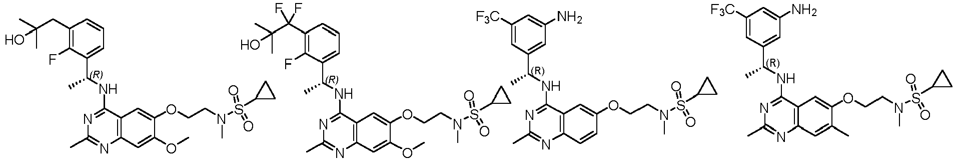

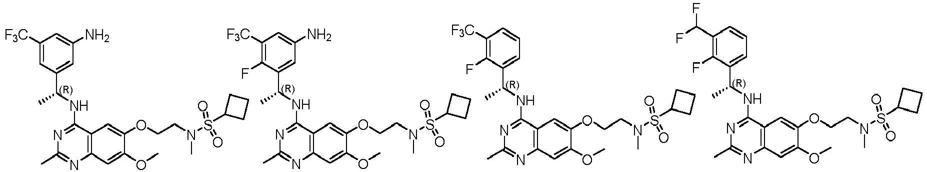

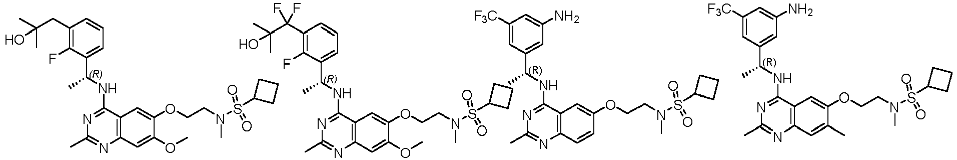

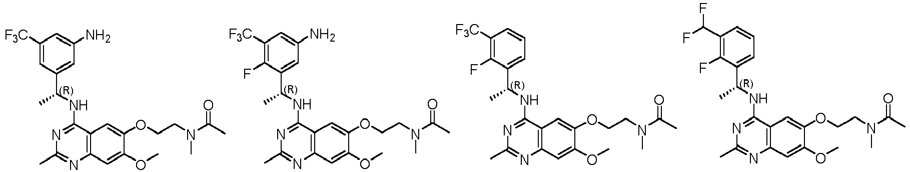

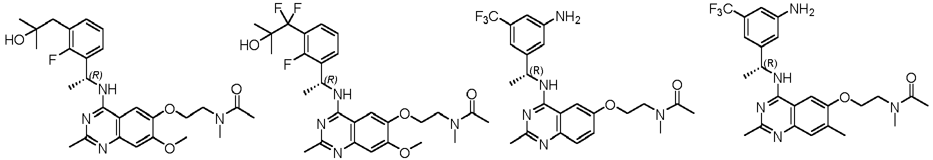

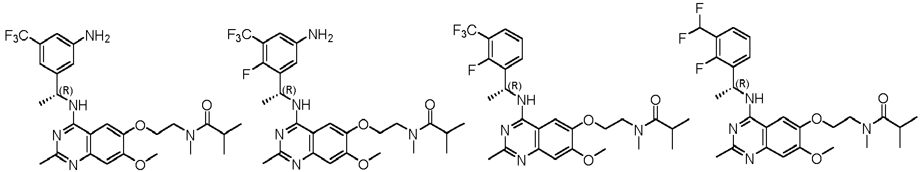

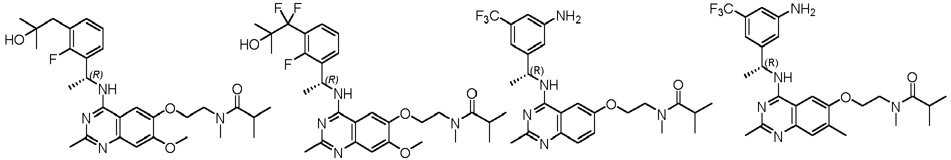

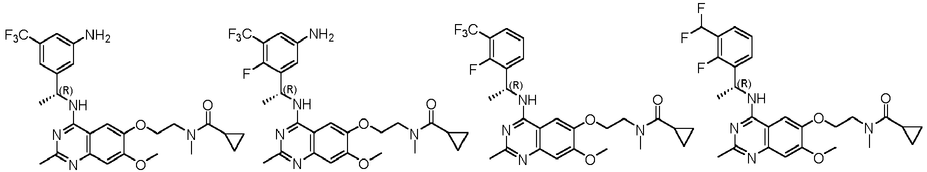

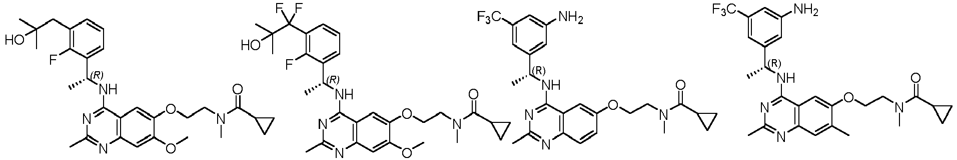

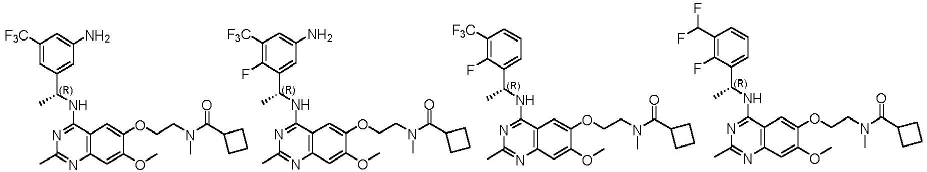

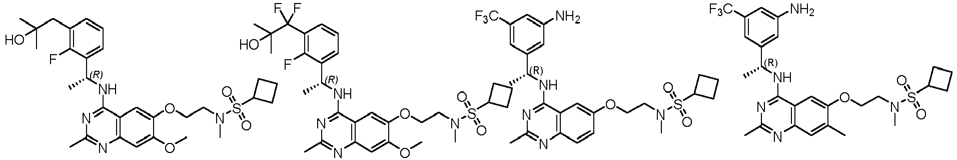

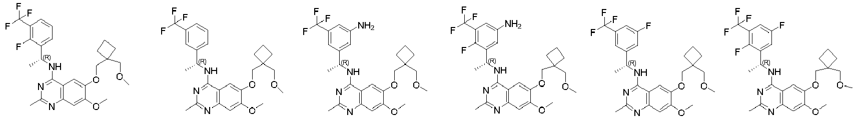

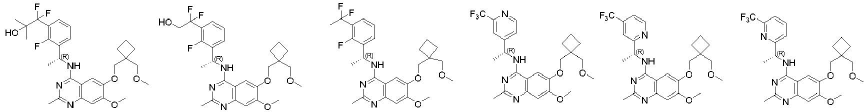

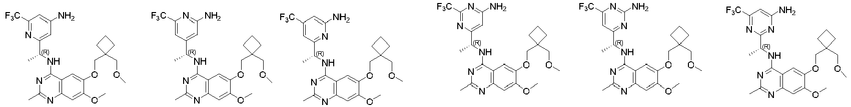

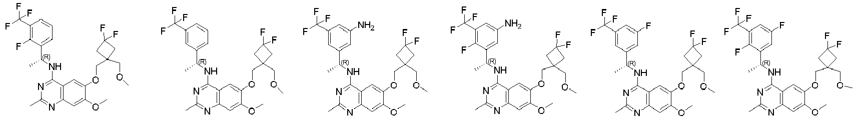

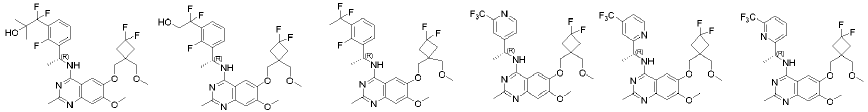

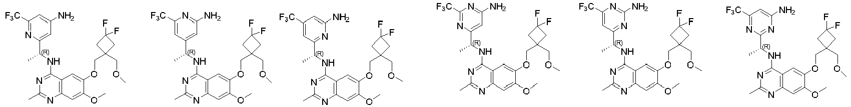

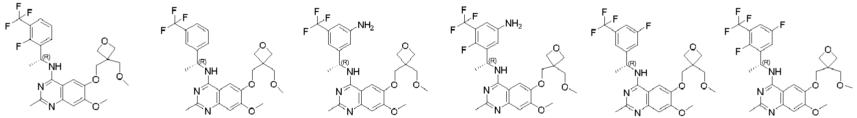

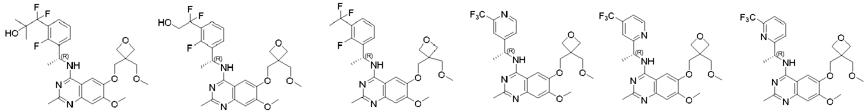

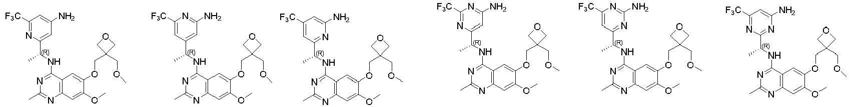

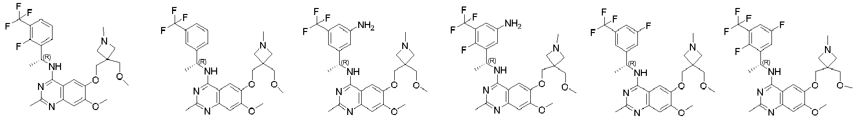

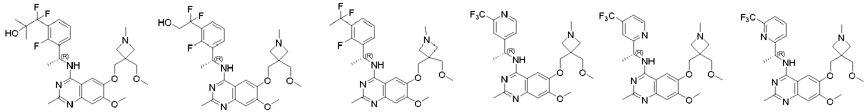

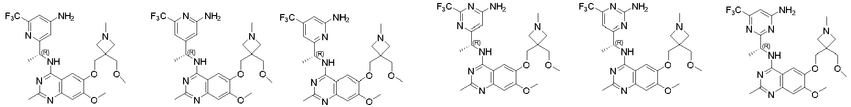

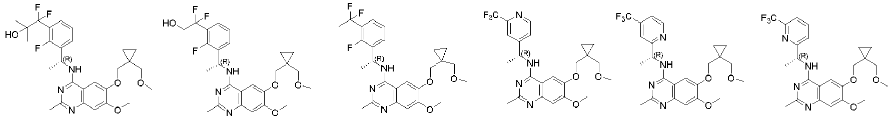

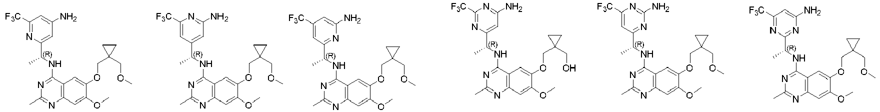

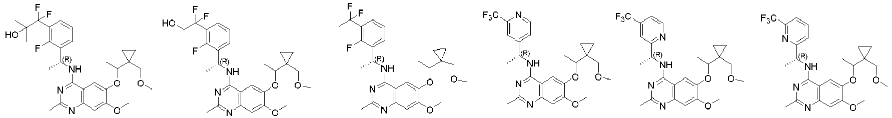

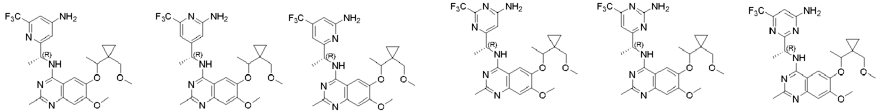

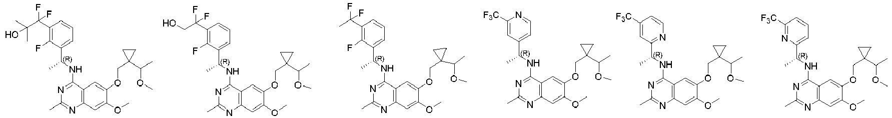

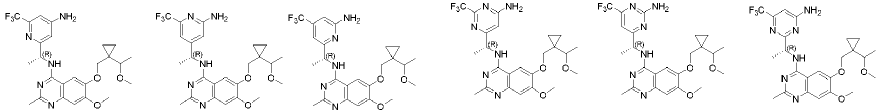

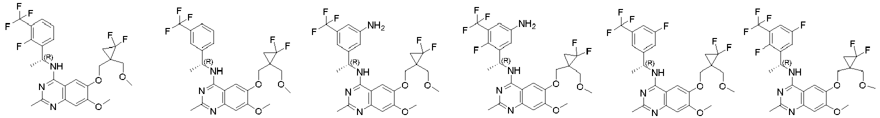

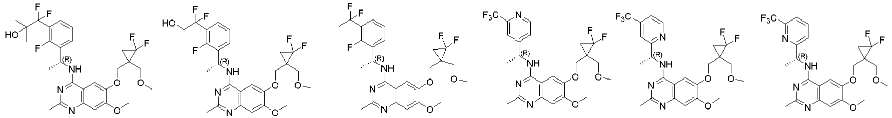

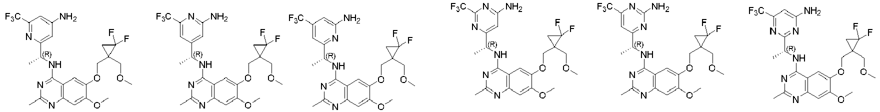

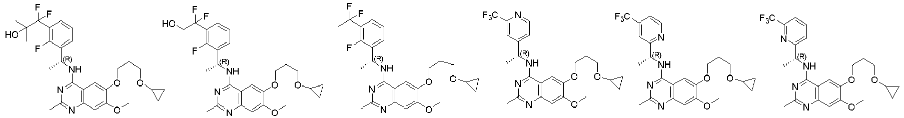

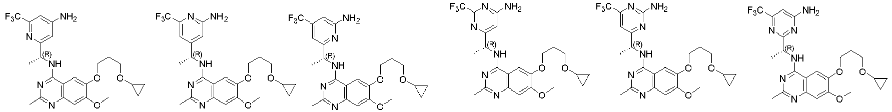

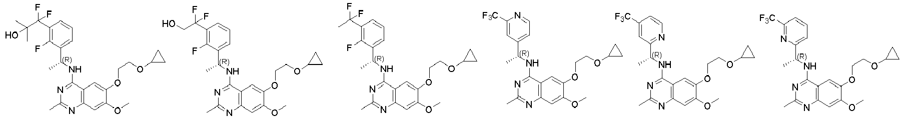

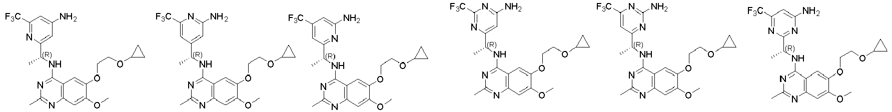

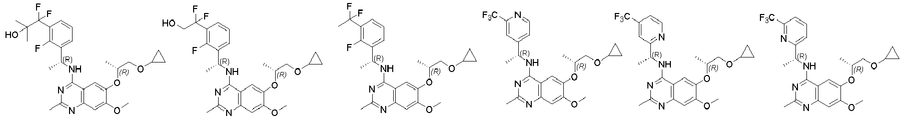

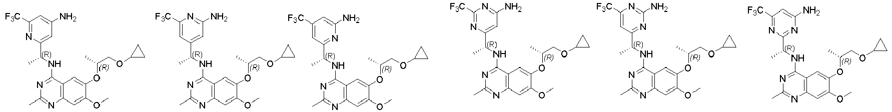

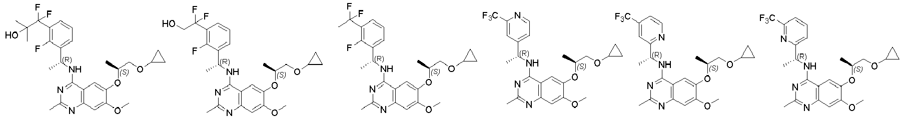

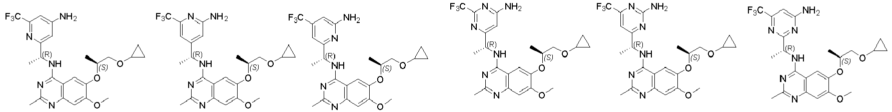

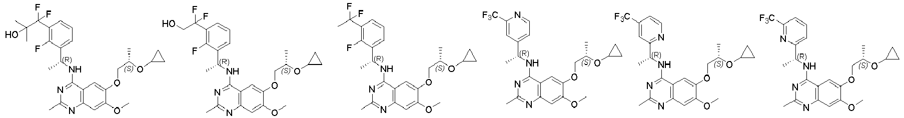

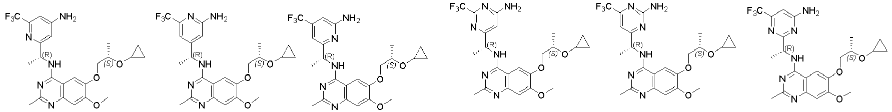

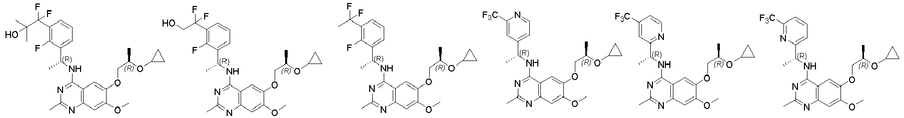

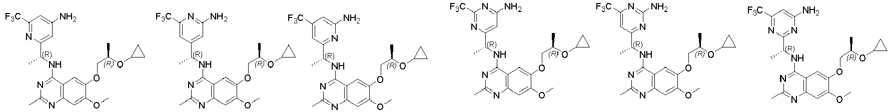

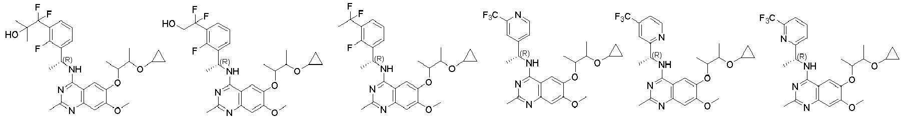

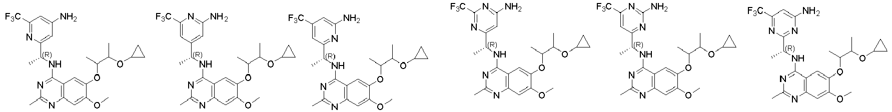

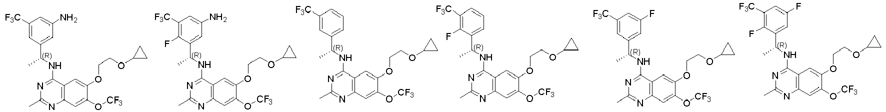

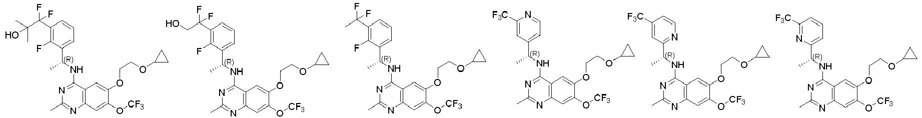

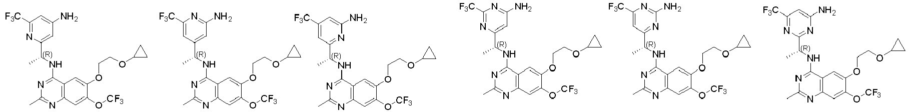

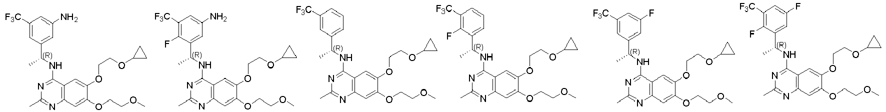

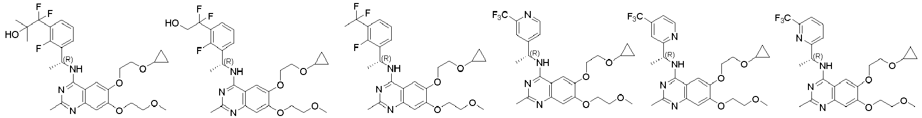

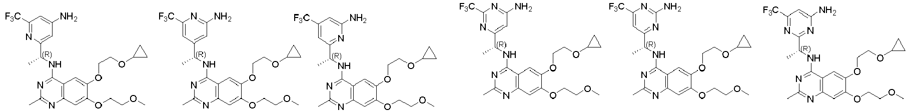

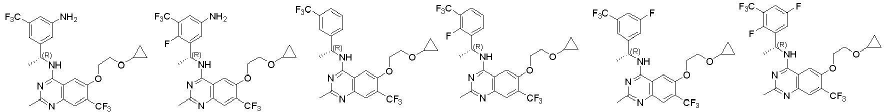

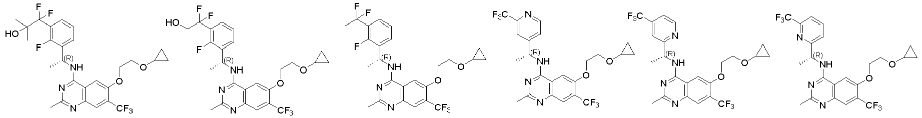

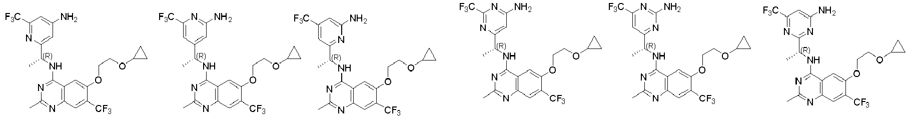

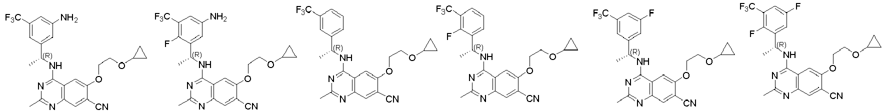

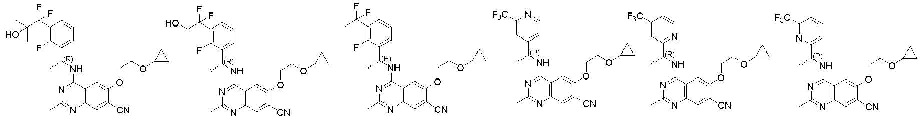

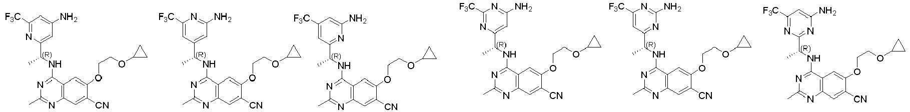

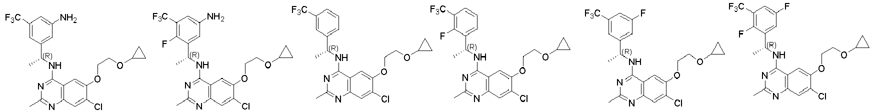

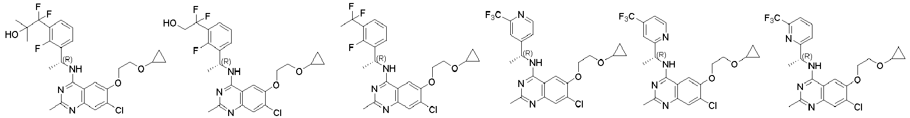

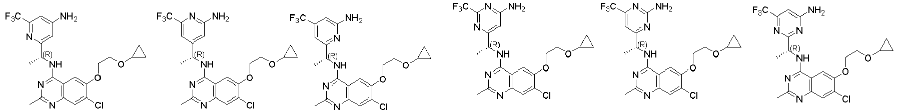

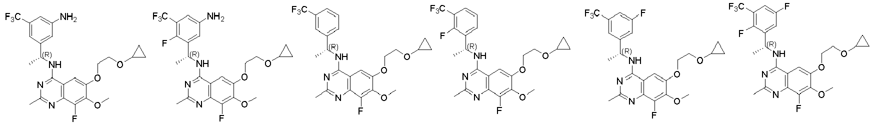

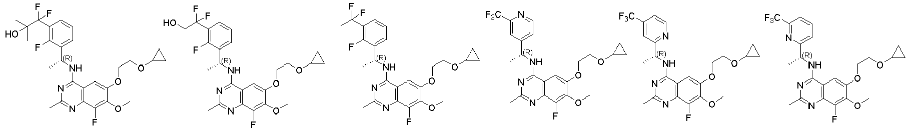

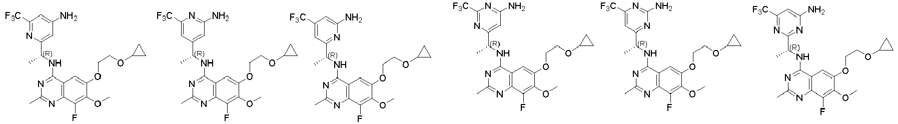

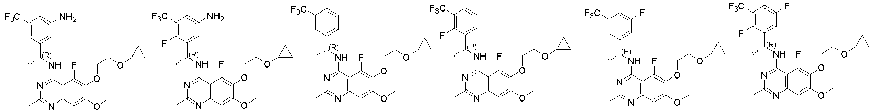

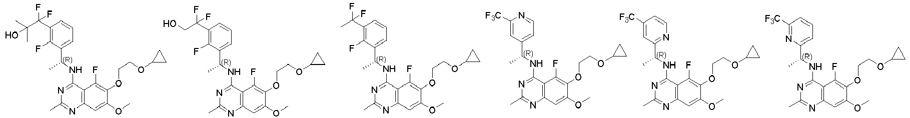

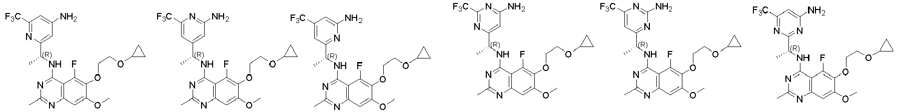

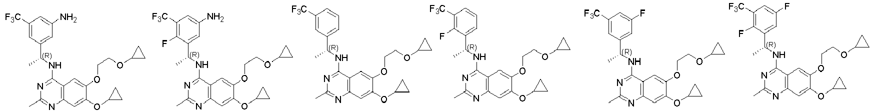

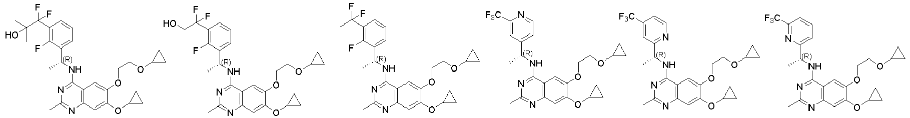

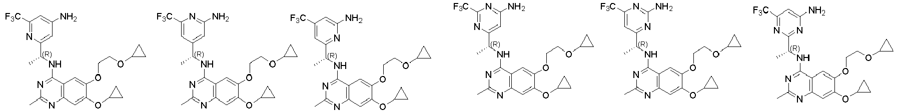

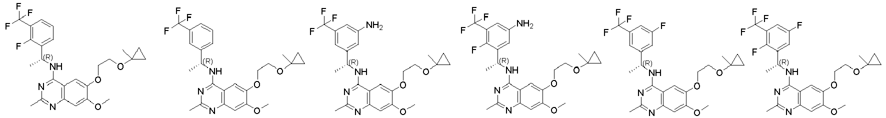

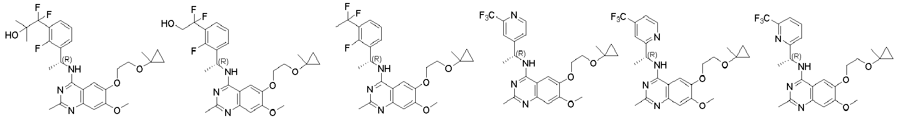

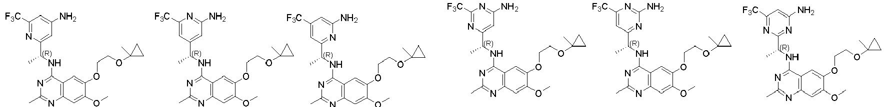

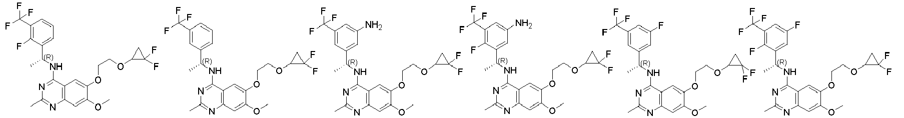

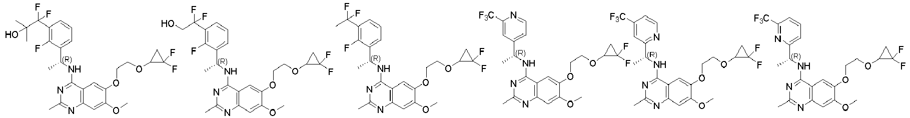

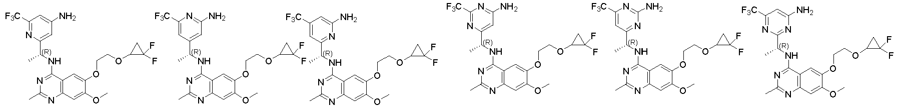

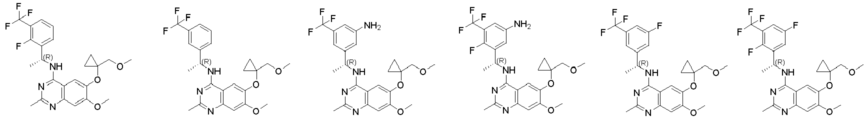

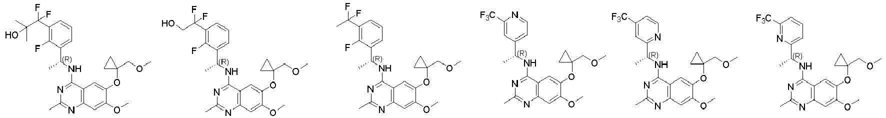

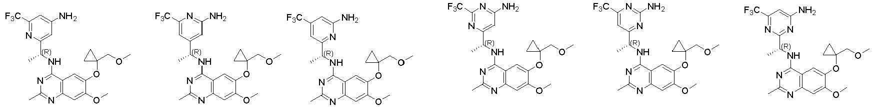

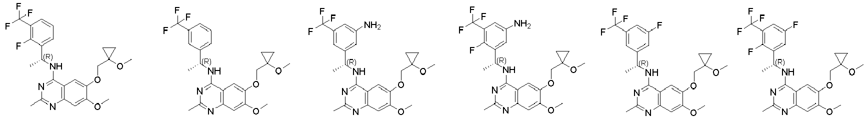

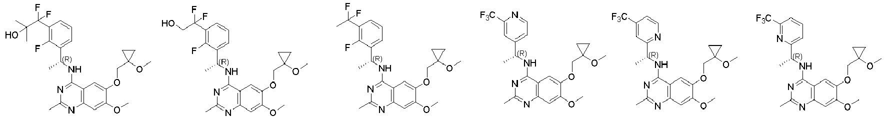

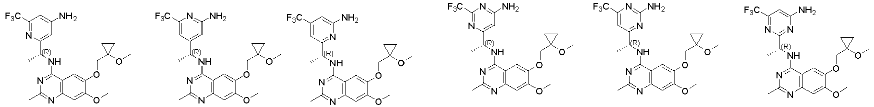

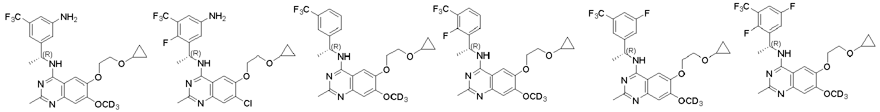

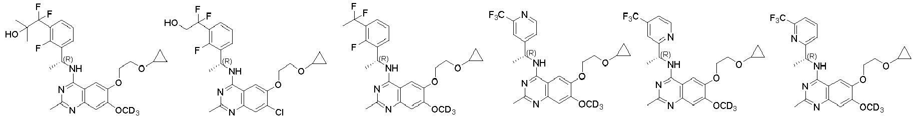

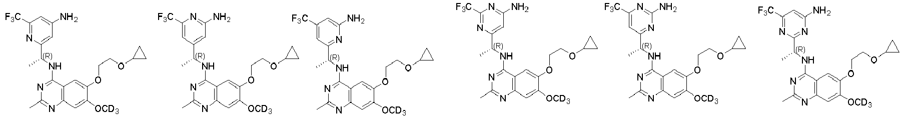

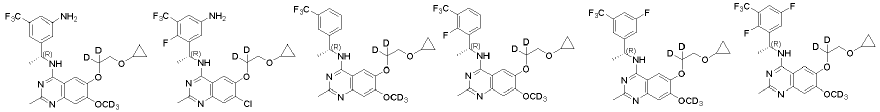

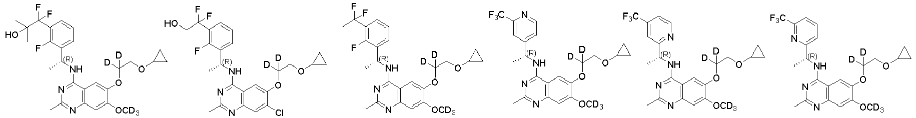

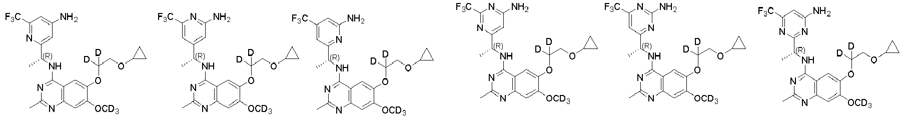

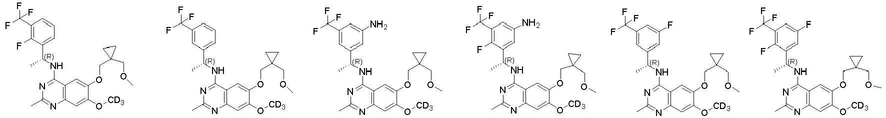

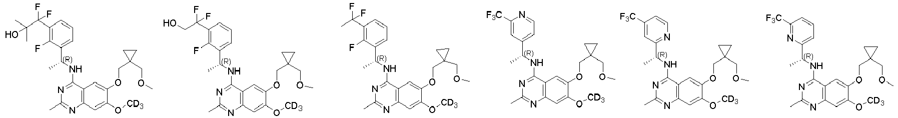

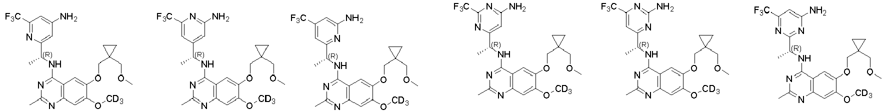

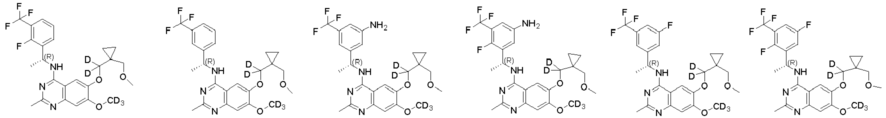

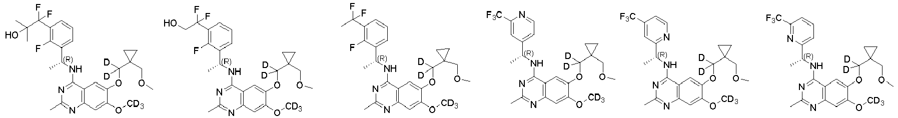

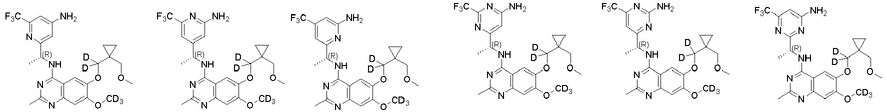

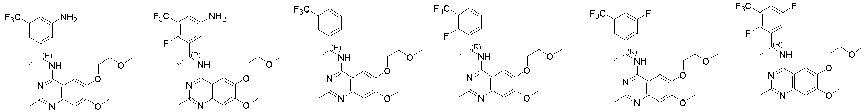

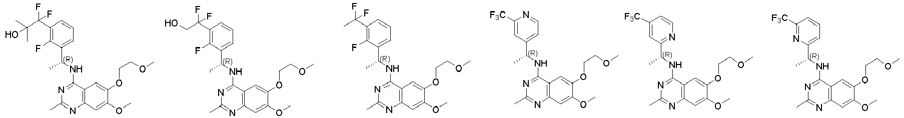

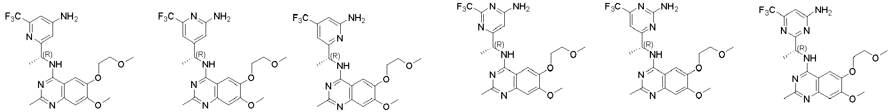

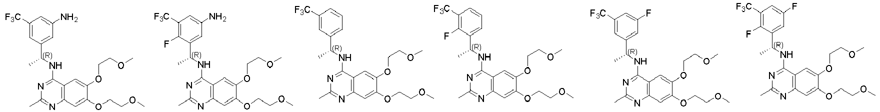

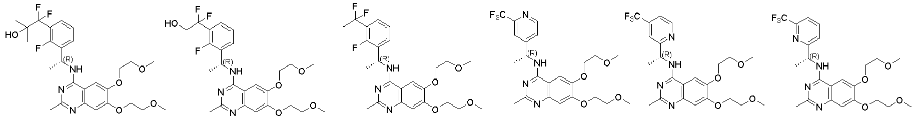

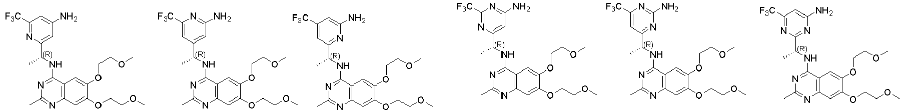

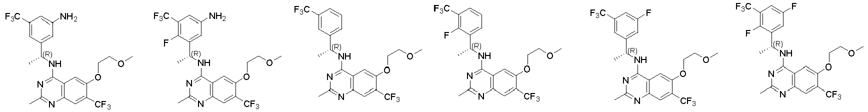

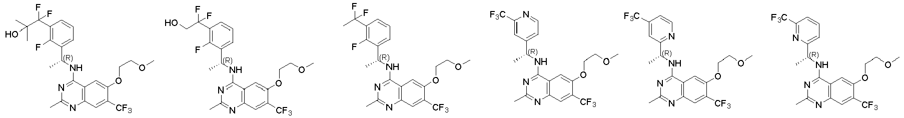

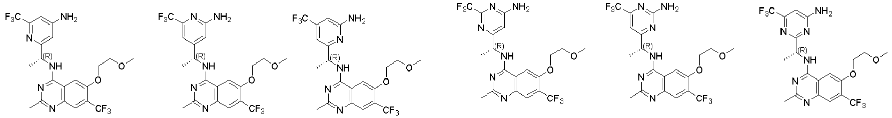

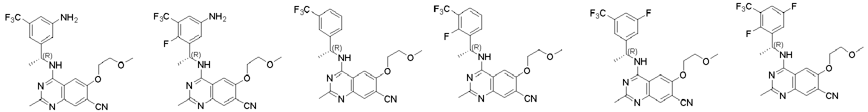

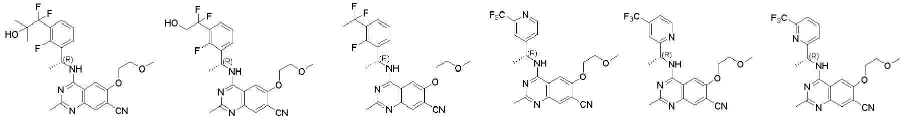

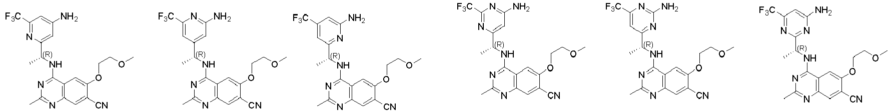

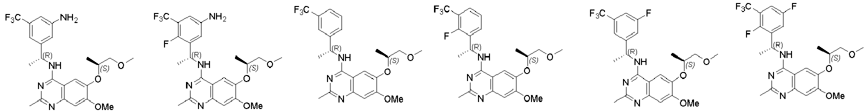

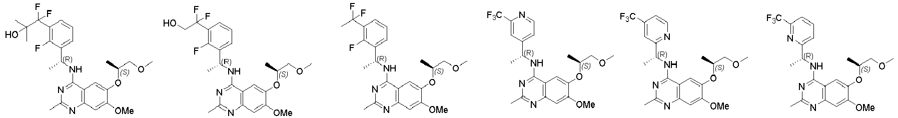

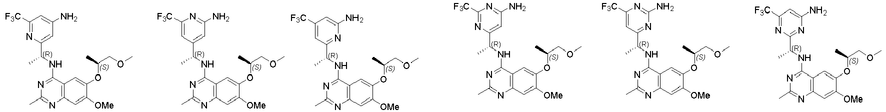

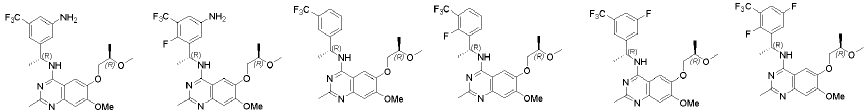

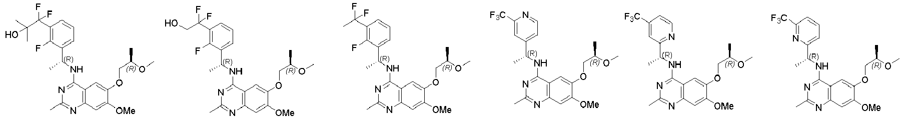

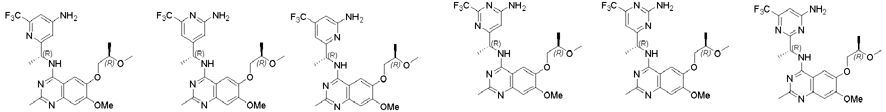

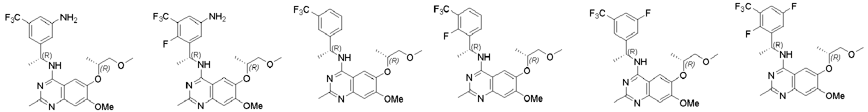

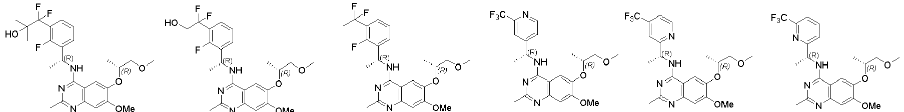

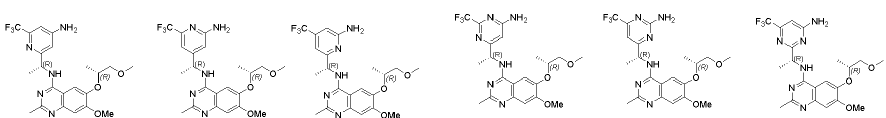

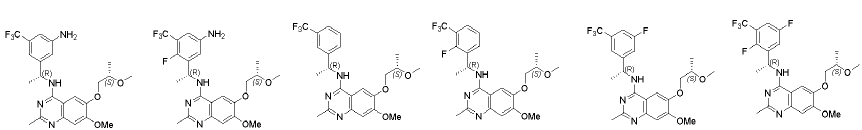

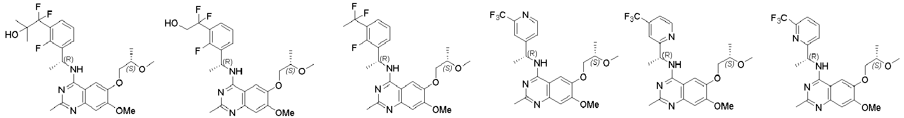

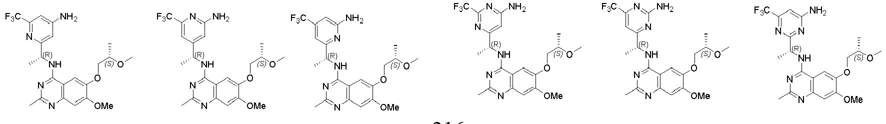

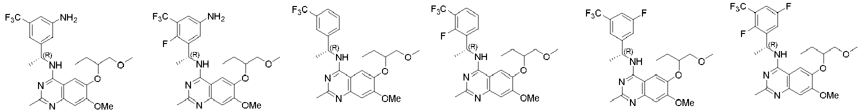

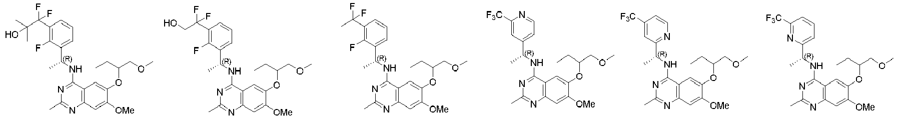

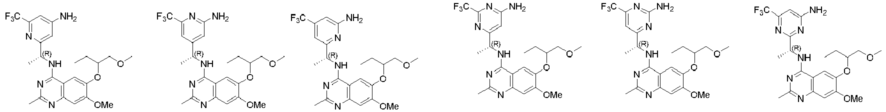

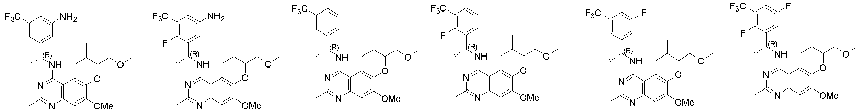

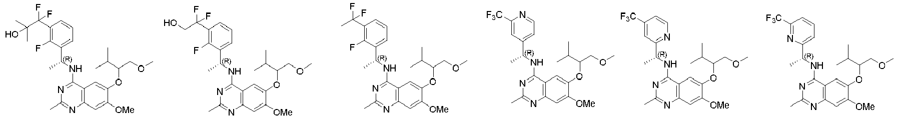

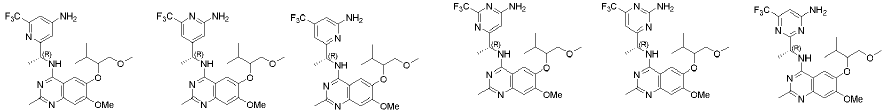

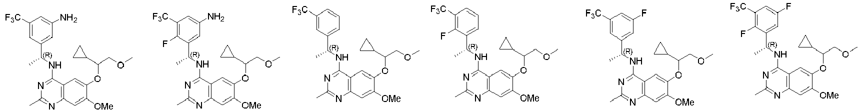

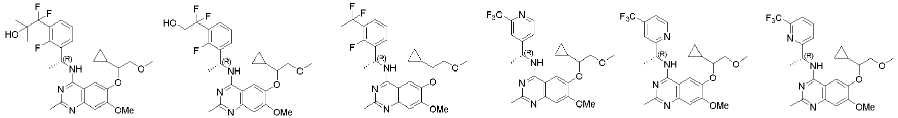

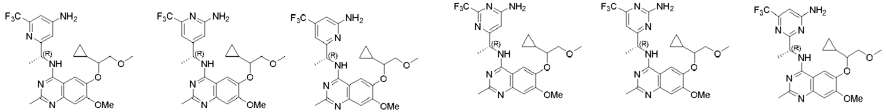

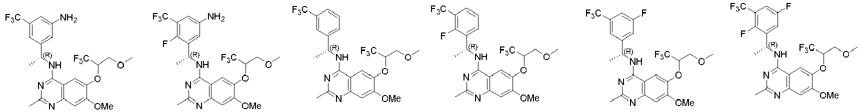

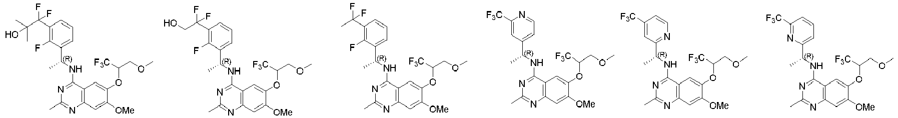

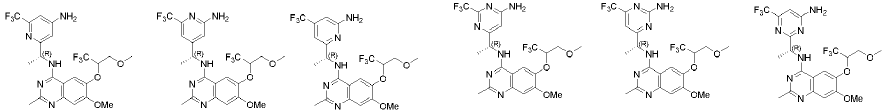

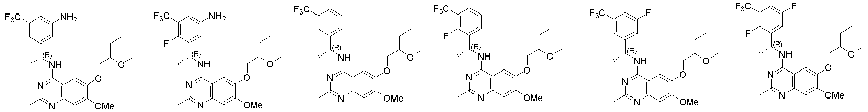

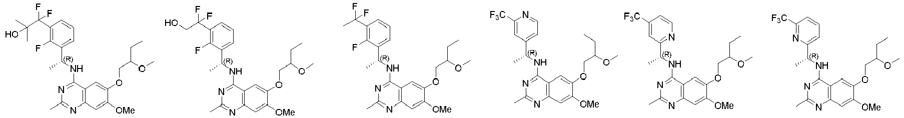

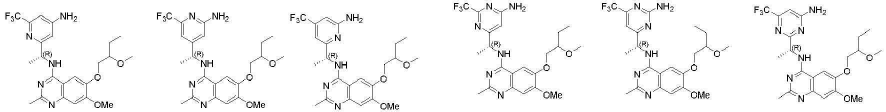

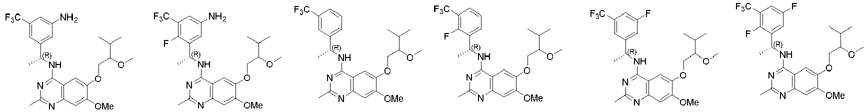

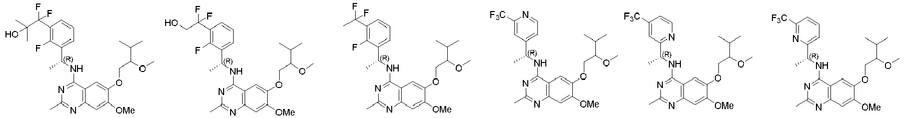

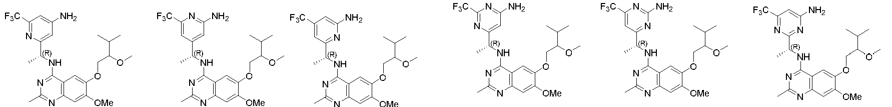

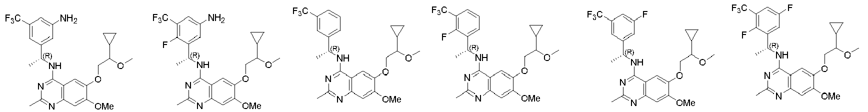

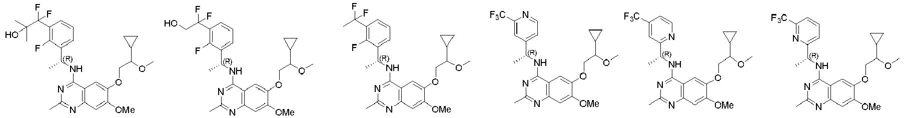

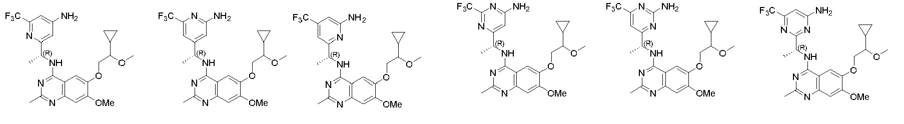

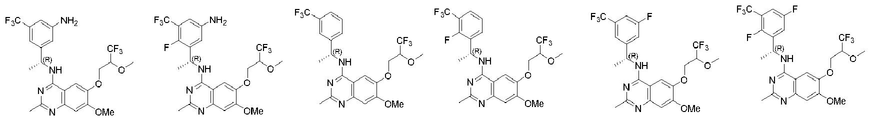

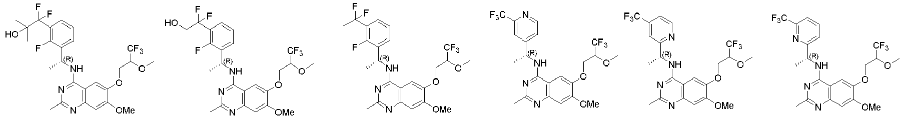

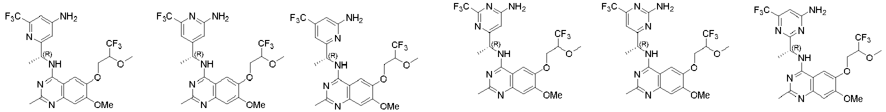

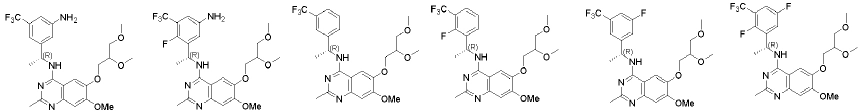

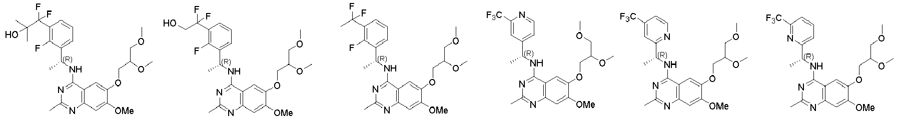

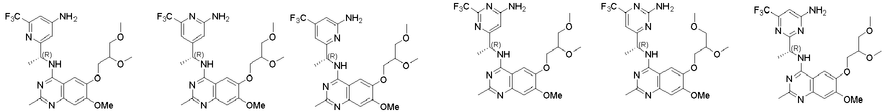

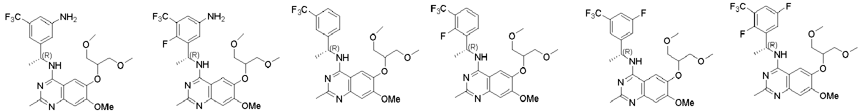

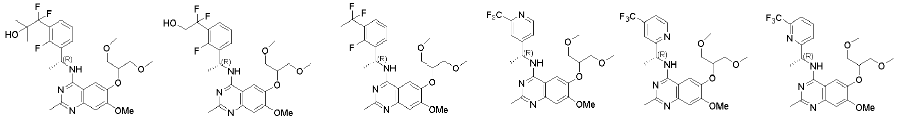

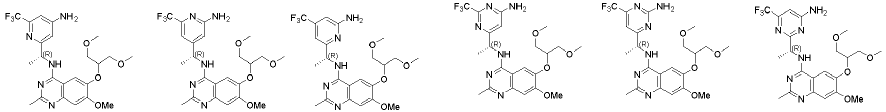

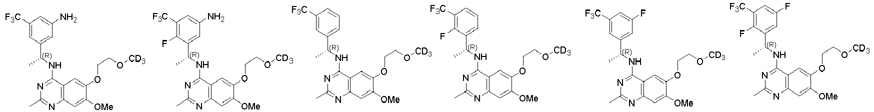

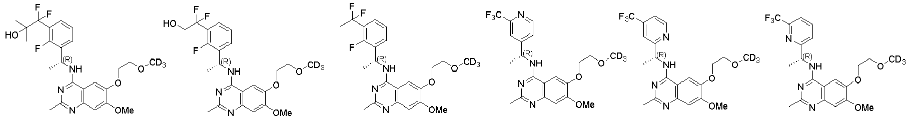

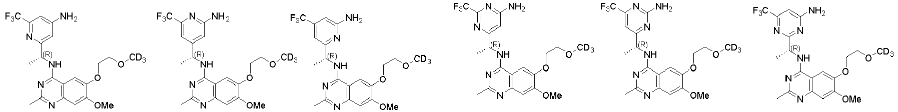

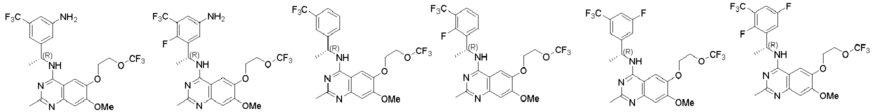

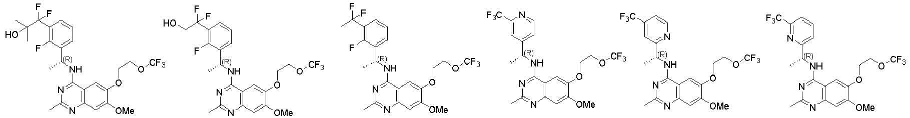

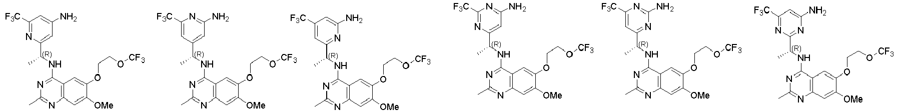

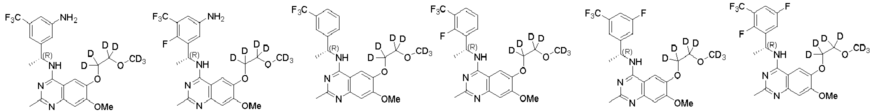

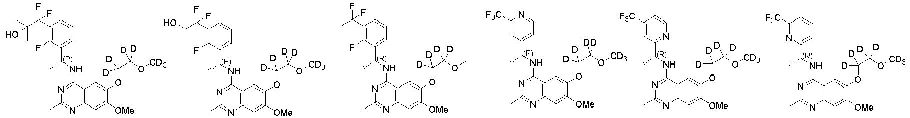

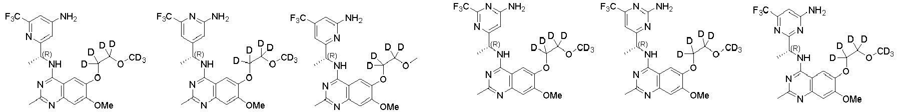

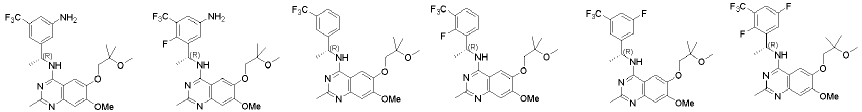

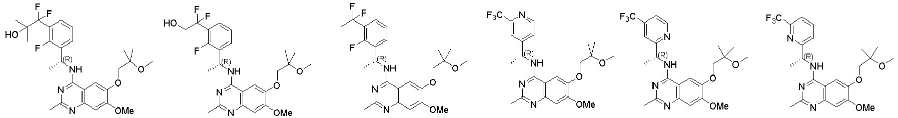

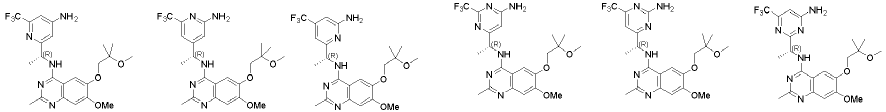

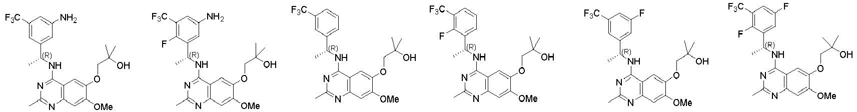

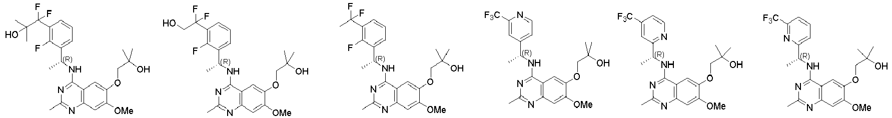

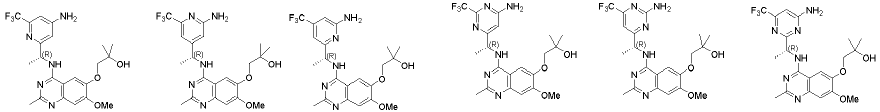

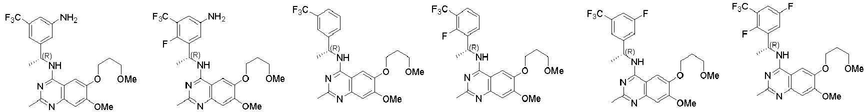

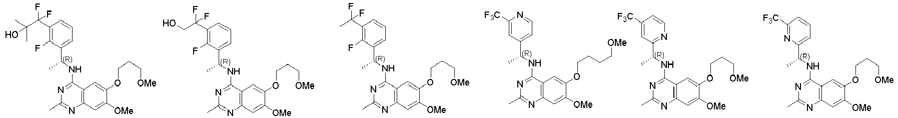

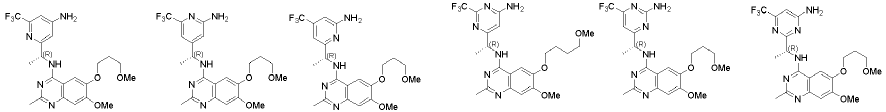

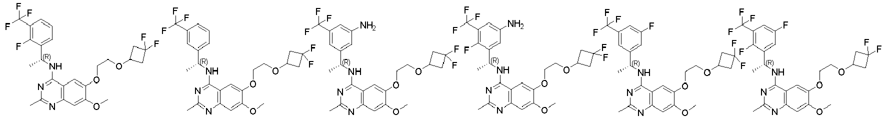

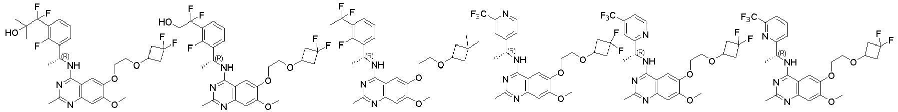

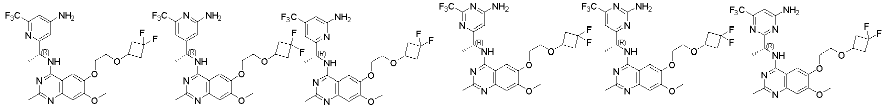

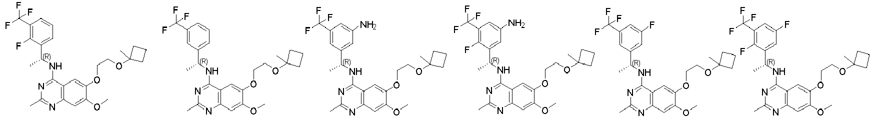

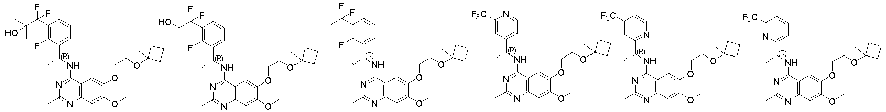

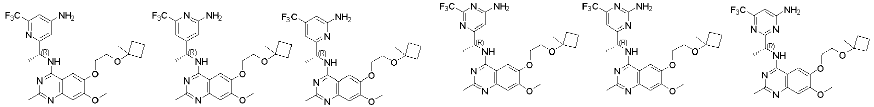

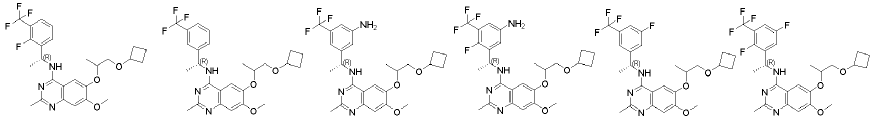

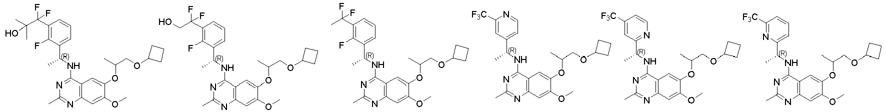

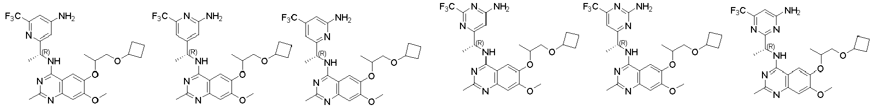

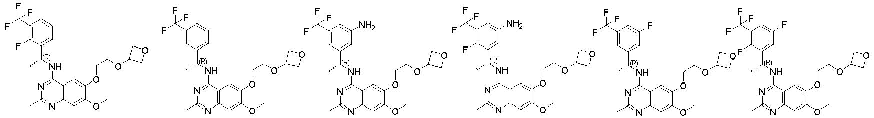

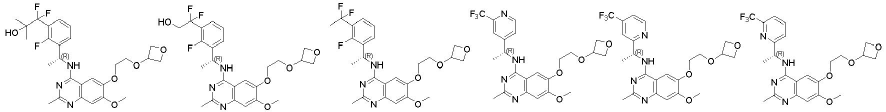

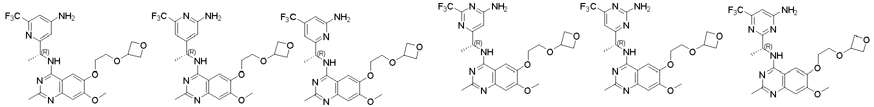

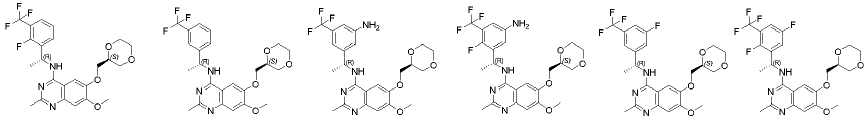

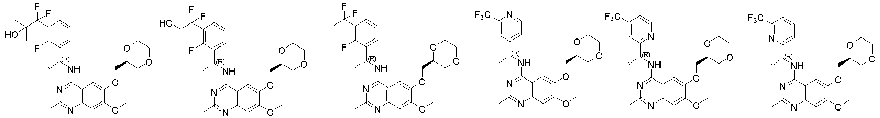

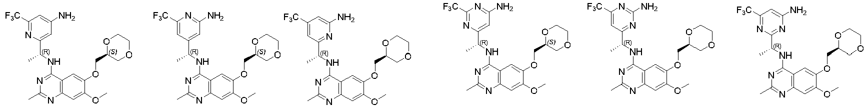

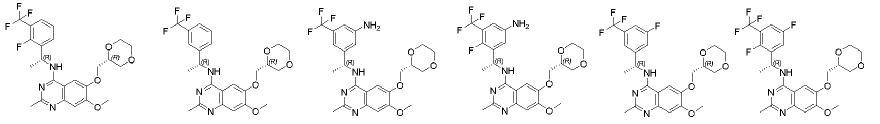

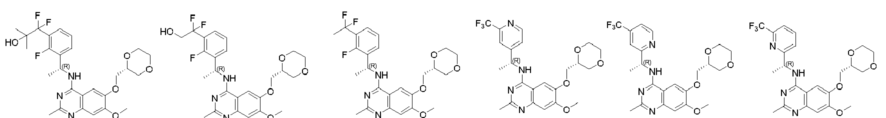

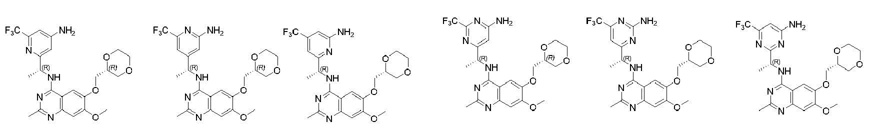

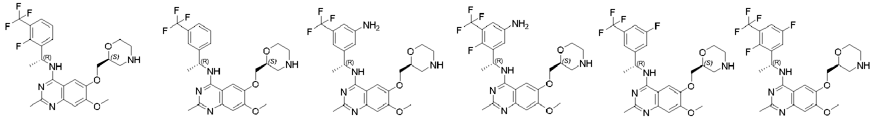

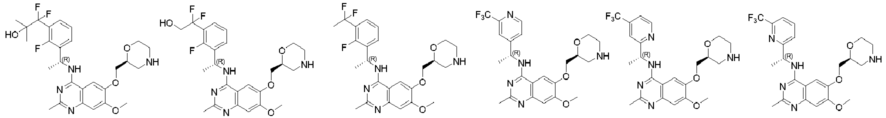

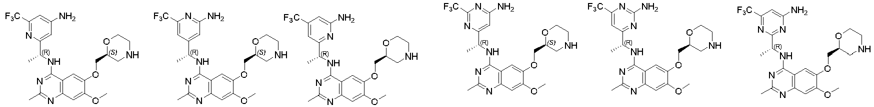

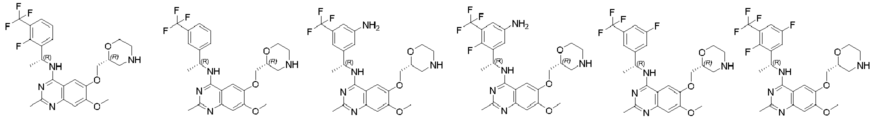

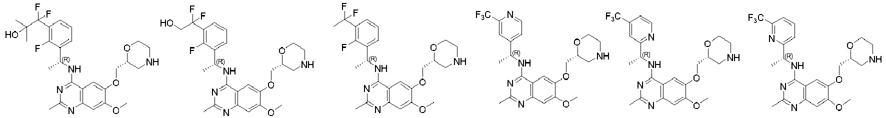

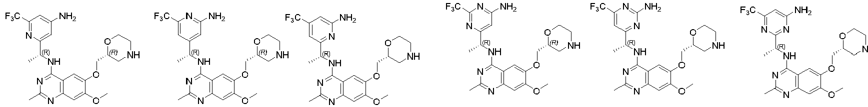

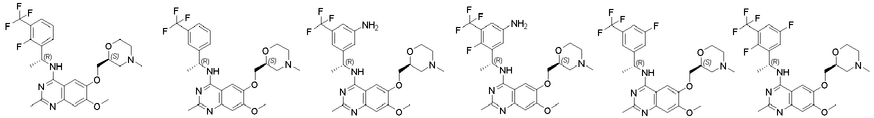

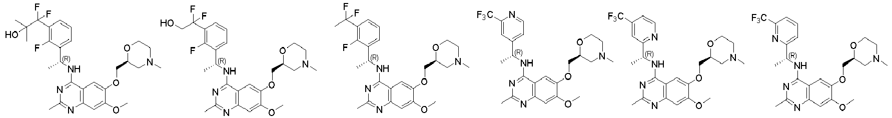

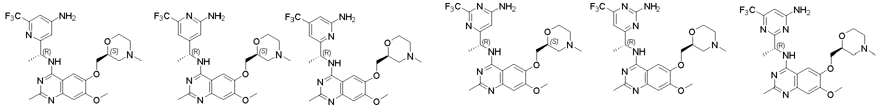

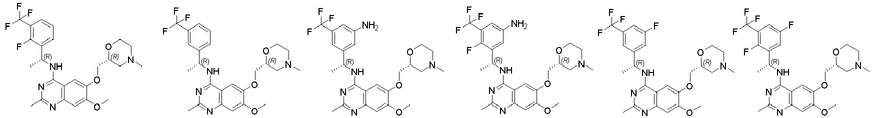

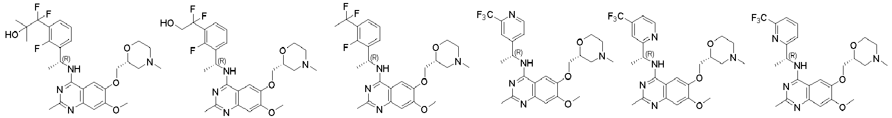

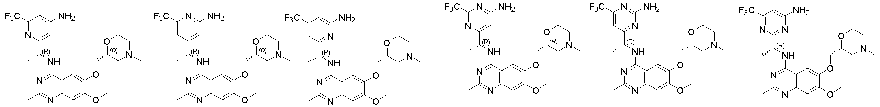

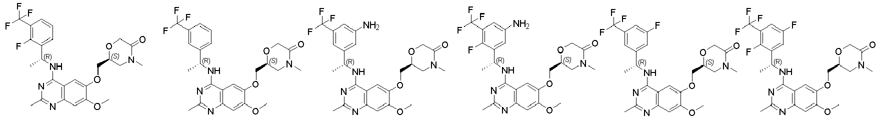

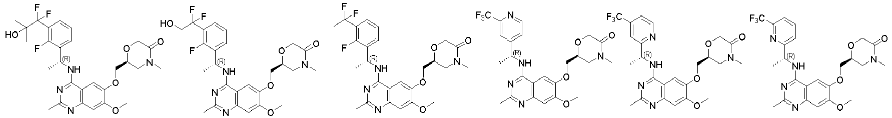

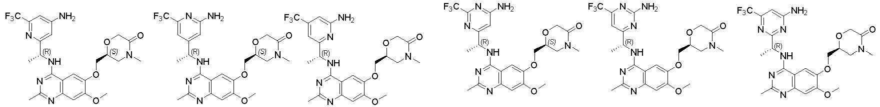

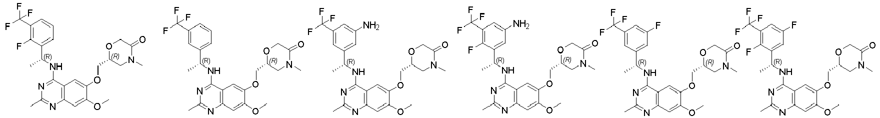

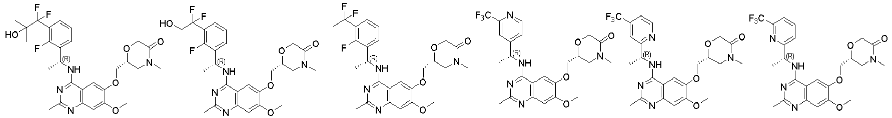

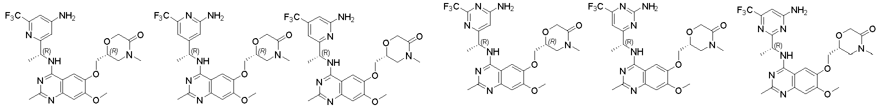

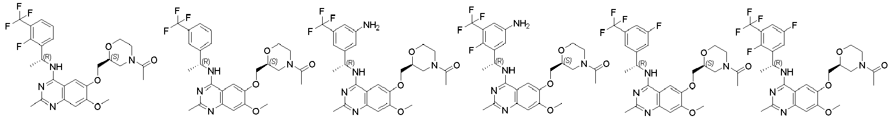

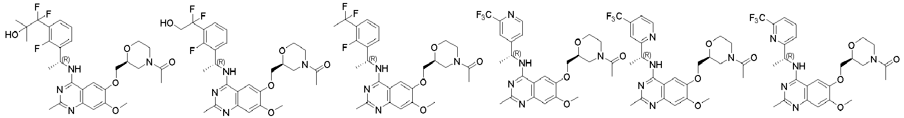

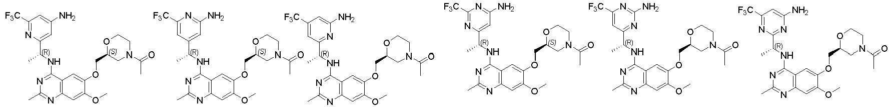

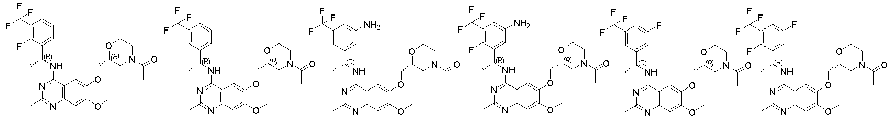

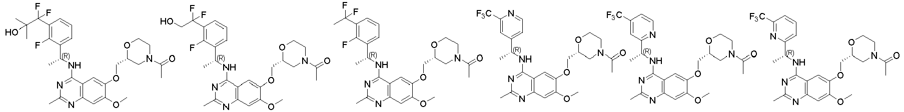

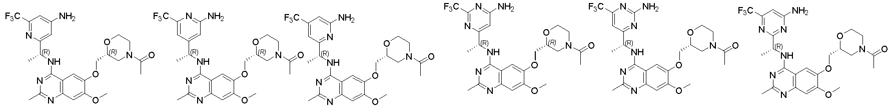

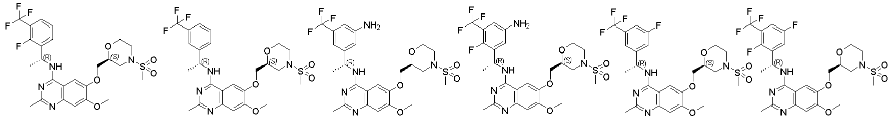

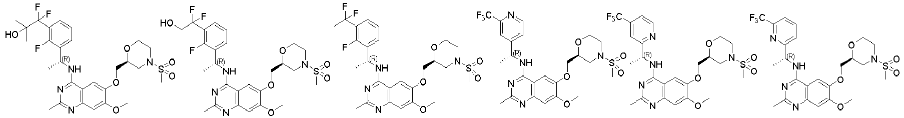

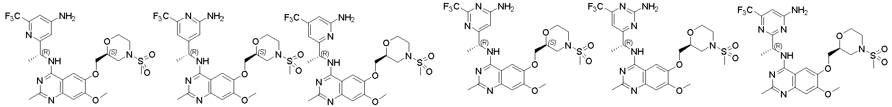

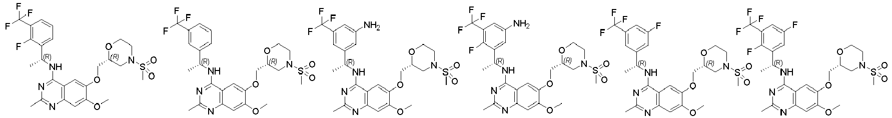

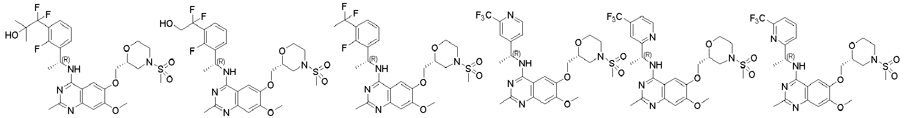

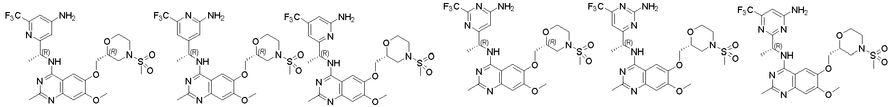

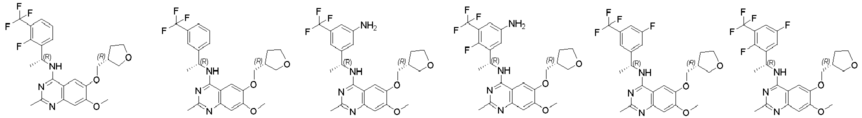

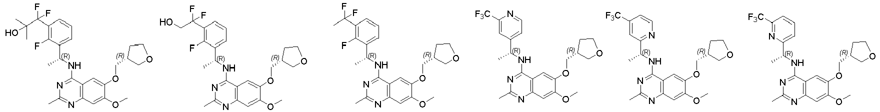

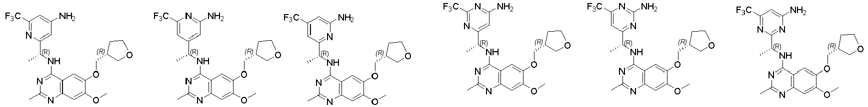

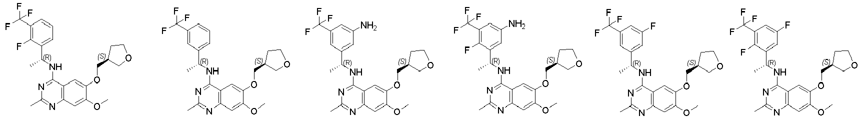

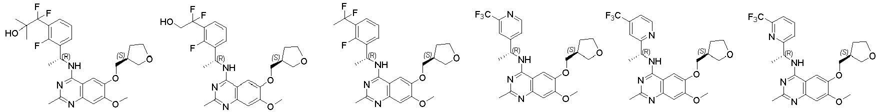

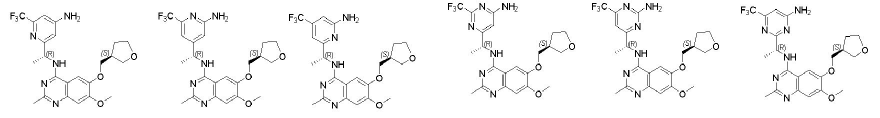

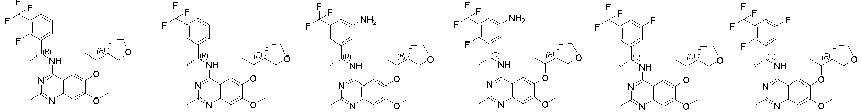

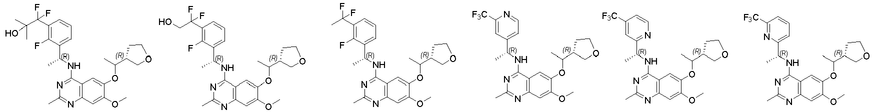

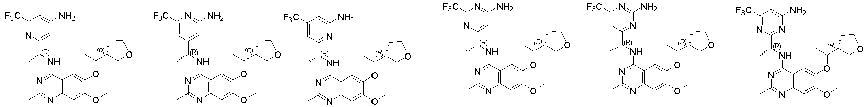

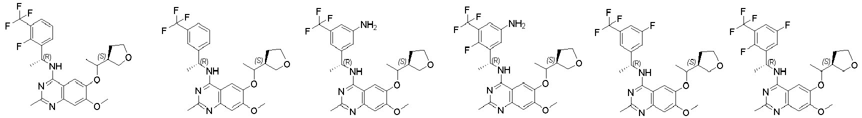

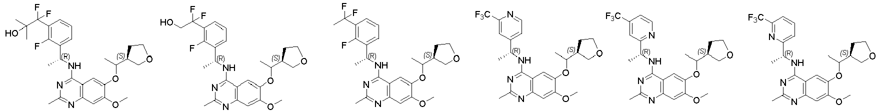

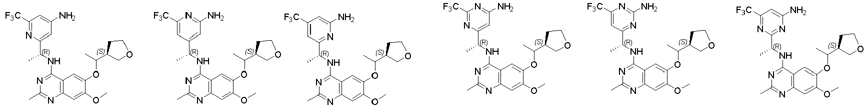

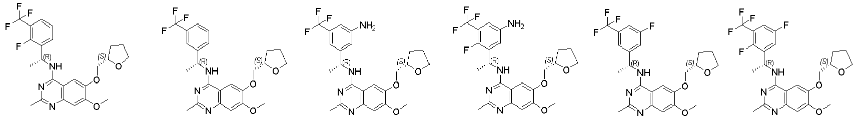

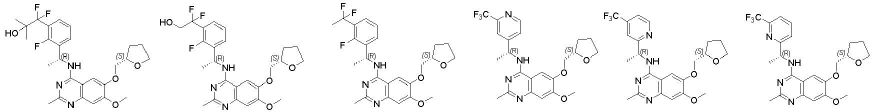

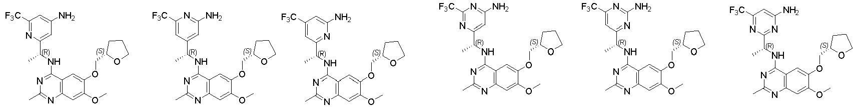

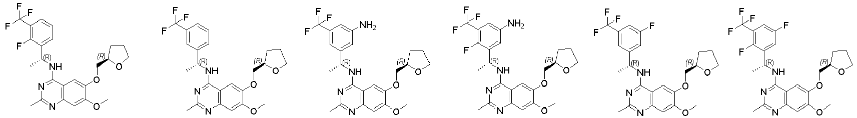

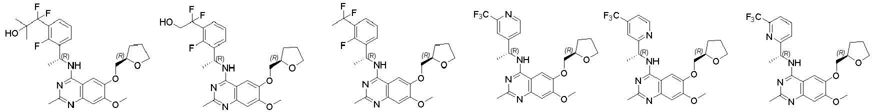

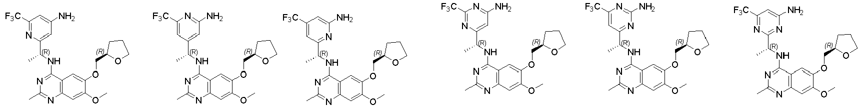

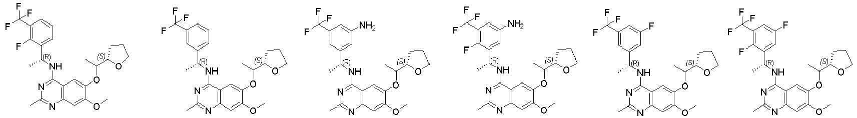

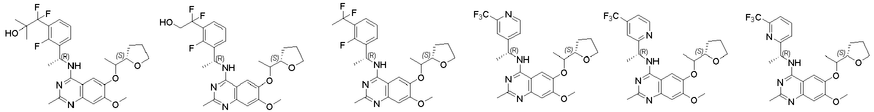

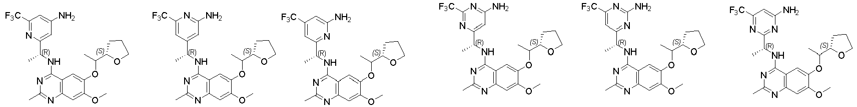

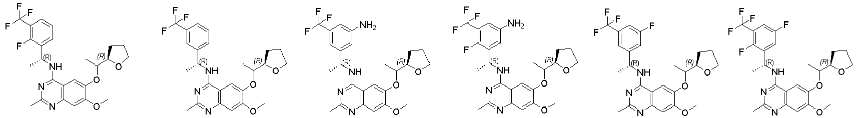

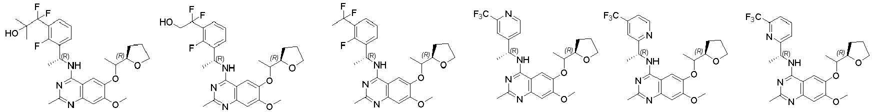

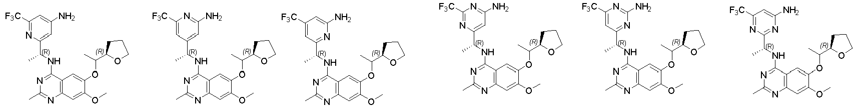

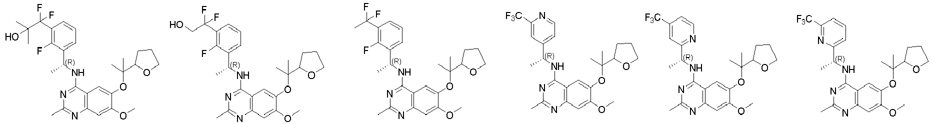

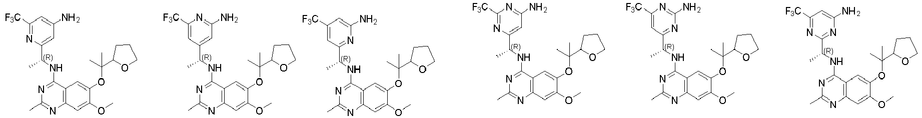

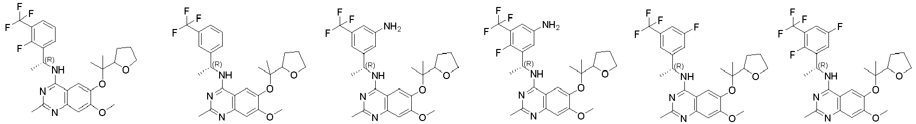

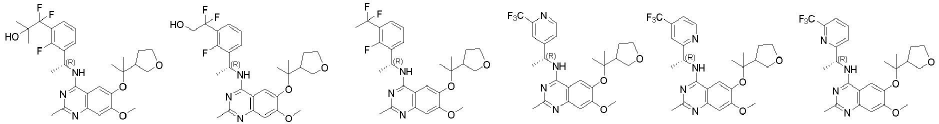

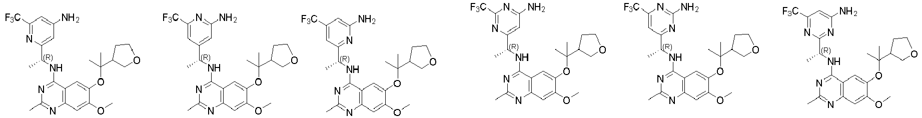

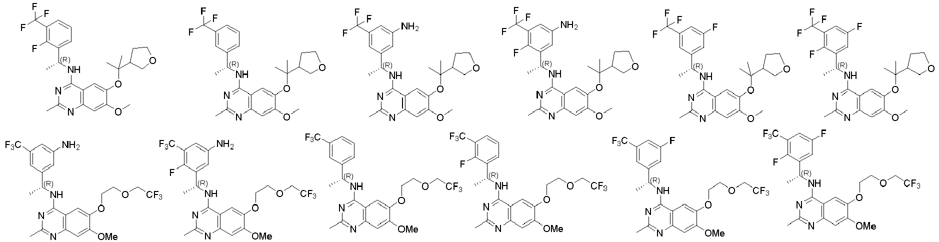

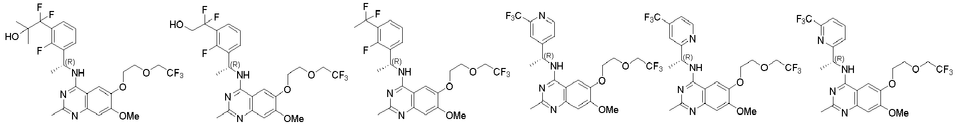

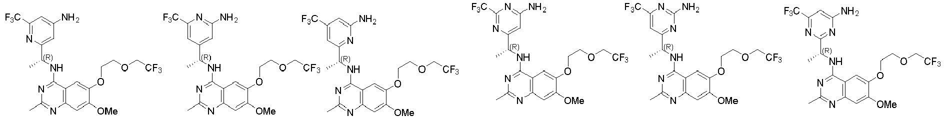

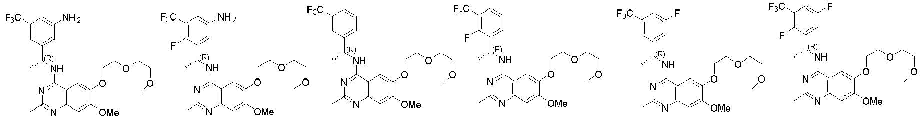

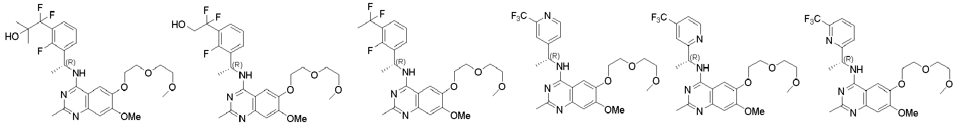

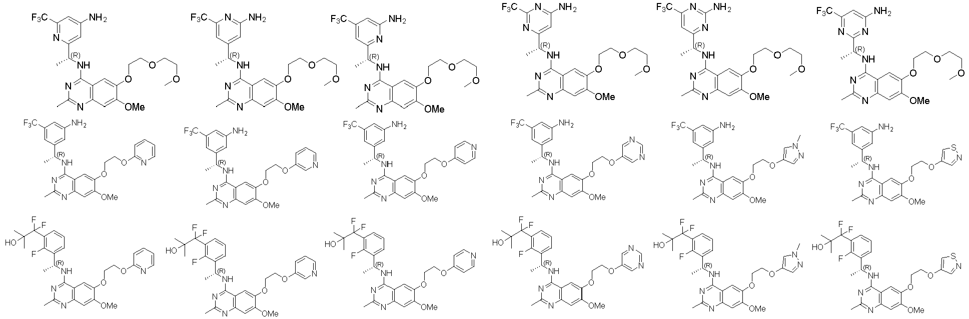

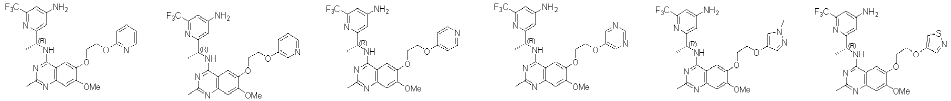

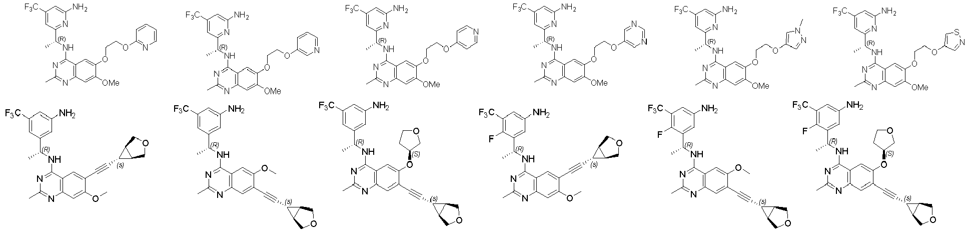

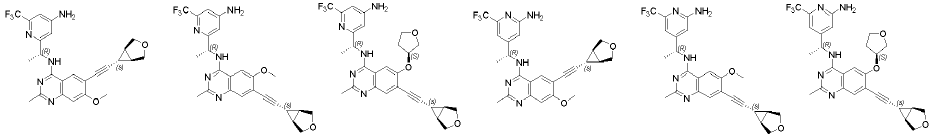

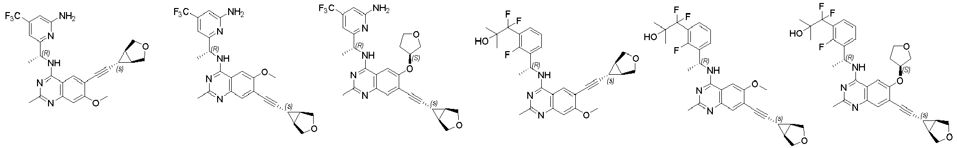

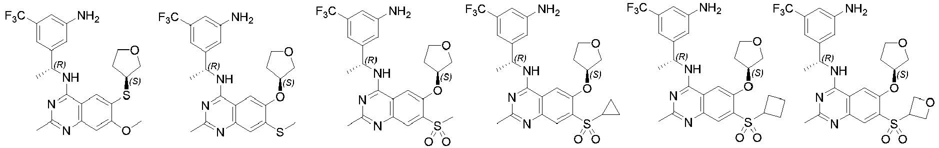

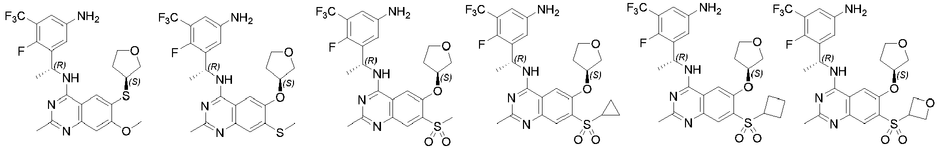

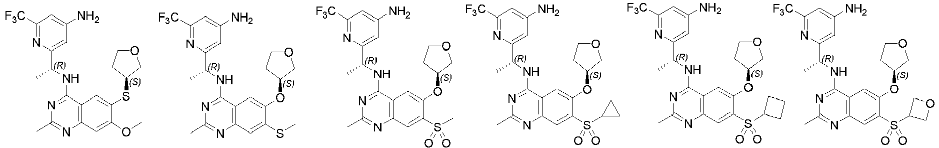

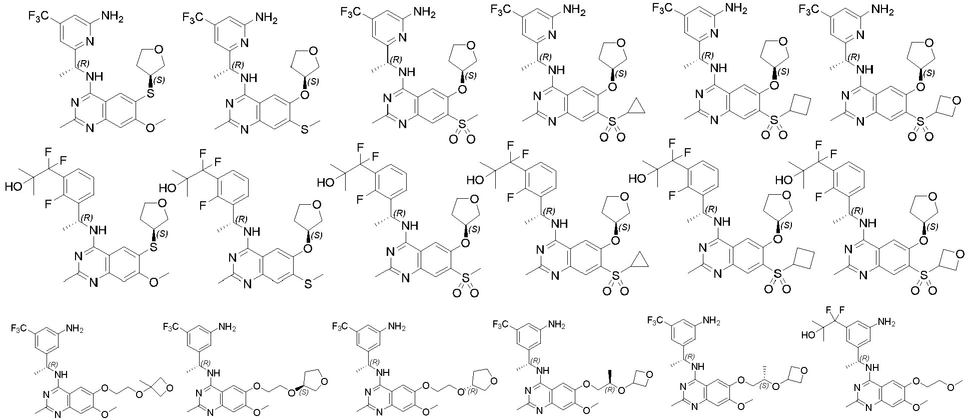

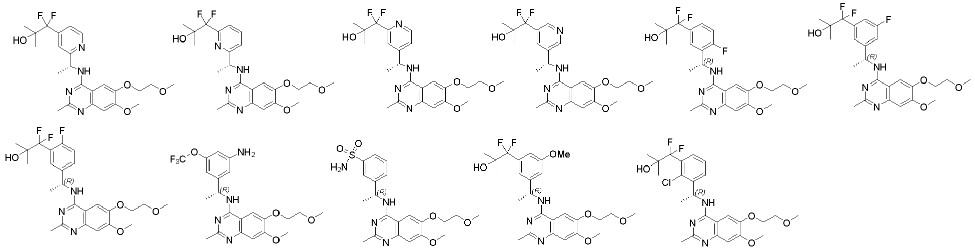

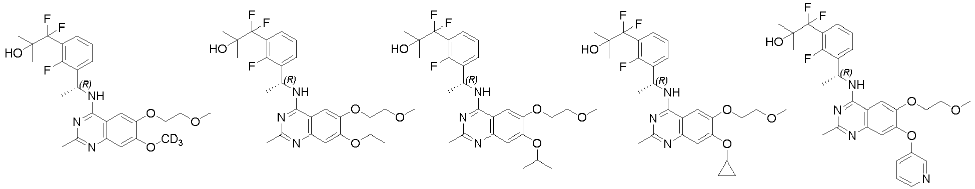

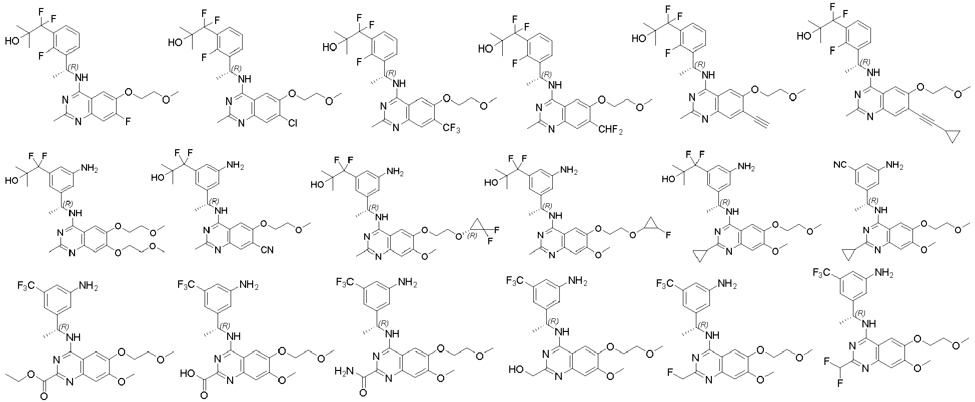

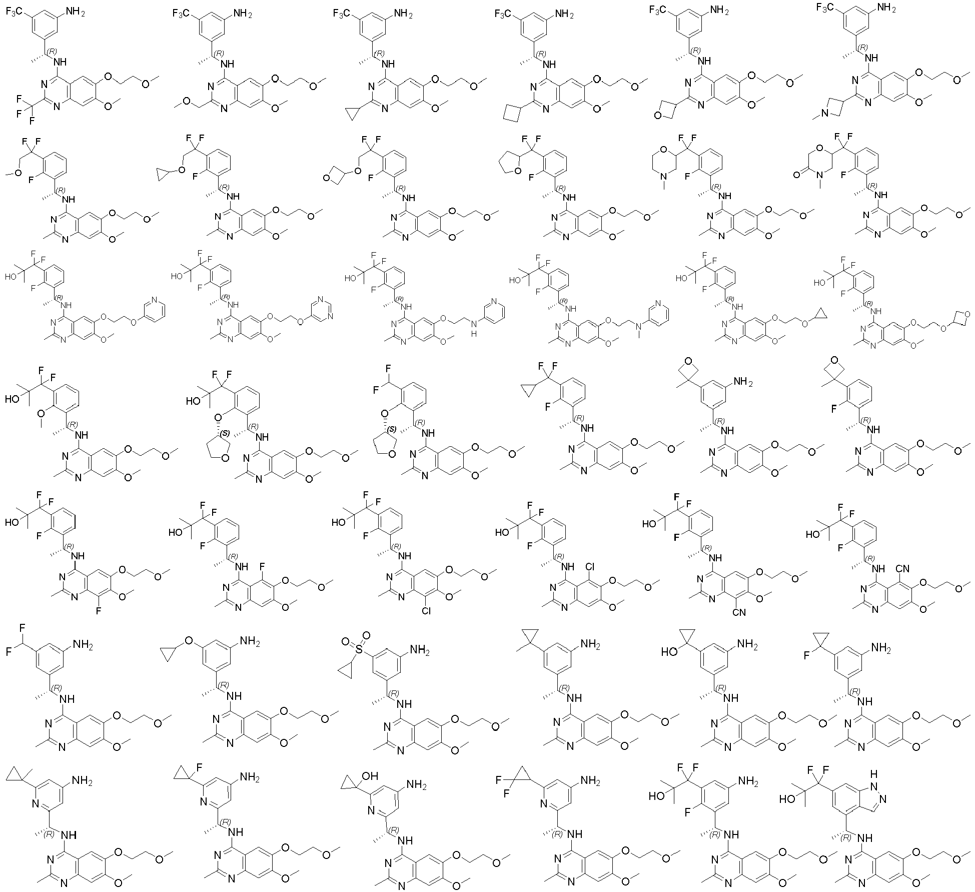

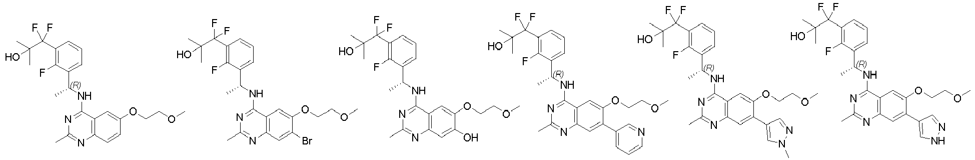

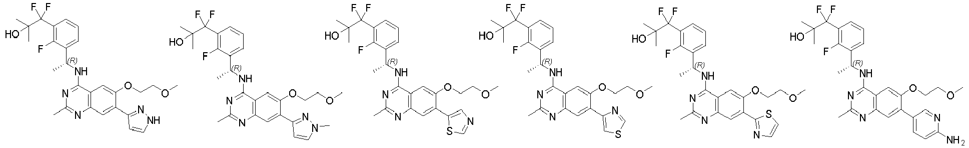

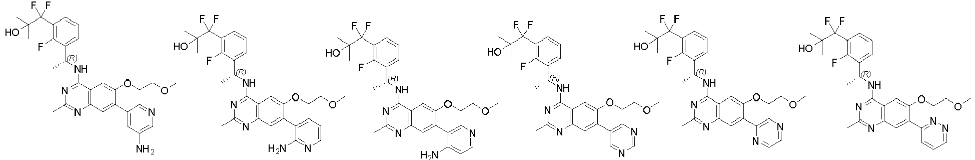

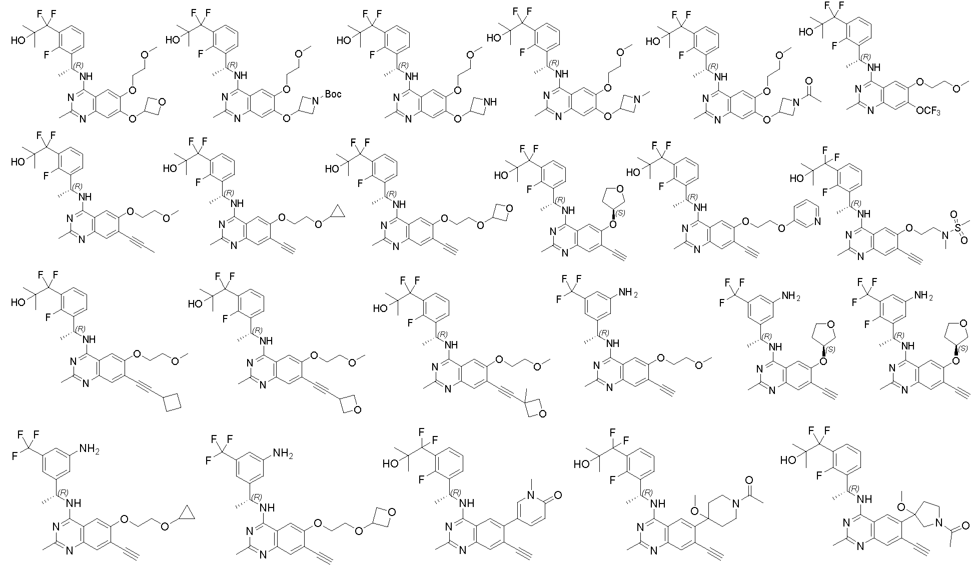

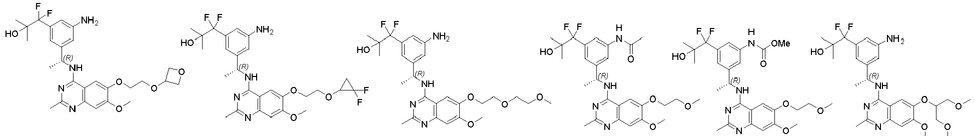

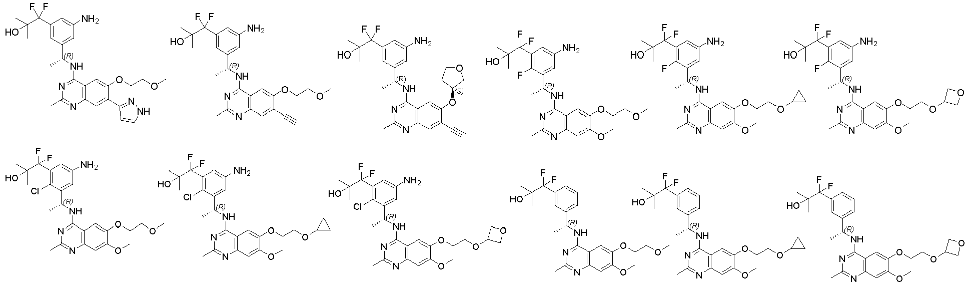

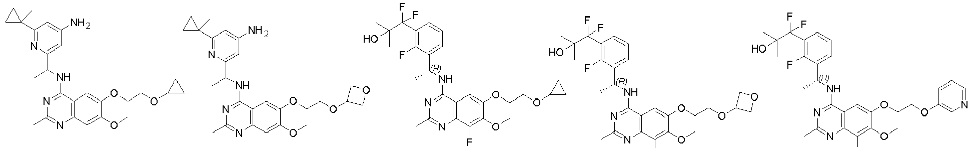

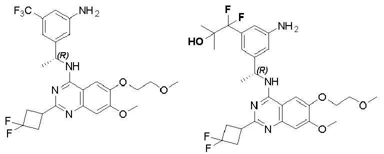

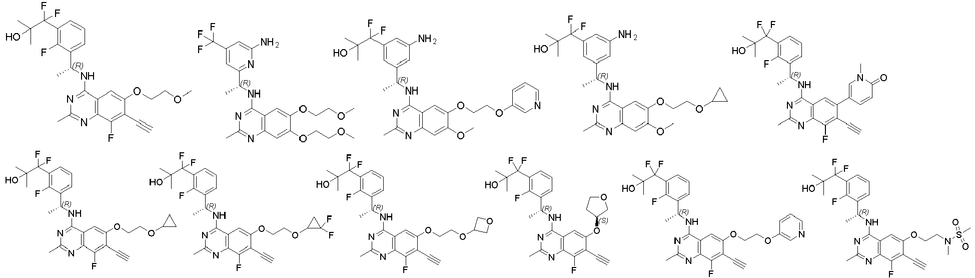

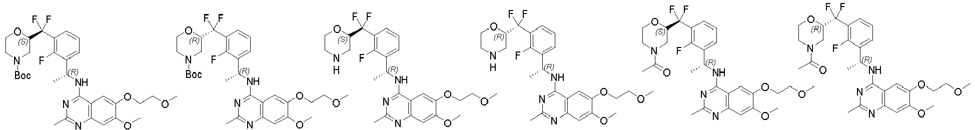

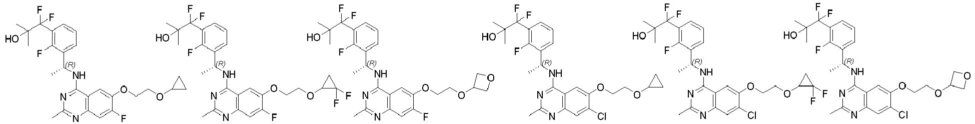

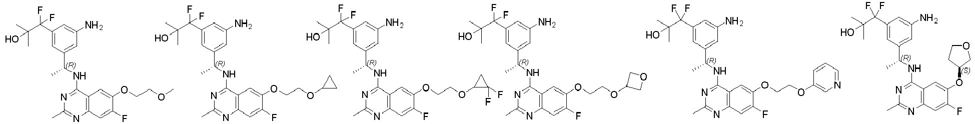

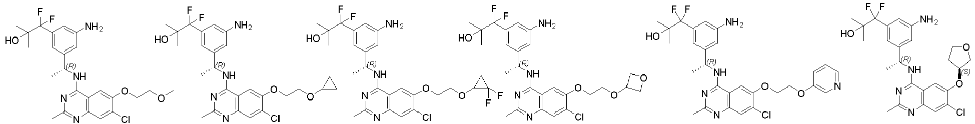

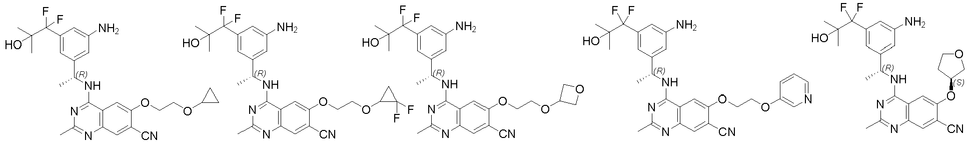

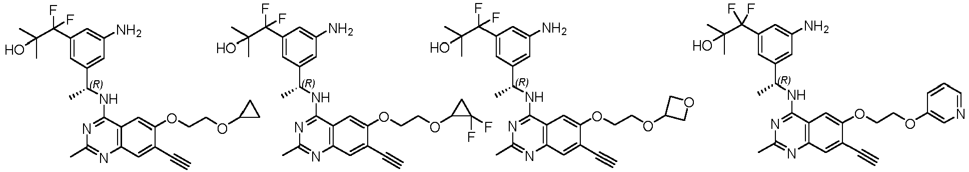

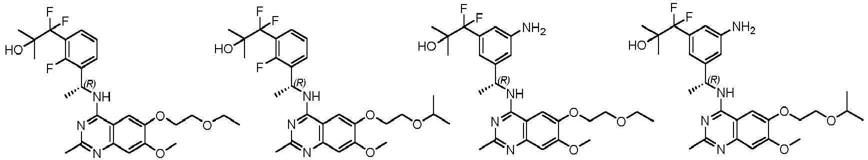

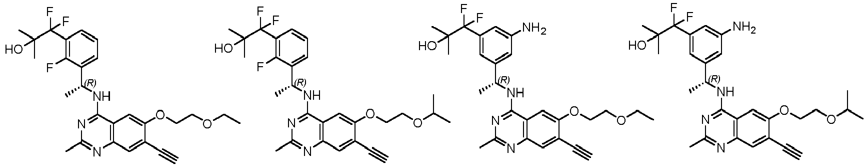

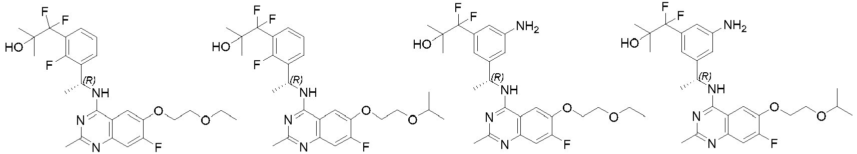

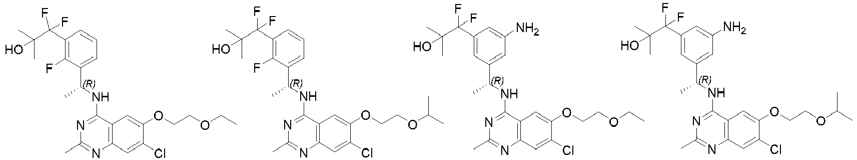

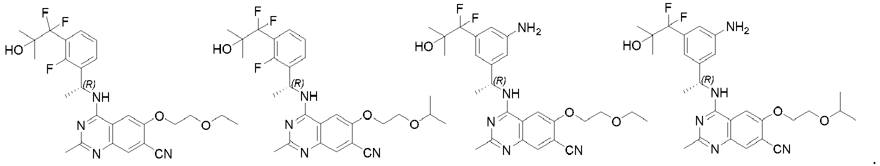

12. A method for preparing the compound, or the stereoisomer, the tautomer, the crystalline form, the pharmaceutically acceptable salt, the hydrate, the solvate or the prodrug thereof according to an one of claims 1-11. comprising the following steps: 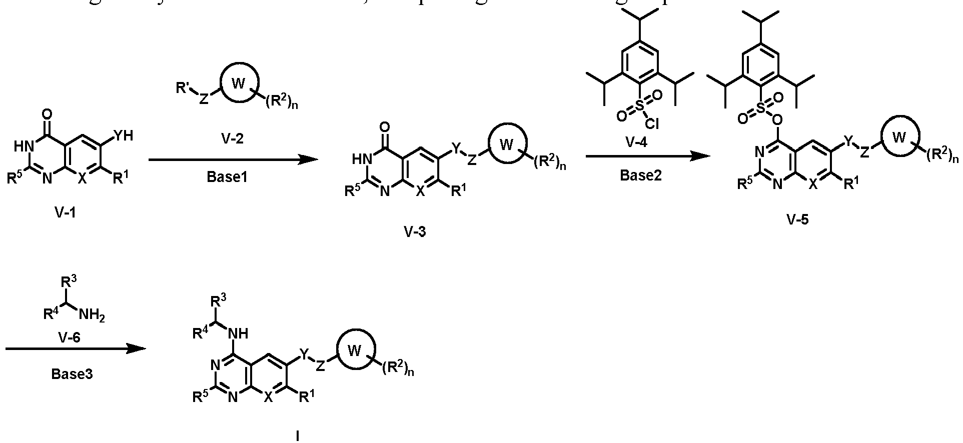
(i) reacting a compound of formula V-1 with a compound of formula V-2 in the presence of a first base to give a compound of formula V-3;
(ii) reacting the compound of formula V-3 with sulfonyl chloride (V-4) in the presence of a second base and a catalyst to give a compound of formula V-5; and
(iii) reacting the compound of formula V-5 with an amine (formula V-6) in the presence of a third base to give a compound of formula (I);
wherein in the formula,
R' is selected from: halogen, OTs and OMs;
R¹, R², R³, R⁴, R⁵, X, Y, Z, Wand n are as defined in claim 1.

13. A pharmaceutical composition comprising i) one or more compounds, or stereoisomers, tautomers, crystalline forms, pharmaceutically acceptable salts, hydrates, solvates or prodrugs thereof according to any one of claims 1-11; and ii) a pharmaceutically acceptable carrier.

14. Use of the compound, the stereoisomer, the tautomer, the crystalline form, the pharmaceutically acceptable salt, the hydrate, the solvate or the prodrug thereof according to any one of claims 1-11, or the pharmaceutical composition according to claim 13 in preparing a pharmaceutical composition for preventing and/or treating a disease associated with the activity or expression level of SOS1.
